# EUROPEAN PATENT SPECIFICATION

(11) **EP 2 972 308 B1**
(45) Date of publication and mention of the grant of the patent: **16.09.2020**
(21) Application number: 14765203.6
(22) Date of filing: 14.03.2014
(51) Int. Cl.: G01N 33/48, G01N 33/68

(54) **BIOMARKERS AND METHODS FOR PREDICTING PRETERM BIRTH**
BIOMARKER UND VERFAHREN ZUR VORHERSAGE EINER FRÜHGEBURT
BIOMARQUEURS ET PROCÉDÉS DE PRÉDICTION D'UNE NAISSANCE PRÉMATURÉE

(30) Priority: 15.03.2013 US 201361798504 P; 20.12.2013 US 201361919586 P
(43) Date of publication of application: 20.01.2016
(73) Proprietor: Sera Prognostics, Inc., Salt Lake City, UT 84109 (US)
(72) Inventor: HICKOK, Durlin Edward, Salt Lake City, UT 84109 (US); BONIFACE, John Jay, Salt Lake City, UT 84109 (US); CRITCHFIELD, Gregory Charles, Salt Lake City, UT 84109 (US); FLEISCHER, Tracey Cristine, Salt Lake City, UT 84109 (US)
(74) Representative: Jones Day
(86) International application number: PCT/US2014/028412
(87) International publication number: WO 2014/144129

(56) References cited:
- WO-A2-2006/034427
- WO-A2-2008/063369
- WO-A2-2009/014987
- US-A1- 2010 017 143
- US-A1- 2010 173 317
- US-A1- 2010 173 786
- US-A1- 2012 315 630
- US-B2- 7 790 463
- SPENCER KEVIN ET AL: "First trimester sex hormone-binding globulin and subsequent development of preeclampsia or other adverse pregnancy outcomes.", HYPERTENSION IN PREGNANCY, vol. 24, no. 3, 2005, pages 303-311, XP009191651, ISSN: 1064-1955
- EREZ, O ET AL.: 'High Tissue Factor Activity And Low Tissue Factor Pathway Inhibitor Concentrations In Patients With Preterm Labor.' THE JOURNAL OF MATERNAL-FETAL AND NEONATAL MEDICINE. vol. 23, no. 1, January 2010, pages 23 - 33, XP055284191
- EASTAUGH, J ET AL.: 'Comparison Of Neural Networks And Statistical Models To Predict Gestational Age at Birth.' NEURAL COMPUTING AND APPLICATIONS. vol. 6, no. 3, 1997, pages 158 - 164, XP055284194

## Description

The invention relates generally to the field of personalized medicine and, more specifically to compositions and methods for determining the probability for preterm birth in a pregnant female.

### BACKGROUND

According to the World Heath Organization, an estimated 15 million babies are born preterm (before 37 completed weeks of gestation) every year. In almost all countries with reliable data, preterm birth rates are increasing. *See,* World Health Organization; March of Dimes; The Partnership for Maternal, Newborn & Child Health; Save the Children, Born too soon: the global action report on preterm birth, ISBN 9789241503433(2012). An estimated 1 million babies die annually from preterm birth complications. Globally, preterm birth is the leading cause of newborn deaths (babies in the first four weeks of life) and the second leading cause of death after pneumonia in children under five years. Many survivors face a lifetime of disability, including learning disabilities and visual and hearing problems.

Across 184 countries with reliable data, the rate of preterm birth ranges from 5% to 18% of babies born. Blencowe et al., "National, regional and worldwide estimates of preterm birth." The Lancet, 9;379(9832):2162-72 (2012). While over 60% of preterm births occur in Africa and south Asia, preterm birth is nevertheless a global problem. Countries with the highest numbers include Brazil, India, Nigeria and the United States of America. Of the 11 countries with preterm birth rates over 15%, all but two are in sub-Saharan Africa. In the poorest countries, on average, 12% of babies are born too soon compared with 9% in higher-income countries. Within countries, poorer families are at higher risk. More than three-quarters of premature babies can be saved with feasible, cost-effective care, for example, antenatal steroid injections given to pregnant women at risk of preterm labour to strengthen the babies' lungs.

Infants born preterm are at greater risk than infants born at term for mortality and a variety of health and developmental problems. Complications include acute respiratory, gastrointestinal, immunologic, central nervous system, hearing, and vision problems, as well as longer-term motor, cognitive, visual, hearing, behavioral, social-emotional, health, and growth problems. The birth of a preterm infant can also bring considerable emotional and economic costs to families and have implications for public-sector services, such as health insurance, educational, and other social support systems. The greatest risk of mortality and morbidity is for those infants born at the earliest gestational ages. However, those infants born nearer to term represent the greatest number of infants born preterm and also experience more complications than infants born at term.

To prevent preterm birth in women who are less than 24 weeks pregnant with an ultrasound showing cervical opening, a surgical procedure known as cervical cerclage can be employed in which the cervix is stitched closed with strong sutures. For women less than 34 weeks pregnant and in active preterm labor, hospitalization may be necessary as well as the administration of medications to temporarily halt preterm labor an/or promote the fetal lung development. If a pregnant women is determined to be at risk for preterm birth, health care providers can implement various clinical strategies that may include preventive medications, for example, hydroxyprogesterone caproate (Makena) injections and/or vaginal progesterone gel, cervical pessaries, restrictions on sexual activity and/or other physical activities, and alterations of treatments for chronic conditions, such as diabetes and high blood pressure, that increase the risk of preterm labor.

There is a great need to identify and provide women at risk for preterm birth with proper antenatal care. Women identified as high-risk can be scheduled for more intensive antenatal surveillance and prophylactic interventions. Current strategies for risk assessment are based on the obstetric and medical history and clinical examination, but these strategies are only able to identify a small percentage of women who are at risk for preterm delivery. Reliable early identification of risk for preterm birth would enable planning appropriate monitoring and clinical management to prevent preterm delivery. Such monitoring and management might include: more frequent prenatal care visits, serial cervical length measurements, enhanced education regarding signs and symptoms of early preterm labor, lifestyle interventions for modifiable risk behaviors, cervical pessaries and progesterone treatment. Finally, reliable antenatal identification of risk for preterm birth also is crucial to cost-effective allocation of monitoring resources.

The present invention addresses this need by providing compositions and methods for determining whether a pregnant woman is at risk for preterm birth. Related advantages are provided as well.

### SUMMARY

The present invention provides biomarkers and methods for predicting the probability of preterm birth in a pregnant female.

Disclosed herein is a panel of isolated biomarkers comprising N of the biomarkers listed in Tables 1 through 63. In some instances, N is a number selected from the group consisting of 2 to 24. In additional instances, the biomarker panel comprises at least two of the isolated biomarkers selected from the group consisting of AFTECCVVASQLR, ELLESYIDGR, and ITLPDFTGDLR. In additional instances, the biomarker panel comprises at least two of the isolated biomarkers selected from the group consisting of FLNWIK, FGFGGSTDSGPIR, LLELTGPK, VEHSDLSFSK, IEGNLIFDPNNYLPK, ALVLELAK, TQILEWAAER, DVLLLVHNLPQNLPGYFWYK, SEPRPGVLLR, ITQDAQLK, ALDLSLK, WWGGQPLWITATK, and LSETNR

In further instances, the biomarker panel comprises at least two of the isolated biomarkers selected from the group consisting of the biomarkers set forth in Table 50 and the biomarkers set forth in Table 52.

Disclosed herein is a panel of isolated biomarkers comprising N of the biomarkers listed in Tables 1 through 63. In some instances, N is a number selected from the group consisting of 2 to 24. In additional instances, the biomarker panel comprises at least two of the isolated biomarkers selected from the group consisting of the biomarkers set forth in Table 50 and the biomarkers set forth in Table 52.

Disclosed herein is a biomarker panel comprising at least two of the isolated biomarkers selected from the group consisting of lipopolysaccharide-binding protein (LBP), prothrombin (THRB), complement component C5 (C5 or CO5), plasminogen (PLMN), and complement component C8 gamma chain (C8G or CO8G).

Disclosed herein is a biomarker panel comprising at least two of the isolated biomarkers selected from the group consisting of Alpha-1B-glycoprotein (A1BG), Disintegrin and metalloproteinase domain-containing protein 12 (ADA12), Apolipoprotein B-100 (APOB), Beta-2-microglobulin (B2MG), CCAAT/enhancer-binding protein alpha/beta (HP8 Peptide), Corticosteroid-binding globulin (CBG), Complement component C6, Endoglin (EGLN), Ectonucleotide pyrophosphatase/phosphodiesterase family member 2 (ENPP2), Coagulation factor VII In a first aspect, the invention provides an isolated biomarker consisting of the sequence IALGGLLFPASNLR.

In a second aspect, the invention provides a method of determining probability for preterm birth or term birth in a pregnant female, the method comprising: detecting a measurable feature of each of one or more biomarkers in a biological sample obtained from said pregnant female, and analyzing said measurable feature to determine the probability for preterm birth or term birth in said pregnant female, wherein the one or more biomarkers comprise a biomarker consisting of the sequence IALGGLLFPASNLR; wherein said measurable feature is defined as the presence, absence, or concentration of a biomarker, or a fragment thereof; an altered structure, such as the presence or amount of a post-translational modification; or the presence of an altered conformation in comparison to the conformation of the biomarker in normal control subjects; and wherein the fragment comprises at least 5, at least 6, at least 7, at least 8, at least 9, at least 10, at least 11, at least 12, or at least 13 consecutive amino acid residues.

In a third aspect, the invention provides a method of predicting gestational age at birth (GAB) or time to birth in a pregnant female, the method comprising: detecting a measurable feature of each of one or more biomarkers in a biological sample obtained from said pregnant female, and analyzing said measurable feature to predict GAB or time to birth in said pregnant female, wherein the one or more biomarkers comprise a biomarker consisting of the sequence IALGGLLFPASNLR; wherein said measurable feature is defined as the presence, absence, or concentration of a biomarker, or a fragment thereof; an altered structure, such as the presence or amount of a post-translational modification; or the presence of an altered conformation in comparison to the conformation of the biomarker in normal control subjects; and wherein the fragment comprises at least 5, at least 6, at least 7, at least 8, at least 9, at least 10, at least 11, at least 12, or at least 13 consecutive amino acid residues.

In one embodiment, the method according to the second aspect further comprises:
(a) quantifying an amount of the one or more biomarkers; (b) multiplying said amount by a predetermined coefficient, (c) determining the probability for preterm birth in the pregnant female comprising adding said individual products to obtain a total risk score that corresponds to said probability. In another embodiment, said analyzing of said measurable feature initially comprises prediction of gestational age at birth (GAB) prior to said determining the probability for preterm birth; optionally wherein said prediction of the GAB is used to determine the probability for preterm birth.

In one embodiment, the method according to the third aspect further comprises:
(a) quantifying an amount of the one or more biomarkers; (b) multiplying and/or thresholding said amount by a predetermined coefficient; (c) determining the predicted GAB in the pregnant female, the determination comprising adding said individual products to obtain a total risk score that corresponds to said predicted GAB; and optionally (d) subtracting the estimated GA at time biological sample was obtained from the predicted GAB to predict time to birth in said pregnant female.

In one embodiment the methods further comprise calculating the probability for preterm birth or term birth, or GAB or time to birth in said pregnant female based on quantifying an amount of each of the one or more biomarkers. In another embodiment, said analyzing comprises a use of a predictive model; and, optionally, comparing the measurable feature with a reference feature. In another embodiment, said analyzing comprises using one or more of: a linear discriminant analysis model, a support vector machine classification algorithm, a recursive feature elimination model, a prediction analysis of microarray model, a logistic regression model, a multiple regression model, a survival model, a CART algorithm, a flex tree algorithm, a LART algorithm, a random forest algorithm, a MART algorithm, a machine learning algorithm, a penalized regression method, and a combination thereof.

In one embodiment, the biological sample is selected from the group consisting of whole blood, plasma, and serum.

In one embodiment, said quantifying comprises mass spectrometry (MS) or liquid chromatography-mass spectrometry (LC-MS). In another embodiment, said MS comprises multiple reaction monitoring (MRM) or selected reaction monitoring (SRM); optionally wherein said MRM (or SRM) comprises scheduled MRM (SRM). In another embodiment, said quantifying comprises an assay that utilizes a capture agent. In another embodiment, said capture agent is selected from the group consisting of an antibody, antibody fragment, nucleic acid-based protein binding reagent, small molecule or variant thereof. In another embodiment, said assay is selected from the group consisting of enzyme immunoassay (EIA), enzyme-linked immunosorbent assay (ELISA), and radioimmunoassay (RIA). In another embodiment, said quantifying further comprises mass spectrometry (MS) or co-immunoprecitipation-mass spectrometry (co-IP MS).

In a further embodiment, the methods comprise detecting one or more risk indicia; optionally wherein the one or more risk indicia are selected from the group consisting of age, prior pregnancy, history of previous low birth weight or preterm delivery, multiple 2nd trimester spontaneous abortion, prior first trimester induced abortion, familial and intergenerational factors, history of infertility, nulliparity, placental abnormalities, cervical and uterine anomalies, gestational bleeding, intrauterine growth restriction, in utero diethylstilbestrol exposure, multiple gestations, infant sex, short stature, low prepregnancy weight/low body mass index, diabetes, hypertension, hypothyroidism, asthma, education level, tobacco use, and urogenital infections.

(FA7), Hyaluronan-binding protein 2 (HABP2), Pregnancy-specific beta-1-glycoprotein 9 (PSG9), Inhibin beta E chain (INHBE).

Disclosed herein is a biomarker panel comprising lipopolysaccharide-binding protein (LBP), prothrombin (THRB), complement component C5 (C5 or CO5), plasminogen (PLMN), complement component C8 gamma chain (C8G or CO8G), complement component 1, q subcomponent, B chain (C1QB), fibrinogen beta chain (FIBB or FIB), C-reactive protein (CRP), inter-alpha-trypsin inhibitor heavy chain H4 (ITIH4), chorionic somatomammotropin hormone (CSH), and angiotensinogen (ANG or ANGT).

Disclosed herein is a biomarker panel comprising Alpha-1B-glycoprotein (A1BG), Disintegrin and metalloproteinase domain-containing protein 12 (ADA12), Apolipoprotein B-100 (APOB), Beta-2-microglobulin (B2MG), CCAAT/enhancer-binding protein alpha/beta (HP8 Peptide), Corticosteroid-binding globulin (CBG), Complement component C6, Endoglin (EGLN), Ectonucleotide pyrophosphatase/phosphodiesterase family member 2 (ENPP2), Coagulation factor VII (FA7), Hyaluronan-binding protein 2 (HABP2), Pregnancy-specific beta-1-glycoprotein 9 (PSG9), Inhibin beta E chain (INHBE).

Disclosed herein is a biomarker panel comprising at least two of the isolated biomarkers selected from the group consisting of the biomarkers set forth in Table 51 and the biomarkers set forth in Table 53.

In some embodiments, the method of determining probability for preterm birth in a pregnant female further comprises detecting a measurable feature of each of N biomarkers selected from the biomarkers listed in Tables 1 through 63 in a biological sample obtained from the pregnant female, and analyzing the measurable feature to determine the probability for preterm birth in the pregnant female. In some embodiments, the invention provides a method of predicting GAB, the method encompassing detecting a measurable feature of each of N biomarkers selected from the biomarkers listed in Tables 1 through 63 in a biological sample obtained from a pregnant female, and analyzing said measurable feature to predict GAB.

In some embodiments, a measurable feature comprises fragments or derivatives of each of the N biomarkers selected from the biomarkers listed in Tables 1 through 63. In some embodiments of the disclosed methods detecting a measurable feature comprises quantifying an amount of each of N biomarkers selected from the biomarkers listed in Tables 1 through 63, combinations or portions and/or derivatives thereof in a biological sample obtained from the pregnant female. In additional embodiments, the disclosed methods of determining probability for preterm birth in a pregnant female further encompass detecting a measurable feature for one or more risk indicia associated with preterm birth.

In some embodiments, the disclosed methods of determining probability for preterm birth in a pregnant female and related methods disclosed herein further comprise detecting a measurable feature of each of N biomarkers, wherein N is selected from the group consisting of 2 to 24. In further embodiments, the disclosed methods of determining probability for preterm birth in a pregnant female and related methods disclosed herein further comprise detecting a measurable feature of each of at least two isolated biomarkers selected from the group consisting of AFTECCVVASQLR, ELLESYIDGR, and ITLPDFTGDLR. In further embodiments, the disclosed methods of determining probability for preterm birth in a pregnant female and related methods disclosed herein further comprise detecting a measurable feature of each of at least two isolated biomarkers selected from the group consisting of FLNWIK, FGFGGSTDSGPIR, LLELTGPK,

### VEHSDLSFSK, IEGNLIFDPNNYLPK, ALVLELAK, TQILEWAAER, DVLLLVHNLPQNLPGYFWYK, SEPRPGVLLR, ITQDAQLK, ALDLSLK,

WWGGQPLWITATK, and LSETNR. In further embodiments, the disclosed methods of determining probability for preterm birth in a pregnant female and related methods disclosed herein further comprise detecting a measurable feature of each of at least two isolated biomarkers selected from the group consisting of the biomarkers set forth in Table 50 and the biomarkers set forth in Table 52.

In other embodiments, the disclosed methods of determining probability for preterm birth in a pregnant female further comprise detecting a measurable feature of each of at least two isolated biomarkers selected from the group consisting of lipopolysaccharide-binding protein (LBP), prothrombin (THRB), complement component C5 (C5 or CO5), plasminogen (PLMN), and complement component C8 gamma chain (C8G or CO8G).

In other embodiments, the disclosed methods of determining probability for preterm birth in a pregnant female further comprise detecting a measurable feature of each of at least two isolated biomarkers selected from the group consisting of of Alpha-1B-glycoprotein (A1BG), Disintegrin and metalloproteinase domain-containing protein 12 (ADA12), Apolipoprotein B-100 (APOB), Beta-2-microglobulin (B2MG), CCAAT/enhancer-binding protein alpha/beta (HP8 Peptide), Corticosteroid-binding globulin (CBG), Complement component C6, Endoglin (EGLN), Ectonucleotide pyrophosphatase/phosphodiesterase family member 2 (ENPP2), Coagulation factor VII (FA7), Hyaluronan-binding protein 2 (HABP2), Pregnancy-specific beta-1-glycoprotein 9 (PSG9), Inhibin beta E chain (INHBE).

In further embodiments, the disclosed methods of determining probability for preterm birth in a pregnant female further comprise detecting a measurable feature of each of at least two isolated biomarkers selected from the group consisting of lipopolysaccharide-binding protein (LBP), prothrombin (THRB), complement component C5 (C5 or CO5), plasminogen (PLMN), complement component C8 gamma chain (C8G or CO8G), complement component 1, q subcomponent, B chain (C1QB), fibrinogen beta chain (FIBB or FIB), C-reactive protein (CRP), inter-alpha-trypsin inhibitor heavy chain H4 (ITIH4), chorionic somatomammotropin hormone (CSH), and angiotensinogen (ANG or ANGT).

In further embodiments, the disclosed methods of determining probability for preterm birth in a pregnant female further comprise detecting a measurable feature of each of at least two isolated biomarkers selected from the group consisting of the biomarkers set forth in Table 51 and the biomarkers set forth in Table 53.

In some embodiments of the methods of determining probability for preterm birth in a pregnant female, the probability for preterm birth in the pregnant female is calculated based on the quantified amount of each of N biomarkers selected from the biomarkers listed in Tables 1 through 63. In some embodiments, the disclosed methods for determining the probability of preterm birth encompass detecting and/or quantifying one or more biomarkers using mass sprectrometry, a capture agent or a combination thereof.

In some embodiments, the disclosed methods of determining probability for preterm birth in a pregnant female encompass an initial step of providing a biomarker panel comprising N of the biomarkers listed in Tables 1 through 63, including the biomarker of the invention. In some instances, the disclosed methods of determining probability for preterm birth in a pregnant female encompass an initial step of providing a biological sample from the pregnant female.

In some embodiments, the disclosed methods of determining probability for preterm birth in a pregnant female encompass communicating the probability to a health care provider. In additional embodiments, the communication informs a subsequent treatment decision for the pregnant female. In further embodiments, the treatment decision of one or more selected from the group of consisting of more frequent prenatal care visits, serial cervical length measurements, enhanced education regarding signs and symptoms of early preterm labor, lifestyle interventions for modifiable risk behaviors and progesterone treatment.

In further embodiments, the disclosed methods of determining probability for preterm birth in a pregnant female encompass analyzing the measurable feature of one or more isolated biomarkers using a predictive model. In some embodiments of the disclosed methods, a measurable feature of one or more isolated biomarkers is compared with a reference feature.

In additional embodiments, the disclosed methods of determining probability for preterm birth in a pregnant female encompass using one or more analyses selected from a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, and a combination thereof. In one embodiment, the disclosed methods of determining probability for preterm birth in a pregnant female encompass logistic regression.

In some embodiments, the invention provides a method of determining probability for preterm birth in a pregnant female, the method encompassing quantifying in a biological sample obtained from the pregnant female an amount of each of N biomarkers selected from the biomarkers listed in Tables 1 through 63; multiplying the amount by a predetermined coefficient, and determining the probability for preterm birth in the pregnant female comprising adding the individual products to obtain a total risk score that corresponds to the probability

In additional embodiments, the invention provides a method of prediciting GAB, the method comprising: (a) quantifying in a biological sample obtained from said pregnant female an amount of each of N biomarkers selected from the biomarkers listed in Tables 1 through 63, including the biomarker of the invention; (b) multiplying or thresholding said amount by a predetermined coefficient, (c) determining the predicted GAB birth in said pregnant female comprising adding said individual products to obtain a total risk score that corresponds to said predicted GAB.

In further embodiments, the invention provides a method of prediciting time to birth in a pregnant female, the method comprising: (a) obtaining a biological sample from said pregnant female; (b) quantifying an amount of each of N biomarkers selected from the biomarkers listed in Tables 1 through 63, including the biomarker of the invention in said biological sample; (c) multiplying or thresholding said amount by a predetermined coefficient, (d) determining predicted GAB in said pregnant female comprising adding said individual products to obtain a total risk score that corresponds to said predicted GAB; and (e) substracting the estimated gestational age (GA) at time biological sample was obtained from the predicted GAB to predict time to birth in said pregnant female.

Other features and advantages of the invention will be apparent from the detailed description, and from the claims.

### BRIEF DESCRIPTION OF THE DRAWINGS

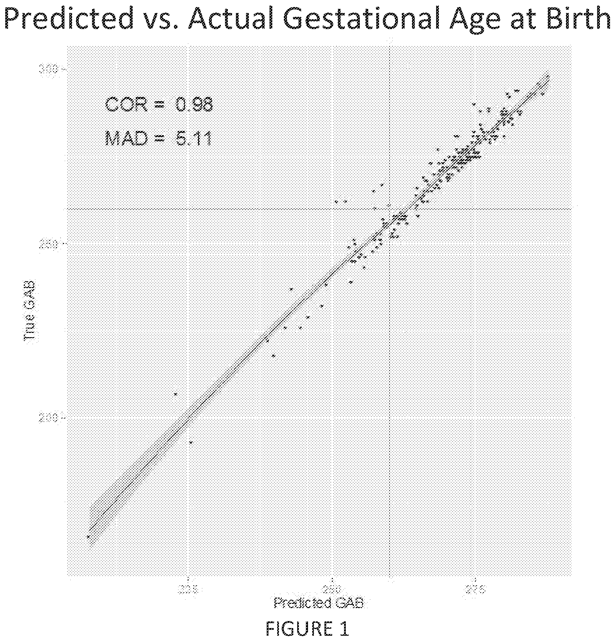

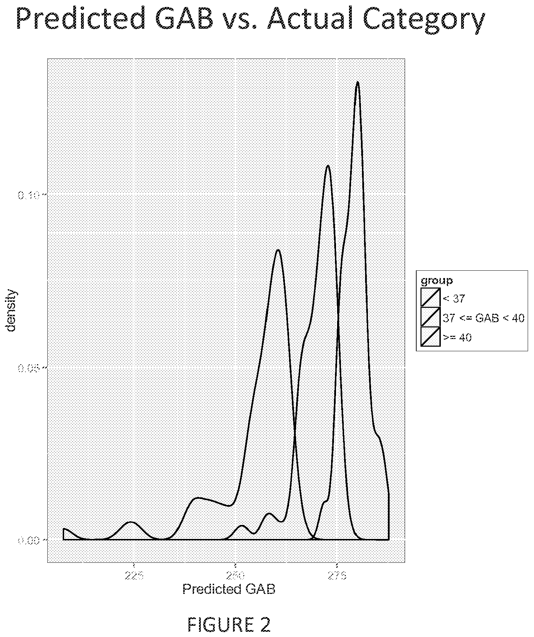

Figure 1. Scatterplot of actual gestational age at birth versus predicted gestational age from random forest regression model.
Firgure 2. Distribution of predicted gestational age from random forest regression model versus actual gestational age at birth (GAB), where actual GAB is given in categories of (i) less than 37 weeks, (ii) 37 to 39 weeks, and (iii) 40 weeks or greater (peaks left to right, respectively).

### DETAILED DESCRIPTION

The present disclosure is based, in part, on the discovery that certain proteins and peptides in biological samples obtained from a pregnant female are differentially expressed in pregnant females that have an increased risk of preterm birth relative to controls. The present disclosure is further based, in part, on the unexpected discovery that panels combining one or more of these proteins and peptides can be utilized in methods of determining the probability for preterm birth in a pregnant female with high sensitivity and specificity. These proteins and peptides disclosed herein serve as biomarkers for classifying test samples, predicting probability of preterm birth, predicting probability of term birth, predicting gestational age at birth (GAB), predicting time to birth and/or monitoring of progress of preventative therapy in a pregnant female, either individually or in a panel of biomarkers.

The disclosure provides biomarker panels, methods and kits for determining the probability for preterm birth in a pregnant female. One major advantage of the present disclosure is that risk of developing preterm birth can be assessed early during pregnancy so that appropriate monitoring and clinical management to prevent preterm delivery can be initiated in a timely fashion. The present invention is of particular benefit to females lacking any risk factors for preterm birth and who would not otherwise be identified and treated.

By way of example, the present disclosure includes methods for generating a result useful in determining probability for preterm birth in a pregnant female by obtaining a dataset associated with a sample, where the dataset at least includes quantitative data about biomarkers and panels of biomarkers that have been identified as predictive of preterm birth, and inputting the dataset into an analytic process that uses the dataset to generate a result useful in determining probability for preterm birth in a pregnant female. As described further below, this quantitative data can include amino acids, peptides, polypeptides, proteins, nucleotides, nucleic acids, nucleosides, sugars, fatty acids, steroids, metabolites, carbohydrates, lipids, hormones, antibodies, regions of interest that serve as surrogates for biological macromolecules and combinations thereof.

In addition to the specific biomarkers identified in this disclosure, for example, by accession number in a public database, sequence, or reference, the invention also contemplates use of biomarker variants that are at least 90% or at least 95% or at least 97% identical to the exemplified sequences and that are now known or later discovered and that have utility for the methods of the invention. These variants may represent polymorphisms, splice variants, mutations, and the like. In this regard, the instant specification discloses multiple art-known proteins in the context of the invention and provides exemplary accession numbers associated with one or more public databases as well as exemplary references to published journal articles relating to these art-known proteins. However, those skilled in the art appreciate that additional accession numbers and journal articles can easily be identified that can provide additional characteristics of the disclosed biomarkers and that the exemplified references are in no way limiting with regard to the disclosed biomarkers. As described herein, various techniques and reagents find use in the methods of the present invention. Suitable samples in the context of the present invention include, for example, blood, plasma, serum, amniotic fluid, vaginal secretions, saliva, and urine. In some embodiments, the biological sample is selected from the group consisting of whole blood, plasma, and serum. In a particular embodiment, the biological sample is serum. As described herein, biomarkers can be detected through a variety of assays and techniques known in the art. As further described herein, such assays include, without limitation, mass spectrometry (MS)-based assays, antibody-based assays as well as assays that combine aspects of the two.

Protein biomarkers associated with the probability for preterm birth in a pregnant female include, but are not limited to, one or more of the isolated biomarkers listed in Tables 1 through 63. In addition to the specific biomarkers, the disclosure further includes biomarker variants that are about 90%, about 95%, or about 97% identical to the exemplified sequences. Variants, as used herein, include polymorphisms, splice variants, mutations, and the like.

Additional markers can be selected from one or more risk indicia, including but not limited to, maternal characteristics, medical history, past pregnancy history, and obstetrical history. Such additional markers can include, for example, previous low birth weight or preterm delivery, multiple 2nd trimester spontaneous abortions, prior first trimester induced abortion, familial and intergenerational factors, history of infertility, nulliparity, placental abnormalities, cervical and uterine anomalies, short cervical length measurements, gestational bleeding, intrauterine growth restriction, in utero diethylstilbestrol exposure, multiple gestations, infant sex, short stature, low prepregnancy weight, low or high body mass index, diabetes, hypertension, urogenital infections (i.e. urinary tract infection), asthma, anxiety and depression, asthma, hypertension, hypothyroidism. Demographic risk indicia for preterm birth can include, for example, maternal age, race/ethnicity, single marital status, low socioeconomic status, maternal age, employment-related physical activity, occupational exposures and environment exposures and stress. Further risk indicia can include, inadequate prenatal care, cigarette smoking, use of marijuana and other illicit drugs, cocaine use, alcohol consumption, caffeine intake, maternal weight gain, dietary intake, sexual activity during late pregnancy and leisure-time physical activities. (Preterm Birth: Causes, Consequences, and Prevention, Institute of Medicine (US) Committee on Understanding Premature Birth and Assuring Healthy Outcomes; Behrman RE, Butler AS, editors. Washington (DC): National Academies Press (US); 2007). Additional risk indicia useful for as markers can be identified using learning algorithms known in the art, such as linear discriminant analysis, support vector machine classification, recursive feature elimination, prediction analysis of microarray, logistic regression, CART, FlexTree, LART, random forest, MART, and/or survival analysis regression, which are known to those of skill in the art and are further described herein.

Disclosed herein are panels of isolated biomarkers comprising N of the biomarkers selected from the group listed in Tables 1 through 63. In the disclosed panels of biomarkers N can be a number selected from the group consisting of 2 to 24. In the disclosed methods, the number of biomarkers that are detected and whose levels are determined, can be 1, or more than 1, such as 2, 3, 4, 5, 6, 7, 8, 9, 10, 11, 12, 12, 14, 15, 16, 17, 18, 19, 20, 21, 22, 23, 24, 25 or more. In certain instances the number of biomarkers that are detected, and whose levels are determined, can be 1, or more than 1, such as 2, 3, 4, 5, 6, 7, 8, 9, 10, or more. The methods of this disclosure are useful for determining the probability for preterm birth in a pregnant female.

While certain of the biomarkers listed in Tables 1 through 63 are useful alone for determining the probability for preterm birth in a pregnant female, methods are also described herein for the grouping of multiple subsets of the biomarkers that are each useful as a panel of three or more biomarkers. In some instances the invention provides panels comprising N biomarkers, wherein N is at least three biomarkers. In other instances N is selected to be any number from 3-23 biomarkers.

In yet other instances N is selected to be any number from 2-5, 2-10, 2-15, 2-20, or 2-23. In other instances N is selected to be any number from 3-5, 3-10, 3-15, 3-20, or 3-23. In other instances N is selected to be any number from 4-5, 4-10, 4-15, 4-20, or 4-23. In other instances N is selected to be any number from 5-10, 5-15, 5-20, or 5-23. In other instances N is selected to be any number from 6-10, 6-15, 6-20, or 6-23. In other instances N is selected to be any number from 7-10, 7-15, 7-20, or 7-23. In other instances N is selected to be any number from 8-10, 8-15, 8-20, or 8-23. In other instances N is selected to be any number from 9-10, 9-15, 9-20, or 9-23. In other instances N is selected to be any number from 10-15, 10-20, or 10-23. It will be appreciated that N can be selected to encompass similar, but higher order, ranges.

In certain instances the panel of isolated biomarkers comprises one or more, two or more, three or more, four or more, or five isolated biomarkers comprising an amino acid sequence selected from AFTECCVVASQLR, ELLESYIDGR, ITLPDFTGDLR, TDAPDLPEENQAR and SFRPFVPR. In some instances the panel of isolated biomarkers comprises one or more, two or more, three or more, four or more, or five isolated biomarkers comprising an amino acid sequence selected from FLNWIK, FGFGGSTDSGPIR, LLELTGPK, VEHSDLSFSK, IEGNLIFDPNNYLPK, ALVLELAK, TQILEWAAER, DVLLLVHNLPQNLPGYFWYK, SEPRPGVLLR, ITQDAQLK, ALDLSLK, WWGGQPLWITATK, and LSETNR.

In some instances the panel of isolated biomarkers comprises one or more, two or more, or three of the isolated biomarkers consisting of an amino acid sequence selected from AFTECCVVASQLR, ELLESYIDGR, and ITLPDFTGDLR. In some instances the panel of isolated biomarkers comprises one or more, two or more, or three of the isolated biomarkers consisting of an amino acid sequence selected from FLNWIK, FGFGGSTDSGPIR, LLELTGPK, VEHSDLSFSK, IEGNLIFDPNNYLPK, ALVLELAK, TQILEWAAER, DVLLLVHNLPQNLPGYFWYK, SEPRPGVLLR, ITQDAQLK, ALDLSLK, WWGGQPLWITATK, and LSETNR.

In some instances the panel of isolated biomarkers comprises one or more, two or more, or three of the isolated biomarkers consisting of an amino acid sequence selected from the biomarkers set forth in Table 50 and the biomarkers set forth in Table 52.

In some instances the panel of isolated biomarkers comprises one or more peptides comprising a fragment from lipopolysaccharide-binding protein (LBP), Schumann et al., Science 249 (4975), 1429-1431 (1990) (UniProtKB/Swiss-Prot: P18428.3); prothrombin (THRB), Walz et al., Proc. Natl. Acad. Sci. U.S.A. 74 (5), 1969-1972(1977) (NCBI Reference Sequence: NP_000497.1); complement component C5 (C5 or CO5) Haviland, J. Immunol. 146 (1), 362-368 (1991) (GenBank: AAA51925.1); plasminogen (PLMN) Petersen et al., J. Biol. Chem. 265 (11), 6104-6111(1990) (NCBI Reference Sequences: NP_000292.1 NP_001161810.1); and complement component C8 gamma chain (C8G or CO8G), Haefliger et al., Mol. Immunol. 28 (1-2), 123-131 (1991) (NCBI Reference Sequence: NP_000597.2).

In some instances the panel of isolated biomarkers comprises one or more peptides comprising a fragment from cell adhesion molecule with homology to complement component 1, q subcomponent, B chain (C1QB), Reid, Biochem. J. 179 (2), 367-371 (1979) (NCBI Reference Sequence: NP_000482.3); fibrinogen beta chain (FIBB or FIB); Watt et al., Biochemistry 18 (1), 68-76 (1979) (NCBI Reference Sequences: NP_001171670.1 and NP_005132.2); C-reactive protein (CRP), Oliveira et al., J. Biol. Chem. 254 (2), 489-502 (1979) (NCBI Reference Sequence: NP_000558.2); inter-alpha-trypsin inhibitor heavy chain H4 (ITIH4) Kim et al., Mol. Biosyst. 7 (5), 1430-1440 (2011) (NCBI Reference Sequences: NP_001159921.1 and NP_002209.2); chorionic somatomammotropin hormone (CSH) Selby et al., J. Biol. Chem. 259 (21), 13131-13138 (1984) (NCBI Reference Sequence: NP_001308.1); and angiotensinogen (ANG or ANGT) Underwood et al., Metabolism 60(8):1150-7 (2011) (NCBI Reference Sequence: NP_000020.1).

In additional instances the invention provides a panel of isolated biomarkers comprising N of the biomarkers listed in Tables 1 through 63. In some instances N is a number selected from the group consisting of 2 to 24. In additional instances the biomarker panel comprises at least two of the isolated biomarkers selected from the group consisting of AFTECCVVASQLR, ELLESYIDGR, and ITLPDFTGDLR. In additional instances the biomarker panel comprises at least two of the isolated biomarkers selected from the group consisting of AFTECCVVASQLR, ELLESYIDGR, ITLPDFTGDLR, TDAPDLPEENQAR and SFRPFVPR. In additional instances the biomarker panel comprises at least two of the isolated biomarkers selected from the group consisting of FLNWIK, FGFGGSTDSGPIR, LLELTGPK, VEHSDLSFSK, IEGNLIFDPNNYLPK, ALVLELAK, TQILEWAAER, DVLLLVHNLPQNLPGYFWYK, SEPRPGVLLR, ITQDAQLK, ALDLSLK, WWGGQPLWITATK, and LSETNR.

In additional instances the biomarker panel comprises at least two of the isolated biomarkers selected from the group consisting of the biomarkers set forth in Table 50 and the biomarkers set forth in Table 52.

In further instances the biomarker panel comprises at least two of the isolated biomarkers selected from the group consisting of lipopolysaccharide-binding protein (LBP), prothrombin (THRB), complement component C5 (C5 or CO5), plasminogen (PLMN), and complement component C8 gamma chain (C8G or CO8G). In another instance the invention provides a biomarker panel comprising at least three isolated biomarkers selected from the group consisting of lipopolysaccharide-binding protein (LBP), prothrombin (THRB), complement component C5 (C5 or CO5), plasminogen (PLMN), and complement component C8 gamma chain (C8G or CO8G).

In further instances the biomarker panel comprises at least two of the isolated biomarkers selected from the group consisting of Alpha-1B-glycoprotein (A1BG), Disintegrin and metalloproteinase domain-containing protein 12 (ADA12), Apolipoprotein B-100 (APOB), Beta-2-microglobulin (B2MG), CCAAT/enhancer-binding protein alpha/beta (HP8 Peptide), Corticosteroid-binding globulin (CBG), Complement component C6, Endoglin (EGLN), Ectonucleotide pyrophosphatase/phosphodiesterase family member 2 (ENPP2), Coagulation factor VII (FA7), Hyaluronan-binding protein 2 (HABP2), Pregnancy-specific beta-1-glycoprotein 9 (PSG9), Inhibin beta E chain (INHBE).

In some instances the biomarker panel comprises lipopolysaccharide-binding protein (LBP), prothrombin (THRB), complement component C5 (C5 or CO5), plasminogen (PLMN), complement component C8 gamma chain (C8G or CO8G), complement component 1, q subcomponent, B chain (C1QB), fibrinogen beta chain (FIBB or FIB), C-reactive protein (CRP), inter-alpha-trypsin inhibitor heavy chain H4 (ITIH4), chorionic somatomammotropin hormone (CSH), and angiotensinogen (ANG or ANGT). In some instances the biomarker panel comprises Alpha-1B-glycoprotein (A1BG), Disintegrin and metalloproteinase domain-containing protein 12 (ADA12), Apolipoprotein B-100 (APOB), Beta-2-microglobulin (B2MG), CCAAT/enhancer-binding protein alpha/beta (HP8 Peptide), Corticosteroid-binding globulin (CBG), Complement component C6, Endoglin (EGLN), Ectonucleotide pyrophosphatase/phosphodiesterase family member 2 (ENPP2), Coagulation factor VII (FA7), Hyaluronan-binding protein 2 (HABP2), Pregnancy-specific beta-1-glycoprotein 9 (PSG9), Inhibin beta E chain (INHBE).

In another instance the biomarker panel comprises at least two isolated biomarkers selected from the group consisting of lipopolysaccharide-binding protein (LBP), prothrombin (THRB), complement component C5 (C5 or CO5), plasminogen (PLMN), complement component C8 gamma chain (C8G or CO8G), complement component 1, q subcomponent, B chain (C1QB), fibrinogen beta chain (FIBB or FIB), C-reactive protein (CRP), inter-alpha-trypsin inhibitor heavy chain H4 (ITIH4), chorionic somatomammotropin hormone (CSH), and angiotensinogen (ANG or ANGT) and the biomarkers set forth in Tables 51 and 53.

In another instance the biomarker panel comprises at least two isolated biomarkers selected from the group consisting of Alpha-1B-glycoprotein (A1BG), Disintegrin and metalloproteinase domain-containing protein 12 (ADA12), Apolipoprotein B-100 (APOB), Beta-2-microglobulin (B2MG), CCAAT/enhancer-binding protein alpha/beta (HP8 Peptide), Corticosteroid-binding globulin (CBG), Complement component C6, Endoglin (EGLN), Ectonucleotide pyrophosphatase/phosphodiesterase family member 2 (ENPP2), Coagulation factor VII (FA7), Hyaluronan-binding protein 2 (HABP2), Pregnancy-specific beta-1-glycoprotein 9 (PSG9), Inhibin beta E chain (INHBE).

It must be noted that, as used in this specification and the appended claims, the singular forms "a", "an" and "the" include plural referents unless the content clearly dictates otherwise. Thus, for example, reference to "a biomarker" includes a mixture of two or more biomarkers, and the like.

The term "about," particularly in reference to a given quantity, is meant to encompass deviations of plus or minus five percent.

As used in this application, including the appended claims, the singular forms "a," "an," and "the" include plural references, unless the content clearly dictates otherwise, and are used interchangeably with "at least one" and "one or more."

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

As used herein, the term "panel" refers to a composition, such as an array or a collection, comprising one or more biomarkers. The term can also refer to a profile or index of expression patterns of one or more biomarkers described herein. The number of biomarkers useful for a biomarker panel is based on the sensitivity and specificity value for the particular combination of biomarker values.

As used herein, and unless otherwise specified, the terms "isolated" and "purified" generally describes a composition of matter that has been removed from its native environment (e.g., the natural environment if it is naturally occurring), and thus is altered by the hand of man from its natural state. An isolated protein or nucleic acid is distinct from the way it exists in nature.

The term "biomarker" refers to a biological molecule, or a fragment of a biological molecule, the change and/or the detection of which can be correlated with a particular physical condition or state. The terms "marker" and "biomarker" are used interchangeably throughout the disclosure. For example, the biomarkers of the present invention are correlated with an increased likelihood of preterm birth. Such biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins). The term also encompasses portions or fragments of a biological molecule, for example, peptide fragment of a protein or polypeptide that comprises at least 5 consecutive amino acid residues, at least 6 consecutive amino acid residues, at least 7 consecutive amino acid residues, at least 8 consecutive amino acid residues, at least 9 consecutive amino acid residues, at least 10 consecutive amino acid residues, at least 11 consecutive amino acid residues, at least 12 consecutive amino acid residues, at least 13 consecutive amino acid residues, at least 14 consecutive amino acid residues, at least 15 consecutive amino acid residues, at least 5 consecutive amino acid residues, at least 16 consecutive amino acid residues, at least 17consecutive amino acid residues, at least 18 consecutive amino acid residues, at least 19 consecutive amino acid residues, at least 20 consecutive amino acid residues, at least 21 consecutive amino acid residues, at least 22 consecutive amino acid residues, at least 23 consecutive amino acid residues, at least 24 consecutive amino acid residues, at least 25 consecutive amino acid residues,or more consecutive amino acid residues.

The invention also provides a method of determining probability for preterm birth in a pregnant female, the method comprising detecting a measurable feature of each of N biomarkers selected from the biomarkers listed in Tables 1 through 63, including the biomarker of the invention, in a biological sample obtained from the pregnant female, and analyzing the measurable feature to determine the probability for preterm birth in the pregnant female. As disclosed herein, a measurable feature comprises fragments or derivatives of each of said N biomarkers selected from the biomarkers listed in Tables 1 through 63. In some embodiments of the disclosed methods detecting a measurable feature comprises quantifying an amount of each of N biomarkers selected from the biomarkers listed in Tables 1 through 63, including the biomarker of the invention, combinations or portions and/or derivatives thereof in a biological sample obtained from said pregnant female.

The invention further provides a method of predicting GAB, the method encompassing detecting a measurable feature of each of N biomarkers selected from the biomarkers listed in Tables 1 through 63, including the biomarker of the invention, in a biological sample obtained from a pregnant female, and analyzing the measurable feature to predict GAB.

The invention also provides a method of prediciting GAB, the method comprising: (a) quantifying in a biological sample obtained from the pregnant female an amount of each of N biomarkers selected from the biomarkers listed in Tables 1 through 63, including the biomarker of the invention; (b) multiplying or thresholding the amount by a predetermined coefficient, (c) determining the predicted GAB birth in the pregnant female comprising adding the individual products to obtain a total risk score that corresponds to the predicted GAB.

The invention further provides a method of prediciting time to birth in a pregnant female, the method comprising: (a) obtaining a biological sample from the pregnant female; (b) quantifying an amount of each of N biomarkers selected from the biomarkers listed in Tables 1 through 63, including the biomarker of the invention, in the biological sample; (c) multiplying or thresholding the amount by a predetermined coefficient, (d) determining predicted GAB in the pregnant female comprising adding the individual products to obtain a total risk score that corresponds to the predicted GAB; and (e) substracting the estimated gestational age (GA) at time biological sample was obtained from the predicted GAB to predict time to birth in said pregnant female. For methods directed to prediciting time to birth, it is understood that "birth" means birth following spontaneous onset of labor, with or without rupture of membranes.

Although described and exemplified with reference to methods of determining probability for preterm birth in a pregnant female, the present disclosure is similarly applicable to the methods of predicting GAB, the methods for predicting term birth, methods for determining the probability of term birth in a pregnant female as well methods of prediciting time to birth in a pregnant female. It will be apparent to one skilled in the art that each of the aforementioned methods has specific and substantial utilities and benefits with regard maternal-fetal health considerations.

In some embodiments, the method of determining probability for preterm birth in a pregnant female and related methods disclosed herein further comprise detecting a measurable feature of each of N biomarkers, wherein N is selected from the group consisting of 2 to 24. In further embodiments, the disclosed methods of determining probability for preterm birth in a pregnant female and related methods disclosed herein further comprise detecting a measurable feature of each of at least two isolated biomarkers selected from the group consisting of AFTECCVVASQLR, ELLESYIDGR, and ITLPDFTGDLR. In further embodiments, the disclosed methods of determining probability for preterm birth in a pregnant female and related methods disclosed herein further comprise detecting a measurable feature of each of at least two isolated biomarkers selected from the group consisting of FLNWIK, FGFGGSTDSGPIR, LLELTGPK, VEHSDLSFSK, IEGNLIFDPNNYLPK, ALVLELAK, TQILEWAAER, DVLLLVHNLPQNLPGYFWYK, SEPRPGVLLR, ITQDAQLK, ALDLSLK, WWGGQPLWITATK, and LSETNR.

In additional embodiments, the disclosed methods of determining probability for preterm birth in a pregnant female and related methods disclosed herein further comprise detecting a measurable feature of each of at least two isolated biomarkers selected from the group consisting of the biomarkers set forth in Table 50 and the biomarkers set forth in Table 52.

In additional embodiments, the method of determining probability for preterm birth in a pregnant female and related methods disclosed herein further comprise detecting a measurable feature of each of at least two isolated biomarkers selected from the group consisting of lipopolysaccharide-binding protein (LBP), prothrombin (THRB), complement component C5 (C5 or CO5), plasminogen (PLMN), and complement component C8 gamma chain (C8G or CO8G).

In additional embodiments, the method of determining probability for preterm birth in a pregnant female and related methods disclosed herein further comprise detecting a measurable feature of each of at least two isolated biomarkers selected from the group consisting of Alpha-1B-glycoprotein (A1BG), Disintegrin and metalloproteinase domain-containing protein 12 (ADA12), Apolipoprotein B-100 (APOB), Beta-2-microglobulin (B2MG), CCAAT/enhancer-binding protein alpha/beta (HP8 Peptide), Corticosteroid-binding globulin (CBG), Complement component C6, Endoglin (EGLN), Ectonucleotide pyrophosphatase/phosphodiesterase family member 2 (ENPP2), Coagulation factor VII (FA7), Hyaluronan-binding protein 2 (HABP2), Pregnancy-specific beta-1-glycoprotein 9 (PSG9), Inhibin beta E chain (INHBE).

In further embodiments, the disclosed method of determining probability for preterm birth in a pregnant female and related methods disclosed herein further comprise detecting a measurable feature of each of at least two isolated biomarkers selected from the group consisting of lipopolysaccharide-binding protein (LBP), prothrombin (THRB), complement component C5 (C5 or CO5), plasminogen (PLMN), complement component C8 gamma chain (C8G or CO8G), complement component 1, q subcomponent, B chain (C1QB), fibrinogen beta chain (FIBB or FIB), C-reactive protein (CRP), inter-alpha-trypsin inhibitor heavy chain H4 (ITIH4), chorionic somatomammotropin hormone (CSH), and angiotensinogen (ANG or ANGT).

In further embodiments, the disclosed method of determining probability for preterm birth in a pregnant female and related methods disclosed herein further comprise detecting a measurable feature of each of at least two isolated biomarkers selected from the group consisting of Alpha-1B-glycoprotein (A1BG), Disintegrin and metalloproteinase domain-containing protein 12 (ADA12), Apolipoprotein B-100 (APOB), Beta-2-microglobulin (B2MG), CCAAT/enhancer-binding protein alpha/beta (HP8 Peptide), Corticosteroid-binding globulin (CBG), Complement component C6, Endoglin (EGLN), Ectonucleotide pyrophosphatase/phosphodiesterase family member 2 (ENPP2), Coagulation factor VII (FA7), Hyaluronan-binding protein 2 (HABP2), Pregnancy-specific beta-1-glycoprotein 9 (PSG9), Inhibin beta E chain (INHBE).

In further embodiments, the disclosed method of determining probability for preterm birth in a pregnant female and related methods disclosed herein further comprise detecting a measurable feature of each of at least two isolated biomarkers selected from the group consisting of Alpha-1B-glycoprotein (A1BG), Disintegrin and metalloproteinase domain-containing protein 12 (ADA12), Apolipoprotein B-100 (APOB), Beta-2-microglobulin (B2MG), CCAAT/enhancer-binding protein alpha/beta (HP8 Peptide), Corticosteroid-binding globulin (CBG), Complement component C6, Endoglin (EGLN), Ectonucleotide pyrophosphatase/phosphodiesterase family member 2 (ENPP2), Coagulation factor VII (FA7), Hyaluronan-binding protein 2 (HABP2), Pregnancy-specific beta-1-glycoprotein 9 (PSG9), Inhibin beta E chain (INHBE).

In further embodiments, the disclosed method of determining probability for preterm birth in a pregnant female and related methods disclosed herein further comprise detecting a measurable feature of each of at least two isolated biomarkers selected from the group consisting of the biomarkers set forth in Table 51 and the biomarkers set forth in Table 53.

As provided by the invention the methods of determining probability for preterm birth in a pregnant female further encompass detecting a measurable feature for one or more risk indicia associated with preterm birth. In additional embodiments the risk indicia are selected form the group consisting of previous low birth weight or preterm delivery, multiple 2nd trimester spontaneous abortions, prior first trimester induced abortion, familial and intergenerational factors, history of infertility, nulliparity, placental abnormalities, cervical and uterine anomalies, gestational bleeding, intrauterine growth restriction, in utero diethylstilbestrol exposure, multiple gestations, infant sex, short stature, low prepregnancy weight, low or high body mass index, diabetes, hypertension, and urogenital infections.

A "measurable feature" is any property, characteristic or aspect that can be determined and correlated with the probability for preterm birth in a subject. The term further encompasses any property, characteristic or aspect that can be determined and correlated in connection with a prediction of GAB, a prediction of term birth, or a prediction of time to birth in a pregnant female. For a biomarker, such a measurable feature can include, for example, the presence, absence, or concentration of the biomarker, or a fragment thereof, in the biological sample, an altered structure, such as, for example, the presence or amount of a post-translational modification, such as oxidation at one or more positions on the amino acid sequence of the biomarker or, for example, the presence of an altered conformation in comparison to the conformation of the biomarker in normal control subjects, and/or the presence, amount, or altered structure of the biomarker as a part of a profile of more than one biomarker. In addition to biomarkers, measurable features can further include risk indicia including, for example, maternal characteristics, age, race, ethnicity, medical history, past pregnancy history, obstetrical history. For a risk indicium, a measurable feature can include, for example, previous low birth weight or preterm delivery, multiple 2nd trimester spontaneous abortions, prior first trimester induced abortion, familial and intergenerational factors, history of infertility, nulliparity, placental abnormalities, cervical and uterine anomalies, short cervical length meansurements, gestational bleeding, intrauterine growth restriction, in utero diethylstilbestrol exposure, multiple gestations, infant sex, short stature, low prepregnancy weight/low body mass index, diabetes, hypertension, urogenital infections, hypothyroidism,asthma, low educational attainment, cigarette smoking, drug use and alcohol consumption.

In some embodiments of the disclosed methods of determining probability for preterm birth in a pregnant female, the probability for preterm birth in the pregnant female is calculated based on the quantified amount of each of N biomarkers selected from the biomarkers listed in Tables 1 through 63, including the biomarker of the invention. In some embodiments, the disclosed methods for determining the probability of preterm birth encompass detecting and/or quantifying one or more biomarkers using mass sprectrometry, a capture agent or a combination thereof.

In some embodiments, the disclosed methods of determining probability for preterm birth in a pregnant female encompass an initial step of providing a biomarker panel comprising N of the biomarkers listed in Tables 1 through 63, including the biomarker of the invention. In some instances the disclosed methods of determining probability for preterm birth in a pregnant female encompass an initial step of providing a biological sample from the pregnant female.

In some embodiments, the disclosed methods of determining probability for preterm birth in a pregnant female encompass communicating the probability to a health care provider. The disclosed of predicting GAB, the methods for predicting term birth, methods for determining the probability of term birth in a pregnant female as well methods of prediciting time to birth in a pregnant female similarly encompass communicating the probability to a health care provider. As stated above, although described and exemplified with reference to determining probability for preterm birth in a pregnant female, all embodiments described throughout this disclosure are similarly applicable to the methods of predicting GAB, the methods for predicting term birth, methods for determining the probability of term birth in a pregnant female as well methods of prediciting time to birth in a pregnant female. Specifically, he biomarkers and panels recited throughout this application with express reference to methods for preterm birth can also be used in methods for predicting GAB, the methods for predicting term birth, methods for determining the probability of term birth in a pregnant female as well methods of prediciting time to birth in a pregnant female. It will be apparent to one skilled in the art that each of the aforementioned methods have specific and substantial utilities and benefits with regard maternal-fetal health considerations.

In additional embodiments, the communication informs a subsequent treatment decision for the pregnant female. In some embodiments, the method of determining probability for preterm birth in a pregnant female encompasses the additional feature of expressing the probability as a risk score.

As used herein, the term "risk score" refers to a score that can be assigned based on comparing the amount of one or more biomarkers in a biological sample obtained from a pregnant female to a standard or reference score that represents an average amount of the one or more biomarkers calculated from biological samples obtained from a random pool of pregnant females. Because the level of a biomarker may not be static throughout pregnancy, a standard or reference score has to have been obtained for the gestational time point that corresponds to that of the pregnant female at the time the sample was taken. The standard or reference score can be predetermined and built into a predictor model such that the comparison is indirect rather than actually performed every time the probability is determined for a subject. A risk score can be a standard (e.g., a number) or a threshold (*e.g.,* a line on a graph). The value of the risk score correlates to the deviation, upwards or downwards, from the average amount of the one or more biomarkers calculated from biological samples obtained from a random pool of pregnant females. In certain embodiments, if a risk score is greater than a standard or reference risk score, the pregnant female can have an increased likelihood of preterm birth. In some embodiments, the magnitude of a pregnant female's risk score, or the amount by which it exceeds a reference risk score, can be indicative of or correlated to that pregnant female's level of risk.

In the context of the present invention, the term "biological sample," encompasses any sample that is taken from pregnant female and contains one or more of the biomarkers listed in Tables 1 through 63 and includes the biomarker of the invention. Suitable samples in the context of the present invention include, for example, blood, plasma, serum, amniotic fluid, vaginal secretions, saliva, and urine. In some embodiments, the biological sample is selected from the group consisting of whole blood, plasma, and serum. In a particular embodiment, the biological sample is serum. As will be appreciated by those skilled in the art, a biological sample can include any fraction or component of blood, without limitation, T cells, monocytes, neutrophils, erythrocytes, platelets and microvesicles such as exosomes and exosome-like vesicles. In a particular embodiment, the biological sample is serum.

Preterm birth refers to delivery or birth at a gestational age less than 37 completed weeks. Other commonly used subcategories of preterm birth have been established and delineate moderately preterm (birth at 33 to 36 weeks of gestation), very preterm (birth at <33 weeks of gestation), and extremely preterm (birth at ≤28 weeks of gestation). With regard to the methods disclosed herein, those skilled in the art understand that the cut-offs that delineate preterm birth and term birth as well as the cut-offs that delineate subcategories of preterm birth can be adjusted in practicing the methods disclosed herein, for example, to maximize a particular health benefit. It is further understood that such adjustments are well within the skill set of individuals considered skilled in the art and encompassed within the scope of the inventions disclosed herein. Gestational age is a proxy for the extent of fetal development and the fetus's readiness for birth. Gestational age has typically been defined as the length of time from the date of the last normal menses to the date of birth. However, obstetric measures and ultrasound estimates also can aid in estimating gestational age. Preterm births have generally been classified into two separate subgroups. One, spontaneous preterm births are those occurring subsequent to spontaneous onset of preterm labor or preterm premature rupture of membranes regardless of subsequent labor augmentation or cesarean delivery. Two, indicated preterm births are those occurring following induction or cesarean section for one or more conditions that the woman's caregiver determines to threaten the health or life of the mother and/or fetus. In some cases the methods of the invention are directed to determining the probability for spontaneous preterm birth. In additional cases the methods of the invention are directed to predicting gestational birth.

As used herein, the term "estimated gestational age" or "estimated GA" refers to the GA determined based on the date of the last normal menses and additional obstetric measures, ultrasound estimates or other clinical parameters including, without limitation, those described in the preceding paragraph. In contrast the term "predicted gestational age at birth" or "predicted GAB" refers to the GAB determined based on the methods of the invention as dislosed herein. As used herein, "term birth" refers to birth at a gestational age equal or more than 37 completed weeks.

In some embodiments, the pregnant female is between 17 and 28 weeks of gestation at the time the biological sample is collected. In other embodiments, the pregnant female is between 16 and 29 weeks, between 17 and 28 weeks, between 18 and 27 weeks, between 19 and 26 weeks, between 20 and 25 weeks, between 21 and 24 weeks, or between 22 and 23 weeks of gestation at the time the biological sample is collected. In further embodiments, the the pregnant female is between about 17 and 22 weeks, between about 16 and 22 weeks between about 22 and 25 weeks, between about 13 and 25 weeks, between about 26 and 28, or between about 26 and 29 weeks of gestation at the time the biological sample is collected. Accordingly, the gestational age of a pregnant female at the time the biological sample is collected can be 15, 16, 17, 18, 19, 20, 21, 22, 23, 24, 25, 26, 27, 28, 29 or 30 weeks.

In some embodiments of the claimed methods the measurable feature comprises fragments or derivatives of each of the N biomarkers selected from the biomarkers listed in Tables 1 through 63, including the biomarker of the invention. In additional embodiments of the claimed methods, detecting a measurable feature comprises quantifying an amount of each of N biomarkers selected from the biomarkers listed in Tables 1 through 63, including the biomarker of the invention, combinations or portions and/or derivatives thereof in a biological sample obtained from said pregnant female.

The term "amount" or "level" as used herein refers to a quantity of a biomarker that is detectable or measurable in a biological sample and/or control. The quantity of a biomarker can be, for example, a quantity of polypeptide, the quantity of nucleic acid, or the quantity of a fragment or surrogate. The term can alternatively include combinations thereof. The term "amount" or "level" of a biomarker is a measurable feature of that biomarker.

In some embodiments, calculating the probability for preterm birth in a pregnant female is based on the quantified amount of each of N biomarkers selected from the biomarkers listed in Tables 1 through 63, including the biomarker of the invention. Any existing, available or conventional separation, detection and quantification methods can be used herein to measure the presence or absence (*e.g.,* readout being present vs. absent; or detectable amount vs. undetectable amount) and/or quantity (*e.g.,* readout being an absolute or relative quantity, such as, for example, absolute or relative concentration) of biomarkers, peptides, polypeptides, proteins and/or fragments thereof and optionally of the one or more other biomarkers or fragments thereof in samples. In some embodiments, detection and/or quantification of one or more biomarkers comprises an assay that utilizes a capture agent. In further embodiments, the capture agent is an antibody, antibody fragment, nucleic acid-based protein binding reagent, small molecule or variant thereof. In additional embodiments, the assay is an enzyme immunoassay (EIA), enzyme-linked immunosorbent assay (ELISA), and radioimmunoassay (RIA). In some embodiments, detection and/or quantification of one or more biomarkers further comprises mass spectrometry (MS). In yet further embodiments, the mass spectrometry is co-immunoprecitipation-mass spectrometry (co-IP MS), where coimmunoprecipitation, a technique suitable for the isolation of whole protein complexes is followed by mass spectrometric analysis.

As used herein, the term "mass spectrometer" refers to a device able to volatilize/ionize analytes to form gas-phase ions and determine their absolute or relative molecular masses. Suitable methods of volatilization/ionization are matrix-assisted laser desorption ionization (MALDI), electrospray, laser/light, thermal, electrical, atomized/sprayed and the like, or combinations thereof. Suitable forms of mass spectrometry include, but are not limited to, ion trap instruments, quadrupole instruments, electrostatic and magnetic sector instruments, time of flight instruments, time of flight tandem mass spectrometer (TOF MS/MS), Fourier-transform mass spectrometers, Orbitraps and hybrid instruments composed of various combinations of these types of mass analyzers. These instruments can, in turn, be interfaced with a variety of other instruments that fractionate the samples (for example, liquid chromatography or solid-phase adsorption techniques based on chemical, or biological properties) and that ionize the samples for introduction into the mass spectrometer, including matrix-assisted laser desorption (MALDI), electrospray, or nanospray ionization (ESI) or combinations thereof.

Generally, any mass spectrometric (MS) technique that can provide precise information on the mass of peptides, and preferably also on fragmentation and/or (partial) amino acid sequence of selected peptides (*e.g.,* in tandem mass spectrometry, MS/MS; or in post source decay, TOF MS), can be used in the methods disclosed herein. Suitable peptide MS and MS/MS techniques and systems are well-known *per se* (see, e.g., Methods in Molecular Biology, vol. 146: "Mass Spectrometry of Proteins and Peptides", by Chapman, ed., Humana Press 2000; Biemann 1990. Methods Enzymol 193: 455-79; or Methods in Enzymology, vol. 402: "Biological Mass Spectrometry", by Burlingame, ed., Academic Press 2005) and can be used in practicing the methods disclosed herein. Accordingly, in some embodiments, the disclosed methods comprise performing quantitative MS to measure one or more biomarkers. Such quantitiative methods can be performed in an automated (Villanueva, et al., Nature Protocols (2006) 1(2):880-891) or semi-automated format. In particular embodiments, MS can be operably linked to a liquid chromatography device (LC-MS/MS or LC-MS) or gas chromatography device (GC-MS or GC-MS/MS). Other methods useful in this context include isotope-coded affinity tag (ICAT), tandem mass tags (TMT), or stable isotope labeling by amino acids in cell culture (SILAC), followed by chromatography and MS/MS.

As used herein, the terms "multiple reaction monitoring (MRM)" or "selected reaction monitoring (SRM)" refer to an MS-based quantification method that is particularly useful for quantifying analytes that are in low abundance. In an SRM experiment, a predefined precursor ion and one or more of its fragments are selected by the two mass filters of a triple quadrupole instrument and monitored over time for precise quantification. Multiple SRM precursor and fragment ion pairs can be measured within the same experiment on the chromatographic time scale by rapidly toggling between the different precursor/fragment pairs to perform an MRM experiment. A series of transitions (precursor/fragment ion pairs) in combination with the retention time of the targeted analyte (*e.g.,* peptide or small molecule such as chemical entity, steroid, hormone) can constitute a definitive assay. A large number of analytes can be quantified during a single LC-MS experiment. The term "scheduled," or "dynamic" in reference to MRM or SRM, refers to a variation of the assay wherein the transitions for a particular analyte are only acquired in a time window around the expected retention time, significantly increasing the number of analytes that can be detected and quantified in a single LC-MS experiment and contributing to the selectivity of the test, as retention time is a property dependent on the physical nature of the analyte. A single analyte can also be monitored with more than one transition. Finally, included in the assay can be standards that correspond to the analytes of interest (*e.g.,* same amino acid sequence), but differ by the inclusion of stable isotopes. Stable isotopic standards (SIS) can be incorporated into the assay at precise levels and used to quantify the corresponding unknown analyte. An additional level of specificity is contributed by the co-elution of the unknown analyte and its corresponding SIS and properties of their transitions (*e.g.,* the similarity in the ratio of the level of two transitions of the unknown and the ratio of the two transitions of its corresponding SIS).

Mass spectrometry assays, instruments and systems suitable for biomarker peptide analysis can include, without limitation, matrix-assisted laser desorption/ionisation time-of-flight (MALDI-TOF) MS; MALDI-TOF post-source-decay (PSD); MALDI-TOF/TOF; surface-enhanced laser desorption/ionization time-of-flight mass spectrometry (SELDI-TOF) MS; electrospray ionization mass spectrometry (ESI-MS); ESI-MS/MS; ESI-MS/(MS)ₙ (n is an integer greater than zero); ESI 3D or linear (2D) ion trap MS; ESI triple quadrupole MS; ESI quadrupole orthogonal TOF (Q-TOF); ESI Fourier transform MS systems; desorption/ionization on silicon (DIOS); secondary ion mass spectrometry (SIMS); atmospheric pressure chemical ionization mass spectrometry (APCI-MS); APCI-MS/MS; APCI- (MS)ₙ; ion mobility spectrometry (IMS); inductively coupled plasma mass spectrometry (ICP-MS)atmospheric pressure photoionization mass spectrometry (APPI-MS); APPI-MS/MS; and APPI- (MS)ₙ. Peptide ion fragmentation in tandem MS (MS/MS) arrangements can be achieved using manners established in the art, such as, e.g., collision induced dissociation (CID). As described herein, detection and quantification of biomarkers by mass spectrometry can involve multiple reaction monitoring (MRM), such as described among others by Kuhn et al. Proteomics 4: 1175-86 (2004). Scheduled multiple-reaction-monitoring (Scheduled MRM) mode acquisition during LC-MS/MS analysis enhances the sensitivity and accuracy of peptide quantitation. Anderson and Hunter, Molecular and Cellular Proteomics 5(4):573 (2006). As described herein, mass spectrometry-based assays can be advantageously combined with upstream peptide or protein separation or fractionation methods, such as for example with the chromatographic and other methods described herein below. As further described herein, shotgun quantitative proteomics can be combined with SRM/MRM-based assays for high-throughput identification and verification of prognostic biomarkers of preterm birth.

A person skilled in the art will appreciate that a number of methods can be used to determine the amount of a biomarker, including mass spectrometry approaches, such as MS/MS, LC-MS/MS, multiple reaction monitoring (MRM) or SRM and production monitoring (PIM) and also including antibody based methods such as immunoassays such as Western blots, enzyme-linked immunosorbant assay (ELISA), immunoprecipitation, immunohistochemistry, immunofluorescence, radioimmunoassay, dot blotting, and FACS. Accordingly, in some embodiments, determining the level of the at least one biomarker comprises using an immunoassay and/or mass spectrometric methods. In additional embodiments, the mass spectrometric methods are selected from MS, MS/MS, LC-MS/MS, SRM, PIM, and other such methods that are known in the art. In other embodiments, LC-MS/MS further comprises ID LC-MS/MS, 2D LC-MS/MS or 3D LC-MS/MS. Immunoassay techniques and protocols are generally known to those skilled in the art (Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and non-competitive immunoassays, can be used (Self et al., Curr. Opin. Biotechnol., 7:60-65 (1996).

In further embodiments, the immunoassay is selected from Western blot, ELISA, immunoprecipitation, immunohistochemistry, immunofluorescence, radioimmunoassay (RIA), dot blotting, and FACS. In certain embodiments, the immunoassay is an ELISA. In yet a further embodiment, the ELISA is direct ELISA (enzyme-linked immunosorbent assay), indirect ELISA, sandwich ELISA, competitive ELISA, multiplex ELISA, ELISPOT technologies, and other similar techniques known in the art. Principles of these immunoassay methods are known in the art, for example John R. Crowther, The ELISA Guidebook, 1st ed., Humana Press 2000, ISBN 0896037282. Typically ELISAs are performed with antibodies but they can be performed with any capture agents that bind specifically to one or more biomarkers of the invention and that can be detected. Multiplex ELISA allows simultaneous detection of two or more analytes within a single compartment (e.g., microplate well) usually at a plurality of array addresses (Nielsen and Geierstanger 2004. J Immunol Methods 290: 107-20 (2004) and Ling et al. 2007. Expert Rev Mol Diagn 7: 87-98 (2007)).

In some embodiments, Radioimmunoassay (RIA) can be used to detect one or more biomarkers in the methods of the invention. RIA is a competition-based assay that is well known in the art and involves mixing known quantities of radioactavely-labelled (e.g.,¹²⁵I or ¹³¹I-labelled) target analyte with antibody specific for the analyte, then adding non-labelled analyte from a sample and measuring the amount of labelled analyte that is displaced (see, e.g., An Introduction to Radioimmunoassay and Related Techniques, by Chard T, ed., Elsevier Science 1995, ISBN 0444821198 for guidance).

A detectable label can be used in the assays described herein for direct or indirect detection of the biomarkers in the methods of the invention. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the biomarkers in the methods of the invention. Suitable detectable labels include, but are not limited to, fluorescent dyes (*e.g.,* fluorescein, fluorescein isothiocyanate (FITC), Oregon Green™, rhodamine, Texas red, tetrarhodimine isothiocynate (TRITC), Cy3, Cy5, etc.), fluorescent markers (*e.g.,* green fluorescent protein (GFP), phycoerythrin, etc.), enzymes (*e.g.,* luciferase, horseradish peroxidase, alkaline phosphatase, etc.), nanoparticles, biotin, digoxigenin, metals, and the like.

For mass-sectrometry based analysis, differential tagging with isotopic reagents, *e.g.,* isotope-coded affinity tags (ICAT) or the more recent variation that uses isobaric tagging reagents, iTRAQ (Applied Biosystems, Foster City, Calif.), or tandem mass tags, TMT, (Thermo Scientific, Rockford, IL), followed by multidimensional liquid chromatography (LC) and tandem mass spectrometry (MS/MS) analysis can provide a further methodology in practicing the methods of the inventon.

A chemiluminescence assay using a chemiluminescent antibody can be used for sensitive, non-radioactive detection of protein levels. An antibody labeled with fluorochrome also can be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase, beta-galactosidase are well known in the art.

A signal from the direct or indirect label can be analyzed, for example, using a spectrophotometer to detect color from a chromogenic substrate; a radiation counter to detect radiation such as a gamma counter for detection of ¹²⁵I; or a fluorometer to detect fluorescence in the presence of light of a certain wavelength. For detection of enzyme-linked antibodies, a quantitative analysis can be made using a spectrophotometer such as an EMAX Microplate Reader (Molecular Devices; Menlo Park, Calif.) in accordance with the manufacturer's instructions. If desired, assays used to practice the invention can be automated or performed robotically, and the signal from multiple samples can be detected simultaneously.

In some embodiments, the methods described herein encompass quantification of the biomarkers using mass spectrometry (MS). In further embodiments, the mass spectrometry can be liquid chromatography-mass spectrometry (LC-MS), multiple reaction monitoring (MRM) or selected reaction monitoring (SRM). In additional embodiments, the MRM or SRM can further encompass scheduled MRM or scheduled SRM.

As described above, chromatography can also be used in practicing the methods of the invention. Chromatography encompasses methods for separating chemical substances and generally involves a process in which a mixture of analytes is carried by a moving stream of liquid or gas ("mobile phase") and separated into components as a result of differential distribution of the analytes as they flow around or over a stationary liquid or solid phase ("stationary phase"), between the mobile phase and said stationary phase. The stationary phase can be usually a finely divided solid, a sheet of filter material, or a thin film of a liquid on the surface of a solid, or the like. Chromatography is well understood by those skilled in the art as a technique applicable for the separation of chemical compounds of biological origin, such as, *e.g.,* amino acids, proteins, fragments of proteins or peptides, *etc.*

Chromatography can be columnar (*i.e.,* wherein the stationary phase is deposited or packed in a column), preferably liquid chromatography, and yet more preferably high-performance liquid chromatography (HPLC), or ultra high performance/pressure liquid chromatography (UHPLC). Particulars of chromatography are well known in the art (Bidlingmeyer, Practical HPLC Methodology and Applications, John Wiley & Sons Inc., 1993). Exemplary types of chromatography include, without limitation, high-performance liquid chromatography (HPLC), UHPLC, normal phase HPLC (NP-HPLC), reversed phase HPLC (RP-HPLC), ion exchange chromatography (IEC), such as cation or anion exchange chromatography, hydrophilic interaction chromatography (HILIC), hydrophobic interaction chromatography (HIC), size exclusion chromatography (SEC) including gel filtration chromatography or gel permeation chromatography, chromatofocusing, affinity chromatography such as immuno-affinity, immobilised metal affinity chromatography, and the like. Chromatography, including single-, two- or more-dimensional chromatography, can be used as a peptide fractionation method in conjunction with a further peptide analysis method, such as for example, with a downstream mass spectrometry analysis as described elsewhere in this specification.

Further peptide or polypeptide separation, identification or quantification methods can be used, optionally in conjunction with any of the above described analysis methods, for measuring biomarkers in the present disclosure. Such methods include, without limitation, chemical extraction partitioning, isoelectric focusing (IEF) including capillary isoelectric focusing (CIEF), capillary isotachophoresis (CITP), capillary electrochromatography (CEC), and the like, one-dimensional polyacrylamide gel electrophoresis (PAGE), two-dimensional polyacrylamide gel electrophoresis (2D-PAGE), capillary gel electrophoresis (CGE), capillary zone electrophoresis (CZE), micellar electrokinetic chromatography (MEKC), free flow electrophoresis (FFE), etc.

In the context of the invention, the term "capture agent" refers to a compound that can specifically bind to a target, in particular a biomarker. The term includes antibodies, antibody fragments, nucleic acid-based protein binding reagents (*e.g.* aptamers, Slow Off-rate Modified Aptamers (SOMAmer™)), protein-capture agents, natural ligands (*i.e.* a hormone for its receptor or vice versa), small molecules or variants thereof.

Capture agents can be configured to specifically bind to a target, in particular a biomarker. Capture agents can include but are not limited to organic molecules, such as polypeptides, polynucleotides and other non polymeric molecules that are identifiable to a skilled person. In the embodiments disclosed herein, capture agents include any agent that can be used to detect, purify, isolate, or enrich a target, in particular a biomarker. Any art-known affinity capture technologies can be used to selectively isolate and enrich/concentrate biomarkers that are components of complex mixtures of biological media for use in the disclosed methods.

Antibody capture agents that specifically bind to a biomarker can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). Antibody capture agents can be any immunoglobulin or derivative therof, whether natural or wholly or partially synthetically produced. All derivatives thereof which maintain specific binding ability are also included in the term. Antibody capture agents have a binding domain that is homologous or largely homologous to an immunoglobulin binding domain and can be derived from natural sources, or partly or wholly synthetically produced. Antibody capture agents can be monoclonal or polyclonal antibodies. In some embodiments, an antibody is a single chain antibody. Those of ordinary skill in the art will appreciate that antibodies can be provided in any of a variety of forms including, for example, humanized, partially humanized, chimeric, chimeric humanized, *etc.* Antibody capture agents can be antibody fragments including, but not limited to, Fab, Fab', F(ab')2, scFv, Fv, dsFv diabody, and Fd fragments. An antibody capture agent can be produced by any means. For example, an antibody capture agent can be enzymatically or chemically produced by fragmentation of an intact antibody and/or it can be recombinantly produced from a gene encoding the partial antibody sequence. An antibody capture agent can comprise a single chain antibody fragment. Alternatively or additionally, antibody capture agent can comprise multiple chains which are linked together, for example, by disulfide linkages.; and, any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

Suitable capture agents useful for practicing the invention also include aptamers. Aptamers are oligonucleotide sequences that can bind to their targets specifically via unique three dimensional (3-D) structures. An aptamer can include any suitable number of nucleotides and different aptamers can have either the same or different numbers of nucleotides. Aptamers can be DNA or RNA or chemically modified nucleic acids and can be single stranded, double stranded, or contain double stranded regions, and can include higher ordered structures. An aptamer can also be a photoaptamer, where a photoreactive or chemically reactive functional group is included in the aptamer to allow it to be covalently linked to its corresponding target. Use of an aptamer capture agent can include the use of two or more aptamers that specifically bind the same biomarker. An aptamer can include a tag. An aptamer can be identified using any known method, including the SELEX (systematic evolution of ligands by exponential enrichment), process. Once identified, an aptamer can be prepared or synthesized in accordance with any known method, including chemical synthetic methods and enzymatic synthetic methods and used in a variety of applications for biomarker detection. Liu et al., Curr Med Chem. 18(27):4117-25 (2011). Capture agents useful in practicing the methods of the invention also include SOMAmers (Slow Off-Rate Modified Aptamers) known in the art to have improved off-rate characteristics. Brody et al., J Mol Biol. 422(5):595-606 (2012). SOMAmers can be generated using using any known method, including the SELEX method.

It is understood by those skilled in the art that biomarkers can be modified prior to analysis to improve their resolution or to determine their identity. For example, the biomarkers can be subject to proteolytic digestion before analysis. Any protease can be used. Proteases, such as trypsin, that are likely to cleave the biomarkers into a discrete number of fragments are particularly useful. The fragments that result from digestion function as a fingerprint for the biomarkers, thereby enabling their detection indirectly. This is particularly useful where there are biomarkers with similar molecular masses that might be confused for the biomarker in question. Also, proteolytic fragmentation is useful for high molecular weight biomarkers because smaller biomarkers are more easily resolved by mass spectrometry. In another example, biomarkers can be modified to improve detection resolution. For instance, neuraminidase can be used to remove terminal sialic acid residues from glycoproteins to improve binding to an anionic adsorbent and to improve detection resolution. In another example, the biomarkers can be modified by the attachment of a tag of particular molecular weight that specifically binds to molecular biomarkers, further distinguishing them. Optionally, after detecting such modified biomarkers, the identity of the biomarkers can be further determined by matching the physical and chemical characteristics of the modified biomarkers in a protein database (*e.g.,* SwissProt).

It is further appreciated in the art that biomarkers in a sample can be captured on a substrate for detection. Traditional substrates include antibody-coated 96-well plates or nitrocellulose membranes that are subsequently probed for the presence of the proteins. Alternatively, protein-binding molecules attached to microspheres, microparticles, microbeads, beads, or other particles can be used for capture and detection of biomarkers. The protein-binding molecules can be antibodies, peptides, peptoids, aptamers, small molecule ligands or other protein-binding capture agents attached to the surface of particles. Each protein-binding molecule can include unique detectable label that is coded such that it can be distinguished from other detectable labels attached to other protein-binding molecules to allow detection of biomarkers in multiplex assays. Examples include, but are not limited to, color-coded microspheres with known fluorescent light intensities (see e.g., microspheres with xMAP technology produced by Luminex (Austin, Tex.); microspheres containing quantum dot nanocrystals, for example, having different ratios and combinations of quantum dot colors (e.g., Qdot nanocrystals produced by Life Technologies (Carlsbad, Calif.); glass coated metal nanoparticles (see *e.g.,* SERS nanotags produced by Nanoplex Technologies, Inc. (Mountain View, Calif.); barcode materials (see e.g., sub-micron sized striped metallic rods such as Nanobarcodes produced by Nanoplex Technologies, Inc.), encoded microparticles with colored bar codes (see e.g., CellCard produced by Vitra Bioscience, vitrabio.com), glass microparticles with digital holographic code images (see *e.g.,* CyVera microbeads produced by Illumina (San Diego, Calif.); chemiluminescent dyes, combinations of dye compounds; and beads of detectably different

Biochips can be used for capture and detection of the biomarkers of the invention. Many protein biochips are known in the art. These include, for example, protein biochips produced by Packard BioScience Company (Meriden Conn.), Zyomyx (Hayward, Calif.) and Phylos (Lexington, Mass.). In general, protein biochips comprise a substrate having a surface. A capture reagent or adsorbent is attached to the surface of the substrate. Frequently, the surface comprises a plurality of addressable locations, each of which location has the capture agent bound there. The capture agent can be a biological molecule, such as a polypeptide or a nucleic acid, which captures other biomarkers in a specific manner. Alternatively, the capture agent can be a chromatographic material, such as an anion exchange material or a hydrophilic material. Examples of protein biochips are well known in the art.

Measuring mRNA in a biological sample can be used as a surrogate for detection of the level of the corresponding protein biomarker in a biological sample. Thus, any of the biomarkers or biomarker panels described herein can also be detected by detecting the appropriate RNA. Levels of mRNA can measured by reverse transcription quantitative polymerase chain reaction (RT-PCR followed with qPCR). RT-PCR is used to create a cDNA from the mRNA. The cDNA can be used in a qPCR assay to produce fluorescence as the DNA amplification process progresses. By comparison to a standard curve, qPCR can produce an absolute measurement such as number of copies of mRNA per cell. Northern blots, microarrays, Invader assays, and RT-PCR combined with capillary electrophoresis have all been used to measure expression levels of mRNA in a sample. See Gene Expression Profiling: Methods and Protocols, Richard A. Shimkets, editor, Humana Press, 2004.

Some embodiments disclosed herein relate to diagnostic and prognostic methods of determining the probability for preterm birth in a pregnant female. The detection of the level of expression of one or more biomarkers and/or the determination of a ratio of biomarkers can be used to determine the probability for preterm birth in a pregnant female. Such detection methods can be used, for example, for early diagnosis of the condition, to determine whether a subject is predisposed to preterm birth, to monitor the progress of preterm birth or the progress of treatment protocols, to assess the severity of preterm birth, to forecast the outcome of preterm birth and/or prospects of recovery or birth at full term, or to aid in the determination of a suitable treatment for preterm birth.

The quantitation of biomarkers in a biological sample can be determined, without limitation, by the methods described above as well as any other method known in the art. The quantitative data thus obtained is then subjected to an analytic classification process. In such a process, the raw data is manipulated according to an algorithm, where the algorithm has been pre-defined by a training set of data, for example as described in the examples provided herein. An algorithm can utilize the training set of data provided herein, or can utilize the guidelines provided herein to generate an algorithm with a different set of data.

In some embodiments, analyzing a measurable feature to determine the probability for preterm birth in a pregnant female encompasses the use of a predictive model. In further embodiments, analyzing a measurable feature to determine the probability for preterm birth in a pregnant female encompasses comparing said measurable feature with a reference feature. As those skilled in the art can appreciate, such comparison can be a direct comparison to the reference feature or an indirect comparison where the reference feature has been incorporated into the predictive model. In further embodiments, analyzing a measurable feature to determine the probability for preterm birth in a pregnant female encompasses one or more of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, or a combination thereof. In particular embodiments, the analysis comprises logistic regression.

An analytic classification process can use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms; etc.

For creation of a random forest for prediction of GAB one skilled in the art can consider a set of k subjects (pregnant women) for whom the gestational age at birth (GAB) is known, and for whom N analytes (transitions) have been measured in a blood specimen taken several weeks prior to birth. A regression tree begins with a root node that contains all the subjects. The average GAB for all subjects can be cacluclated in the root node. The variance of the GAB within the root node will be high, because there is a mixture of women with different GAB's. The root node is then divided (partitioned) into two branches, so that each branch contains women with a similar GAB. The average GAB for subjects in each branch is again caluclated. The variance of the GAB within each branch will be lower than in the root node, because the subset of women within each branch has relatively more similar GAB's than those in the root node. The two branches are created by selecting an analyte and a threshold value for the analyte that creates branches with similar GAB. The analyte and threshold value are chosen from among the set of all analytes and threshold values, usually with a random subset of the analytes at each node. The procedure continues recursively producing branches to create leaves (terminal nodes) in which the subjects have very similar GAB's. The predicted GAB in each terminal node is the average GAB for subjects in that terminal node. This procedure creates a single regression tree. A random forest can consist of several hundred or several thousand such trees.

Classification can be made according to predictive modeling methods that set a threshold for determining the probability that a sample belongs to a given class. The probability preferably is at least 50%, or at least 60%, or at least 70%, or at least 80% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model can be evaluated according to its ability to provide a quality metric, e.g. AUROC (area under the ROC curve) or accuracy, of a particular value, or range of values. Area under the curve measures are useful for comparing the accuracy of a classifier across the complete data range. Classifiers with a greater AUC have a greater capacity to classify unknowns correctly between two groups of interest. In some embodiments, a desired quality threshold is a predictive model that will classify a sample with an accuracy of at least about 0.5, at least about 0.55, at least about 0.6, at least about 0.7, at least about 0.75, at least about 0.8, at least about 0.85, at least about 0.9, at least about 0.95, or higher. As an alternative measure, a desired quality threshold can refer to a predictive model that will classify a sample with an AUC of at least about 0.7, at least about 0.75, at least about 0.8, at least about 0.85, at least about 0.9, or higher.

As is known in the art, the relative sensitivity and specificity of a predictive model can be adjusted to favor either the selectivity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about 0.7, at least about 0.75, at least about 0.8, at least about 0.85, at least about 0.9, or higher.

The raw data can be initially analyzed by measuring the values for each biomarker, usually in triplicate or in multiple triplicates. The data can be manipulated, for example, raw data can be transformed using standard curves, and the average of triplicate measurements used to calculate the average and standard deviation for each patient. These values can be transformed before being used in the models, *e.g.* log-transformed, Box-Cox transformed (Box and Cox, Royal Stat. Soc., Series B, 26:211-246(1964). The data are then input into a predictive model, which will classify the sample according to the state. The resulting information can be communicated to a patient or health care provider.

To generate a predictive model for preterm birth, a robust data set, comprising known control samples and samples corresponding to the preterm birth classification of interest is used in a training set. A sample size can be selected using generally accepted criteria. As discussed above, different statistical methods can be used to obtain a highly accurate predictive model. Examples of such analysis are provided in Example 2.

In one embodiment, hierarchical clustering is performed in the derivation of a predictive model, where the Pearson correlation is employed as the clustering metric. One approach is to consider a preterm birth dataset as a "learning sample" in a problem of "supervised learning." CART is a standard in applications to medicine (Singer, Recursive Partitioning in the Health Sciences, Springer(1999)) and can be modified by transforming any qualitative features to quantitative features; sorting them by attained significance levels, evaluated by sample reuse methods for Hotelling's T² statistic; and suitable application of the lasso method. Problems in prediction are turned into problems in regression without losing sight of prediction, indeed by making suitable use of the Gini criterion for classification in evaluating the quality of regressions.

This approach led to what is termed FlexTree (Huang, Proc. Nat. Acad. Sci. U.S.A 101:10529-10534(2004)). FlexTree performs very well in simulations and when applied to multiple forms of data and is useful for practicing the claimed methods. Software automating FlexTree has been developed. Alternatively, LARTree or LART can be used (Turnbull (2005) Classification Trees with Subset Analysis Selection by the Lasso, Stanford University). The name reflects binary trees, as in CART and FlexTree; the lasso, as has been noted; and the implementation of the lasso through what is termed LARS by Efron et al. (2004) Annals of Statistics 32:407-451 (2004). See, also, Huang et al.., Proc. Natl. Acad. Sci. USA. 101(29):10529-34 (2004). Other methods of analysis that can be used include logic regression. One method of logic regression Ruczinski, Journal of Computational and Graphical Statistics 12:475-512 (2003). Logic regression resembles CART in that its classifier can be displayed as a binary tree. It is different in that each node has Boolean statements about features that are more general than the simple "and" statements produced by CART.

Another approach is that of nearest shrunken centroids (Tibshirani, Proc. Natl. Acad. Sci. U.S.A 99:6567-72(2002)). The technology is k-means-like, but has the advantage that by shrinking cluster centers, one automatically selects features, as is the case in the lasso, to focus attention on small numbers of those that are informative. The approach is available as PAM software and is widely used. Two further sets of algorithms that can be used are random forests (Breiman, Machine Learning 45:5-32 (2001)) and MART (Hastie, The Elements of Statistical Learning, Springer (2001)). These two methods are known in the art as "committee methods," that involve predictors that "vote" on outcome.

To provide significance ordering, the false discovery rate (FDR) can be determined. First, a set of null distributions of dissimilarity values is generated. In one embodiment, the values of observed profiles are permuted to create a sequence of distributions of correlation coefficients obtained out of chance, thereby creating an appropriate set of null distributions of correlation coefficients (Tusher et al., Proc. Natl. Acad. Sci. U.S.A 98, 5116-21 (2001)). The set of null distribution is obtained by: permuting the values of each profile for all available profiles; calculating the pair-wise correlation coefficients for all profile; calculating the probability density function of the correlation coefficients for this permutation; and repeating the procedure for N times, where N is a large number, usually 300. Using the N distributions, one calculates an appropriate measure (mean, median, *etc*.) of the count of correlation coefficient values that their values exceed the value (of similarity) that is obtained from the distribution of experimentally observed similarity values at given significance level.

The FDR is the ratio of the number of the expected falsely significant correlations (estimated from the correlations greater than this selected Pearson correlation in the set of randomized data) to the number of correlations greater than this selected Pearson correlation in the empirical data (significant correlations). This cut-off correlation value can be applied to the correlations between experimental profiles. Using the aforementioned distribution, a level of confidence is chosen for significance. This is used to determine the lowest value of the correlation coefficient that exceeds the result that would have obtained by chance. Using this method, one obtains thresholds for positive correlation, negative correlation or both. Using this threshold(s), the user can filter the observed values of the pair wise correlation coefficients and eliminate those that do not exceed the threshold(s). Furthermore, an estimate of the false positive rate can be obtained for a given threshold. For each of the individual "random correlation" distributions, one can find how many observations fall outside the threshold range. This procedure provides a sequence of counts. The mean and the standard deviation of the sequence provide the average number of potential false positives and its standard deviation.

In an alternative analytical approach, variables chosen in the cross-sectional analysis are separately employed as predictors in a time-to-event analysis (survival analysis), where the event is the occurrence of preterm birth, and subjects with no event are considered censored at the time of giving birth. Given the specific pregnancy outcome (preterm birth event or no event), the random lengths of time each patient will be observed, and selection of proteomic and other features, a parametric approach to analyzing survival can be better than the widely applied semi-parametric Cox model. A Weibull parametric fit of survival permits the hazard rate to be monotonically increasing, decreasing, or constant, and also has a proportional hazards representation (as does the Cox model) and an accelerated failure-time representation. All the standard tools available in obtaining approximate maximum likelihood estimators of regression coefficients and corresponding functions are available with this model.

In addition the Cox models can be used, especially since reductions of numbers of covariates to manageable size with the lasso will significantly simplify the analysis, allowing the possibility of a nonparametric or semi-parametric approach to prediction of time to preterm birth. These statistical tools are known in the art and applicable to all manner of proteomic data. A set of biomarker, clinical and genetic data that can be easily determined, and that is highly informative regarding the probability for preterm birth and predicted time to a preterm birth event in said pregnant female is provided. Also, algorithms provide information regarding the probability for preterm birth in the pregnant female.

Accordingly, one skilled in the art understands that the probability for preterm birth according to the invention can be determined using either a quantitative or a categorical variable. For example, in practicing the methods of the invention the measurable feature of each of N biomarkers can be subjected to categorical data analysis to determine the probability for preterm birth as a binary categorical outcome. Alternatively, the methods of the invention may analyze the measurable feature of each of N biomarkers by initially calculating quantitative variables, in particular, predicted gestational age at birth. The predicted gestational age at birth can subsequently be used as a basis to predict risk of preterm birth. By initially using a quantitative variable and subsequently converting the quantitative variable into a categorical variable the methods of the invention take into account the continuum of measurements detected for the measurable features. For example, by predicting the gestational age at birth rather than making a binary prediction of preterm birth versus term birth, it is possible to tailor the treatment for the pregnant female. For example, an earlier predicted gestational age at birth will result in more intensive prenatal intervention, *i.e.* monitoring and treatment, than a predicted gestational age that approaches full term.

Among women with a predicted GAB of j days plus or minus k days, p(PTB) can estimated as the proportion of women in the PAPR clinical trial (*see* Example 1) with a predicted GAB of j days plus or minus k days who actually deliver before 37 weeks gestational age. More generally, for women with a predicted GAB of j days plus or minus k days, the probability that the actual gestational age at birth will be less than a specified gestational age, p(actual GAB < specified GAB), was estimated as the proportion of women in the PAPR clinical trial with a predicted GAB of j days plus or minus k days who actually deliver before the specified gestational age.

In the development of a predictive model, it can be desirable to select a subset of markers, i.e. at least 3, at least 4, at least 5, at least 6, up to the complete set of markers. Usually a subset of markers will be chosen that provides for the needs of the quantitative sample analysis, e.g. availability of reagents, convenience of quantitation, etc., while maintaining a highly accurate predictive model. The selection of a number of informative markers for building classification models requires the definition of a performance metric and a user-defined threshold for producing a model with useful predictive ability based on this metric. For example, the performance metric can be the AUC, the sensitivity and/or specificity of the prediction as well as the overall accuracy of the prediction model.

As will be understood by those skilled in the art, an analytic classification process can use any one of a variety of statistical analytic methods to manipulate the quantitative data and provide for classification of the sample. Examples of useful methods include, without limitation, linear discriminant analysis, recursive feature elimination, a prediction analysis of microarray, a logistic regression, a CART algorithm, a FlexTree algorithm, a LART algorithm, a random forest algorithm, a MART algorithm, and machine learning algorithms.

As described in Example 2, various methods are used in a training model. The selection of a subset of markers can be for a forward selection or a backward selection of a marker subset. The number of markers can be selected that will optimize the performance of a model without the use of all the markers. One way to define the optimum number of terms is to choose the number of terms that produce a model with desired predictive ability (*e.g.* an AUC>0.75, or equivalent measures of sensitivity/specificity) that lies no more than one standard error from the maximum value obtained for this metric using any combination and number of terms used for the given algorithm.

**Table 1. Transitions with p-values less than 0.05 in univariate Cox Proportional Hazards analyses to predict Gestational Age at Birth**

| **Transition** | **Protein** | **p-value Cox univariate** |
|---|---|---|
| ITLPDFTGDLR 624.34_920.4 | LBP_HUMAN | 0.006 |
| ELLESYIDGR_ 597.8_710.3 | THRB_HUMAN | 0.006 |
| TDAPDLPEENQAR_ 728.34_613.3 | CO5_HUMAN | 0.007 |
| AFTECCVVASQLR_ 770.87_574.3 | CO5_HUMAN | 0.009 |
| SFRPFVPR_335.86_272.2 | LBP_HUMAN | 0.011 |
| ITLPDFTGDLR_ 624.34_288.2 | LBP_HUMAN | 0.012 |
| SFRPFVPR_335.86_635.3 | LBP_HUMAN | 0.015 |
| ELLESYIDGR 597.8_839.4 | THRB_HUMAN | 0.018 |
| LEQGENVFLQATDK_ 796.4_822.4 | C1QB_HUMAN | 0.019 |
| ETAASLLQAGYK_626.33_679.4 | THRB_HUMAN | 0.021 |
| VTGWGNLK_437.74_617.3 | THRB_HUMAN | 0.021 |
| EAQLPVIENK_ 570.82_699.4 | PLMN_HUMAN | 0.023 |
| EAQLPVIENK_ 570.82_329.1 | PLMN_HUMAN | 0.023 |
| FLQEQGHR_ 338.84_497.3 | CO8G_HUMAN | 0.025 |
| IRPFFPQQ_516.79_661.4 | FIBB_HUMAN | 0.028 |
| ETAASLLQAGYK 626.33 - 879.5 | THRB_HUMAN | 0.029 |
| AFTECCVVASQLR770.87_673.4 | CO5_HUMAN | 0.030 |
| TLLPVSKPEIR_ 418.26_288.2 | CO5_HUMAN | 0.030 |
| LSSPAVITDK_ 515.79_743.4 | PLMN_HUMAN | 0.033 |
| YEVQGEVFTKPQLWP_ 910.96_392.2 | CRP_HUMAN | 0.036 |
| LQGTLPVEAR_ 542.31_571.3 | CO5_HUMAN | 0.036 |
| VRPQQLVK_ 484.31_609.3 | ITIH4_HUMAN | 0.036 |
| IEEIAAK_ 387.22_531.3 | CO5_HUMAN | 0.041 |
| TLLPVSKPEIR_418.26_514.3 | CO5_HUMAN | 0.042 |
| VQEAHLTEDQIFYFPK_ 655.66_701.4 | CO8G_HUMAN | 0.047 |
| ISLLLIESWLEPVR_ 834.49_371.2 | CSH_HUMAN | 0.048 |
| ALQDQLVLVAAK_ 634.88_289.2 | ANGT_HUMAN | 0.048 |
| YEFLNGR_ 449.72_293.1 | PLMN_HUMAN | 0.049 |

**Table 2. Transitions selected by the Cox stepwise AIC analysis**

| **Transition** | **coef** | **exp(coef)** | **se(coef)** | **z** | **Pr(>\|z\|)** |
|---|---|---|---|---|---|
| Collection.Window.GA.in.Day s | 1.28E-01 | 1.14E+00 | 2.44E-02 | 5.26 | 1.40E-07 |
| ITLPDFTGDLR 624.34_920. 4 | 2.02E+00 | 7.52E+00 | 1.14E+00 | 1.77 | 0.07667 |
| TPSAAYLWVGTGASEAEK _919.45_849.4 | 2.85E+01 | 2.44E+12 | 3.06E+00 | 9.31 | <2e-16 |
| TATSEYQTFFNPR_781.37_3 86.2 | 5.14E+00 | 1.70E+02 | 6.26E-01 | 8.21 | 2.20E-16 |
| TASDFITK_ 441.73_781.4 | -1.25E+00 | 2.86E-01 | 1.58E+00 | -0.79 | 0.42856 |
| IITGLLEFEVYLEYLQNR_ 7 38.4_530.3 | 1.30E+01 | 4.49E+05 | 1.45E+00 | 9 | <2e-16 |
| IIGGSDADIK_ 494.77_762.4 | -6.43E+01 | 1.16E-28 | 6.64E+00 | -9.68 | <2e-16 |
| YTTEIIK 434.25_603.4 | 6.96E+01 | 1.75E+30 | 7.06E+00 | 9.86 | <2e-16 |
| EDTPNSVWEPAK_ 686.82_3 15.2 | 7.91E+00 | 2.73E+03 | 2.66E+00 | 2.98 | 0.00293 |
| LYYGDDEK_ 501.72_726.3 | 8.74E+00 | 6.23E+03 | 1.57E+00 | 5.57 | 2.50E-08 |
| VRPQQLVK_484.31_609.3 | 4.64E+01 | 1.36E+20 | 3.97E+00 | 11.66 | < 2e-16 |
| GGEIEGFR_432.71_379.2 | -3.33E+00 | 3.57E-02 | 2.19E+00 | -1.52 | 0.12792 |
| DGSPDVTTADIGANTPDAT K_973.45_844.4 | -1.52E+01 | 2.51E-07 | 1.41E+00 | -10.8 | < 2e-16 |
| VQEAHLTEDQIFYFPK_655. 66_391.2 | -2.02E+01 | 1.77E-09 | 2.45E+00 | -8.22 | 2.20E-16 |
| VEIDTK_ 352.7_476.3 | 7.06E+00 | 1.17E+03 | 1.45E+00 | 4.86 | 1.20E-06 |
| AVLTIDEK_ 444.76_605.3 | 7.85E+00 | 2.56E+03 | 9.46E-01 | 8.29 | < 2e-16 |
| FSVVYAK_407.23_579.4 | -2.44E+01 | 2.42E-11 | 3.08E+00 | -7.93 | 2.20E-15 |
| YYLQGAK_ 421.72_516.3 | -1.82E+01 | 1.22E-08 | 2.45E+00 | -7.44 | 1.00E-13 |
| EENFYVDETTVVK_ 786.88_ 259.1 | -1.90E+01 | 5.36E-09 | 2.71E+00 | -7.03 | 2.00E-12 |
| YGFYTHVFR 397.2_421.3 | 1.90E+01 | 1.71E+08 | 2.73E+00 | 6.93 | 4.20E-12 |
| HTLNQIDEVK_ 598.82_951.5 | 1.03E+01 | 3.04E+04 | 2.11E+00 | 4.89 | 9.90E-07 |
| AFIQLWAFDAVK_704.89_8 36.4 | 1.08E+01 | 4.72E+04 | 2.59E+00 | 4.16 | 3.20E-05 |
| SGFSFGFK_438.72_585.3 | 1.35E+01 | 7.32E+05 | 2.56E+00 | 5.27 | 1.40E-07 |
| GWVTDGFSSLK_598.8_854. 4 | -3.12E+00 | 4.42E-02 | 9.16E-01 | -3.4 | 0.00066 |
| ITENDIQIALDDAK_779.9_6 32.3 | 1.91E+00 | 6.78E+00 | 1.36E+00 | 1.4 | 0.16036 |

**Table 3. Transitions selected by Cox lasso model**

| Transition | coef | exp(coef) | se(coef) | z | Pr(>\|z\|) |
|---|---|---|---|---|---|
| Collection.Window.GA.in.Days | 0.0233 | 1.02357 | 0.00928 | 2.51 | 0.012 |
| AFTECCVVASQLR_770.87_574.3 | 1.07568 | 2.93198 | 0.84554 | 1.27 | 0.203 |
| ELLESYIDGR_ 597.8_710.3 | 1.3847 | 3.99365 | 0.70784 | 1.96 | 0.05 |
| ITLPDFTGDLR_ 624.34_920.4 | 0.814 | 2.25691 | 0.40652 | 2 | 0.045 |

**Table 4. Area under the ROC (AUROC) curve for individual analytes to discriminate pre-term birth subjects from non-pre-term birth subjects. The 77 transitions with the highest AUROC area are shown.**

| **Transition** | **AUROC** |
|---|---|
| ELLESYIDGR_ 597.8_710.3 | 0.71 |
| AFTECCVVASQLR770.87_574.3 | 0.70 |
| ITLPDFTGDLR 624.34_920.4 | 0.70 |
| IRPFFPQQ_516.79_661.4 | 0.68 |
| TDAPDLPEENQAR_ 728.34_613.3 | 0.67 |
| ITLPDFTGDLR_ 624.34_288.2 | 0.67 |
| ELLESYIDGR_ 597.8_839.4 | 0.67 |
| SFRPFVPR_335.86_635.3 | 0.67 |
| ETAASLLQAGYK_ 626.33 - 879.5 | 0.67 |
| TLLPVSKPEIR_ 418.26_288.2 | 0.66 |
| ETAASLLQAGYK_626.33_679.4 | 0.66 |
| SFRPFVPR 335.86_272.2 | 0.66 |
| LQGTLPVEAR_ 542.31_571.3 | 0.66 |
| VEPLYELVTATDFAYSSTVR_ 754.38_712.4 | 0.66 |
| DPDQTDGLGLSYLSSHIANVER 796.39_328.1 | 0.66 |
| VTGWGNLK_437.74_617.3 | 0.65 |
| ALQDQLVLVAAK_ 634.88_289.2 | 0.65 |
| EAQLPVIENK_ 570.82_329.1 | 0.65 |
| VRPQQLVK_ 484.31_609.3 | 0.65 |
| AFTECCVVASQLR770.87_673.4 | 0.65 |
| YEFLNGR_ 449.72_293.1 | 0.65 |
| VGEYSLYIGR_ 578.8_871.5 | 0.64 |
| EAQLPVIENK_ 570.82_699.4 | 0.64 |
| TLLPVSKPEIR_ 418.26_514.3 | 0.64 |
| IEEIAAK_387.22_531.3 | 0.64 |
| LEQGENVFLQATDK_ 796.4_822.4 | 0.64 |
| LQGTLPVEAR_ 542.31_842.5 | 0.64 |
| FLQEQGHR_ 338.84_497.3 | 0.63 |
| ISLLLIESWLEPVR_ 834.49_371.2 | 0.63 |
| IITGLLEFEVYLEYLQNR_738.4_530.3 | 0.63 |
| LSSPAVITDK_ 515.79_743.4 | 0.63 |
| VRPQQLVK_ 484.31_722.4 | 0.63 |
| SLPVSDSVLSGFEQR_810.92_723.3 | 0.63 |
| VQEAHLTEDQIFYFPK_ 655.66_701.4 | 0.63 |
| NADYSYSVWK_ 616.78_333.2 | 0.63 |
| DAQYAPGYDK_564.25_813.4 | 0.62 |
| FQLPGQK_ 409.23_276.1 | 0.62 |
| TASDFITK_ 441.73_781.4 | 0.62 |
| YGLVTYATYPK_ 638.33_334.2 | 0.62 |
| GSFALSFPVESDVAPIAR_931.99_363.2 | 0.62 |
| TLLIANETLR_ 572.34_703.4 | 0.62 |
| VILGAHQEVNLEPHVQEIEVSR_832.78_860.4 | 0.62 |
| TATSEYQTFFNPR_ 781.37_386.2 | 0.62 |
| YEVQGEVFTKPQLWP_ 910.96_392.2 | 0.62 |
| DISEVVTPR_ 508.27_472.3 | 0.62 |
| GSFALSFPVESDVAPIAR_931.99_456.3 | 0.62 |
| YGFYTHVFR_ 397.2_421.3 | 0.62 |
| TLEAQLTPR_ 514.79_685.4 | 0.62 |
| YGFYTHVFR_ 397.2_659.4 | 0.62 |
| AVGYLITGYQR_ 620.84_737.4 | 0.61 |
| DPDQTDGLGLSYLSSHIANVER_ 796.39_456.2 | 0.61 |
| FNAVLTNPQGDYDTSTGK_ 964.46_262.1 | 0.61 |
| SPEQQETVLDGNLIIR_906.48_685.4 | 0.61 |
| ALNHLPLEYNSALYSR_ 620.99_538.3 | 0.61 |
| GGEIEGFR_432.71_508.3 | 0.61 |
| GIVEECCFR_585.26_900.3 | 0.61 |
| DAQYAPGYDK_564.25_315.1 | 0.61 |
| FAFNLYR_465.75_712.4 | 0.61 |
| YTTEIIK_ 434.25_603.4 | 0.61 |
| AVLTIDEK_ 444.76_605.3 | 0.61 |
| AITPPHPASQANIIFDITEGNLR_825.77_459.3 | 0.60 |
| EPGLCTWQSLR_673.83_790.4 | 0.60 |
| AVYEAVLR_ 460.76_587.4 | 0.60 |
| ALQDQLVLVAAK_634.88_956.6 | 0.60 |
| AWVAWR_ 394.71_531.3 | 0.60 |
| TNLESILSYPK_ 632.84_807.5 | 0.60 |
| HLSLLTTLSNR_ 418.91_376.2 | 0.60 |
| FTFTLHLETPKPSISSSNLNPR_ 829.44_787.4 | 0.60 |
| AVGYLITGYQR_ 620.84_523.3 | 0.60 |
| FQLPGQK_ 409.23_429.2 | 0.60 |
| YGLVTYATYPK_ 638.33_843.4 | 0.60 |
| TELRPGETLNVNFLLR_ 624.68_662.4 | 0.60 |
| LSSPAVITDK_ 515.79_830.5 | 0.60 |
| TATSEYQTFFNPR_ 781.37_272.2 | 0.60 |
| LPTAVVPLR_ 483.31_385.3 | 0.60 |
| APLTKPLK_ 289.86_260.2 | 0.60 |

**Table 5. AUROCs for random forest, boosting, lasso, and logistic regression models for a specific number of transitions permitted in the model, as estimated by 100 rounds of bootstrap resampling.**

| Number of transitions | rf | boosting | logit | lasso |
|---|---|---|---|---|
| 1 | 0.59 | 0.67 | 0.64 | 0.69 |
| 2 | 0.66 | 0.70 | 0.63 | 0.68 |
| 3 | 0.69 | 0.70 | 0.58 | 0.71 |
| 4 | 0.68 | 0.72 | 0.58 | 0.71 |
| 5 | 0.73 | 0.71 | 0.58 | 0.68 |
| 6 | 0.72 | 0.72 | 0.56 | 0.68 |
| 7 | 0.74 | 0.70 | 0.60 | 0.67 |
| 8 | 0.73 | 0.72 | 0.62 | 0.67 |
| 9 | 0.72 | 0.72 | 0.60 | 0.67 |
| 10 | 0.74 | 0.71 | 0.62 | 0.66 |
| 11 | 0.73 | 0.69 | 0.58 | 0.67 |
| 12 | 0.73 | 0.69 | 0.59 | 0.66 |
| 13 | 0.74 | 0.71 | 0.57 | 0.66 |
| 14 | 0.73 | 0.70 | 0.57 | 0.65 |
| 15 | 0.72 | 0.70 | 0.55 | 0.64 |

**Table 6. Top 15 transitions selected by each multivariate method, ranked by importance for that method.**

| | rf | boosting | lasso | logit |
|---|---|---|---|---|
| 1 | ELLESYIDGR 597.8 710.3 | AFTECCVVASQL R 770.87 574.3 | AFTECCVVASQLR 770.87 574.3 | ALQDQLVLVAAK_ 634.88 289.2 |
| 2 | TATSEYQTFF NPR_781.37_38 6.2 | DPDQTDGLGLSY LSSHIANVER_796 .39 328.1 | ISLLLIESWLEPVR_ 834.49_371.2 | AVLTIDEK_ 444.76_ 605.3 |
| 3 | ITLPDFTGDLR 624.34_920.4 | ELLESYIDGR 597 .8 710.3 | LPTAVVPLR_ 483.31 385.3 | Collection.Window.G A.in.Days |
| 4 | AFTECCVVAS QLR_770.87_57 4.3 | TATSEYQTFFNPR _781.37_386.2 | ALQDQLVLVAAK_ 634.88_289.2 | AHYDLR_ 387.7_566 .3 |
| 5 | VEPLYELVTA TDFAYSSTVR 754.38 712.4 | ITLPDFTGDLR_62 4.34_920.4 | ETAASLLQAGYK_ 626.33_679.4 | AEAQAQYSAAVA K_ 654.33_908.5 |
| 6 | GSFALSFPVES DVAPIAR_931. 99_363.2 | GGEIEGFR_432.71 _379.2 | IITGLLEFEVYLEYL QNR_738.4_530.3 | AEAQAQYSAAVA K_ 654.33_709.4 |
| 7 | VGEYSLYIGR_ 578.8_871.5 | ALQDQLVLVAAK _634.88_289.2 | ADSQAQLLLSTVV GVFTAPGLHLK_82 2.46 983.6 | ADSQAQLLLSTVV GVFTAPGLHLK_82 2.46 983.6 |
| 8 | SFRPFVPR_335 .86_635.3 | VGEYSLYIGR_57 8.8_871.5 | SLPVSDSVLSGFEQ R_810.92_723.3 | AITPPHPASQANIIF DITEGNLR_ 825.77_ 459.3 |
| 9 | ALQDQLVLVA AK 634.88_289 .2 | VEPLYELVTATD FAYSSTVR 754.3 8 712.4 | SFRPFVPR_335.86_ 272.2 | ADSQAQLLLSTVV GVFTAPGLHLK_82 2.46 664.4 |
| 10 | EDTPNSVWEP AK_686.82_315 .2 | SPEQQETVLDGN LIIR_906.48_685.4 | IIGGSDADIK_494.7 7_260.2 | AYSDLSR_ 406.2_37 5.2 |
| 11 | YGFYTHVFR 397.2_421.3 | YEFLNGR 449.72 293.1 | NADYSYSVWK 61 6.78 333.2 | DALSSVQESQVAQ QAR 572.96 672.4 |
| 12 | DPDQTDGLGL SYLSSHIANVE R_ 796.39 328.1 | LEQGENVFLQAT DK_796.4_822.4 | GSFALSFPVESDVA PIAR_ 931.99_456.3 | ANRPFLVFIR_411.5 8 435.3 |
| 13 | LEQGENVFLQ ATDK_ 796.4_8 22.4 | LQGTLPVEAR_54 2.31_571.3 | LSSPAVITDK 515.7 9_743.4 | DALSSVQESQVAQ QAR_572.96_502.3 |
| 14 | LQGTLPVEAR _542.31_571.3 | ISLLLIESWLEPVR _834.49_371.2 | ELPEHTVK_ 476.76_ 347.2 | ALEQDLPVNIK_62 0.35 570.4 |
| 15 | SFRPFVPR_335 .86 272.2 | TASDFITK 441.73 781.4 | EAQLPVIENK_ 570. 82 699.4 | AVLTIDEK_ 444.76_ 718.4 |

Disclosed herein are kits for determining probability of preterm birth, wherein the kits can be used to detect N of the isolated biomarkers listed in Tables 1 through 63. For example, the kits can be used to detect one or more, two or more, or three of the isolated biomarkers selected from the group consisting of AFTECCVVASQLR, ELLESYIDGR, and ITLPDFTGDLR. For example, the kits can be used to detect one or more, two or more, or three of the isolated biomarkers selected from the group consisting of FLNWIK, FGFGGSTDSGPIR, LLELTGPK, VEHSDLSFSK, IEGNLIFDPNNYLPK, ALVLELAK, TQILEWAAER, DVLLLVHNLPQNLPGYFWYK, SEPRPGVLLR, ITQDAQLK, ALDLSLK, WWGGQPLWITATK, and LSETNR.

The kits can be used to detect one or more, two or more, three or more, four or more, five or more, six or more, seven or more, or eight of the isolated biomarkers selected from the group consisting of lipopolysaccharide-binding protein (LBP), prothrombin (THRB), complement component C5 (C5 or CO5), plasminogen (PLMN), and complement component C8 gamma chain (C8G or CO8G).

The kits can be used to detect one or more, two or more, three or more, four or more, five or more, six or more, seven or more, or eight of the isolated biomarkers selected from the group consisting of Alpha-1B-glycoprotein (A1BG), Disintegrin and metalloproteinase domain-containing protein 12 (ADA12), Apolipoprotein B-100 (APOB), Beta-2-microglobulin (B2MG), CCAAT/enhancer-binding protein alpha/beta (HP8 Peptide), Corticosteroid-binding globulin (CBG), Complement component C6, Endoglin (EGLN), Ectonucleotide pyrophosphatase/phosphodiesterase family member 2 (ENPP2), Coagulation factor VII (FA7), Hyaluronan-binding protein 2 (HABP2), Pregnancy-specific beta-1-glycoprotein 9 (PSG9), Inhibin beta E chain (INHBE).

The kit can include one or more agents for detection of biomarkers, a container for holding a biological sample isolated from a pregnant female; and printed instructions for reacting agents with the biological sample or a portion of the biological sample to detect the presence or amount of the isolated biomarkers in the biological sample. The agents can be packaged in separate containers. The kit can further comprise one or more control reference samples and reagents for performing an immunoassay.

In one instances, the kit comprises agents for measuring the levels of at least N of the isolated biomarkers listed in Tables 1 through 63. The kit can include antibodies that specifically bind to these biomarkers, for example, the kit can contain at least one of an antibody that specifically binds to lipopolysaccharide-binding protein (LBP), an antibody that specifically binds to prothrombin (THRB), an antibody that specifically binds to complement component C5 (C5 or CO5), an antibody that specifically binds to plasminogen (PLMN), and an antibody that specifically binds to complement component C8 gamma chain (C8G or CO8G).

In one instance, the kit comprises agents for measuring the levels of at least N of the isolated biomarkers listed in Tables 1 through 63. The kit can include antibodies that specifically bind to these biomarkers, for example, the kit can contain at least one of an antibody that specifically binds to Alpha-1B-glycoprotein (A1BG), Disintegrin and metalloproteinase domain-containing protein 12 (ADA12), Apolipoprotein B-100 (APOB), Beta-2-microglobulin (B2MG), CCAAT/enhancer-binding protein alpha/beta (HP8 Peptide), Corticosteroid-binding globulin (CBG), Complement component C6, Endoglin (EGLN), Ectonucleotide pyrophosphatase/phosphodiesterase family member 2 (ENPP2), Coagulation factor VII (FA7), Hyaluronan-binding protein 2 (HABP2), Pregnancy-specific beta-1-glycoprotein 9 (PSG9), Inhibin beta E chain (INHBE).

The kit can comprise one or more containers for compositions contained in the kit. Compositions can be in liquid form or can be lyophilized. Suitable containers for the compositions include, for example, bottles, vials, syringes, and test tubes. Containers can be formed from a variety of materials, including glass or plastic. The kit can also comprise a package insert containing written instructions for methods of determining probability of preterm birth.

The following examples are provided by way of illustration, not limitation.

### EXAMPLES

### Example 1. Development of Sample Set for Discovery and Validation of Biomarkers for Preterm Birth

A standard protocol was developed governing conduct of the Proteomic Assessment of Preterm Risk (PAPR) clinical study. This protocol also specified that the samples and clinical information could be used to study other pregnancy complications for some of the subjects. Specimens were obtained from women at 11 Internal Review Board (IRB) approved sites across the United States. After providing informed consent, serum and plasma samples were obtained, as well as pertinent information regarding the patient's demographic characteristics, past medical and pregnancy history, current pregnancy history and concurrent medications. Following delivery, data were collected relating to maternal and infant conditions and complications. Serum and plasma samples were processed according to a protocol that requires standardized refrigerated centrifugation, aliquoting of the samples into 0.5 ml 2-D bar-coded cryovials and subsequent freezing at -80°C.

Following delivery, preterm birth cases were individually reviewed to determine their status as either a spontaneous preterm birth or a medically indicated preterm birth. Only spontaneous preterm birth cases were used for this analysis. For discovery of biomarkers of preterm birth, 80 samples were analyzed in two gestational age groups: a) a late window composed of samples from 23-28 weeks of gestation which included 13 cases, 13 term controls matched within one week of sample collection and 14 term random controls, and, b) an early window composed of samples from 17-22 weeks of gestation included 15 cases, 15 term controls matched within one week of sample collection and 10 random term controls.

The samples were subsequently depleted of high abundance proteins using the Human 14 Multiple Affinity Removal System (MARS 14), which removes 14 of the most abundant proteins that are treated as uninformative with regard to the identification for disease-relevant changes in the serum proteome. To this end, equal volumes of each clinical or a pooled human serum sample (HGS) sample were diluted with column buffer and filtered to remove precipitates. Filtered samples were depleted using a MARS-14 column (4.6 x 100 mm, Cat. #5188-6558, Agilent Technologies). Samples were chilled to 4°C in the autosampler, the depletion column was run at room temperature, and collected fractions were kept at 4°C until further analysis. The unbound fractions were collected for further analysis.

A second aliquot of each clinical serum sample and of each HGS was diluted into ammonium bicarbonate buffer and depleted of the 14 high and approximately 60 additional moderately abundant proteins using an IgY14-SuperMix (Sigma) hand-packed column, comprised of 10 mL of bulk material (50% slurry, Sigma). Shi et al., Methods, 56(2):246-53 (2012). Samples were chilled to 4°C in the autosampler, the depletion column was run at room temperature, and collected fractions were kept at 4°C until further analysis. The unbound fractions were collected for further analysis.

Depleted serum samples were denatured with trifluorethanol, reduced with dithiotreitol, alkylated using iodoacetamide, and then digested with trypsin at a 1:10 trypsin: protein ratio. Following trypsin digestion, samples were desalted on a C18 column, and the eluate lyophilized to dryness. The desalted samples were resolubilized in a reconstitution solution containing five internal standard peptides.

Depleted and trypsin digested samples were analyzed using a scheduled Multiple Reaction Monitoring method (sMRM). The peptides were separated on a 150 mm x 0.32 mm Bio-Basic C18 column (ThermoFisher) at a flow rate of 5 µl/min using a Waters Nano Acquity UPLC and eluted using an acetonitrile gradient into a AB SCIEX QTRAP 5500 with a Turbo V source (AB SCIEX, Framingham, MA). The sMRM assay measured 1708 transitions that correspond to 854 peptides and 236 proteins. Chromatographic peaks were integrated using Rosetta Elucidator software (Ceiba Solutions).

Transitions were excluded from analysis, if their intensity area counts were less than 10000 and if they were missing in more than three samples per batch. Intensity area counts were log transformed and Mass Spectrometry run order trends and depletion batch effects were minimized using a regression analysis.

### Example 2. Analysis I of Transitions to Identify Preterm Birth Biomarkers

The objective of these analyses was to examine the data collected in Example 1 to identify transitions and proteins that predict preterm birth. The specific analyses employed were (i) Cox time-to-event analyses and (ii) models with preterm birth as a binary categorical dependent variable. The dependent variable for all the Cox analyses was Gestational Age of time to event (where event is preterm birth). For the purpose of the Cox analyses, preterm birth subjects have the event on the day of birth. Term subjects are censored on the day of birth. Gestational age on the day of specimen collection is a covariate in all Cox analyses.

The assay data were previously adjusted for run order and depletion batch, and log transformed. Values for gestational age at time of sample collection were adjusted as follows. Transition values were regressed on gestational age at time of sample collection using only controls (non-pre-term subjects). The residuals from the regression were designated as adjusted values. The adjusted values were used in the models with pre-term birth as a binary categorical dependent variable. Unadjusted values were used in the Cox analyses.

### Univariate Cox Proportional Hazards Analyses

Univariate Cox Proportional Hazards analyses was performed to predict Gestational Age at Birth, including Gestational age on the day of specimen collection as a covariate. Table 1 shows the transitions with p-values less than 0.05. Five proteins have multiple transitions among those with p-value less than 0.05: lipopolysaccharide-binding protein (LBP), prothrombin (THRB), complement component C5 (C5 or CO5), plasminogen (PLMN), and complement component C8 gamma chain (C8G or CO8G).

### Multivariate Cox Proportional Hazards Analyses: Stepwise AIC selection

Cox Proportional Hazards analyses was performed to predict Gestational Age at Birth, including Gestational age on the day of specimen collection as a covariate, using stepwise and lasso models for variable selection. These analyses include a total of n= 80 subjects, with number of PTB events= 28. The stepwise variable selection analysis used the Akaike Information Criterion (AIC) as the stopping criterion. Table 2 shows the transitions selected by the stepwise AIC analysis. The coefficient of determination (R²) for the stepwise AIC model is 0.86 (not corrected for multiple comparisons).

### Multivariate Cox Proportional Hazards Analyses: lasso selection

Lasso variable selection was used as the second method of multivariate Cox Proportional Hazards analyses to predict Gestational Age at Birth, including Gestational age on the day of specimen collection as a covariate. This analysis uses a lambda penalty for lasso estimated by cross validation. Table 3 shows the results. The lasso variable selection method is considerably more stringent than the stepwise AIC, and selects only 3 transitions for the final model, representing 3 different proteins. These 3 proteins give the top 4 transitions from the univariate analysis; 2 of the top 4 univariate are from the same protein, and hence are not both selected by the lasso method. Lasso tends to select a relatively small number of variables with low mutual correlation. The coefficient of determination (R²) for the lasso model is 0.21 (not corrected for multiple comparisons).

### Univariate AUROC analysis of preterm birth as a binary categorical dependent variable

Univariate analyses was performed to discriminate pre-term subjects from non-pre-term subjects (pre-term as a binary categorical variable) as estimated by area under the receiver operating characteristic (AUROC) curve. These analyses use transition values adjusted for gestational age at time of sample collection, as described above. Table 4 shows the AUROC curve for the 77 transitions with the highest AUROC area of 0.6 or greater.

### Multivariate analysis of preterm birth as a binary categorical dependent variable

Multivariate analyses was performed to predict preterm birth as a binary categorical dependent variable, using random forest, boosting, lasso, and logistic regression models. Random forest and boosting models grow many classification trees. The trees vote on the assignment of each subject to one of the possible classes. The forest chooses the class with the most votes over all the trees.

For each of the four methods (random forest, boosting, lasso, and logistic regression) each method was allowed to select and rank its own best 15 transitions. We then built models with 1 to 15 transitions. Each method sequentially reduces the number of nodes from 15 to 1 independently. A recursive option was used to reduce the number of nodes at each step: To determine which node to remove, the nodes were ranked at each step based on their importance from a nested cross-validation procedure. The least important node was eliminated. The importance measures for lasso and logistic regression are z-values. For random forest and boosting, the variable importance was calculated from permuting out-of-bag data: for each tree, the classification error rate on the out-of-bag portion of the data was recorded; the error rate was then recalculated after permuting the values of each variable (i.e., transition); if the transition was in fact important, there would have been be a big difference between the two error rates; the difference between the two error rates were then averaged over all trees, and normalized by the standard deviation of the differences. The AUCs for these models are shown in Table 5, as estimated by 100 rounds of bootstrap resampling. Table 6 shows the top 15 transitions selected by each multivariate method, ranked by importance for that method. These multivariate analyses suggest that models that combine 3 or more transitions give AUC greater than 0.7, as estimated by bootstrap.

In multivariate models, random forest (rf), boosting, and lasso models gave the best area under the AUROC curve. The following transitions were selected by these models, as significant in Cox univariate models, and/or having high univariate ROC's:
AFTECCVVASQLR_770.87_574.3
ELLESYIDGR_597.8_710.3
ITLPDFTGDLR_624.34_920.4
TDAPDLPEENQAR_728.34_613.3
SFRPFVPR_335.86_635.3

In summary, univariate and multivariate Cox analyses was performed using transitions to predict Gestational Age at Birth (GAB), including Gestational age on the day of specimen collection as a covariate. In the univariate Cox analysis, five proteins were identified that have multiple transitions among those with p-value less than 0.05: lipopolysaccharide-binding protein (LBP), prothrombin (THRB), complement component C5 (C5 or CO5), plasminogen (PLMN), and complement component C8 gamma chain (C8G or CO8G).

In multivariate Cox analyses, stepwise AIC variable analysis selects 24 transitions, while the lasso model selects 3 transitions, which include the 3 top proteins in the univariate analysis. Univariate (AUROC) and multivariate (random forest, boosting, lasso, and logistic regression) analyses were performed to predict pre-term birth as a binary categorical variable. Univariate analyses identified 63 analytes with AUROC of 0.6 or greater. Multivariate analyses suggest that models that combine 3 or more transitions give AUC greater than 0.7, as estimated by bootstrap.

### Example 3. Study II to Identify and Confirm Preterm Birth Biomarkers

A further study was performed using essentially the same methods described in the preceding Examples unless noted below. In this study, 2 gestational aged matched controls were used for each case of 28 cases and 56 matched controls, all from the early gestational window only (17-22 weeks).

The samples were processed in 4 batches with each batch composed of 7 cases, 14 matched controls and 3 HGS controls. Serum samples were depleted of the 14 most abundant serum samples by MARS14 as described in Example 1. Depleted serum was then reduced with dithiothreitol, alkylated with iodacetamide, and then digested with trypsin at a 1:20 trypsin to protein ratio overnight at 37°C. Following trypsin digestion, the samples were desalted on an Empore C18 96-well Solid Phase Extraction Plate (3M Company) and lyophilized to dryness. The desalted samples were resolubilized in a reconstitution solution containing five internal standard peptides .

The LC-MS/MS analysis was performed with an Agilent Poroshell 120 EC-C18 column (2.1x50mm, 2.7 µm) and eluted with an acetonitrile gradient into a Agilent 6490 Triple Quadrapole mass spectrometer.

Data analysis included the use of conditional logistic regression where each matching triplet (case and 2 matched controls) was a stratum. The p-value reported in the table indicates whether there is a significant difference between cases and matched controls.

**Table 7. Results of Study II**

| **Transition** | **Protein** | **Annotation** | **p-value** |
|---|---|---|---|
| DFHINLFQVLPWLK | CFAB_ HUMAN | Complement factor B | 0.006729512 |
| ITLPDFTGDLR | LBP_HUMAN | Lipopolysaccharide-binding protein | 0.012907017 |
| WWGGQPLWITATK | ENPP2 HUMAN | Ectonucleotide pyrophosphatase/phosp hodiesterase family member 2 | 0.013346 |
| TASDFITK | GELS_ HUMAN | Gelsolin | 0.013841221 |
| AGLLRPDYALLGHR | PGRP2 HUMAN | N-acetylmuramoyl-L-alanine amidase | 0.014241979 |
| FLQEQGHR | CO8G_ HUMAN | Complement component C8 gamma chain | 0.014339596 |
| FLNWIK | HABP2 HUMAN | Hyaluronan -binding protein 2 | 0.014790418 |
| EKPAGGIPVLGSLVNTVL K | BPIB1_ HUMAN | BPI fold-containing family B member 1 | 0.019027746 |
| ITGFLKPGK | LBP HUMAN | Lipopolysaccharide-binding protein | 0.019836986 |
| YGLVTYATYPK | CFAB_ HUMAN | Complement factor B | 0.019927774 |
| SLLQPNK | CO8A_ HUMAN | Complement component C8 alpha chain | 0.020930939 |
| DISEVVTPR | CFAB_ HUMAN | Complement factor B | 0.021738046 |
| VQEAHLTEDQIFYFPK | CO8G_ HUMAN | Complement component C8 gamma chain | 0.021924548 |
| SPELQAEAK | APOA2_HUMAN | Apolipoprotein A-II | 0.025944285 |
| TYLHTYESEI | ENPP2_HUMAN | Ectonucleotide pyrophosphatase/phosp hodiesterase family member 2 | 0.026150038 |
| DSPSVWAAVPGK | PROF1_ HUMAN | Profilin-1 | 0.026607371 |
| HYINLITR | NPY HUMAN | Pro-neuropeptide Y | 0.027432804 |
| SLPVSDSVLSGFEQR | CO8G_ HUMAN | Complement component C8 gamma chain | 0.029647857 |
| IPGIFELGISSQSDR | CO8B_ HUMAN | Complement component C8 beta chain | 0.030430996 |
| IQTHSTTYR | F13B HUMAN | Coagulation factor XIII B chain | 0.031667664 |
| DGSPDVTTADIGANTPDA TK | PGRP2_HUMAN | N-acetylmuramoyl-L-alanine amidase | 0.034738338 |
| QLGLPGPPDVPDHAAYHP F | ITIH4_HUMAN | Inter-alpha-trypsin inhibitor heavy chain H4 | 0.043130591 |
| FPLGSYTIQNIVAGSTYLF STK | LCAP_HUMAN | Leucyl-cystinyl aminopeptidase | 0.044698045 |
| AHYDLR | FETUA_HUMAN | Alpha-2-HS-glycoprotein | 0.046259201 |
| SFRPFVPR | LBP_HUMAN | Lipopolysaccharide-binding protein | 0.047948847 |

### Example 4. Study III Shotgun Identification of Preterm Birth Biomarkers

A further study used a hypothesis-independent shotgun approach to identify and quantify additional biomarkers not present on our multiplexed hypothesis dependent MRM assay. Samples were processed as described in the preceding Examples unless noted below.

Tryptic digests of MARS depleted patient (preterm birth cases and term controls) samples were fractionated by two-dimensional liquid chromatography and analyzed by tandem mass spectrometry. Aliquots of the samples, equivalent to 3-4 µl of serum, were injected onto a 6 cm x 75 µm self-packed strong cation exchange (Luna SCX, Phenomenex) column. Peptides were eluded from the SCX column with salt (15, 30, 50, 70, and 100% B, where B = 250mM ammonium acetate, 2% acetonitrile, 0.1% formic acid in water) and consecutively for each salt elution, were bound to a 0.5 µl C18 packed stem trap (Optimize Technologies, Inc.) and further fractionated on a 10 cm x 75 µm reversed phase ProteoPep II PicoFrit column (New Objective). Peptides were eluted from the reversed phase column with an acetonitrile gradient containing 0.1% formic acid and directly ionized on an LTQ-Orbitrap (ThermoFisher). For each scan, peptide parent ion masses were obtained in the Orbitrap at 60K resolution and the top seven most abundant ions were fragmented in the LTQ to obtain peptide sequence information.

Parent and fragment ion data were used to search the Human RefSeq database using the Sequest (Eng et al., J. Am. Soc. Mass Spectrom 1994; 5:976-989) and X!Tandem (Craig and Beavis, Bioinformatics 2004; 20:1466-1467) algorithms. For Sequest, data was searched with a 20 ppm tolerance for the parent ion and 1 AMU for the fragment ion. Two missed trypsin cleavages were allowed, and modifications included static cysteine carboxyamidomethylation and methionine oxidation. After searching the data was filtered by charge state vs. Xcorr scores (charge +1 ≥ 1.5 Xcorr, charge +2 ≥ 2.0, charge +3 ≥ 2.5). Similar search parameters were used for X!tandem, except the mass tolerance for the fragment ion was 0.8 AMU and there is no Xcorr filtering. Instead, the PeptideProphet algorithm (Keller et al., Anal. Chem 2002;74:5383-5392) was used to validate each X!Tandem peptide-spectrum assignment and Protein assignments were validated using ProteinProphet algorithm (Nesvizhskii et al., Anal. Chem 2002; 74:5383-5392). Data was filtered to include only the peptide-spectrum matches that had PeptideProphet probability of 0.9 or more. After compiling peptide and protein identifications, spectral count data for each peptide were imported into DAnTE software (Polpitiya et al., Bioinformatics. 2008; 24:1556-1558). Log transformed data was mean centered and missing values were filtered, by requiring that a peptide had to be identified in at least 4 cases and 4 controls. To determine the significance of an analyte, Receiver Operating Characteristic (ROC) curves for each analyte were created where the true positive rate (Sensitivity) is plotted as a function of the false positive rate (1-Specificity) for different thresholds that separate the SPTB and Term groups. The area under the ROC curve (AUC) is equal to the probability that a classifier will rank a randomly chosen positive instance higher than a randomly chosen negative one. Peptides with AUC greater than or equal to 0.6 found uniquely by Sequest or Xtandem are found in Tables 8 and 9, respectively, and those identified by both approaches are found in Table 10.

**Table 8. Significant peptides (AUC>0.6) for Sequest only**

| **Protein Description** | **Uniprot ID (name)** | **Peptide** | **S_AUC** |
|---|---|---|---|
| 5'-AM P-activated protein kinase subunit gamma-3 | **Q9UGI9** (AAKG3_HUMAN) | K.LVIFDTM*LEIK.K | 0.78 |
| afamin precursor | **P43652** (AFAM_HUMAN) | K.FIEDNIEYITIIAFAQYVQEATFEEME K.L | 0.79 |
| afamin precursor | **P43652** (AFAM_HUMAN) | K.IAPQLSTEELVSLGEK.M | 0.71 |
| afamin precursor | **P43652** (AFAM_HUMAN) | K.LKHELTDEELQSLFTNFANVVDK.C | 0.60 |
| afamin precursor | **P43652** (AFAM_HUMAN) | K.LPNNVLQEK.I | 0.60 |
| afamin precursor | **P43652** (AFAM_HUMAN) | K.SDVGFLPPFPTLDPEEK.C | 0.71 |
| afamin precursor | **P43652** (AFAM_HUMAN) | K.VMNHICSK.Q | 0.68 |
| afamin precursor | **P43652** (AFAM_HUMAN) | R.ESLLNHFLYEVAR.R | 0.69 |
| afamin precursor | **P43652** (AFAM_HUMAN) | R.LCFFYNKK.S | 0.69 |
| alpha-1-antichymotrypsin precursor | **P01011** (AACT_HUMAN) | K.AVLDVFEEGTEASAATAVK.I | 0.72 |
| alpha-1-anti chymotrypsin precursor | **P01011** (AACT_HUMAN) | K.EQLSLLDR.F | 0.65 |
| alpha-1-anti chymotrypsin precursor | **P01011** (AACT_HUMAN) | K.EQLSLLDRFTEDAK.R | 0.64 |
| alpha-1-anti chymotrypsin precursor | **P01011** (AACT_HUMAN) | K.EQLSLLDRFTEDAKR.L | 0.60 |
| alpha-1-anti chymotrypsin precursor | **P01011** (AACT_HUMAN) | K.ITDLIKDLDSQTMM*VLVNYIFFK.A | 0.65 |
| alpha-1-anti chymotrypsin precursor | **P01011** (AACT_HUMAN) | K.ITLLSALVETR.T | 0.62 |
| alpha-1-antichymotrypsin precursor | **P01011** (AACT_HUMAN) | K.RLYGSEAFATDFQDSAAAK.K | 0.62 |
| alpha-1-antichymotrypsin precursor | **P01011** (AACT_HUMAN) | R.EIGELYLPK.F | 0.65 |
| alpha-1B-glycoprotein precursor | **P04217** (A1BG_HUMAN) | R.CEGPIPDVTFELLR.E | 0.67 |
| alpha-1B-glycoprotein precursor | **P04217** (A1BG_HUMAN) | R.FALVR.E | 0.79 |
| alpha-2-antiplasmin isoform a precursor | **P08697** (A2AP_HUMAN) | K.SPPGVCSR.D | 0.81 |
| alpha-2-antiplasmin isoform a precursor | **P08697** (A2AP_HUMAN) | R.DSFHLDEQFTVPVEMMQAR.T | 0.69 |
| alpha-2-HS-glycoprotein preproprotein | **P02765** (FETUA_HUMAN) | K.CNLLAEK.Q | 0.67 |
| alpha-2-HS-glycoprotein preproprotein | **P02765** (FETUA_HUMAN) | K.EHAVEGDCDFQLLK.L | 0.67 |
| alpha-2-HS-glycoprotein preproprotein | **P02765** (FETUA_HUMAN) | K. HTLNQI DEVKVWPQQPSGELFEI EI D TLETTCHVLDPTPVAR.C | 0.64 |
| alpha-2-macroglobulin precursor | **P01023** (A2MG_HUMAN) | K.MVSGFIPLKPTVK.M | 0.73 |
| alpha-2-macroglobulin precursor | **P01023** (A2MG_HUMAN) | R.AFQPFFVELTM*PYSVIR.G | 0.68 |
| alpha-2-macroglobulin precursor | **P01023** (A2MG_HUMAN) | R.AFQPFFVELTMPYSVIR.G | 0.62 |
| alpha-2-macroglobulin precursor | **P01023** (A2MG_HUMAN) | R.NQGNTWLTAFVLK.T | 0.73 |
| angiotensinogen preproprotein | **P01019** (ANGT_HUMAN) | K.IDRFMQAVTGWK.T | 0.81 |
| angiotensinogen preproprotein | **P01019** (ANGT_HUMAN) | K.LDTEDKLR.A | 0.72 |
| angiotensinogen preproprotein | **P01019** (ANGT_HUMAN) | K.TGCSLMGASVDSTLAFNTYVHFQGK .M | 0.64 |
| angiotensinogen preproprotein | **P01019** (ANGT_HUMAN) | R.AAMVGMLANFLGFR.I | 0.62 |
| antithrombin-III precursor | **P01008** (ANT3_HUMAN) | K.NDNDNIFLSPLSISTAFAMTK.L | 0.64 |
| antithrombin-III precursor | **P01008** (ANT3_HUMAN) | K.SKLPGIVAEGRDDLYVSDAFHK.A | 0.81 |
| antithrombin-III precursor | **P01008** (ANT3_HUMAN) | R.EVPLNTIIFMGR.V | 0.61 |
| antithrom bin-III precursor | **P01008** (ANT3_HUMAN) | R.FATTFYQHLADSKNDNDNIFLSPLSIS TAFAMTK.L | 0.66 |
| antithrombin-III precursor | **P01008** (ANT3_HUMAN) | R.ITDVIPSEAINELTVLVLVNTIYFK.G | 0.60 |
| antithrombin-III precursor | **P01008** (ANT3_HUMAN) | R.RVWELSK.A | 0.63 |
| antithrombin-III precursor | **P01008** (ANT3_HUMAN) | R.VAEGTQVLELPFKGDDITM*VLILPK PEK.S | 0.62 |
| antithrombin-III precursor | **P01008** (ANT3_HUMAN) | R.VAEGTQVLELPFKGDDITMVLILPKP EK.S | 0.62 |
| apolipoprotein A-II preproprotein | **P02652** (APOA2_HUMAN) | K.AGTELVNFLSYFVELGTQPATQ.- | 0.61 |
| apolipoprotein A-II preproprotein | **P02652** (APOA2_HUMAN) | K.EPCVESLVSQYFQTVTDYGK.D | 0.63 |
| apolipoprotein A-IV precursor | **P06727** (APOA4_HUMAN) | K.ALVQQMEQLR.Q | 0.61 |
| apolipoprotein A-IV precursor | **P06727** (APOA4_HUMAN) | K.LGPHAGDVEGHLSFLEK.D | 0.61 |
| apolipoprotein A-IV precursor | **P06727** (APOA4_HUMAN) | K.SELTQQLNALFQDK.L | 0.71 |
| apolipoprotein A-IV precursor | **P06727** (APOA4_HUMAN) | K.SLAELGGHLDQQVEEFRR.R | 0.61 |
| apolipoprotein A-IV precursor | **P06727** (APOA4_HUMAN) | K.VKIDQTVEELRR.S | 0.75 |
| apolipoprotein A-IV precursor | **P06727** (APOA4_HUMAN) | K.VNSFFSTFK.E | 0.63 |
| apolipoprotein B-100 precursor | **P04114** (APOB_HUMAN) | K.ATFQTPDFIVPLTDLR.I | 0.65 |
| apolipoprotein B-100 precursor | **P04114** (APOB_HUMAN) | K.AVSM*PSFSILGSDVR.V | 0.65 |
| apolipoprotein B-100 precursor | **P04114** (APOB_HUMAN) | K.AVSMPSFSILGSDVR.V | 0.67 |
| apolipoprotein B-100 precursor | **P04114** (APOB_HUMAN) | K.EQHLFLPFSYK.N | 0.65 |
| apolipoprotein B-100 precursor | **P04114** (APOB_HUMAN) | K.KIISDYHQQFR.Y | 0.63 |
| apolipoprotein B-100 precursor | **P04114** (APOB_HUMAN) | K.QVFLYPEKDEPTYILNIK.R | 0.64 |
| apolipoprotein B-100 precursor | **P04114** (APOB_HUMAN) | K.SPAFTDLHLR.Y | 0.69 |
| apolipoprotein B-100 precursor | **P04114** (APOB_HUMAN) | K.TILGTMPAFEVSLQALQK.A | 0.62 |
| apolipoprotein B-100 precursor | **P04114** (APOB_HUMAN) | K.VLADKFIIPGLK.L | 0.72 |
| apolipoprotein B-100 precursor | **P04114** (APOB_HUMAN) | K.YSQPEDSLIPFFEITVPESQLTVSQFTL PK.S | 0.61 |
| apolipoprotein B-100 precursor | **P04114** (APOB_HUMAN) | R.DLKVEDIPLAR.I | 0.64 |
| apolipoprotein B-100 precursor | **P04114** (APOB_HUMAN) | R.GIISALLVPPETEEAK.Q | 0.81 |
| apolipoprotein B-100 | **P04114** (APOB_HUMAN) | R.ILGEELGFASLHDLQLLGK.L | 0.62 |
| precursor | | | |
| apolipoprotein B-100 precursor | **P04114** (APOB_HUMAN) | R.LELELRPTGEIEQYSVSATYELQR.E | 0.60 |
| apolipoprotein B-100 precursor | **P04114** (APOB_HUMAN) | R.NIQEYLSILTDPDGK.G | 0.68 |
| apolipoprotein B-100 precursor | **P04114** (APOB_HUMAN) | R.TFQIPGYTVPVVNVEVSPFTIEMSAF GYVFPK.A | 0.75 |
| apolipoprotein B-100 precursor | **P04114** (APOB_HUMAN) | R.TIDQMLNSELQWPVPDIYLR.D | 0.70 |
| apolipoprotein C-I precursor | **P02654** (APOC1_HUMAN) | K.MREWFSETFQK.V | 0.61 |
| apolipoprotein C-II precursor | **P02655** (APOC2_HUMAN) | K.STAAMSTYTGIFTDQVLSVLKGEE.- | 0.61 |
| apolipoprotein C-III precursor | **P02656** (APOC3_HUMAN) | R.GWVTDGFSSLK.D | 0.62 |
| apolipoprotein E precursor | **P02649** (APOE_HUMAN) | R.AATVGSLAGQPLQER.A | 0.61 |
| apolipoprotein E precursor | **P02649** (APOE_HUMAN) | R.LKSWFEPLVEDMQR.Q | 0.65 |
| apolipoprotein E precursor | **P02649** (APOE_HUMAN) | R.WVQTLSEQVQEELLSSQVTQELR.A | 0.64 |
| ATP-binding cassette sub-family D member 4 | **014678** (ABCD4_HUMAN) | K.LCGGGRWELM*R.I | 0.60 |
| ATP-binding cassette sub-family F member 3 | **Q9NUQ8** (ABCF3_HUMAN) | K.LPGLLK.R | 0.73 |
| beta-2-glycoprotein 1 precursor | **P02749** (APOH_HUMAN) | K.EHSSLAFWK.T | 0.64 |
| beta-2-glycoprotein 1 precursor | **P02749** (APOH_HUMAN) | R.TCPKPDDLPFSTVVPLK.T | 0.60 |
| beta-2-glycoprotein 1 precursor | **P02749** (APOH_HUMAN) | R.VCPFAGILENGAVR.Y | 0.68 |
| beta-Ala-His dipeptidase precursor | **Q96KN2** (CNDP1_HUMAN) | K.LFAAFFLEMAQLH.- | 0.68 |
| biotinidase precursor | **P43251** (BTD_HUMAN) | K.SHLIIAQVAK.N | 0.62 |
| carboxypeptidase B2 preproprotein | **Q961Y4** (CBPB2_HUMAN) | K.NAIWIDCGIHAR.E | 0.62 |
| carboxypeptidase N catalytic chain precursor | **P15169** (CBPN_HUMAN) | R.EALIQFLEQVHQGIK.G | 0.69 |
| carboxypeptidase N subunit 2 precursor | **P22792** (CPN2_HUMAN) | R.LLNIQTYCAGPAYLK.G | 0.62 |
| catalase | **P04040** (CATA_HUMAN) | R.LCENIAGHLKDAQIFIQK.K | 0.62 |
| ceruloplasmin precursor | **P00450** (CERU_HUMAN) | K.AETGDKVYVHLK.N | 0.61 |
| ceruloplasmin precursor | **P00450** (CERU_HUMAN) | K.AGLQAFFQVQECNK.S | 0.62 |
| ceruloplasmin precursor | **P00450** (CERU_HUMAN) | K.DIASGLIGPLIICK.K | 0.63 |
| ceruloplasmin precursor | **P00450** (CERU_HUMAN) | K.DIFTGLIGPM*K.I | 0.63 |
| ceruloplasmin precursor | **P00450** (CERU_HUMAN) | K.DIFTGLIGPMK.I | 0.68 |
| ceruloplasmin precursor | **P00450** (CERU_HUMAN) | K.M*YYSAVDPTKDIFTGLIGPMK.I | 0.62 |
| ceruloplasmin precursor | **P00450** (CERU_HUMAN) | K.MYYSAVDPTKDIFTGLIGPM*K.I | 0.63 |
| ceruloplasmin precursor | **P00450** (CERU_HUMAN) | K.PVWLGFLGPIIK.A | 0.63 |
| ceruloplasmin precursor | **P00450** (CERU_HUMAN) | R.ADDKVYPGEQYTYMLLATEEQSPGE GDGNCVTR.I | 0.64 |
| ceruloplasmin precursor | **P00450** (CERU_HUMAN) | R.DTANLFPQTSLTLHM*WPDTEGTF NVECLTTDHYTGGMK.Q | 0.71 |
| ceruloplasmin precursor | **P00450** (CERU_HUMAN) | R.DTANLFPQTSLTLHMWPDTEGTFN VECLTTDHYTGGMK.Q | 0.68 |
| ceruloplasmin precursor | **P00450** (CERU_HUMAN) | R.FNKNNEGTYYSPNYNPQSR.S | 0.74 |
| ceruloplasmin precursor | **P00450** (CERU_HUMAN) | R.IDTINLFPATLFDAYM*VAQNPGEW M*LSCQNLNHLK.A | 0.75 |
| ceruloplasmin precursor | **P00450** (CERU_HUMAN) | R.IDTINLFPATLFDAYM*VAQNPGEW MLSCQNLNHLK.A | 0.86 |
| ceruloplasmin precursor | **P00450** (CERU_HUMAN) | R.IDTINLFPATLFDAYMVAQNPGEW M*LSCQNLNHLK.A | 0.60 |
| ceruloplasmin precursor | **P00450** (CERU_HUMAN) | R.KAEEEHLGILGPQLHADVGDKVK.I | 0.71 |
| ceruloplasmin precursor | **P00450** (CERU_HUMAN) | R.TTIEKPVWLGFLGPIIK.A | 0.63 |
| cholinesterase precursor | **P06276** (CHLE_HUMAN) | R.FWTSFFPK.V | 0.76 |
| clusterin preproprotein | **P10909** (CLUS_HUMAN) | K.LFDSDPITVTVPVEVSR.K | 0.78 |
| clusterin preproprotein | **P10909** (CLUS_HUMAN) | R.ASSIIDELFQDR.F | 0.68 |
| coagulation factor IX preproprotein | **P00740** (FA9_HUMAN) | K.WIVTAAHCVETGVK.I | 0.60 |
| coagulation factor VII isoform a preproprotein | **P08709** (FA7_HUMAN) | R.FSLVSGWGQLLDR.G | 0.78 |
| coagulation factor X preproprotein | **P00742** (FA10_HUMAN) | K.ETYDFDIAVLR.L | 0.75 |
| coiled-coil domain-containing protein 13 | **Q8IYE1** (CCD13_HUMAN) | K.VRQLEMEIGQLNVHYLR.N | 0.67 |
| complement C1q subcomponent subunit A precursor | **P02745** (C1QA_HUMAN) | R.PAFSAIR.R | 0.66 |
| complement C1q subcomponent | **P02746** (C1QB_HUMAN) | K.VVTFCDYAYNTFQVTTGGMVLK.L | 0.63 |
| subunit B precursor | | | |
| complement C1q subcomponent subunit C precursor | **P02747** (C1QC_HUMAN) | K.FQSVFTVTR.Q | 0.63 |
| complement C1r subcomponent precursor | **P00736** (C1R_HUMAN) | K.TLDEFTIIQNLQPQYQFR.D | 0.62 |
| complement C1r subcomponent precursor | **P00736** (C1R_HUMAN) | R.MDVFSQNMFCAGHPSLK.Q | 0.68 |
| complement C1r subcomponent precursor | **P00736** (C1R_HUMAN) | R.WILTAAHTLYPK.E | 0.74 |
| complement C1s subcomponent precursor | **P09871** (C1S_HUMAN) | K.FYAAGLVSWGPQCGTYGLYTR.V | 0.68 |
| complement C1s subcomponent precursor | **P09871** (C1S_HUMAN) | K.GFQVVVTLR.R | 0.63 |
| complement C2 isoform 3 | **P06681** (CO2_HUMAN) | R.GALISDQWVLTAAHCFR.D | 0.61 |
| complement C2 isoform 3 | **P06681** (CO2_HUMAN) | R.PICLPCTMEANLALR.R | 0.66 |
| complement C3 precursor | **P01024** (CO3_HUMAN) | R.YYGGGYGSTQATFMVFQALAQYQK .D | 0.75 |
| complement C4-A isoform 1 | **P0C0L4** (CO4A_HUMAN) | K.GLCVATPVQLR.V | 0.74 |
| complement C4-A isoform 1 | **P0C0L4** (CO4A_HUMAN) | K.M*RPSTDTITVM*VENSHGLR.V | 0.83 |
| complement C4-A isoform 1 | **P0C0L4** (CO4A_HUMAN) | K.MRPSTDTITVM*VENSHGLR.V | 0.72 |
| complement C4-A isoform 1 | **P0C0L4** (CO4A_HUMAN) | K.VGLSGM*AIADVTLLSGFHALR.A | 0.71 |
| complement C4-A isoform 1 | **P0C0L4** (CO4A_HUMAN) | K.VLSLAQEQVGGSPEK.L | 0.63 |
| complement C4-A isoform 1 | **P0C0L4** (CO4A_HUMAN) | R.EMSGSPASGIPVK.V | 0.65 |
| complement C4-A isoform 1 | **P0C0L4** (CO4A_HUMAN) | R.GCGEQTM*IYLAPTLAASR.Y | 0.75 |
| complement C4-A isoform 1 | **P0C0L4** (CO4A_HUMAN) | R.GLQDEDGYR.M | 0.75 |
| complement C4-A isoform 1 | **P0C0L4** (CO4A_HUMAN) | R.GQIVFMNREPK.R | 0.93 |
| complement C4-A isoform 1 | **P0C0L4** (CO4A_HUMAN) | R.KKEVYM*PSSIFQDDFVIPDISEPGT WK.I | 0.72 |
| complement C4-A isoform 1 | **P0C0L4** (CO4A_HUMAN) | R.LPMSVR.R | 0.78 |
| complement C4-A isoform 1 | **P0C0L4** (CO4A_HUMAN) | R.LTVAAPPSGGPGFLSIER.P | 0.84 |
| complement C4-A | **P0C0L4** (CO4A_HUMAN) | R.NFLVR.A | 0.75 |
| isoform 1 | | | |
| complement C4-A isoform 1 | **P0C0L4** (CO4A_HUMAN) | R.NGESVKLHLETDSLALVALGALDTAL YAAGSK.S | 0.88 |
| complement C4-A isoform 1 | **P0C0L4** (CO4A_HUMAN) | R.QGSFQGGFR.S | 0.60 |
| complement C4-A isoform 1 | **P0C0L4** (CO4A_HUMAN) | R.TLEIPGNSDPNMIPDGDFNSYVR.V | 0.69 |
| complement C4-A isoform 1 | **P0C0L4** (CO4A_HUMAN) | R.VTASDPLDTLGSEGALSPGGVASLLR .L | 0.63 |
| complement C4-A isoform 1 | **P0C0L4** (CO4A_HUMAN) | R.YLDKTEQWSTLPPETK.D | 0.67 |
| complement C5 preproprotein | **P01031** (CO5_HUMAN) | K.ADNFLLENTLPAQSTFTLAISAYALSL GDK.T | 0.63 |
| complement C5 preproprotein | **P01031** (CO5_HUMAN) | K.ALVEGVDQLFTDYQIK.D | 0.63 |
| complement C5 preproprotein | **P01031** (CO5_HUMAN) | K.DGHVILQLNSIPSSDFLCVR.F | 0.62 |
| complement C5 preproprotein | **P01031** (CO5_HUMAN) | K.DVFLEMNIPYSVVR.G | 0.63 |
| complement C5 preproprotein | **P01031** (CO5_HUMAN) | K.EFPYRIPLDLVPK.T | 0.60 |
| complement C5 preproprotein | **P01031** (CO5_HUMAN) | K.FQNSAILTIQPK.Q | 0.67 |
| complement C5 preproprotein | **P01031** (CO5_HUMAN) | K.VFKDVFLEMNIPYSVVR.G | 0.63 |
| complement C5 preproprotein | **P01031** (CO5_HUMAN) | R.VFQFLEK.S | 0.61 |
| complement component C6 precursor | **P13671** (CO6_HUMAN) | K.DLHLSDVFLK.A | 0.60 |
| complement component C6 precursor | **P13671** (CO6_HUMAN) | R.TECIKPVVQEVLTITPFQR.L | 0.62 |
| complement component C7 precursor | **P10643** (CO7_HUMAN) | K.SSGWHFVVK.F | 0.61 |
| complement component C7 precursor | **P10643** (CO7_HUMAN) | R.ILPLTVCK.M | 0.75 |
| complement component C8 alpha chain precursor | **P07357** (CO8A_HUMAN) | R.ALDQYLMEFNACR.C | 0.65 |
| complement component C8 gamma chain precursor | **P07360** (CO8G_HUMAN) | K.YGFCEAADQFHVLDEVR.R | 0.60 |
| complement component C9 precursor | **P02748** (CO9_HUMAN) | R.AIEDYINEFSVRK.C | 0.69 |
| complement | **P02748** (CO9_HUMAN) | R.TAGYGINILGMDPLSTPFDNEFYNGL | 0.69 |
| component C9 precursor | | CNR.D | |
| complement factor B preproprotein | **P00751** (CFAB_HUMAN) | K.ALFVSEEEKK.L | 0.64 |
| complement factor B preproprotein | **P00751** (CFAB_HUMAN) | K.CLVNLIEK.V | 0.70 |
| complement factor B preproprotein | **P00751** (CFAB_HUMAN) | K.EAGIPEFYDYDVALIK.L | 0.66 |
| complement factor B preproprotein | **P00751** (CFAB_HUMAN) | K.VSEADSSNADWVTK.Q | 0.73 |
| complement factor B preproprotein | **P00751** (CFAB_HUMAN) | K.YGQTIRPICLPCTEGTTR.A | 0.67 |
| complement factor B preproprotein | **P00751** (CFAB_HUMAN) | R.DLEIEVVLFHPNYNINGK.K | 0.71 |
| complement factor B preproprotein | **P00751** (CFAB_HUMAN) | R.FLCTGGVSPYADPNTCR.G | 0.64 |
| complement factor H isoform a precursor | **P08603** (CFAH_HUMAN) | K.DGWSAQPTCIK.S | 0.80 |
| complement factor H isoform a precursor | **P08603** (CFAH_HUMAN) | K.EGWIHTVCINGR.W | 0.67 |
| complement factor H isoform a precursor | **P08603** (CFAH_HUMAN) | K.TDCLSLPSFENAIPMGEK.K | 0.61 |
| complement factor H isoform a precursor | **P08603** (CFAH_HUMAN) | R.DTSCVNPPTVQNAYIVSR.Q | 0.60 |
| complement factor H isoform b precursor | **P08603** (CFAH_HUMAN) | K.CTSTGWIPAPR.C | 0.68 |
| complement factor H isoform b precursor | **P08603** (CFAH_HUMAN) | K.IIYKENER.F | 0.76 |
| complement factor H isoform b precursor | **P08603** (CFAH_HUMAN) | K.IVSSAM*EPDREYHFGQAVR.F | 0.75 |
| complement factor H isoform b precursor | **P08603** (CFAH_HUMAN) | K.IVSSAMEPDREYHFGQAVR.F | 0.68 |
| complement factor H isoform b precursor | **P08603** (CFAH_HUMAN) | R.CTLKPCDYPDIK.H | 0.81 |
| complement factor H isoform b precursor | **P08603** (CFAH_HUMAN) | R.KGEWVALNPLR.K | 0.60 |
| complement factor H isoform b precursor | **P08603** (CFAH_HUMAN) | R.KGEWVALNPLRK.C | 0.69 |
| complement factor H isoform b precursor | **P08603** (CFAH_HUMAN) | R.RPYFPVAVGK.Y | 0.68 |
| complement factor H-related protein 1 precursor | **Q03591** (FHR1_HUMAN) | R.EIMENYNIALR.W | 0.64 |
| complement factor I preproprotein | **P05156** (CFAI_HUMAN) | K.DASGITCGGIYIGGCWILTAAHCLR.A | 0.71 |
| complement factor I preproprotein | **P05156** (CFAI_HUMAN) | K.VANYFDWISYHVGR.P | 0.72 |
| complement factor I preproprotein | **P05156** (CFAI_HUMAN) | R.IIFHENYNAGTYQNDIALIEMK.K | 0.63 |
| complement factor I preproprotein | **P05156** (CFAI_HUMAN) | R.YQIWTTVVDWIHPDLK.R | 0.63 |
| conserved oligomeric Golgi complex subunit 6 isoform | **Q9Y2V7** (COG6_HUMAN) | K.ISNLLK.F | 0.65 |
| corticosteroid-binding globulin precursor | **P08185** (CBG_HUMAN) | R.WSAGLTSSQVDLYIPK.V | 0.62 |
| C-reactive protein precursor | **P02741** (CRP_HUMAN) | K.YEVQGEVFTKPQLWP.- | 0.60 |
| dopamine beta-hydroxylase precursor | **P09172** (DOPO_HUMAN) | R.HVLAAWALGAK.A | 0.88 |
| double-stranded RNA-specific editase B2 | **Q9NS39** (RED2_HUMAN) | R.AGLRYVCLAEPAER.R | 0.75 |
| dual oxidase 2 precursor | **Q9NRD8** (DUOX2_HUMAN) | R.FTQLCVKGGGGGGNGIR.D | 0.65 |
| FERM domain-containing protein 8 | **Q9BZ67** (FRMD8_HUMAN) | R.VQLGPYQPGRPAACDLR.E | 0.65 |
| fetuin-B precursor | **Q9UGM5** (FETUB_HUMAN) | R.GGLGSLFYLTLDVLETDCHVLR.K | 0.83 |
| ficolin-3 isoform 1 precursor | **075636** (FCN3_HUMAN) | R.ELLSQGATLSGWYHLCLPEGR.A | 0.69 |
| gastric intrinsic factor precursor | **P27352** (IF_HUMAN) | K.KTTDM*ILNEIKQGK.F | 0.60 |
| gelsolin isoform d | **P06396** (GELS_HUMAN) | K. NWRDPDQTDGLGLSYLSSHIANVER .V | 0.72 |
| gelsolin isoform d | **P06396** (GELS_HUMAN) | K.TPSAAYLWVGTGASEAEK.T | 0.80 |
| gelsolin isoform d | **P06396** (GELS_HUMAN) | R.VEKFDLVPVPTNLYGDFFTGDAYVIL K.T | 0.60 |
| gelsolin isoform d | **P06396** (GELS_HUMAN) | R.VPFDAATLHTSTAMAAQHGM DDD GTGQK.Q | 0.67 |
| glutathione peroxidase 3 precursor | **P22352** (GPX3_HUMAN) | K.FYTFLK.N | 0.63 |
| hemopexin precursor | **P02790** (HEMO_HUMAN) | K.GDKVWVYPPEKK.E | 0.65 |
| hemopexin precursor | **P02790** (HEMO_HUMAN) | K.LLQDEFPGIPSPLDAAVECHR.G | 0.71 |
| hemopexin precursor | **P02790** (HEMO_HUMAN) | K.SGAQATWTELPWPHEK.V | 0.64 |
| hemopexin precursor | **P02790** (HEMO_HUMAN) | K.SGAQATWTELPWPHEKVDGALCM EK.S | 0.61 |
| hemopexin precursor | **P02790** (HEMO_HUMAN) | K.VDGALCMEK.S | 0.66 |
| hemopexin precursor | **P02790** (HEMO_HUMAN) | R.DYFMPCPGR.G | 0.68 |
| hemopexin precursor | **P02790** (HEMO_HUMAN) | R.EWFWDLATGTM*K.E | 0.64 |
| hemopexin precursor | **P02790** (HEMO_HUMAN) | R.QGHNSVFLIK.G | 0.71 |
| heparin cofactor 2 precursor | **P05546** (HEP2_HUMAN) | K.HQGTITVNEEGTQATTVTTVGFMPL STQVR.F | 0.60 |
| heparin cofactor 2 precursor | **P05546** (HEP2_HUMAN) | K.YEITTIHNLFR.K | 0.62 |
| heparin cofactor 2 | **P05546** (HEP2_HUMAN) | R.LNILNAK.F | 0.68 |
| precursor | | | |
| heparin cofactor 2 precursor | **P05546** (HEP2_HUMAN) | R.NFGYTLR.S | 0.64 |
| heparin cofactor 2 precursor | **P05546** (HEP2_HUMAN) | R.VLKDQVNTFDNIFIAPVGISTAMGM *ISLGLK.G | 0.63 |
| hepatocyte cell adhesion molecule precursor | **Q14CZ8** (HECAM_HUMAN) | K.PLLNDSRMLLSPDQK.V | 0.61 |
| hepatocyte growth factor activator preproprotein | **Q04756** (HGFA_HUMAN) | R.VQLSPDLLATLPEPASPGR.Q | 0.82 |
| histidine-rich glycoprotein precursor | **P04196** (HRG_HUMAN) | R.DGYLFQLLR.I | 0.63 |
| hyaluronan-binding protein 2 isoform 1 preproprotein | **Q14520** (HABP2_HUMAN) | K.FLNWIK.A | 0.82 |
| hyaluronan-binding protein 2 isoform 1 preproprotein | **Q14520** (HABP2_HUMAN) | K.LKPVDGHCALESK.Y | 0.61 |
| hyaluronan-binding protein 2 isoform 1 preproprotein | **Q14520** (HABP2_HUMAN) | K.RPGVYTQVTK.F | 0.74 |
| inactive caspase-12 | **Q6UXS9** (CASPC_HUMAN) | K.AGADTHGRLLQGNICNDAVTK.A | 0.74 |
| insulin-degrading enzyme isoform 1 | **P14735** (IDE_HUMAN) | K.KIIEKM*ATFEIDEK.R | 0.85 |
| insulin-like growth factor-binding protein complex acid labile subunit isoform 2 precursor | **P35858** (ALS_HUMAN) | R.SFEGLGQLEVLTLDHNQLQEVK.A | 0.62 |
| inter-alpha-trypsin inhibitor heavy chain H1 isoform a precursor | **P19827** (ITIH1_HUMAN) | K.ELAAQTIKK.S | 0.81 |
| inter-alpha-trypsin inhibitor heavy chain H1 isoform a precursor | **P19827** (ITIH1_HUMAN) | K.GSLVQASEANLQAAQDFVR.G | 0.71 |
| inter-alpha-trypsin inhibitor heavy chain H1 isoform a precursor | **P19827** (ITIH1_HUMAN) | K.QLVHHFEIDVDIFEPQGISK.L | 0.70 |
| inter-alpha-trypsin inhibitor heavy chain H1 isoform a precursor | **P19827** (ITIH1_HUMAN) | K.QYYEGSEIVVAGR.I | 0.83 |
| inter-alpha-trypsin inhibitor heavy chain H1 isoform a | **P19827** (ITIH1_HUMAN) | R.EVAFDLEIPKTAFISDFAVTADGNAFI GDIK.D | 0.70 |
| precursor | | | |
| inter-alpha-trypsin inhibitor heavy chain H1 isoform a precursor | **P19827** (ITIH1_HUMAN) | R.GMADQDGLKPTIDKPSEDSPPLEM* LGPR.R | 0.63 |
| inter-alpha-trypsin inhibitor heavy chain H1 isoform a precursor | **P19827** (ITIH1_HUMAN) | R.GMADQDGLKPTIDKPSEDSPPLEML GPR.R | 0.60 |
| inter-alpha-trypsin inhibitor heavy chain H2 precursor | **P19823** (ITIH2_HUMAN) | K.FDPAKLDQIESVITATSANTQLVLETL AQM*DDLQDFLSK.D | 0.80 |
| inter-alpha-trypsin inhibitor heavy chain H2 precursor | **P19823** (ITIH2_HUMAN) | K.KFYNQVSTPLLR.N | 0.76 |
| inter-alpha-trypsin inhibitor heavy chain H2 precursor | **P19823** (ITIH2_HUMAN) | K.NILFVIDVSGSM*WGVK.M | 0.68 |
| inter-alpha-trypsin inhibitor heavy chain H2 precursor | **P19823** (ITIH2_HUMAN) | K.NILFVIDVSGSMWGVK.M | 0.62 |
| inter-alpha-trypsin inhibitor heavy chain H2 precursor | **P19823** (ITIH2_HUMAN) | R.KLGSYEHR.I | 0.72 |
| inter-alpha-trypsin inhibitor heavy chain H2 precursor | **P19823** (ITIH2_HUMAN) | R.LSNENHGIAQR.I | 0.66 |
| inter-alpha-trypsin inhibitor heavy chain H2 precursor | **P19823** (ITIH2_HUMAN) | R.MATTMIQSK.V | 0.60 |
| inter-alpha-trypsin inhibitor heavy chain H2 precursor | **P19823** (ITIH2_HUMAN) | R.SILQM*SLDHHIVTPLTSLVIENEAG DER.M | 0.63 |
| inter-alpha-trypsin inhibitor heavy chain H2 precursor | **P19823** (ITIH2_HUMAN) | R.SILQMSLDHHIVTPLTSLVIENEAGDE R.M | 0.65 |
| inter-alpha-trypsin inhibitor heavy chain H2 precursor | **P19823** (ITIH2_HUMAN) | R.TEVNVLPGAK.V | 0.69 |
| inter-a lpha-trypsin inhibitor heavy chain H4 isoform 1 precursor | **Q14624** (ITIH4_HUMAN) | K.NVVFVIDK.S | 0.68 |
| inter-alpha-trypsin inhibitor heavy chain H4 isoform 1 precursor | **Q14624** (ITIH4_HUMAN) | K.WKETLFSVMPGLK.M | 0.65 |
| inter-alpha-trypsin inhibitor heavy chain H4 isoform 1 | **Q14624** (ITIH4_HUMAN) | K.YIFHNFM*ER.L | 0.67 |
| precursor | | | |
| inter-alpha-trypsin inhibitor heavy chain H4 isoform 1 precursor | **Q14624** (ITIH4_HUMAN) | R.FAHTVVTSR.V | 0.63 |
| inter-alpha-trypsin inhibitor heavy chain H4 isoform 1 precursor | **Q14624** (ITIH4_HUMAN) | R.FKPTLSQQQK.S | 0.60 |
| inter-alpha-trypsin inhibitor heavy chain H4 isoform 1 precursor | **Q14624** (ITIH4_HUMAN) | R.IHEDSDSALQLQDFYQEVANPLLTA VTFEYPSNAVEEVTQNNFR.L | 0.64 |
| inter-alpha-trypsin inhibitor heavy chain H4 isoform 1 precursor | **Q14624** (ITIH4_HUMAN) | R.MNFRPGVLSSR.Q | 0.63 |
| inter-alpha-trypsin inhibitor heavy chain H4 isoform 1 precursor | **Q14624** (ITIH4_HUMAN) | R.NVHSAGAAGSR.M | 0.62 |
| inter-alpha-trypsin inhibitor heavy chain H4 isoform 1 precursor | **Q14624** (ITIH4_HUMAN) | R.NVHSGSTFFK.Y | 0.75 |
| inter-alpha-trypsin inhibitor heavy chain H4 isoform 1 precursor | **Q14624** (ITIH4_HUMAN) | R.RLGVYELLLK.V | 0.66 |
| kallistatin precursor | **P29622** (KAIN_HUMAN) | K.KLELHLPK.F | 0.78 |
| kallistatin precursor | **P29622** (KAIN_HUMAN) | R.EIEEVLTPEMLMR.W | 0.60 |
| kininogen-1 isoform 2 precursor | **P01042** (KNG1_HUMAN) | K.AATGECTATVGKR.S | 0.67 |
| kininogen-1 isoform 2 precursor | **P01042** (KNG1_HUMAN) | K.LGQSLDCNAEVYVVPWEK.K | 0.72 |
| kininogen-1 isoform 2 precursor | **P01042** (KNG1_HUMAN) | K.YNSQNQSNNQFVLYR.I | 0.62 |
| kininogen-1 isoform 2 precursor | **P01042** (KNG1_HUMAN) | R.QVVAGLNFR.I | 0.64 |
| leucine-rich alpha-2-glycoprotein precursor | **P02750** (A2GL_HUMAN) | K.DLLLPQPDLR.Y | 0.64 |
| leucine-rich alpha-2-glycoprotein precursor | **P02750** (A2GL_HUMAN) | R.LHLEGNKLQVLGK.D | 0.76 |
| leucine-rich alpha-2-glycoprotein precursor | **P02750** (A2GL_HUMAN) | R.TLDLGENQLETLPPDLLR.G | 0.61 |
| lipopolysaccharide-binding protein | **P18428** (LBP_HUMAN) | K.GLQYAAQEGLLALQSELLR.I | 0.82 |
| precursor | | | |
| lipopolysaccharide-binding protein precursor | **P18428** (LBP_HUMAN) | K.LAEGFPLPLLK.R | 0.66 |
| lumican precursor | **P51884** (LUM_HUMAN) | K.SLEYLDLSFNQIAR.L | 0.65 |
| lumican precursor | **P51884** (LUM_HUMAN) | R.LKEDAVSAAFK.G | 0.74 |
| m7GpppX diphosphatase | **Q96C86** (DCPS_HUMAN) | R.IVFENPDPSDGFVLIPDLK.W | 0.62 |
| matrix metalloproteinase-19 isoform 1 preproprotein | **Q99542** (MMP19_HUMAN) | R.VYFFK.G | 0.63 |
| MBT domain-containing protein 1 | **Q05BQ5** (MBTD1_HUMAN) | K.WFDYLR.E | 0.65 |
| monocyte differentiation antigen CD14 precursor | **P08571** (CD14_HUMAN) | R.LTVGAAQVPAQLLVGALR.V | 0.66 |
| pappalysin-1 preproprotein | **Q13219** (PAPP1_HUMAN) | R.VSFSSPLVAISGVALR.S | 0.66 |
| phosphatidylinositol-glycan-specific phospholipase D precursor | **P80108** (PHLD_HUMAN) | K.GIVAAFYSGPSLSDKEK.L | 0.71 |
| phosphatidylinositol-glycan-specific phospholipase D precursor | **P80108** (PHLD_HUMAN) | R.WYVPVKDLLGIYEK.L | 0.71 |
| pigment epithelium-derived factor precursor | **P36955** (PEDF_HUMAN) | K.LQSLFDSPDFSK.I | 0.61 |
| pigment epithelium-derived factor precursor | **P36955** (PEDF_HUMAN) | R.ALYYDLISSPDIHGTYK.E | 0.72 |
| plasma kallikrein preproprotein | **P03952** (KLKB1_HUMAN) | R.CLLFSFLPASSINDMEKR.F | 0.60 |
| plasma protease C1 inhibitor precursor | **P05155** (IC1_HUMAN) | K.FQPTLLTLPR.I | 0.70 |
| plasma protease C1 inhibitor precursor | **P05155** (IC1_HUMAN) | K.GVTSVSQIFHSPDLAIR.D | 0.66 |
| plasminogen isoform 1 precursor | **P00747** (PLMN_HUMAN) | K.VIPACLPSPNYVVADR.T | 0.63 |
| plasminogen isoform 1 precursor | **P00747** (PLMN_HUMAN) | R.FVTWIEGVMR.N | 0.60 |
| plasminogen isoform 1 precursor | **P00747** (PLMN_HUMAN) | R.HSIFTPETNPR.A | 0.63 |
| platelet basic protein preproprotein | **P02775** (CXCL7_HUMAN) | K.GKEESLDSDLYAELR.C | 0.70 |
| platelet glycoprotein V precursor | **P40197** (GPV_HUMAN) | K.MVLLEQLFLDHNALR.G | 0.66 |
| platelet glycoprotein V precursor | **P40197** (GPV_HUMAN) | R.LVSLDSGLLNSLGALTELQFHR.N | 0.88 |
| pregnancy zone protein precursor | **P20742** (PZP_HUMAN) | K.ALLAYAFSLLGK.Q | 0.66 |
| pregnancy zone protein precursor | **P20742** (PZP_HUMAN) | K.DLFHCVSFTLPR.I | 0.86 |
| pregnancy zone protein precursor | **P20742** (PZP_HUMAN) | K.MLQITNTGFEMK.L | 0.84 |
| pregnancy zone protein precursor | **P20742** (PZP_HUMAN) | R.NELIPLIYLENPRR.N | 0.65 |
| pregnancy zone protein precursor | **P20742** (PZP_HUMAN) | R.SYIFIDEAHITQSLTWLSQMQK.D | 0.68 |
| pregnancy-specific beta-1-glycoprotein 2 precursor | **P11465** (PSG2_HUMAN) | R.SDPVTLNLLHGPDLPR.I | 0.66 |
| pregnancy-specific beta-1-glycoprotein 3 precursor | **Q16557** (PSG3_HUMAN) | R.TLFLFGVTK.Y | 0.62 |
| pregnancy-specific beta-1-glycoprotein 5 precursor | **Q15238** (PSG5_HUMAN) | R.ILILPSVTR.N | 0.76 |
| pregnancy-specific beta-1-glycoprotein 6 isoform a | **Q00889** (PSG6_HUMAN) | R.SDPVTLNLLPK.L | 0.63 |
| progesterone-induced-blocking factor 1 | **Q8WXW3** (PIBF1_HUMAN) | R.VLQLEK.Q | 0.71 |
| protein AMBP preproprotein | **P02760** (AMBP_HUMAN) | R.VVAQGVGIPEDSIFTMADR.G | 0.60 |
| protein CBFA2T2 isoform MTGR1b | **043439** (MTG8R_HUMAN) | R.LTEREWADEWKHLDHALNCIMEM VEK.T | 0.70 |
| protein FAM98C | **Q17RN3** (FA98C_HUMAN) | R.ALCGGDGAAALREPGAGLR.L | 0.75 |
| protein NLRC3 | **Q7RTR2** (NLRC3_HUMAN) | K.ALM*DLLAGKGSQGSQAPQALDR.T | 0.92 |
| protein Z-dependent protease inhibitor precursor | **Q9UK55** (ZPI_HUMAN) | K.MGDHLALEDYLTTDLVETWLR.N | 0.60 |
| prothrombin preproprotein | **P00734** (THRB_HUMAN) | K.SPQELLCGASLISDR.W | 0.84 |
| prothrombin preproprotein | **P00734** (THRB_HUMAN) | R.LAVTTHGLPCLAWASAQAK.A | 0.62 |
| prothrombin preproprotein | **P00734** (THRB_HUMAN) | R.SEGSSVNLSPPLEQCVPDR.G | 0.70 |
| prothrombin preproprotein | **P00734** (THRB_HUMAN) | R.SGIECQLWR.S | 0.68 |
| prothrombin preproprotein | **P00734** (THRB_HUMAN) | R.TATSEYQTFFNPR.T | 0.60 |
| prothrombin preproprotein | **P00734** (THRB_HUMAN) | R.VTGWGNLKETWTANVGK.G | 0.69 |
| putative hydroxypyruvate | **Q5T013** (HYI_HUMAN) | R.IHLM*AGR.V | 0.69 |
| isomerase isoform 1 | | | |
| putative hydroxypyruvate isomerase isoform 1 | **Q5T013** (HYI_HUMAN) | R.IHLMAGR.V | 0.66 |
| ras-like protein family member 10A precursor | **Q92737** (RSLAA_HUMAN) | R.PAHPALR.L | 0.71 |
| ras-related GTP-binding protein A | **Q7L523** (RRAGA_HUMAN) | K.ISNIIK.Q | 0.82 |
| retinol-binding protein 4 precursor | **P02753** (RET4_HUMAN) | K.M*KYWGVASFLQK.G | 0.73 |
| retinol-binding protein 4 precursor | **P02753** (RET4_HUMAN) | R.FSGTWYAM*AK.K | 0.63 |
| retinol-binding protein 4 precursor | **P02753** (RET4_HUMAN) | R.LLNLDGTCADSYSFVFSR.D | 0.79 |
| retinol-binding protein 4 precursor | **P02753** (RET4_HUMAN) | R.LLNNWDVCADMVGTFTDTEDPAKF K.M | 0.77 |
| sex hormone-binding globulin isoform 1 precursor | **P04278** (SHBG_HUMAN) | R.LFLGALPGEDSSTSFCLNGLWAQGQ R.L | 0.66 |
| sex hormone-binding globulin isoform 4 precursor | **P04278** (SHBG_HUMAN) | K.DDWFMLGLR.D | 0.60 |
| sex hormone-binding globulin isoform 4 precursor | **P04278** (SHBG_HUMAN) | R.SCDVESNPGIFLPPGTQAEFNLR.G | 0.64 |
| sex hormone-binding globulin isoform 4 precursor | **P04278** (SHBG_HUMAN) | R.TWDPEGVIFYGDTNPKDDWFM*L GLR.D | 0.65 |
| sex hormone-binding globulin isoform 4 precursor | **P04278** (SHBG_HUMAN) | R.TWDPEGVIFYGDTNPKDDWFMLGL R.D | 0.66 |
| signal transducer and activator of transcription 2 | **P52630** (STAT2_HUMAN) | R.KFCRDIQDPTQLAEMIFNLLLEEK.R | 0.73 |
| spectrin beta chain, non-erythrocytic 1 | **Q13813** (SPTN1_HUMAN) | R.NELIRQEKLEQLAR.R | 0.60 |
| stabilin-1 precursor | **Q9NY15** (STAB1_HUMAN) | R.KNLSER.W | 0.88 |
| succinate-semialdehyde dehydrogenase, mitochondrial | **P51649** (SSDH_HUMAN) | R.KWYNLMIQNK.D | 0.88 |
| tetranectin precursor | **P05452** (TETN_HUMAN) | K.SRLDTLAQEVALLK.E | 0.75 |
| THAP domain-containing protein 6 | **Q8TBB0** (THAP6_HUMAN) | K.RLDVNAAGIWEPKK.G | 0.69 |
| thyroxine- binding globulin precursor | **P05543** (THBG_HUMAN) | R.SILFLGK.V | 0.79 |
| tripartite motif-containing protein 5 | **Q9C035** (TRIM5_HUMAN) | R.ELISDLEHRLQGSVM*ELLQGVDGVI K.R | 0.60 |
| vitamin D-binding | **P02774** (VTDB_HUMAN) | K.EDFTSLSLVLYSR.K | 0.66 |
| protein isoform 1 precursor | | | |
| vitamin D-binding protein isoform 1 precursor | **P02774** (VTDB_HUMAN) | K.ELSSFIDKGQELCADYSENTFTEYK.K | 0.67 |
| vitamin D-binding protein isoform 1 precursor | **P02774** (VTDB_HUMAN) | K.ELSSFIDKGQELCADYSENTFTEYKK. K | 0.66 |
| vitamin D-binding protein isoform 1 precursor | **P02774** (VTDB_HUMAN) | K.EVVSLTEACCAEGADPDCYDTR.T | 0.65 |
| vitamin D-binding protein isoform 1 precursor | **P02774** (VTDB_HUMAN) | K.TAMDVFVCTYFMPAAQLPELPDVEL PTNKDVCDPGNTK.V | 0.84 |
| vitamin D-binding protein isoform 1 precursor | **P02774** (VTDB_HUMAN) | R.RTHLPEVFLSK.V | 0.69 |
| vitamin D-binding protein isoform 1 precursor | **P02774** (VTDB_HUMAN) | R.VCSQYAAYGEK.K | 0.66 |
| vitronectin precursor | **P04004** (VTNC_HUMAN) | K.LIRDVWGIEGPIDAAFTR.I | 0.61 |
| vitronectin precursor | **P04004** (VTNC_HUMAN) | R.DVWGIEGPIDAAFTR.I | 0.63 |
| vitronectin precursor | **P04004** (VTNC_HUMAN) | R.ERVYFFK.G | 0.81 |
| vitronectin precursor | **P04004** (VTNC_HUMAN) | R.FEDGVLDPDYPR.N | 0.64 |
| vitronectin precursor | **P04004** (VTNC_HUMAN) | R.IYISGM*APRPSLAK.K | 0.75 |
| zinc finger protein 142 | **P52746** (ZN142_HUMAN) | K.TRFLLR.T | 0.66 |

**Table 9. Significant peptides (AUC>0.6) forfor X!Tandem only**

| **Protein description** | **Uniprot ID (name)** | **Peptide** | **XT_AUC** |
|---|---|---|---|
| afamin precursor | **P43652** (AFAM_HUMAN) | K.HELTDEELQSLFTNFANVVDK.C | 0.65 |
| afamin precursor | **P43652** (AFAM_HUMAN) | R.NPFVFAPTLLTVAVHFEEVAK.S | 0.91 |
| alpha-1-antichymotrypsin precursor | **P01011** (AACT_HUMAN) | K.ADLSGITGAR.N | 0.67 |
| alpha-1-antichymotrypsin precursor | **P01011** (AACT_HUMAN) | K.MEEVEAMLLPETLKR.W | 0.60 |
| alpha-1-antichymotrypsin precursor | **P01011** (AACT_HUMAN) | K.WEMPFDPQDTHQSR.F | 0.64 |
| alpha-1-antichymotrypsin precursor | **P01011** (AACT_HUMAN) | R.LYGSEAFATDFQDSAAAK.K | 0.62 |
| alpha-1B-glycoprotein precursor | **P04217** (A1BG_HUMAN) | K.HQFLLTGDTQGR.Y | 0.72 |
| alpha-1B-glycoprotein precursor | **P04217** (A1BG_HUMAN) | K.NGVAQEPVHLDSPAIK.H | 0.63 |
| alpha-1B-glycoprotein precursor | **P04217** (A1BG_HUMAN) | K.SLPAPWLSM*APVSWITPGLK.T | 0.72 |
| alpha-1B-glycoprotein precursor | **P04217** (A1BG_HUMAN) | K.VTLTCVAPLSGVDFQLRR.G | 0.67 |
| alpha-1B-glycoprotein precursor | **P04217** (A1BG_HUMAN) | R.C*EGPIPDVTFELLR.E | 0.67 |
| alpha-1B-glycoprotein precursor | **P04217** (A1BG_HUMAN) | R.C*LAPLEGAR.F | 0.79 |
| alpha-1B-glycoprotein precursor | **P04217** (A1BG_HUMAN) | R.CLAPLEGAR.F | 0.63 |
| alpha-1B-glycoprotein precursor | **P04217** (A1BG_HUMAN) | R.GVTFLLR.R | 0.69 |
| alpha-1B-glycoprotein precursor | **P04217** (A1BG_HUMAN) | R.LHDNQNGWSGDSAPVELILSDETL PAPEFSPEPESGR.A | 0.60 |
| alpha-1B-glycoprotein precursor | **P04217** (A1BG_HUMAN) | R.TPGAAANLELIFVGPQHAGNYR.C | 0.62 |
| alpha-2-antiplasmin isoform a precursor | **P08697** (A2AP_HUMAN) | K.HQM*DLVATLSQLGLQELFQAPDL R.G | 0.61 |
| alpha-2-antiplasmin isoform a precursor | **P08697** (A2AP_HUMAN) | R.LCQDLGPGAFR.L | 0.68 |
| alpha-2-antiplasmin isoform a precursor | **P08697** (A2AP_HUMAN) | R.WFLLEQPEIQVAHFPFK.N | 0.60 |
| alpha-2-HS-glycoprotein preproprotein | **P02765** (FETUA_HUMAN) | K.VWPQQPSGELFEIEIDTLETTCHVL DPTPVAR.C | 0.61 |
| alpha-2-HS-glycoprotein preproprotein | **P02765** (FETUA_HUMAN) | R.HTFMGVVSLGSPSGEVSHPR.K | 0.68 |
| alpha-2-HS-glycoprotein preproprotein | **P02765** (FETUA_HUMAN) | R.Q*PNCDDPETEEAALVAIDYINQNL PWGYK.H | 0.69 |
| alpha-2-HS-glycoprotein preproprotein | **P02765** (FETUA_HUMAN) | R.QPNCDDPETEEAALVAIDYINQNLP WGYK.H | 0.64 |
| alpha-2-HS-glycoprotein preproprotein | **P02765** (FETUA_HUMAN) | R.TVVQPSVGAAAGPVVPPCPGR.I | 0.64 |
| angiotensinogen preproprotein | **P01019** (ANGT_HUMAN) | K.QPFVQGLALYTPVVLPR.S | 0.73 |
| angiotensinogen preproprotein | **P01019** (ANGT_HUMAN) | R.AAM*VGM*LANFLGFR.I | 0.62 |
| apolipoprotein A-IV precursor | **P06727** (APOA4_HUMAN) | K.LVPFATELHER.L | 0.64 |
| apolipoprotein A-IV precursor | **P06727** (APOA4_HUMAN) | R.LLPHANEVSQK.I | 0.61 |
| apolipoprotein A-IV precursor | **P06727** (APOA4_HUMAN) | R.SLAPYAQDTQEKLNHQLEGLTFQM K.K | 0.70 |
| apolipoprotein B-100 precursor | **P04114** (APOB_HUMAN) | K.FPEVDVLTK.Y | 0.61 |
| apolipoprotein B-100 precursor | **P04114** (APOB_HUMAN) | K.HINIDQFVR.K | 0.70 |
| apolipoprotein B-100 precursor | **P04114** (APOB_HUMAN) | K.LLSGGNTLHLVSTTK.T | 0.66 |
| apolipoprotein B-100 precursor | **P04114** (APOB_HUMAN) | K.Q*VFLYPEKDEPTYILNIKR.G | 0.81 |
| apolipoprotein B-100 precursor | **P04114** (APOB_HUMAN) | K.QVFLYPEKDEPTYILNIKR.G | 0.77 |
| apolipoprotein B-100 precursor | **P04114** (APOB_HUMAN) | K.SLHMYANR.L | 0.83 |
| apolipoprotein B-100 precursor | **P04114** (APOB_HUMAN) | K.SVSDGIAALDLNAVANK.I | 0.62 |
| apolipoprotein B-100 precursor | **P04114** (APOB_HUMAN) | K.SVSLPSLDPASAKIEGNLIFDPNNYL PK.E | 0.67 |
| apolipoprotein B-100 precursor | **P04114** (APOB_HUMAN) | K.TEVIPPLIENR.Q | 0.63 |
| apolipoprotein B-100 precursor | **P04114** (APOB_HUMAN) | K.VLVDHFGYTK.D | 0.76 |
| apolipoprotein B-100 precursor | **P04114** (APOB_HUMAN) | R.TSSFALNLPTLPEVKFPEVDVLTK.Y | 0.62 |
| apolipoprotein C-III precursor | **P02656** (APOC3_HUMAN) | R.GWVTDGFSSLKDYWSTVK.D | 0.66 |
| apolipoprotein E precursor | **P02649** (APOE_HUMAN) | R.GEVQAMLGQSTEELR.V | 0.81 |
| apolipoprotein E precursor | **P02649** (APOE_HUMAN) | R.LAVYQAGAR.E | 0.63 |
| apolipoprotein E precursor | **P02649** (APOE_HUMAN) | R.LGPLVEQGR.V | 0.69 |
| attractin isoform 2 preproprotein | **O75882** (ATRN_HUMAN) | K.LTLTPWVGLR.K | 0.69 |
| beta-2-glycoprotein 1 precursor | **P02749** (APOH_HUMAN) | K.FICPLTGLWPINTLK.C | 0.63 |
| beta-2-glycoprotein 1 precursor | **P02749** (APOH_HUMAN) | K.TFYEPGEEITYSCKPGYVSR.G | 0.62 |
| beta-Ala-His dipeptidase precursor | **Q96KN2** (CNDP1_HUMAN) | K.MVVSMTLGLHPWIANIDDTQYLA AK.R | 0.81 |
| beta-Ala-His dipeptidase precursor | **Q96KN2** (CNDP1_HUMAN) | K.VFQYIDLHQDEFVQTLK.E | 0.65 |
| biotinidase precursor | **P43251** (BTD_HUMAN) | R.TSIYPFLDFM*PSPQVVR.W | 0.79 |
| carboxypeptidase N catalytic chain precursor | **P15169** (CBPN_HUMAN) | R.ELMLQLSEFLCEEFR.N | 0.61 |
| ceruloplasmin precursor | **P00450** (CERU_HUMAN) | K.AEEEHLGILGPQLHADVGDKVK.I | 0.73 |
| ceruloplasmin precursor | **P00450** (CERU_HUMAN) | K.ALYLQYTDETFR.T | 0.64 |
| ceruloplasmin precursor | **P00450** (CERU_HUMAN) | K.DVDKEFYLFPTVFDENESLLLEDNIR .M | 0.62 |
| ceruloplasmin precursor | **P00450** (CERU_HUMAN) | K.HYYIGIIETTWDYASDHGEK.K | 0.61 |
| ceruloplasmin precursor | **P00450** (CERU_HUMAN) | R.EYTDASFTNRK.E | 0.67 |
| ceruloplasmin precursor | **P00450** (CERU_HUMAN) | R.HYYIAAEEIIWNYAPSGIDIFTK.E | 0.63 |
| ceruloplasmin precursor | **P00450** (CERU_HUMAN) | R.IYHSHIDAPK.D | 0.62 |
| ceruloplasmin precursor | **P00450** (CERU_HUMAN) | R.Q*KDVDKEFYLFPTVFDENESLLLE DNIR.M | 0.74 |
| ceruloplasmin precursor | **P00450** (CERU_HUMAN) | R.QKDVDKEFYLFPTVFDENESLLLED NIR.M | 0.65 |
| ceruloplasmin precursor | **P00450** (CERU_HUMAN) | R.TYYIAAVEVEWDYSPQR.E | 0.90 |
| coagulation factor IX preproprotein | **P00740** (FA9_HUMAN) | R.SALVLQYLR.V | 0.69 |
| coagulation factor V precursor | **P12259** (FA5_HUMAN) | K.EFNPLVIVGLSK.D | 0.61 |
| coagulation factor XII precursor | **P00748** (FA12_HUMAN) | R.NPDNDIRPWCFVLNR.D | 0.65 |
| coagulation factor XII precursor | **P00748** (FA12_HUMAN) | R.VVGGLVALR.G | 0.61 |
| complement C1q subcomponent subunit B precursor | **P02746** (C1QB_HUMAN) | K.NSLLGMEGANSIFSGFLLFPDMEA. - | 0.64 |
| complement C1q subcomponent subunit B precursor | **P02746** (C1QB_HUMAN) | K.VPGLYYFTYHASSR.G | 0.63 |
| complement C1q subcomponent subunit C precursor | **P02747** (C1QC_HUMAN) | R.Q*THQPPAPNSLIR.F | 0.60 |
| complement C1r subcomponent precursor | **P00736** (C1R_HUMAN) | R.LPVANPQACENWLR.G | 0.72 |
| complement C2 isoform 3 | **P06681** (CO2_HUMAN) | K.NQGILEFYGDDIALLK.L | 0.74 |
| complement C2 isoform 3 | **P06681** (CO2_HUMAN) | K.RNDYLDIYAIGVGK.L | 0.61 |
| complement C2 isoform 3 | **P06681** (CO2_HUMAN) | R.QPYSYDFPEDVAPALGTSFSHMLG ATNPTQK.T | 0.78 |
| complement C3 precursor | **P01024** (CO3_HUMAN) | R.IHWESASLLR.S | 0.69 |
| complement C4-A isoform 1 | **P0C0L4** (CO4A_HUMAN) | K.FACYYPR.V | 0.64 |
| complement C4-A isoform 1 | **P0C0L4** (CO4A_HUMAN) | K.LHLETDSLALVALGALDTALYAAGS K.S | 0.74 |
| complement C4-A isoform 1 | **P0C0L4** (CO4A_HUMAN) | K.LVNGQSHISLSK.A | 0.64 |
| complement C4-A isoform 1 | **P0C0L4** (CO4A_HUMAN) | K.M*RPSTDTITVMVENSHGLR.V | 0.60 |
| complement C4-A isoform 1 | **P0C0L4** (CO4A_HUMAN) | K.MRPSTDTITVMVENSHGLR.V | 0.65 |
| complement C4-A isoform 1 | **P0C0L4** (CO4A_HUMAN) | K.SCGLHQLLR.G | 0.74 |
| complement C4-A isoform 1 | **P0C0L4** (CO4A_HUMAN) | K.VGLSGMAIADVTLLSGFHALR.A | 0.61 |
| complement C4-A isoform 1 | **P0C0L4** (CO4A_HUMAN) | K.YVLPNFEVK.I | 0.64 |
| complement C4-A isoform 1 | **P0C0L4** (CO4A_HUMAN) | R.ALEILQEEDLIDEDDIPVR.S | 0.64 |
| complement C4-A isoform 1 | **P0C0L4** (CO4A_HUMAN) | R.ECVGFEAVQEVPVGLVQPASATLY DYYNPER.R | 0.62 |
| complement C4-A isoform 1 | **P0C0L4** (CO4A_HUMAN) | R.EELVYELNPLDHR.G | 0.66 |
| complement C4-A isoform 1 | **P0C0L4** (CO4A_HUMAN) | R.STQDTVIALDALSAYWIASHTTEER. G | 0.70 |
| complement C4-A isoform 1 | **P0C0L4** (CO4A_HUMAN) | R.VGDTLNLNLR.A | 0.79 |
| complement C4-A isoform 1 | **P0C0L4** (CO4A_HUMAN) | R.VHYTVCIWR.N | 0.65 |
| complement C4-B-like preproprotein | **P0C0L5** (CO4B_HUMAN) | K.GLCVATPVQLR.V | 1.00 |
| complement C4-B-like preproprotein | **P0C0L5** (CO4B_HUMAN) | K.KYVLPNFEVK.I | 0.60 |
| complement C4-B-like preproprotein | **P0C0L5** (CO4B_HUMAN) | K.VDFTLSSERDFALLSLQVPLKDAK.S | 0.74 |
| complement C4-B-like preproprotein | **P0C0L5** (CO4B_HUMAN) | R.EMSGSPASGIPVK.V | 0.72 |
| complement C4-B-like preproprotein | **P0C0L5** (CO4B_HUMAN) | R.GCGEQTM*IYLAPTLAASR.Y | 0.75 |
| complement C4-B-like preproprotein | **P0C0L5** (CO4B_HUMAN) | R.NGESVKLHLETDSLALVALGALDTA LYAAGSK.S | 0.85 |
| complement C5 preproprotein | **P01031** (CO5_HUMAN) | R.IPLDLVPK.T | 0.65 |
| complement C5 preproprotein | **P01031** (CO5_HUMAN) | R.SYFPESWLWEVHLVPR.R | 0.63 |
| complement C5 preproprotein | **P01031** (CO5_HUMAN) | R.YGGGFYSTQDTINAIEGLTEYSLLVK .Q | 0.62 |
| complement component C6 precursor | **P13671** (CO6_HUMAN) | K.ENPAVIDFELAPIVDLVR.N | 0.63 |
| complement component C8 alpha chain precursor | **P07357** (CO8A_HUMAN) | K.YNPVVIDFEMQPIHEVLR.H | 0.61 |
| complement component C8 alpha chain precursor | **P07357** (CO8A_HUMAN) | R.HTSLGPLEAK.R | 0.65 |
| complement component C8 beta chain preproprotein | **P07358** (CO8B_HUMAN) | K.C*QHEMDQYWGIGSLASGINLFTN SFEGPVLDHR.Y | 0.61 |
| complement component C8 beta chain preproprotein | **P07358** (CO8B_HUMAN) | K.SGFSFGFK.I | 0.64 |
| complement component C8 beta chain preproprotein | **P07358** (CO8B_HUMAN) | R.DTMVEDLVVLVR.G | 0.77 |
| complement component C8 gamma chain precursor | **P07360** (CO8G_HUMAN) | K.ANFDAQQFAGTWLLVAVGSACR.F | 0.63 |
| complement component C8 gamma chain precursor | **P07360** (CO8G_HUMAN) | R.AEATTLHVAPQGTAMAVSTFR.K | 0.61 |
| complement component C9 precursor | **P02748** (CO9_HUMAN) | R.DVVLTTTFVDDIK.A | 0.73 |
| complement component C9 precursor | **P02748** (CO9_HUMAN) | R.RPWNVASLIYETK.G | 0.66 |
| complement factor B preproprotein | **P00751** (CFAB_HUMAN) | K.ISVIRPSK.G | 0.70 |
| complement factor B preproprotein | **P00751** (CFAB_HUMAN) | K.VASYGVKPR.Y | 0.63 |
| complement factor B preproprotein | **P00751** (CFAB_HUMAN) | R.DFHINLFQVLPWLK.E | 0.68 |
| complement factor B preproprotein | **P00751** (CFAB_HUMAN) | R.DLLYIGK.D | 0.63 |
| complement factor B preproprotein | **P00751** (CFAB_HUMAN) | R.GDSGGPLIVHK.R | 0.63 |
| complement factor B preproprotein | **P00751** (CFAB_HUMAN) | R.LEDSVTYHCSR.G | 0.68 |
| complement factor B preproprotein | **P00751** (CFAB_HUMAN) | R.LPPTTTCQQQK.E | 0.68 |
| complement factor H isoform a precursor | **P08603** (CFAH_HUMAN) | K.CLHPCVISR.E | 0.62 |
| complement factor H isoform a precursor | **P08603** (CFAH_HUMAN) | K.CTSTGWIPAPR.C | 0.74 |
| complement factor H isoform a precursor | **P08603** (CFAH_HUMAN) | K.IDVHLVPDR.K | 0.66 |
| complement factor H isoform a precursor | **P08603** (CFAH_HUMAN) | K.IVSSAMEPDREYHFGQAVR.F | 0.67 |
| complement factor H isoform a precursor | **P08603** (CFAH_HUMAN) | K.SIDVACHPGYALPK.A | 0.67 |
| complement factor H isoform a precursor | **P08603** (CFAH_HUMAN) | K.VSVLCQENYLIQEGEEITCKDGR.W | 0.63 |
| complement factor H isoform a precursor | **P08603** (CFAH_HUMAN) | K.WSSPPQCEGLPCK.S | 0.60 |
| complement factor H isoform a precursor | **P08603** (CFAH_HUMAN) | R.EIMENYNIALR.W | 0.61 |
| complement factor H isoform a precursor | **P08603** (CFAH_HUMAN) | R.RPYFPVAVGK.Y | 0.83 |
| complement factor H isoform a precursor | **P08603** (CFAH_HUMAN) | R.WQSIPLCVEK.I | 0.63 |
| complement factor I preproprotein | **P05156** (CFAI_HUMAN) | R.YQIWTTVVDWIHPDLKR.I | 0.72 |
| corticosteroid-binding globulin precursor | **P08185** (CBG_HUMAN) | K. AVLQLN EEGVDTAGST GVTLN LTSK PIILR.F | 0.61 |
| corticosteroid-binding globulin precursor | **P08185** (CBG_HUMAN) | R.GLASANVDFAFSLYK.H | 0.66 |
| fibrinogen alpha chain isoform alpha-E preproprotein | **P02671** (FIBA_HUMAN) | K.TFPGFFSPMLGEFVSETESR.G | 0.62 |
| gelsolin isoform b | **P06396** (GELS_HUMAN) | K.FDLVPVPTNLYGDFFTGDAYVILK.T | 0.66 |
| gelsolin isoform b | **P06396** (GELS_HUMAN) | K.QTQVSVLPEGGETPLFK.Q | 0.66 |
| gelsolin isoform b | **P06396** (GELS_HUMAN) | K.TPSAAYLWVGTGASEAEK.T | 0.71 |
| gelsolin isoform b | **P06396** (GELS_HUMAN) | R.AQPVQVAEGSEPDGFWEALGGK. A | 0.67 |
| gelsolin isoform b | **P06396** (GELS_HUMAN) | R.IEGSNKVPVDPATYGQFYGGDSYIIL YNYR.H | 0.60 |
| gelsolin isoform b | **P06396** (GELS_HUMAN) | R.VEKFDLVPVPTNLYGDFFTGDAYVI LK.T | 0.73 |
| gelsolin isoform b | **P06396** (GELS_HUMAN) | R.VPFDAATLHTSTAMAAQHGMDD DGTGQK.Q | 0.63 |
| glutathione peroxidase 3 precursor | **P22352** (GPX3_HUMAN) | K.FLVGPDGIPIMR.W | 0.60 |
| hemopexin precursor | **P02790** (HEMO_HUMAN) | K.ALPQPQNVTSLLGCTH.- | 0.63 |
| hemopexin precursor | **P02790** (HEMO_HUMAN) | K.SLGPNSCSANGPGLYLIHGPNLYCY SDVEK.L | 0.68 |
| hemopexin precursor | **P02790** (HEMO_HUMAN) | R.DGWHSWPIAHQWPQGPSAVDAA FSWEEK.L | 0.63 |
| hemopexin precursor | **P02790** (HEMO_HUMAN) | R.GECQAEGVLFFQGDR.E | 0.67 |
| hemopexin precursor | **P02790** (HEMO_HUMAN) | R.GECQAEGVLFFQGDREWFWDLAT GTM*K.E | 0.67 |
| hemopexin precursor | **P02790** (HEMO_HUMAN) | R.LEKEVGTPHGIILDSVDAAFICPGSS R.L | 0.75 |
| hemopexin precursor | **P02790** (HEMO_HUMAN) | R.LWWLDLK.S | 0.62 |
| hemopexin precursor | **P02790** (HEMO_HUMAN) | R.WKNFPSPVDAAFR.Q | 0.68 |
| heparin cofactor 2 precursor | **P05546** (HEP2_HUMAN) | K.DQVNTFDNIFIAPVGISTAMGMISL GLK.G | 0.60 |
| insulin-like growth factor-binding protein complex acid labile subunit isoform 2 precursor | **P35858** (ALS_HUMAN) | K.ANVFVQLPR.L | 0.71 |
| insulin-like growth factor-binding protein complex acid labile subunit isoform 2 precursor | **P35858** (ALS_HUMAN) | R.LEALPNSLLAPLGR.L | 0.61 |
| insulin-like growth factor-binding protein complex acid labile subunit isoform 2 precursor | **P35858** (ALS_HUMAN) | R.LFQGLGK.L | 0.68 |
| insulin-like growth factor-binding protein complex acid labile subunit isoform 2 precursor | **P35858** (ALS_HUMAN) | R.NLIAAVAPGAFLGLK.A | 0.76 |
| insulin-like growth factor-binding protein complex acid labile subunit isoform 2 precursor | **P35858** (ALS_HUMAN) | R.TFTPQPPGLER.L | 0.73 |
| inter-alpha-trypsin inhibitor heavy chain H1 isoform a precursor | **P19827** (ITIH1_HUMAN) | K.Q*LVHHFEIDVDIFEPQGISK.L | 0.69 |
| inter-alpha-trypsin inhibitor heavy chain H1 isoform a precursor | **P19827** (ITIH1_HUMAN) | K.VTFQLTYEEVLK.R | 0.61 |
| inter-alpha-trypsin inhibitor heavy chain H1 isoform a precursor | **P19827** (ITIH1_HUMAN) | K.VTFQLTYEEVLKR.N | 0.70 |
| inter-alpha-trypsin inhibitor heavy chain H1 isoform a precursor | **P19827** (ITIH1_HUMAN) | R.GIEILNQVQESLPELSNHASILIMLT DGDPTEGVTDR.S | 0.62 |
| inter-alpha-trypsin inhibitor heavy chain H1 isoform a precursor | **P19827** (ITIH1_HUMAN) | R.GM*ADQDGLKPTIDKPSEDSPPLE M*LGPR.R | 0.79 |
| inter-alpha-trypsin inhibitor heavy chain H1 isoform a precursor | **P19827** (ITIH1_HUMAN) | R.KAAISGENAGLVR.A | 0.78 |
| inter-alpha-trypsin inhibitor heavy chain H2 precursor | **P19823** (ITIH2_HUMAN) | K.AGELEVFNGYFVHFFAPDNLDPIPK .N | 0.64 |
| inter-alpha-trypsin inhibitor heavy chain H2 precursor | **P19823** (ITIH2_HUMAN) | K.FYNQVSTPLLR.N | 0.68 |
| inter-alpha-trypsin inhibitor heavy chain H2 precursor | **P19823** (ITIH2_HUMAN) | K.VQFELHYQEVK.W | 0.68 |
| inter-alpha-trypsin inhibitor heavy chain H2 precursor | **P19823** (ITIH2_HUMAN) | R.ETAVDGELVVLYDVK.R | 0.63 |
| inter-alpha-trypsin inhibitor heavy chain H2 precursor | **P19823** (ITIH2_HUMAN) | R.IYLQPGR.L | 0.75 |
| inter-alpha-trypsin inhibitor heavy chain H3 preproprotein | **Q06033** (ITIH3_HUMAN) | R.LWAYLTIEQLLEK.R | 0.60 |
| inter-alpha-trypsin inhibitor heavy chain H4 isoform 1 precursor | **Q14624** (ITIH4_HUMAN) | K.ITFELVYEELLK.R | 0.60 |
| inter-alpha-trypsin inhibitor heavy chain H4 isoform 1 precursor | **Q14624** (ITIH4_HUMAN) | K.LQDRGPDVLTATVSGK.L | 0.67 |
| inter-alpha-trypsin inhibitor heavy chain H4 isoform 1 precursor | **Q14624** (ITIH4_HUMAN) | K.TGLLLLSDPDKVTIGLLFWDGRGEG LR.L | 0.63 |
| inter-alpha-trypsin inhibitor heavy chain H4 isoform 1 precursor | **Q14624** (ITIH4_HUMAN) | K.WKETLFSVM*PGLK.M | 0.79 |
| inter-alpha-trypsin inhibitor heavy chain H4 isoform 1 precursor | **Q14624** (ITIH4_HUMAN) | R.AISGGSIQIENGYFVHYFAPEGLTT M*PK.N | 0.60 |
| inter-alpha-trypsin inhibitor heavy chain H4 isoform 1 precursor | **Q14624** (ITIH4_HUMAN) | R.AISGGSIQIENGYFVHYFAPEGLTT MPK.N | 0.65 |
| inter-alpha-trypsin inhibitor heavy chain H4 isoform 1 precursor | **Q14624** (ITIH4_HUMAN) | R.ANTVQEATFQMELPK.K | 0.68 |
| inter-alpha-trypsin inhibitor heavy chain H4 isoform 1 precursor | **Q14624** (ITIH4_HUMAN) | R.SFAAGIQALGGTNINDAMLMAVQ LLDSSNQEER.L | 0.64 |
| inter-alpha-trypsin inhibitor heavy chain H4 isoform 1 precursor | **Q14624** (ITIH4_HUMAN) | R.VQGNDHSATR.E | 0.63 |
| inter-alpha-trypsin inhibitor heavy chain H4 isoform 2 precursor | **Q14624** (ITIH4_HUMAN) | K.ITFELVYEELLKR.R | 0.60 |
| inter-alpha-trypsin inhibitor heavy chain H4 isoform 2 precursor | **Q14624** (ITIH4_HUMAN) | K.VTIGLLFWDGR.G | 0.65 |
| inter-alpha-trypsin inhibitor heavy chain H4 isoform 2 precursor | **Q14624** (ITIH4_HUMAN) | R.LWAYLTIQQLLEQTVSASDADQQA LR.N | 0.68 |
| kallistatin precursor | **P29622** (KAIN_HUMAN) | K.LFHTNFYDTVGTIQLINDHVK.K | 0.73 |
| kininogen-1 isoform 2 precursor | **P01042** (KNG1_HUMAN) | K.ENFLFLTPDCK.S | 0.64 |
| kininogen-1 isoform 2 precursor | **P01042** (KNG1_HUMAN) | K.IYPTVNCQPLGMISLMK.R | 0.64 |
| kininogen-1 isoform 2 precursor | **P01042** (KNG1_HUMAN) | K.KIYPTVNCQPLGMISLMK.R | 0.78 |
| kininogen-1 isoform 2 precursor | **P01042** (KNG1_HUMAN) | K.SLWNGDTGECTDNAYIDIQLR.I | 0.67 |
| lumican precursor | **P51884** (LUM_HUMAN) | K.ILGPLSYSK.I | 0.60 |
| N-acetylmuramoyl-L-alanine amidase precursor | **Q96PD5** (PGRP2_HUMAN) | K.EYGVVLAPDGSTVAVEPLLAGLEAG LQGR.R | 0.61 |
| N-acetylmuramoyl-L-alanine amidase precursor | **Q96PD5** (PGRP2_HUMAN) | R.EGKEYGVVLAPDGSTVAVEPLLAGL EAGLQGR.R | 0.69 |
| N-acetylmuramoyl-L-alanine amidase precursor | **Q96PD5** (PGRP2_HUMAN) | R.Q*NGAALTSASILAQQVWGTLVLL QR.L | 0.60 |
| pigment epithelium-derived factor precursor | **P36955** (PEDF_HUMAN) | K.IAQLPLTGSMSIIFFLPLK.V | 0.65 |
| pigment epithelium-derived factor precursor | **P36955** (PEDF_HUMAN) | R.SSTSPTTNVLLSPLSVATALSALSLG AEQR.T | 0.79 |
| plasma kallikrein preproprotein | **P03952** (KLKB1_HUMAN) | K.VAEYMDWILEK.T | 0.62 |
| plasma kallikrein preproprotein | **P03952** (KLKB1_HUMAN) | R.C*LLFSFLPASSINDMEKR.F | 0.60 |
| plasma kallikrein preproprotein | **P03952** (KLKB1_HUMAN) | R.C*QFFSYATQTFHK.A | 0.60 |
| plasma kallikrein preproprotein | **P03952** (KLKB1_HUMAN) | R.CLLFSFLPASSINDMEK.R | 0.76 |
| plasma protease C1 inhibitor precursor | **P05155** (IC1_HUMAN) | R.LVLLNAIYLSAK.W | 0.96 |
| pregnancy zone protein precursor | **P20742** (PZP_HUMAN) | R.NALFCLESAWNVAK.E | 0.67 |
| pregnancy zone protein precursor | **P20742** (PZP_HUMAN) | R.NQGNTWLTAFVLK.T | 0.61 |
| pregna ncy-specific beta-1-glycoprotein 9 precursor | **Q00887** (PSG9_HUMAN) | R.SNPVILNVLYGPDLPR.I | 0.62 |
| prenylcysteine oxidase 1 precursor | **Q9UHG3** (PCYOX_HUMAN) | K.IAIIGAGIGGTSAAYYLR.Q | 0.71 |
| protein AMBP preproprotein | **P02760** (AMBP_HUMAN) | K.WYNLAIGSTCPWLK.K | 0.77 |
| protein AMBP preproprotein | **P02760** (AMBP_HUMAN) | R.TVAACNLPIVR.G | 0.66 |
| prothrombin preproprotein | **P00734** (THRB_HUMAN) | R.IVEGSDAEIGMSPWQVMLFR.K | 0.62 |
| prothrombin preproprotein | **P00734** (THRB_HUMAN) | R.RQECSIPVCGQDQVTVAMTPR.S | 0.69 |
| prothrombin preproprotein | **P00734** (THRB_HUMAN) | R.TFGSGEADCGLRPLFEK.K | 0.61 |
| retinol-binding protein 4 precursor | **P02753** (RET4_HUMAN) | R.FSGTWYAMAK.K | 0.60 |
| retinol-binding protein 4 precursor | **P02753** (RET4_HUMAN) | R.LLNNWDVCADMVGTFTDTEDPAK .F | 0.64 |
| serum amyloid P-component precursor | **P02743** (SAMP_HUMAN) | R.GYVIIKPLVWV.- | 0.62 |
| sex hormone-binding globulin isoform 1 precursor | **P04278** (SHBG_HUMAN) | K.VVLSSGSGPGLDLPLVLGLPLQLK.L | 0.60 |
| sex hormone-binding globulin isoform 1 precursor | **P04278** (SHBG_HUMAN) | R.TWDPEGVIFYGDTNPKDDWFM*L GLR.D | 0.75 |
| sex hormone-binding globulin isoform 1 precursor | **P04278** (SHBG_HUMAN) | R.TWDPEGVIFYGDTNPKDDWFMLG LR.D | 0.74 |
| thrombospondin-1 precursor | **P07996** (TSP1_HUMAN) | K.GFLLLASLR.Q | 0.70 |
| thyroxine-binding globulin precursor | **P05543** (THBG_HUMAN) | K.AVLHIGEK.G | 0.85 |
| thyroxine-binding globulin precursor | **P05543** (THBG_HUMAN) | K.FSISATYDLGATLLK.M | 0.65 |
| thyroxine-binding globulin precursor | **P05543** (THBG_HUMAN) | K.KELELQIGNALFIGK.H | 0.61 |
| thyroxine- binding globulin precursor | **P05543** (THBG_HUMAN) | K.MSSINADFAFNLYR.R | 0.67 |
| transforming growth factor-beta-induced protein ig-h3 precursor | **Q15582** (BGH3_HUMAN) | R.LTLLAPLNSVFK.D | 0.65 |
| transthyretin precursor | **P02766** (TTHY_HUMAN) | R.GSPAINVAVHVFR.K | 0.67 |
| uncharacterized protein C3orf20 isoform 1 | **Q8ND61** (CC020_HUMAN) | K.MPSHLMLAR.K | 0.64 |
| vitamin D-binding protein isoform 1 precursor | **P02774** (VTDB_HUMAN) | K.ELPEHTVK.L | 0.75 |
| vitamin D-binding protein isoform 1 precursor | **P02774** (VTDB_HUMAN) | K.EYANQFMWEYSTNYGQAPLSLLVS YTK.S | 0.69 |
| vitamin D-binding protein isoform 1 precursor | **P02774** (VTDB_HUMAN) | K.HLSLLTTLSNR.V | 0.65 |
| vitamin D-binding protein isoform 1 precursor | **P02774** (VTDB_HUMAN) | K.HQPQEFPTYVEPTNDEICEAFR.K | 0.64 |
| vitamin D-binding protein isoform 1 precursor | **P02774** (VTDB_HUMAN) | K.LAQKVPTADLEDVLPLAEDITNILSK. C | 0.73 |
| vitamin D-binding protein isoform 1 precursor | **P02774** (VTDB_HUMAN) | K.LCDNLSTK.N | 0.70 |
| vitamin D-binding protein isoform 1 precursor | **P02774** (VTDB_HUMAN) | K.LCMAALK.H | 0.63 |
| vitamin D-binding protein isoform 1 precursor | **P02774** (VTDB_HUMAN) | K.SCESNSPFPVHPGTAECCTK.E | 0.63 |
| vitamin D-binding protein isoform 1 precursor | **P02774** (VTDB_HUMAN) | K.SYLSMVGSCCTSASPTVCFLK.E | 0.61 |
| vitamin D-binding protein isoform 1 precursor | **P02774** (VTDB_HUMAN) | K.TAMDVFVCTYFM*PAAQLPELPDV ELPTNK.D | 0.61 |
| vitamin D-binding protein isoform 1 precursor | **P02774** (VTDB_HUMAN) | K.VLEPTLK.S | 0.69 |
| vitamin D-binding protein isoform 1 precursor | **P02774** (VTDB_HUMAN) | R.KFPSGTFEQVSQLVK.E | 0.66 |
| vitamin D-binding protein isoform 1 precursor | **P02774** (VTDB_HUMAN) | R.THLPEVFLSK.V | 0.62 |
| vitamin D-binding protein isoform 1 precursor | **P02774** (VTDB_HUMAN) | R.TSALSAK.S | 0.74 |
| vitronectin precursor | **P04004** (VTNC_HUMAN) | R.GQYCYELDEK.A | 0.73 |
| vitronectin precursor | **P04004** (VTNC_HUMAN) | R.M*DWLVPATCEPIQSVFFFSGDK.Y | 0.64 |
| vitronectin precursor | **P04004** (VTNC_HUMAN) | R.Q*PQFISR.D | 0.63 |

**Table 10. Significant peptides (AUC>0.6) for both X!Tandem and Sequest**

| **Protein description** | **Uniprot ID (name)** | **Peptide** | **XT_AUC** | **S_AUC** |
|---|---|---|---|---|
| afamin precursor | P43652 (AFAM_HUMAN ) | K.HFQNLGK.D | 0.74 | 0.61 |
| afamin precursor | P43652 (AFAM_HUMAN ) | R.RHPDLSIPELL R.I | 0.67 | 0.63 |
| afamin precursor | P43652 (AFAM_ HUMAN | R.TINPAVDHCC K.T | 0.66 | 0.86 |
| | ) | | | |
| alpha- 1 -antichymotrypsin precursor | P01011 (AACT _HUMAN) | K.ITDLIKDLDSQ TMMVLVNYIFF K.A | 0.71 | 0.73 |
| alpha-1-antichymotrypsin precursor | P01011 (AACT HUMAN) | R.DYNLNDILLQ LGIEEAFTSK.A | 0.74 | 0.62 |
| alpha-1-antichymotrypsin precursor | P01011 (AACT _HUMAN) | R.GTHVDLGLAS ANVDFAFSLYK. Q | 0.76 | 0.61 |
| alpha-1B-glycoprotein precursor | P04217 (A1BG HUMAN) | K.SLPAPWLSMA PVSWITPGLK.T | 0.71 | 0.65 |
| alpha-2-antiplasmin isoform a precursor | P08697 (A2AP _HUMAN) | K.GFPIKEDFLEQ SEQLFGAKPVSL TGK.Q | 0.66 | 0.69 |
| alpha-2-antiplasmin isoform a precursor | P08697 (A2AP _HUMAN) | K.HQMDLVATL SQLGLQELFQAP DLR.G | 0.67 | 0.60 |
| alpha-2-antiplasmin isoform a precursor | P08697 (A2AP HUMAN) | R.QLTSGPNQEQ VSPLTLLK.L | 0.66 | 0.61 |
| alpha-2-HS-glycoprotein preproprotein | P02765 (FETUA_ HUMA N) | R.AQLVPLPPST YVEFTVSGTDC VAK.E | 0.64 | 0.63 |
| angiotensinogen preproprotein | P01019 (ANGT _HUMAN) | K.DPTFIPAPIQA K.T | 0.69 | 0.69 |
| angiotensinogen preproprotein | P01019 (ANGT _HUMAN) | R.FM*QAVTGW K.T | 0.65 | 0.65 |
| antithrombin-III precursor | P01008 (ANT3 _HUMAN) | K.ANRPFL VFIR. E | 0.72 | 0.60 |
| antithrombin-III precursor | P01008 (ANT3_ HUMAN) | K.GDDITMVLIL PKPEK.S | 0.69 | 0.68 |
| antithrombin-III precursor | P01008 (ANT3_ HUMAN) | R.DIPMNPMCIY R.S | 0.63 | 0.78 |
| apolipoprotein A-IV precursor | P06727 (APOA4_HUMA N) | K.KLVPFATELH ER.L | 0.65 | 0.77 |
| apolipoprotein A-IV precursor | P06727 (APOA4_HUMA N) | K.SLAELGGHLD QQVEEFR.R | 0.60 | 0.75 |
| apolipoprotein B-100 precursor | P04114 (APOB_ HUMAN) | K.ALYWVNGQV PDGVSK.V | 0.61 | 0.63 |
| apolipoprotein B-100 precursor | P04114 (APOB_ HUMAN) | K.FIIPGLK.L | 0.64 | 0.68 |
| apolipoprotein B-100 precursor | P04114 (APOB_ HUMAN) | K.FSVPAGIVIPS FQALTAR.F | 0.63 | 0.63 |
| apolipoprotein B-100 precursor | P04114 (APOB_ HUMAN) | K.IEGNLIFDPNN YLPK.E | 0.63 | 0.65 |
| apolipoprotein B-100 precursor | P04114 (APOB_ HUMAN) | K.LNDLNSVLV MPTFHVPFTDL | 0.91 | 0.88 |
| | | QVPSCK.L | | |
| apolipoprotein B-100 precursor | P04114 (APOB_ HUMAN) | K.VELEVPQLCS FILK.T | 0.60 | 0.61 |
| apolipoprotein B-100 precursor | P04114 (APOB_ HUMAN) | K.VNWEEEAAS GLLTSLK.D | 0.60 | 0.73 |
| apolipoprotein B-100 precursor | P04114 (APOB_ HUMAN) | R.ATLYALSHAV NNYHK.T | 0.78 | 0.80 |
| apolipoprotein B-100 precursor | P04114 (APOB_ HUMAN) | R.TGISPLALIK.G | 0.64 | 0.77 |
| apolipoprotein B-100 precursor | P04114 (APOB_ HUMAN) | R.TLQGIPQMIG EVIR.K | 0.65 | 0.66 |
| apolipoprotein C-III precursor | P02656 (APOC3_HUMA N) | K.DALSSVQESQ VAQQAR.G | 0.80 | 0.69 |
| apolipoprotein C-IV precursor | P55056 (APOC4_HUMA N) | R.DGWQWFWSP STFR.G | 0.63 | 0.67 |
| apolipoprotein E precursor | P02649 (APOE_ HUMAN) | K.VQAAVGTSA APVPSDNH.- | 0.70 | 0.72 |
| apolipoprotein E precursor | P02649 (APOE_ HUMAN) | R.WELALGR.F | 0.88 | 0.60 |
| beta-2-microglobulin precursor | P61769 (B2MG _HUMAN) | K.SNFLNCYVSG FHPSDIEVDLLK. N | 0.60 | 0.70 |
| bone marrow proteoglycan isoform 1 preproprotein | P13727 (PRG2 _HUMAN) | R.GGHCVALCT R.G | 0.83 | 0.86 |
| carboxypeptidase B2 preproprotein | Q96IY4 (CBPB2_HUMAN ) | R.LVDFYVMPV VNVDGYDYSW K.K | 0.61 | 0.65 |
| carboxypeptidase B2 preproprotein | Q96IY4 (CBPB2_HUMAN ) | R.YTHGHGSETL YLAPGGGDDWI YDLGIK.Y | 0.60 | 0.68 |
| carboxypeptidase N subunit 2 precursor | P22792 (CPN2_ HUMAN) | K.LSNNALSGLP QGVFGK.L | 0.65 | 0.67 |
| carboxypeptidase N subunit 2 precursor | P22792 (CPN2_ _HUMAN) | K.TLNLAQNLLA QLPEELFHPLTS LQTLK.L | 0.67 | 0.69 |
| carboxypeptidase N subunit 2 precursor | P22792 (CPN2_ HUMAN) | R.WLNVQLSPR. Q | 0.74 | 0.67 |
| ceruloplasmin precursor | P00450 (CERU_ HUMAN) | K.GDSVVWYLF SAGNEADVHGI YFSGNTYL WR. G | 0.90 | 0.72 |
| ceruloplasmin precursor | P00450 (CERU_ HUMAN) | K.MYYSAVDPT K.D | 0.70 | 0.82 |
| ceruloplasmin precursor | P00450 (CERU_ HUMAN) | R.GPEEEHLGIL GPVIWAEVGDTI | 0.60 | 0.65 |
| | | R.V | | |
| ceruloplasmin precursor | P00450 (CERU_ HUMAN) | R.IDTINLFPATL FDAYMVAQNP GEWMLSCQNL NHLK.A | 0.66 | 0.70 |
| ceruloplasmin precursor | P00450 (CERU_ HUMAN) | R.SGAGTEDSAC IPWAYYSTVDQ YSGLIGPL IVCR.R | 0.88 | 0.92 |
| cholinesterase precursor | P06276 (CHLE_ HUMAN) | K.IFFPGVSEFGK .E | 0.70 | 0.63 |
| cholinesterase precursor | P06276 (CHLE_ _HUMAN) | R.AILQSGSFNAP WAVTSLYEAR. N | 0.75 | 0.77 |
| chorionic gonadotropin, beta polypeptide 8 precursor | P01233 (CGHB _HUMAN) | R.VLQGVLPALP QVVCNYR.D | 0.60 | 0.75 |
| chorionic somatomammotropin hormone 2 isoform 2 precursor | P01243 (CSH_HUMAN) | R.ISLLLIESWLE PVR.F | 0.83 | 0.63 |
| coagulation factor XII precursor | P00748 (FA12_ HUMAN) | R.LHEAFSPVSY QHDLALLR.L | 0.60 | 0.66 |
| coagulation factor XII precursor | P00748 (FA12 HUMAN) | R.TTLSGAPCQP WASEATYR.N | 0.69 | 0.82 |
| complement C1q subcomponent subunit A precursor | P02745 (C1QA_HUMAN) | K.GLFQVVSGG MVLQLQQGDQ VWVEKDPK.K | 0.65 | 0.60 |
| complement C1r subcomponent precursor | P00736 (C1R_HUMAN) | K.VLNYVDWIK K.E | 0.80 | 0.76 |
| complement C1s subcomponent precursor | P09871 (C1S_ HUMAN) | K.SNALDIIFQTD LTGQK.K | 0.62 | 0.77 |
| complement C4-A isoform 1 | P0C0L4 (CO4A_ HUMAN) | K.EGAIHREELV YELNPLDHR.G | 0.76 | 0.75 |
| complement C4-A isoform 1 | P0C0L4 (CO4A_ HUMAN) | K.ITQVLHFTK.D | 0.63 | 0.62 |
| complement C4-A isoform 1 | P0C0L4 (CO4A_ HUMAN) | K.SHALQLNNR. Q | 0.66 | 0.71 |
| complement C4-A isoform 1 | P0C0L4 (CO4A_ HUMAN) | R.AVGSGATFSH YYYM*ILSR.G | 0.65 | 0.60 |
| complement C4-A isoform 1 | P0C0L4 (CO4A_ HUMAN) | R.EPFLSCCQFA ESLR.K | 0.64 | 0.72 |
| complement C4-A isoform 1 | P0C0L4 (CO4A_ HUMAN) | R.GHLFLQTDQP IYNPGQR.V | 0.63 | 0.76 |
| complement C4-A isoform 1 | P0C0L4 (CO4A_ HUMAN) | R.GLEEELQFSL GSK.I | 0.68 | 0.68 |
| complement C4-A isoform 1 | P0C0L4 (CO4A_ HUMAN) | R.GSFEFPVGDA VSK.V | 0.67 | 0.70 |
| complement C4-A isoform 1 | P0C0L4 (CO4A_ HUMAN) | R.LLATLCSAEV CQCAEGK.C | 0.61 | 0.71 |
| complement C4-A isoform 1 | P0C0L4 (CO4A_ HUMAN) | R.VQQPDCREPF LSCCQFAESLRK .K | 0.65 | 0.83 |
| complement C4-A isoform 1 | P0C0L4 (CO4A_ HUMAN) | R.YIYGKPVQGV AYVR.F | 0.82 | 0.76 |
| complement C5 preproprotein | P01031 (CO5_ HUMAN) | K.ITHYNYLILSK .G | 0.66 | 0.69 |
| complement C5 preproprotein | P01031 (CO5_ HUMAN) | R.ENSLYLTAFT VIGIR.K | 0.60 | 0.68 |
| complement C5 preproprotein | P01031 (CO5_ HUMAN) | R.KAFDICPLVK. I | 0.77 | 0.65 |
| complement C5 preproprotein | P01031 (CO5_ _HUMAN) | R.VDDGVASFVL NLPSGVTVLEFN VK.T | 0.68 | 0.61 |
| complement component C6 precursor | P13671 (CO6 _HUMAN) | K.TFSEWLESVK ENPAVIDFELAP IVDLVR.N | 0.94 | 0.64 |
| complement component C6 precursor | P13671 (CO6_ _HUMAN) | R.IFDDFGTHYF TSGSLGGVYDL YQFSSEELK.N | 0.78 | 0.75 |
| complement component C7 precursor | P10643 (CO7_ HUMAN) | K.ELSHLPSLYD YSAYR.R | 0.69 | 0.71 |
| complement component C7 precursor | P10643 (CO7_ HUMAN) | R.RYSAWAESV TNLPQVIK.Q | 0.71 | 0.70 |
| complement component C8 alpha chain precursor | P07357 (CO8A_ HUMAN) | K.YNPVVIDFEM *QPIHEVLR.H | 0.68 | 0.73 |
| complement component C8 beta chain preproprotein | P07358 (CO8B_HUMAN) | K.VEPLYELVTA TDFAYSSTVR.Q | 0.69 | 0.70 |
| complement component C8 beta chain preproprotein | P07358 (CO8B_HUMAN) | R.SLM*LHYEFL QR.V | 0.61 | 0.65 |
| complement component C8 gamma chain precursor | P07360 (CO8G _HUMAN) | K.YGFCEAADQF HVLDEVRR.- | 0.78 | 0.76 |
| complement component C8 gamma chain precursor | P07360 (CO8G_HUMAN) | R.FLQEQGHR.A | 0.63 | 0.69 |
| complement component C8 gamma chain precursor | P07360 (CO8G_HUMAN) | R.KLDGICWQV R.Q | 0.75 | 0.70 |
| complement component C8 gamma chain precursor | P07360 (CO8G _HUMAN) | R.SLPVSDSVLS GFEQR.V | 0.70 | 0.60 |
| complement component C9 precursor | P02748 (C09 HUMAN) | R.GTVIDVTDFV NWASSINDAPV | 0.68 | 0.69 |
| | | LISQK.L | | |
| complement factor B preproprotein | P00751 (CFAB _HUMAN) | K.NPREDYLDV YVFGVGPLVNQ VNINALASK.K | 0.72 | 0.77 |
| complement factor B preproprotein | P00751 (CFAB HUMAN) | R.GDSGGPLIVH KR.S | 0.60 | 0.76 |
| complement factor B preproprotein | P00751 (CFAB _HUMAN) | R.HVIILMTDGL HNM*GGDPITVI DEIR.D | 0.60 | 0.64 |
| complement factor B preproprotein | P00751 (CFAB _HUMAN) | R.KNPREDYLDV YVFGVGPLVNQ VNINALASK.K | 0.63 | 0.63 |
| complement factor H isoform a precursor | P08603 (CFAH_ HUMAN) | K.SCDIPVFMNA R.T | 0.62 | 0.71 |
| complement factor H isoform a precursor | P08603 (CFAH_ _HUMAN) | K.SPPEISHGVV AHMSDSYQYGE EVTYK.C | 0.88 | 0.88 |
| complement factor H isoform a precursor | P08603 (CFAH_ HUMAN) | K.TDCLSLPSFE NAIPMGEKK.D | 0.61 | 0.66 |
| complement factor I preproprotein | P05156 (CFAI_ HUMAN) | K.RAQLGDLPW QVAIK.D | 0.71 | 0.74 |
| complement factor I preproprotein | P05156 (CFAI_ HUMAN) | K.SLECLHPGTK. F | 0.64 | 0.81 |
| complement factor I preproprotein | P05156 (CFAI_ HUMAN) | R.TMGYQDFAD VVCYTQK.A | 0.73 | 0.75 |
| extracellular matrix protein 1 isoform 3 precursor | Q16610 (ECM1_HUMAN) | R.ELLALIQLER. E | 0.69 | 0.65 |
| gelsolin isoform a precursor | P06396 (GELS_ HUMAN) | R.VPEARPNSMV VEHPEFLK.A | 0.76 | 0.62 |
| glutathione peroxidase 3 precursor | P22352 (GPX3_ HUMAN) | R.LFWEPMK.V | 0.69 | 0.67 |
| hemopexin precursor | P02790 (HEMO_HUMAN ) | R.DVRDYFMPCP GR.G | 0.70 | 0.72 |
| heparin cofactor 2 precursor | P05546 (HEP2 _HUMAN) | K.DALENIDPAT QMMILNCIYFK. G | 0.61 | 0.65 |
| heparin cofactor 2 precursor | P05546 (HEP2_HUMAN) | K.GLIKDALENI DPATQMMILNC IYFK.G | 0.64 | 0.64 |
| heparin cofactor 2 precursor | P05546 (HEP2 HUMAN) | K.QFPILLDFK.T | 0.61 | 0.69 |
| heparin cofactor 2 precursor | P05546 (HEP2 _HUMAN) | R.VLKDQVNTF DNIFIAPVGISTA MGMISLGLK.G | 0.88 | 0.75 |
| insulin-like growth factor-binding protein | P35858 (ALS_ HUMAN) | R.AFWLDVSHN R.L | 0.61 | 0.82 |
| complex acid labile subunit isoform 2 precursor | | | | |
| inter-alpha-trypsin inhibitor heavy chain H1 isoform a precursor | P19827 (ITIH1_ HUMAN) | K.ADVQAHGEG QEFSITCLVDEE EMKK.L | 0.61 | 0.74 |
| inter-alpha-trypsin inhibitor heavy chain H1 isoform a precursor | P19827 (ITIH1_ HUMAN) | K.ILGDM*QPGD YFDLVLFGTR.V | 0.71 | 0.63 |
| inter-alpha-trypsin inhibitor heavy chain H1 isoform a precursor | P19827 (ITIH1_ HUMAN) | K.ILGDMQPGDY FDLVLFGTR.V | 0.68 | 0.60 |
| inter-alpha-trypsin inhibitor heavy chain H1 isoform a precursor | P19827 (ITIH1_HUMAN) | K.NVVFVIDISGS MR.G | 0.76 | 0.83 |
| inter-alpha-trypsin inhibitor heavy chain H1 isoform a precursor | P19827 (ITIH1_ HUMAN) | K.TAFISDFAVT ADGNAFIGDIKD K.V | 0.74 | 0.63 |
| inter-alpha-trypsin inhibitor heavy chain H1 isoform a precursor | P19827 (ITIH1_ HUMAN) | R.GHMLENHVE R.L | 0.78 | 0.80 |
| inter-alpha-trypsin inhibitor heavy chain H1 isoform a precursor | P19827 (ITIH1_ HUMAN) | R.GM*ADQDGL KPTIDKPSEDSP PLEMLGPR.R | 0.61 | 0.62 |
| inter-alpha-trypsin inhibitor heavy chain H1 isoform a precursor | P19827 (ITIH1_ HUMAN) | R.LWAYLTIQEL LAK.R | 0.68 | 0.62 |
| inter-alpha-trypsin inhibitor heavy chain H1 isoform a precursor | P19827 (ITIH1_ HUMAN) | R.NHM*QYEIVI K.V | 0.67 | 0.65 |
| inter-alpha-trypsin inhibitor heavy chain H2 precursor | P19823 (ITIH2 _HUMAN) | K.AHVSFKPTVA QQR.I | 0.75 | 0.61 |
| inter-alpha-trypsin inhibitor heavy chain H2 precursor | P19823 (ITIH2 _HUMAN) | K.ENIQDNISLFS LGM*GFDVDYD FLKR.L | 0.80 | 0.93 |
| inter-alpha-trypsin inhibitor heavy chain H2 precursor | P19823 (ITIH2_HUMAN) | K.ENIQDNISLFS LGMGFDVDYDF LKR.L | 0.63 | 0.80 |
| inter-alpha-trypsin inhibitor heavy chain H2 precursor | P19823 (ITIH2 _HUMAN) | K.HLEVDVWVIE PQGLR.F | 0.61 | 0.61 |
| inter-alpha-trypsin inhibitor heavy chain H2 precursor | P19823 (ITIH2 _HUMAN) | K.LWAYLTINQL LAER.S | 0.69 | 0.62 |
| inter-alpha-trypsin inhibitor heavy chain H2 precursor | P19823 (ITIH2 _HUMAN) | R.AEDHFSVIDF NQNIR.T | 0.65 | 0.63 |
| inter-alpha-trypsin inhibitor heavy chain H2 precursor | P19823 (ITIH2 _HUMAN) | R.FLHVPDTFEG HFDGVPVISK.G | 0.66 | 0.62 |
| inter-alpha-trypsin inhibitor heavy chain H4 isoform 1 precursor | Q14624 (ITIH4_HUMAN) | K.ILDDLSPR.D | 0.67 | 0.65 |
| inter-alpha-trypsin inhibitor heavy chain H4 isoform 1 precursor | Q14624 (ITIH4_HUMAN) | K.IPKPEASFSPR. R | 0.69 | 0.77 |
| inter-alpha-trypsin inhibitor heavy chain H4 isoform 1 precursor | Q14624 (ITIH4 _HUMAN) | K.SPEQQETVLD GNLIIR.Y | 0.63 | 0.69 |
| inter-alpha-trypsin inhibitor heavy chain H4 isoform 1 precursor | Q14624 (ITIH4_HUMAN) | K.YIFHNFMER.L | 0.66 | 0.61 |
| inter-alpha-trypsin inhibitor heavy chain H4 isoform 1 precursor | Q14624 (ITIH4 _HUMAN) | R.FSSHVGGTLG QFYQEVLWGSP AASDDGRR.T | 0.69 | 0.71 |
| inter-alpha-trypsin inhibitor heavy chain H4 isoform 1 precursor | Q14624 (ITIH4_HUMAN) | R.GPDVLTATVS GK.L | 0.63 | 0.82 |
| inter-alpha-trypsin inhibitor heavy chain H4 isoform 1 precursor | Q14624 (ITIH4 _HUMAN) | R.NMEQFQVSVS VAPNAK.I | 0.78 | 0.60 |
| inter-alpha-trypsin inhibitor heavy chain H4 isoform 1 precursor | Q14624 (ITIH4 _HUMAN) | R.RLDYQEGPPG VEISCWSVEL.- | 0.68 | 0.62 |
| kallistatin precursor | P29622 (KAIN HUMAN) | K.IVDL VSELKK. D | 0.75 | 0.67 |
| kallistatin precursor | P29622 (KAIN_ HUMAN) | R.VGSALFLSHN LK.F | 0.70 | 0.74 |
| kininogen-1 isoform 2 precursor | P01042 (KNG1_ HUMAN) | K.IYPTVNCQPL GM*ISLM*K.R | 0.89 | 0.62 |
| kininogen-1 isoform 2 precursor | P01042 (KNG1_ HUMAN) | K.TVGSDTFYSF K.Y | 0.61 | 0.68 |
| kininogen-1 isoform 2 precursor | P01042 (KNG1_ HUMAN) | R.DIPTNSPELEE TLTHTITK.L | 0.61 | 0.76 |
| kininogen-1 isoform 2 precursor | P01042 (KNG1_ HUMAN) | R.VQVVAGK.K | 0.67 | 0.71 |
| lumican precursor | P51884 (LUM_ HUMAN) | R.FNALQYLR.L | 0.68 | 0.76 |
| macrophage colony-stimulating factor 1 receptor precursor | P09603 (CSF1_HUMAN) | K.VIPGPPALTLV PAELVR.I | 0.68 | 0.60 |
| monocyte differentiation antigen CD14 precursor | P08571 (CD14_ HUMAN) | K.ITGTMPPLPLE ATGLALSSLR.L | 0.80 | 0.67 |
| N-acetylmuramoyl-L-alanine amidase | Q96PD5 (PGRP2_ HUMAN | K.EFTEAFLGCP AIHPR.C | 0.62 | 0.64 |
| precursor | ) | | | |
| N-acetylmuramoyl-L-alanine amidase precursor | Q96PD5 (PGRP2_ HUMAN ) | R.RVINLPLDSM AAPWETGDTFP DVVAIAPDVR.A | 0.63 | 0.62 |
| phosphatidylinositol-glycan-specific phospholipase D precursor | P80108 (PHLD _HUMAN) | R.GVFFSVNSWT PDSMSFIYK.A | 0.67 | 0.78 |
| pigment epithelium-derived factor precursor | **P36955** (PEDF_HUMAN) | K.EIPDEISILLLGVAHF K.G | 0.63 | 0.61 |
| pigment epithelium-derived factor precursor | **P36955** (PEDF_HUMAN) | K.IAQLPLTGSM*SIIF FLPLK.V | 0.79 | 0.61 |
| pigment epithelium-derived factor precursor | **P36955** (PEDF_HUMAN) | K.TVQAVLTVPK.L | 0.75 | 0.79 |
| pigment epithelium-derived factor precursor | **P36955** (PEDF_HUMAN) | R.ALYYDLISSPDIHGT YKELLDTVTAPQK.N | 0.60 | 0.73 |
| pigment epithelium-derived factor precursor | **P36955** (PEDF_HUMAN) | R.DTDTGALLFIGK.I | 0.85 | 0.62 |
| plasminogen isoform 1 precursor | **P00747** (PLMN_HUMAN) | R.ELRPWCFTTDPNK R.W | 0.70 | 0.68 |
| plasminogen isoform 1 precursor | **P00747** (PLMN_HUMAN) | R.TECFITGWGETQGT FGAGLLK.E | 0.63 | 0.68 |
| platelet basic protein preproprotein | **P02775** (CXCL7_HUMAN) | K.GTHCNQVEVIATLK .D | 0.60 | 0.61 |
| pregnancy zone protein precursor | **P20742** (PZP_HUMAN) | K.AVGYLITGYQR.Q | 0.87 | 0.73 |
| pregnancy zone protein precursor | **P20742** (PZP_HUMAN) | R.AVDQSVLLM*KPE AELSVSSVYNLLTVK.D | 0.64 | 0.62 |
| pregnancy zone protein precursor | **P20742** (PZP_HUMAN) | R.IQHPFTVEEFVLPK. F | 0.66 | 0.74 |
| pregnancy zone protein precursor | **P20742** (PZP_HUMAN) | R.NELIPLIYLENPR.R | 0.61 | 0.61 |
| protein AMBP preproprotein | **P02760** (AMBP_HUMAN) | R.AFIQLWAFDAVK.G | 0.72 | 0.67 |
| proteoglycan 4 isoform B precursor | **Q92954** (PRG4_HUMAN) | K.GFGGLTGQIVAALS TAK.Y | 0.70 | 0.72 |
| prothrombin preproprotein | **P00734** (THRB_HUMAN) | K.YGFYTHVFR.L | 0.70 | 0.63 |
| prothrombin preproprotein | **P00734** (THRB_HUMAN) | R.IVEGSDAEIGM*SP WQVMLFR.K | 0.63 | 0.71 |
| retinol-binding protein 4 precursor | **P02753** (RET4_HUMAN) | K.KDPEGLFLQDNIVA EFSVDETGQMSATAK .G | 0.67 | 0.67 |
| thyroxine-binding globulin precursor | **P05543** (THBG_HUMAN) | K.AQWANPFDPSKTE DSSSFLIDK.T | 0.67 | 0.80 |
| thyroxine-binding globulin precursor | **P05543** (THBG_HUMAN) | K.GWVDLFVPK.F | 0.67 | 0.64 |
| thyroxine-binding globulin precursor | **P05543** (THBG_HUMAN) | R.SFM*LLILER.S | 0.65 | 0.68 |
| thyroxine-binding globulin precursor | **P05543** (THBG_HUMAN) | R.SFMLLILER.S | 0.64 | 0.62 |
| vitamin D-binding protein isoform 1 precursor | **P02774** (VTDB_HUMAN) | K.EFSHLGKEDFTSLSL VLYSR.K | 0.74 | 0.61 |
| vitamin D-binding protein isoform 1 precursor | **P02774** (VTDB_HUMAN) | K.EYANQFM*WEYST NYGQAPLSLLVSYTK. S | 0.73 | 0.61 |
| vitamin D-binding protein isoform 1 precursor | **P02774** (VTDB_HUMAN) | K.HQPQEFPTYVEPTN DEICEAFRK.D | 0.67 | 0.69 |
| vitamin D-binding protein isoform 1 precursor | **P02774** (VTDB_HUMAN) | K.SYLSM*VGSCCTSA SPTVCFLK.E | 0.63 | 0.62 |
| vitamin D-binding protein isoform 1 precursor | **P02774** (VTDB_HUMAN) | K.TAM*DVFVCTYFM PAAQLPELPDVELPT NK.D | 0.63 | 0.60 |
| vitamin D-binding protein isoform 1 precursor | **P02774** (VTDB_HUMAN) | K.VPTADLEDVLPLAE DITNILSK.C | 0.70 | 0.71 |
| vitronectin precursor | **P04004** (VTNC_HUMAN) | K.AVRPGYPK.L | 0.68 | 0.77 |
| vitronectin precursor | **P04004** (VTNC_HUMAN) | R.MDWLVPATCEPIQ SVFFFSGDK.Y | 0.67 | 0.65 |
| zinc-alpha-2-glycoprotein precursor | **P25311** (ZA2G_HUMAN) | K.EIPAWVPFDPAAQI TK.Q | 0.63 | 0.67 |

The differentially expressed proteins identified by the hypothesis-independent strategy above, not already present in our MRM-MS assay, were candidates for incorporation into the MRM-MS assay. Two additional proteins (AFP, PGH1) of functional interest were also selected for MRM development. Candidates were prioritized by AUC and biological function, with preference give for new pathways. Sequences for each protein of interest, were imported into Skyline software which generated a list of tryptic peptides, m/z values for the parent ions and fragment ions, and an instrument-specific collision energy (McLean et al. Bioinformatics (2010) 26 (7): 966-968; McLean et al. Anal. Chem (2010) 82 (24): 10116-10124).

The list was refined by eliminating peptides containing cysteines and methionies, and by using the shotgun data to select the charge state(s) and a subset of potential fragment ions for each peptide that had already been observed on a mass spectrometer.

After prioritizing parent and fragment ions, a list of transitions was exported with a single predicted collision energy. Approximately 100 transitions were added to a single MRM run. For development, MRM data was collected on either a QTRAP 5500 (AB Sciex) or a 6490 QQQ (Agilent). Commercially available human female serum (from pregnant and non-pregnant donors), was depleted and processed to tryptic peptides, as described above, and used to "scan" for peptides of interest. In some cases, purified synthetic peptides were used for further optimization. For development, digested serum or purified synthetic peptides were separated with a 15 min acetonitrile gradient at 100 ul/min on a 2.1 x 50 mM Poroshell 120 EC-C18 column (Agilent) at 40°C.

The MS/MS data was imported back into Skyline, where all chromatograms for each peptide were overlayed and used to identify a concensus peak corresponding to the peptide of interest and the transitions with the highest intensities and the least noise. Table 11, contains a list of the most intensely observed candidate transitions and peptides for transfer to the MRM assay.

**Table 11. Candidate peptides and transitions for transferring to the MRM assay**

| Protein | Peptide | m/z, charge | fragment ion, m/z, charge, rank | area |
|---|---|---|---|---|
| alpha-1-antichymotrypsin | K.ADLSGITGAR.N | 480.7591++ | S [y7] - 661.3628+[1] | 1437602 |
| | | | G [y6] - 574.3307+[2] | 637584 |
| | | | T [y4] - 404.2252+[3] | 350392 |
| | | | L [y8] - 774.4468+[4] | 191870 |
| | | | G [y3] - 303.1775+[5] | 150575 |
| | | | I [y5] - 517.3093+[6] | 97828 |
| alpha-1-antichymotrypsin | K.EQLSLLDR.F | 487.2693++ | S [y5] - 603.3461+[1] | 345602 |
| | | | L [y6] - 716.4301+[2] | 230046 |
| | | | L [y4] - 516.3140+[3] | 143874 |
| | | | D [y2] - 290.1459+[4] | 113381 |
| | | | D [y2] - 290.1459+[5] | 113381 |
| | | | Q [b2] - 258.1084+[6] | 78157 |
| alpha-1-antichymotrypsin | K.ITLLSALVETR.T | 608.3690++ | S [y7] - 775.4308+[1] | 1059034 |
| | | | L [y8] - 888.5149+[2] | 541969 |
| | | | T [b2] - 215.1390+[3] | 408819 |
| | | | L [y9] - 1001.5990+[4] | 438441 |
| | | | V [y4] - 504.2776+[5] | 311293 |
| | | | L [y5] - 617.3617+[6] | 262544 |
| | | | L [b3] - 328.2231+[7] | 197526 |
| | | | T [y2] - 276.1666+[8] | 212816 |
| | | | E [y3] - 405.2092+[9] | 207163 |
| alpha-1-antichymotrypsin | R.EIGELYLPK.F | 531.2975++ | G [y7] - 819.4611+[2] | 977307 |
| | | | L [y5] - 633.3970+[3] | 820582 |
| | | | Y [y4] - 520.3130+[4] | 400762 |
| | | | L [y3] - 357.2496+[5] | 498958 |
| | | | P [y2] - 244.1656+[1] | 1320591 |
| | | | I [b2] - 243.1339+[6] | 303268 |
| | | | G [b3] - 300.1554+[7] | 305120 |
| alpha-1-antichymotrypsin | R.GTHVDLGLASA NVDFAFSLYK.Q | 742.3794+++ | D [y8] - 990.4931+[1] | 154927 |
| | | | L [b8] - 793.4203+[2] | 51068 |
| | | | D [b5] - 510.2307+[3] | 45310 |
| | | | F [y7] - 875.4662+[4] | 42630 |
| | | | A [b9] - 864.4574+[5] | 43355 |
| | | | S [y4] - 510.2922+[6] | 45310 |
| | | | F [y5] - 657.3606+[7] | 37330 |
| | | | V [y9] - 1089.5615+[8] | 32491 |
| | | | G [b7] - 680.3362+[9] | 38185 |
| | | | Y [y2] - 310.1761+[10] | 36336 |
| | | | N [b12] - 1136.5695+[11] | 16389 |
| | | | S [b10] - 951.4894+[12] | 16365 |
| | | | L [b6] - 623.3148+[13] | 13687 |
| | | | L [y3] - 423.2602+[14] | 17156 |
| | | | V [b4] - 395.2037+[15] | 10964 |
| alpha-1-antichymotrypsin | R.NLAVSQVVHK. A | 547.8195++ | A [y8] - 867.5047+[1] | 266203 |
| | | | L [b2] - 228.1343+[2] | 314232 |
| | | | V [y7] - 796.4676+[3] | 165231 |
| | | | A [b3] - 299.1714+[4] | 173694 |
| | | | S [y6] - 697.3991+[5] | 158512 |
| | | | H [y2] - 284.1717+[6] | 136431 |
| | | | V [b4] - 398.2398+[7] | 36099 |
| | | | S [b5] - 485.2718+[8] | 23836 |
| | | 365.5487+++ | S [y6] - 697.3991+[1] | 223443 |
| | | | V [y3] - 383.2401+[2] | 112952 |
| | | | V [y4] - 482.3085+[3] | 84872 |
| | | | Q [y5] - 610.3671+[4] | 30835 |
| inter-alpha-trypsin inhibitor heavy chain H1 | K.AAISGENAGLVR .A | 579.3173++ | S [y9] - 902.4690+[1] | 518001 |
| | | | G [y8] - 815.4370+[2] | 326256 |
| | | | N [y6] - 629.3729+[3] | 296670 |
| | | | S [b4] - 343.1976+[4] | 258172 |
| inter-alpha-trypsin inhibitor heavy chain H1 | K.GSLVQASEANL QAAQDFVR.G | 668.6763+++ | A [y7] - 806.4155+[1] | 304374 |
| | | | A [y6] - 735.3784+[2] | 193844 |
| | | | V [b4] - 357.2132+[3] | 294094 |
| | | | F [y3] - 421.2558+[4] | 167816 |
| | | | A [b6] - 556.3089+[5] | 149216 |
| | | | L [b11] - 535.7775++[6] | 156882 |
| | | | A [b13] - 635.3253++[7] | 249287 |
| | | | A [y14] - 760.3786++[8] | 123723 |
| | | | F [b17] - 865.9208++[9] | 23057 |
| inter-alpha-trypsin | K.TAFISDFAVTAD | 1087.0442++ | G [y4] - 432.2453+[1] | 22362 |
| inhibitor heavy chain H1 | GNAFIGDIK.D | | I [y5] - 545.3293+[2] | 8319 |
| | | | A [b8] - 853.4090+[3] | 7006 |
| | | | G [y9] - 934.4993+[4] | 6755 |
| | | | F [y6] - 692.3978+[5] | 6193 |
| | | | V [b9] - 952.4775+[6] | 9508 |
| inter-alpha-trypsin inhibitor heavy chain H1 | K.VTYDVSR.D | 420.2165++ | Y [y5] - 639.3097+[1] | 609348 |
| | | | T [b2] - 201.1234+[2] | 792556 |
| | | | D [y4] - 476.2463+[3] | 169546 |
| | | | V [y3] - 361.2194+[4] | 256946 |
| | | | Y [y5] - 320.1585++[5] | 110608 |
| | | | S [y2] - 262.1510+[6] | 50268 |
| | | | Y [b3] - 182.5970++[7] | 10947 |
| | | | D [b4] - 479.2136+[8] | 13662 |
| inter-alpha-trypsin inhibitor heavy chain H1 | R.EVAFDLEIPK.T | 580.8135++ | P [y2] - 244.1656+[1] | 2032509 |
| | | | D [y6] - 714.4032+[2] | 672749 |
| | | | A [y8] - 932.5088+[3] | 390837 |
| | | | L [y5] - 599.3763+[4] | 255527 |
| | | | F [y7] - 861.4716+[5] | 305087 |
| inter-alpha-trypsin inhibitor heavy chain H1 | R.LWAYLTIQELLA K.R | 781.4531++ | W [b2] - 300.1707+[1] | 602601 |
| | | | A [b3] - 371.2078+[2] | 356967 |
| | | | T [y8] - 915.5510+[3] | 150419 |
| | | | Y [b4] - 534.2711+[4] | 103449 |
| | | | I [y7] - 814.5033+[5] | 72044 |
| | | | Q [y6] - 701.4192+[6] | 66989 |
| | | | L [b5] - 647.3552+[7] | 99820 |
| | | | E [y5] - 573.3606+[8] | 44843 |
| inter-alpha-trypsin inhibitor heavy chain H2 | K.FYNQVSTPLLR. N | 669.3642++ | S [y6] - 686.4196+[1] | 367330 |
| | | | V [y7] - 785.4880+[2] | 182396 |
| | | | P [y4] - 498.3398+[3] | 103638 |
| | | | Y [b2] - 311.1390+[4] | 52172 |
| | | | Q [b4] - 553.2405+[5] | 54270 |
| | | | N [b3] - 425.1819+[6] | 34567 |
| inter-alpha-trypsin inhibitor heavy chain H2 | K.HLEVDVWVIEP QGLR.F | 597.3247+++ | I [y7] - 812.4625+[1] | 206996 |
| | | | P [y5] - 570.3358+[2] | 303693 |
| | | | E [y6] - 699.3784+[3] | 126752 |
| | | | P [y5] - 285.6715++[4] | 79841 |
| inter-alpha-trypsin inhibitor heavy chain H2 | K.TAGLVR.S | 308.6925++ | A [b2] - 173.0921+[1] | 460019 |
| | | | G [y4] - 444.2929+[2] | 789068 |
| | | | V [y2] - 274.1874+[3] | 34333 |
| | | | G [b3] - 230.1135+[4] | 15169 |
| | | | L [y3] - 387.2714+[5] | 29020 |
| inter-alpha-trypsin inhibitor heavy chain H2 | R.IYLQPGR.L | 423.7452++ | L [y5] - 570.3358+[1] | 638209 |
| | | | P [y3] - 329.1932+[2] | 235194 |
| | | | Y [b2] - 277.1547+[3] | 266889 |
| | | | Q [y4] - 457.2518+[4] | 171389 |
| inter-alpha-trypsin inhibitor heavy chain H2 | R.LSNENHGIAQR. I | 413.5461+++ | N [y9] - 519.7574++[1] | 325409 |
| | | | N [y7] - 398.2146++[2] | 39521 |
| | | | G [y5] - 544.3202+[3] | 139598 |
| | | | S [b2] - 201.1234+[4] | 54786 |
| | | | E [y8] - 462.7359++[5] | 30623 |
| inter-alpha-trypsin inhibitor heavy chain H2 | R.SLAPTAAAKR.R | 415.2425++ | A [y7] - 629.3617+[1] | 582421 |
| | | | L [b2] - 201.1234+[2] | 430584 |
| | | | P [y6] - 558.3246+[3] | 463815 |
| | | | A [b3] - 272.1605+[4] | 204183 |
| | | | T [y5] - 461.2718+[5] | 47301 |
| inter-alpha-trypsin inhibitor heavy chain H3 | K.EVSFDVELPK.T | 581.8032++ | P [y2] - 244.1656+[1] | 132304 |
| | | | V [b2] - 229.1183+[2] | 48895 |
| | | | L [y3] - 357.2496+[3] | 20685 |
| inter-alpha-trypsin inhibitor heavy chain H3 | K.IQENVR.N | 379.7114++ | E [y4] - 517.2729+[1] | 190296 |
| | | | E [b3] - 371.1925+[2] | 51697 |
| | | | Q [b2] - 242.1499+[3] | 54241 |
| | | | N [y3] - 388.2303+[4] | 21156 |
| | | | V [y2] - 274.1874+[5] | 8309 |
| inter-alpha-trypsin inhibitor heavy chain H3 | R.ALDLSLK.Y | 380.2342++ | D [y5] - 575.3399+[1] | 687902 |
| | | | L [b2] - 185.1285+[2] | 241010 |
| | | | L [y2] - 260.1969+[3] | 29365 |
| inter-alpha-trypsin inhibitor heavy chain H3 | R.LIQDAVTGLTVN GQITGDK.R | 972.0258++ | V [b6] - 640.3665+[1] | 139259 |
| | | | G [b8] - 798.4356+[2] | 53886 |
| | | | G [y7] - 718.3730+[3] | 12518 |
| pigment epithelium-derived factor precursor | K.SSFVAPLEK.S | 489.2687++ | A [y5] - 557.3293+[1] | 13436 |
| | | | V [y6] - 656.3978+[2] | 9350 |
| | | | F [y7] - 803.4662+[3] | 6672 |
| | | | P [y4] - 486.2922+[4] | 6753 |
| pigment epithelium-derived factor precursor | K.TVQAVLTVPK.L | 528.3266++ | Q [y8] - 855.5298+[1] | 26719 |
| | | | V [b2] - 201.1234+[2] | 21239 |
| | | | Q [y8] - 428.2686++[3] | 16900 |
| | | | A [y7] - 727.4713+[4] | 9518 |
| | | | L [y5] - 557.3657+[5] | 5108 |
| | | | Q [b3] - 329.1819+[6] | 5450 |
| | | | V [y6] - 656.4341+[7] | 4391 |
| pigment epithelium-derived factor precursor | R.ALYYDLISSPDIH GTYK.E | 652.6632+++ | Y [y15] - 886.4305++[1] | 78073 |
| | | | Y [y14] - 804.8988++[2] | 26148 |
| pigment epithelium-derived factor precursor | R.DTDTGALLFIGK. I | 625.8350++ | G [y8] - 818.5135+[1] | 25553 |
| | | | T [b2] - 217.0819+[2] | 22716 |
| | | | T [b4] - 217.0819++[3] | 22716 |
| | | | L [y5] - 577.3708+[4] | 11600 |
| | | | I [y3] - 317.2183+[5] | 11089 |
| | | | A [b6] - 561.2151+[6] | 6956 |
| pigment epithelium-derived factor precursor | K.ELLDTVTAPQK. N | 607.8350++ | T [y5] - 544.3089+[1] | 17139 |
| | | | D [y8] - 859.4520+[2] | 17440 |
| | | | L [y9] - 972.5360+[3] | 14344 |
| | | | A [y4] - 443.2613+[4] | 11474 |
| | | | T [y7] - 744.4250+[5] | 10808 |
| | | | V [y6] - 643.3774+[6] | 9064 |
| pregnancy-specific beta-1-glycoprotein 1 | K.FQLPGQK.L | 409.2320++ | L [y5] - 542.3297+[1] | 116611 |
| | | | P [y4] - 429.2456+[2] | 91769 |
| | | | Q [b2] - 276.1343+[3] | 93301 |
| pregnancy-specific beta-1-glycoprotein 1 | R.DLYHYITSYVVD GEIIIYGPAYSGR.E | 955.4762+++ | G [y7] - 707.3471+[1] | 5376 |
| | | | Y [y8] - 870.4104+[2] | 3610 |
| | | | P [y6] - 650.3257+[3] | 2770 |
| | | | I [y9] - 983.4945+[4] | 3361 |
| pregnancy-specific beta-1-glycoprotein 11 | K.LFIPQITPK.H | 528.8262++ | P [y6] - 683.4087+[1] | 39754 |
| | | | F [b2] - 261.1598+[2] | 29966 |
| | | | I [y7] - 796.4927+[3] | 13162 |
| pregnancy-specific beta-1-glycoprotein 11 | NSATGEESSTSLTI R | 776.8761++ | E [b7] - 689.2737+[1] | 11009 |
| | | | T [y6] - 690.4145+[2] | 11284 |
| | | | L [y4] - 502.3348+[3] | 2265 |
| | | | S [y7] - 389.2269++[4] | 1200 |
| | | | T [y3] - 389.2507+[5] | 1200 |
| | | | I [y2] - 288.2030+[6] | 2248 |
| pregnancy-specific beta-1-glycoprotein 2 | K.FQQSGQNLFIP QITTK.H | 617.3317+++ | F [y8] - 474.2817++[1] | 43682 |
| | | | G [y12] - 680.3852++[2] | 24166 |
| | | | S [b4] - 491.2249+[3] | 23548 |
| | | | Q [b3] - 404.1928+[4] | 17499 |
| | | | I [y4] - 462.2922+[5] | 17304 |
| | | | F [b9] - 525.7538++[6] | 17206 |
| | | | I [b10] - 582.2958++[7] | 16718 |
| | | | L [b8] - 452.2196++[8] | 16490 |
| | | | P [y6] - 344.2054++[9] | 16198 |
| | | | G [b5] - 548.2463+[10] | 15320 |
| pregnancy-specific beta-1-glycoprotein 2 | IHPSYTNYR | 575.7856++ | N [b7] - 813.3890+[1] | 16879 |
| | | | Y [b5] - 598.2984+[2] | 18087 |
| | | | T [y4] - 553.2729+3 | 2682 |
| pregnancy-specific beta-1-glycoprotein 2 | FQLSETNR | 497.7513++ | L [y6] - 719.3682+[1] | 358059 |
| | | | S [y5] - 606.2842+[2] | 182330 |
| | | | Q [b2] - 276.1343+[3] | 292482 |
| pregnancy-specific beta-1-glycoprotein 3 | VSAPSGTGHLPGL NPL | 506.2755+++ | T [b7] - 300.6530++[1] | 25346 |
| | | | H [y8] - 860.4989+[2] | 12159 |
| | | | H [y8] - 430.7531++[3] | 15522 |
| pregnancy-specific beta-1-glycoprotein 3 | EDAGSYTLHIVK | 666.8433++ | Y [b6] - 623.2307+[1] | 23965 |
| | | | Y [y7] - 873.5193+[2] | 21686 |
| | | | L [b8] - 837.3625+[3] | 4104 |
| | | | A [b3] - 316.1139+[4] | 1987 |
| pregnancy-specific beta-1-glycoprotein 4 | R.TLFIFGVTK.Y | 513.3051++ | F [y7] - 811.4713+[1] | 62145 |
| | | | L [b2] - 215.1390+[2] | 31687 |
| | | | F [y5] - 551.3188+[3] | 972 |
| pregnancy-specific beta-1-glycoprotein 4 | NYTYIWWLNGQS LPVSPR | 1097.5576++ | W [b6] - 841.3879+[1] | 25756 |
| | | | G [y9] - 940.5211+[2] | 25018 |
| | | | Y [b4] - 542.2245+[3] | 19778 |
| | | | Q [y8] - 883.4996+[4] | 6642 |
| | | | P [y2] - 272.1717+[5] | 5018 |
| pregnancy-specific beta-1-glycoprotein 5 | GVTGYFTFNLYLK | 508.2695+++ | L [y2] - 260.1969+[1] | 176797 |
| | | | T [y11] - 683.8557++[2] | 136231 |
| | | | F [b6] - 625.2980+[3] | 47523 |
| | | | L [y4] - 536.3443+[4] | 23513 |
| pregnancy-specific beta-1-glycoprotein 6 | SNPVTLNVLYGPD LPR | 585.6527+++ | Y [y7] - 817.4203+[1] | 14118 |
| | | | G [y6] - 654.3570+[2] | 10433 |
| | | | P [b3] - 299.1350+[3] | 87138* |
| | | | P [y5] - 299.1714++[4] | 77478* |
| | | | P [y5] - 597.3355+[5] | 68089* |
| pregnancy-specific beta-1-glycoprotein 7 | DVLLLVHNLPQNL TGHIWYK | 791.7741+++ | L [y8] - 1017.5516+[3] | 141169 |
| | | | G [y6] - 803.4199+[5] | 115905 |
| | | | W [y3] - 496.2554+[6] | 108565 |
| | | | P [y11] - 678.8566++[7] | 105493 |
| | | | V [b2] - 215.1026+[1] | 239492 |
| | | | L [b3] - 328.1867+[2] | 204413 |
| | | | N [b8] - 904.5251+[4] | 121880 |
| pregnancy-specific beta-1-glycoprotein 7 | YGPAYSGR | 435.7089++ | A [y5] - 553.2729+[1] | 25743* |
| | | | Y [y4] - 482.2358+[2] | 25580* |
| | | | P [y6] - 650.3257+[3] | 10831* |
| | | | S [y3] - 319.1724+[4] | 10559* |
| | | | G [b2] - 221.0921+[5] | 7837* |
| pregnancy-specific beta-1-glycoprotein 8 | LQLSETNR | 480.7591++ | S [b4] - 442.2660+[1] | 18766 |
| | | | L [b3] - 355.2340+[2] | 12050 |
| | | | Q [b2] - 242.1499+[3] | 1339 |
| | | | T [b6] - 672.3563+[4] | 2489 |
| pregnancy-specific beta-1-glycoprotein 9 | K.LFIPQITR.N | 494.3029++ | P [y5] - 614.3620+[1] | 53829 |
| | | | I [y6] - 727.4461+[2] | 13731 |
| | | | I [b3] - 374.2438+[3] | 4178 |
| | | | Q [y4] - 517.3093+[4] | 2984 |
| pregnancy-specific beta-1-glycoprotein 9 | K.LPIPYITINNLNP R.E | 819.4723++ | P [b2] - 211.1441+[1] | 18814* |
| | | | P [b4] - 211.1441++[2] | 18814* |
| | | | T [b7] - 798.4760+[3] | 17287* |
| | | | T [y8] - 941.5163+[4] | 10205* |
| | | | Y [b5] - 584.3443+[5] | 10136* |
| | | | N [y6] - 727.3846+[6] | 9511* |
| pregnancy-specific beta-1-glycoprotein 9 | R.SNPVILNVLYGP DLPR.I | 589.6648+++ | P [y5] - 597.3355+[1] | 3994 |
| | | | Y [y7] - 817.4203+[2] | 3743 |
| | | | G [y6] - 654.3570+[3] | 3045 |
| pregnancy-specific beta-1-glycoprotein 9 | DVLLLVHNLPQNL PGYFWYK | 810.4387+++ | P [y7] - 960.4614+[1] | 120212 |
| | | | V [b2] - 215.1026+[2] | 65494 |
| | | | L [b3] - 328.1867+[3] | 54798 |
| pregnancy-specific beta-1-glycoprotein 9 | SENYTYIWWLNG QSLPVSPGVK | 846.7603+++ | W [y15] - 834.4488++[1] | 14788 |
| | | | P [y4] - 200.6314++[2] | 19000 |
| | | | Y [y17] - 972.5225++[3] | 4596 |
| | | | L [b10] - 678.8166++[4] | 2660 |
| | | | Y [b6] - 758.2992+[5] | 1705 |
| | | | P [y4] - 400.2554+[6] | 1847 |
| Pan-PSG | ILILPSVTR | 506.3317++ | P [y5] - 559.3198+[1] | 484395 |
| | | | L [b2] - 227.1754+[2] | 102774 |
| | | | L [b4] - 227.1754++[3] | 102774 |
| | | | I [y7] - 785.4880+[4] | 90153 |
| | | | I [b3] - 340.2595+[5] | 45515 |
| | | | L [y6] - 672.4039+[6] | 40368 |
| thyroxine-binding globulin precursor | K.AQWANPFDPS K.T | 630.8040++ | A [b4] - 457.2194+[1] | 30802 |
| | | | S [y2] - 234.1448+[2] | 28255 |
| | | | D [y4] - 446.2245+[3] | 24933 |
| thyroxine-binding globulin precursor | K.AVLHIGEK.G | 289.5080+++ | I [y4] - 446.2609+[1] | 220841 |
| | | | H [y5] - 292.1636++[2] | 303815 |
| | | | H [y5] - 583.3198+[3] | 133795 |
| | | | V [b2] - 171.1128+[4] | 166139 |
| | | | L [y6] - 348.7056++[5] | 823533 |
| thyroxine-binding globulin precursor | K.FLNDVK.T | 368.2054++ | N [y4] - 475.2511+[1] | 296859 |
| | | | V [y2] - 246.1812+[2] | 219597 |
| | | | L [b2] - 261.1598+[3] | 87504 |
| thyroxine-binding globulin precursor | K.FSISATYDLGATL LK.M | 800.4351++ | Y [y9] - 993.5615+[1] | 34111 |
| | | | G [y6] - 602.3872+[2] | 17012 |
| | | | D [y8] - 830.4982+ | 45104 |
| | | | S [b2] - 235.1077+[4] | 15480 |
| thyroxine-binding globulin precursor | K.GWVDLFVPK.F | 530.7949++ | W [b2] - 244.1081+[1] | 1261810 |
| | | | P [y2] - 244.1656+[2] | 1261810 |
| | | | V [b7] - 817.4243+[3] | 517675 |
| | | | V [y7] - 817.4818+[4] | 517675 |
| | | | D [y6] - 718.4134+[5] | 306994 |
| | | | F [b6] - 718.3559+[6] | 306994 |
| | | | V [y3] - 343.2340+[7] | 112565 |
| | | | V [b3] - 343.1765+[8] | 112565 |
| thyroxine-binding globulin precursor | K.NALALFVLPK.E | 543.3395++ | A [y7] - 787.5076+[1] | 198085 |
| | | | L [b3] - 299.1714+[2] | 199857 |
| | | | P [y2] - 244.1656+[3] | 129799 |
| | | | L [y8] - 900.5917+[4] | 111572 |
| | | | L [y6] - 716.4705+[5] | 88773 |
| | | | F [y5] - 603.3865+[6] | 54020 |
| | | | L [y3] - 357.2496+[7] | 43353 |
| thyroxine-binding globulin precursor | R.SILFLGK.V | 389.2471++ | L [y5] - 577.3708+[1] | 1878736 |
| | | | I [b2] - 201.1234+[2] | 946031 |
| | | | G [y2] - 204.1343+[3] | 424248 |
| | | | L [y3] - 317.2183+[4] | 291162 |
| | | | F [y4] - 464.2867+[5] | 391171 |
| AFP | R.DFNQFSSGEK.N | 386.8402+++ | N [b3] - 189.0764++[1] | 42543 |
| | | | S [y4] - 210.6081++[2] | 21340 |
| | | | G [y3] - 333.1769+[3] | 53766 |
| | | | N [b3] - 377.1456+[4] | 58644 |
| | | | F [b2] - 263.1026+[5] | 5301 |
| AFP | K.GYQELLEK.C | 490.2584++ | E [y5] - 631.3661+[1] | 110518 |
| | | | L [y4] - 502.3235+[2] | 74844 |
| | | | E [y2] - 276.1554+[3] | 42924 |
| | | | E [b4] - 478.1932+[4] | 20953 |
| AFP | K.GEEELQK.Y | 416.7060++ | E [b2] - 187.0713+[1] | 37843 |
| | | | E [y4] - 517.2980+[2] | 56988 |
| AFP | K.FIYEIAR.R | 456.2529++ | I [y3] - 359.2401+[1] | 34880 |
| | | | I [b2] - 261.1598+[2] | 7931 |
| AFP | R.HPFLYAPTILLW AAR.Y | 590.3348+++ | I [y7] - 421.7660++[1] | 11471 |
| | | | L [y6] - 365.2239++[2] | 5001 |
| | | | A [b6] - 365.1896++[3] | 5001 |
| | | | L [y6] - 729.4406+[4] | 3218 |
| | | | F [b3] - 382.1874+[5] | 6536 |
| | | | A [b6] - 729.3719+[6] | 3218 |
| AFP | R.TFQAITVTK.L | 504.7898++ | T [b6] - 662.3508+[1] | 11241 |
| | | | T [y4] - 448.2766+[2] | 7541 |
| | | | A [b4] - 448.2191+[3] | 7541 |
| AFP | K.LTTLER.G | 366.7162++ | T [y4] - 518.2933+[1] | 7836 |
| | | | L [b4] - 215.1390++[2] | 4205 |
| | | | T [b2] - 215.1390+[3] | 4205 |
| AFP | R.HPQLAVSVILR. V | | L [y2] - 288.2030+[1] | 3781 |
| | | | I [y3] - 401.2871+[2] | 2924 |
| | | | L [b4] - 476.2616+[3] | 2647 |
| AFP | K.LGEYYLQNAFLV AYTK.K | 631.6646+++ | G [b2] - 171.1128+[1] | 10790 |
| | | | Y [y3] - 411.2238+[2] | 2303 |
| | | | F [b10] - 600.2902++[3] | 1780 |
| | | | Y [b4] - 463.2187+[4] | 2214 |
| | | | F [y7] - 421.2445++[6] | 3072 |
| PGH1 | R.ILPSVPK.D | 377.2471++ | P [y5] - 527.3188+[1] | 5340492 |
| | | | S [y4] - 430.2660+[5] | 419777 |
| | | | P [y2] - 244.1656+[2] | 4198508 |
| | | | P [y5] - 264.1630++[3] | 2771328 |
| | | | L [b2] - 227.1754+[4] | 2331263 |
| PGH1 | K.AEHPTWGDEQL FQTTR.L | 639.3026+++ | E [b9] - 512.2120++[1] | 64350 |
| | | | P [b4] - 218.1030++[2] | 38282 |
| | | | L [b11] - 632.7833++[3] | 129128 |
| | | | G [y10] - 597.7911++[4] | 19406 |
| | | | G [b7] - 779.3471+[5] | 51467 |
| | | | T [y3] - 189.1108++[6] | 10590 |
| | | | D [y9] - 569.2804++[7] | 12460 |
| | | | L [y6] - 765.4254+[8] | 6704 |
| | | | D [b8] - 447.6907++[9] | 4893 |
| | | | P [b4] - 435.1987+[10] | 8858 |
| | | | Q [y7] - 893.4839+[11] | 6101 |
| | | | T [b5] - 268.6268++[12] | 5456 |
| | | | T [b5] - 536.2463+[13] | 5549 |
| PGH1 | R.LILIGETIK.I | 500.3261++ | G [y5] - 547.3086+[1] | 7649 |
| | | | T [y3] - 361.2445+[2] | 6680 |
| | | | E [y4] - 490.2871+[3] | 5234 |
| | | | L [y7] - 773.4767+[4] | 3342 |
| PGH1 | R.LQPFNEYR.K | 533.7694++ | N [b5] - 600.3140+[1] | 25963 |
| | | | F [b4] - 486.2711+[2] | 6915 |
| | | | E [y3] - 467.2249+[3] | 15079 |

| | | | | |
|---|---|---|---|---|
| * QTRAP5500 data, all other peak areas are from Agilent 6490 | | | | |

Next, the top 2-10 transitions per peptide and up to 7 peptides per protein were selected for collision energy (CE) optimization on the Agilent 6490. Using Skyline or MassHunter Qual software, the optimized CE value for each transition was determined based on the peak area or signal to noise. The two transitions with the largest peak areas per peptide and at least two peptides per protein were chosen for the final MRM method. Substitutions of transitions with lower peak areas were made when a transition with a larger peak area had a high background level or had a low m/z value that has more potential for interference.

Lastly, the retention times of selected peptides were mapped using the same column and gradient as our established sMRM assay. The newly discovered analytes were subsequently added to the sMRM method and used in a further hypothesis-dependent discovery study described in Example 5 below.

The above method was typical for most proteins. However, in some cases, the differentially expressed peptide identified in the shotgun method did not uniquely identify a protein, for example, in protein families with high sequence identity. In these cases, a MRM method was developed for each family member. Also, let it be noted that, for any given protein, peptides in addition to those found to be significant and fragment ions not observed on the Orbitrap may have been included in MRM optimization and added to the final sMRM method if those yielded the best signal intensities.

### Example 5. Study IV to Identify and Confirm Preterm Birth Biomarkers

A further hypothesis-dependent discovery study was performed with the scheduled MRM assay used in Examples 3 but now augmented with newly discovered analytes from the Example 4. Less robust transitions (from the original 1708 described in Example 1) were removed to improve analytical performance and make room for the newly discovered analytes. Samples included approximately 30 cases and 60 matched controls from each of three gestational periods (early, 17-22 weeks, middle, 23-25 weeks and late, 26-28 weeks). Log transformed peak areas for each transition were corrected for run order and batch effects by regression. The ability of each analyte to separate cases and controls was determined by calculating univariate AUC values from ROC curves. Ranked univariate AUC values (0.6 or greater) are reported for individual gestational age window sample sets (Tables 12, 13, 15) and a combination of the middle and late window (Table 14). Multivariate classifiers were built using different subsets of analytes (described below) by Lasso and Random Forest methods. Lasso significant transitions correspond to those with non-zero coefficients and Random Forest analye ranking was determined by the Gini importance values (mean decrease in model accuracy if that variable is removed). We report all analytes with non-zero Lasso coefficients (Tables 16-32) and the top 30 analytes from each Random Forest analysis (Tables 33-49). Models were built considering the top univariate 32 or 100 analytes, the single best univariate analyte for the top 50 proteins or all analytes. Lastly 1000 rounds of bootstrap resampling were performed and the nonzero Lasso coefficients or Random Forest Gini importance values were summed for each analyte amongst panels with AUCs of 0.85 or greater.

**Table 12. Early Window Individual Stats**

| **Transition** | **Protein** | **AUC** |
|---|---|---|
| ELIEELVNITQNQK_557.6_517.3 | IL13_HUMAN | 0.834 |
| ITLPDFTGDLR_624.3_288.2 | LBP_HUMAN | 0.822 |
| FLNWIK_410.7_560.3 | HABP2_HUMAN | 0.820 |
| ITLPDFTGDLR_624.3_920.5 | LBP_HUMAN | 0.808 |
| SFRPFVPR_335.9_635.3 | LBP_HUMAN | 0.800 |
| LIQDAVTGLTVNGQITGDK_972.0_798.4 | ITIH3_HUMAN | 0.800 |
| FSVVYAK_407.2_579.4 | FETUA_HUMAN | 0.796 |
| ITGFLKPGK_320.9_429.3 | LBP_HUMAN | 0.796 |
| AHYDLR_387.7_288.2 | FETUA_HUMAN | 0.796 |
| FSVVYAK_407.2_381.2 | FETUA_HUMAN | 0.795 |
| SFRPFVPR_335.9_272.2 | LBP_HUMAN | 0.795 |
| DVLLLVHNLPQNLPGYFWYK_810.4_967.5 | PSG9_HUMAN | 0.794 |
| ELIEELVNITQNQK_557.6_618.3 | IL13_HUMAN | 0.794 |
| QALEEFQK_496.8_680.3 | CO8B_HUMAN | 0.792 |
| DAGLSWGSAR_510.3_390.2 | NEUR4_HUMAN | 0.792 |
| AHYDLR_387.7_566.3 | FETUA_HUMAN | 0.791 |
| VFQFLEK_455.8_811.4 | CO5_HUMAN | 0.786 |
| ITGFLKPGK_320.9_301.2 | LBP_HUMAN | 0.783 |
| VFQFLEK_455.8_276.2 | CO5_HUMAN | 0.782 |
| SLLQPNK_400.2_599.4 | CO8A_HUMAN | 0.781 |
| VQTAHFK_277.5_431.2 | CO8A_HUMAN | 0.780 |
| SDLEVAHYK_531.3_617.3 | CO8B_HUMAN | 0.777 |
| SLLQPNK_400.2_358.2 | CO8A_HUMAN | 0.776 |
| TLLPVSKPEIR_418.3_288.2 | CO5_HUMAN | 0.776 |
| ALNHLPLEYNSALYSR_621.0_538.3 | CO6_HUMAN | 0.774 |
| DISEVVTPR_508.3_787.4 | CFAB_HUMAN | 0.774 |
| VSEADSSNADWVTK_754.9_533.3 | CFAB_HUMAN | 0.773 |
| LSSPAVITDK_ 515.8_743.4 | PLMN_HUMAN | 0.773 |
| VQEAHLTEDQIFYFPK_655.7_701.4 | CO8G_HUMAN | 0.772 |
| DVLLLVHNLPQNLPGYFWYK_810.4_594.3 | PSG9_HUMAN | 0.771 |
| ALVLELAK_428.8_672.4 | INHBE_HUMAN | 0.770 |
| FLNWIK_410.7_561.3 | HABP2_HUMAN | 0.770 |
| LSSPAVITDK_ 515.8_830.5 | PLMN_HUMAN | 0.769 |
| LPNNVLQEK_527.8_844.5 | AFAM_HUMAN | 0.769 |
| VSEADSSNADWVTK_754.9_347.2 | CFAB_HUMAN | 0.768 |
| HTLNQIDEVK_598.8_951.5 | FETUA_HUMAN | 0.767 |
| TTSDGGYSFK_531.7_860.4 | INHA HUMAN | 0.761 |
| YENYTSSFFIR_713.8_756.4 | IL12B_HUMAN | 0.760 |
| HTLNQIDEVK_598.8_958.5 | FETUA_HUMAN | 0.760 |
| DISEVVTPR_508.3_472.3 | CFAB_HUMAN | 0.760 |
| LIQDAVTGLTVNGQITGDK_972.0_640.4 | ITIH3_HUMAN | 0.759 |
| EAQLPVIENK_570.8_699.4 | PLMN_HUMAN | 0.759 |
| SLPVSDSVLSGFEQR_810.9_836.4 | CO8G_HUMAN | 0.757 |
| AVLHIGEK_289.5_348.7 | THBG_HUMAN | 0.755 |
| GLQYAAQEGLLALQSELLR_1037.1_929.5 | LBP_HUMAN | 0.752 |
| FLQEQGHR_338.8_497.3 | CO8G_HUMAN | 0.750 |
| LPNNVLQEK_527.8_730.4 | AFAM_HUMAN | 0.750 |
| AVLHIGEK_289.5_292.2 | THBG_HUMAN | 0.749 |
| QLYGDTGVLGR_589.8_501.3 | CO8G_HUMAN | 0.748 |
| WWGGQPLWITATK_772.4_929.5 | ENPP2_HUMAN | 0.747 |
| NADYSYSVWK_616.8_769.4 | CO5_HUMAN | 0.746 |
| GLQYAAQEGLLALQSELLR_1037.1_858.5 | LBP_HUMAN | 0.746 |
| SLPVSDSVLSGFEQR_810.9_723.3 | CO8G_HUMAN | 0.745 |
| IEEIAAK_387.2_531.3 | CO5_HUMAN | 0.743 |
| TYLHTYESEI_628.3_908.4 | ENPP2_HUMAN | 0.742 |
| WWGGQPLWITATK_772.4_373.2 | ENPP2_HUMAN | 0.742 |
| FQLSETNR_497.8_605.3 | PSG2_HUMAN | 0.741 |
| NIQSVNVK_451.3_674.4 | GROA_HUMAN | 0.741 |
| TGVAVNKPAEFTVDAK_549.6_258.1 | FLNA_HUMAN | 0.740 |
| LQGTLPVEAR_542.3_571.3 | CO5_HUMAN | 0.740 |
| SGFSFGFK_438.7_732.4 | CO8B_HUMAN | 0.740 |
| HELTDEELQSLFTNFANVVDK_817.1_906.5 | AFAM_HUMAN | 0.740 |
| VQTAHFK_277.5_502.3 | CO8A_HUMAN | 0.739 |
| YENYTSSFFIR_713.8_293.1 | IL12B_HUMAN | 0.739 |
| AFTECCVVASQLR_770.9_574.3 | CO5_HUMAN | 0.736 |
| EAQLPVIENK_570.8_329.2 | PLMN_HUMAN | 0.734 |
| QALEEFQK_496.8_551.3 | CO8B_HUMAN | 0.734 |
| DAQYAPGYDK_564.3_813.4 | CFAB_HUMAN | 0.734 |
| TEFLSNYLTNVDDITLVPGTLGR_846.8_600.3 | ENPP2_HUMAN | 0.734 |
| IAIDLFK_410.3_635.4 | HEP2_HUMAN | 0.733 |
| TASDFITK_441.7_781.4 | GELS_HUMAN | 0.731 |
| YEFLNGR_449.7_606.3 | PLMN_HUMAN | 0.731 |
| TVQAVLTVPK_528.3_428.3 | PEDF_HUMAN | 0.731 |
| LIENGYFHPVK_439.6_627.4 | F13B_HUMAN | 0.730 |
| DALSSVQESQVAQQAR_573.0_672.4 | APOC3_HUMAN | 0.730 |
| TVQAVLTVPK_528.3_855.5 | PEDF _HUMAN | 0.730 |
| ALQDQLVLVAAK_634.9_289.2 | ANGT_HUMAN | 0.727 |
| TYLHTYESEI_628.3_515.3 | ENPP2_HUMAN | 0.727 |
| SDLEVAHYK_531.3_746.4 | CO8B_HUMAN | 0.726 |
| FLPCENK_454.2_550.2 | IL10_HUMAN | 0.725 |
| HPWIVHWDQLPQYQLNR_744.0_1047.0 | KS6A3_HUMAN | 0.725 |
| AFTECCVVASQLR_770.9_673.4 | CO5_HUMAN | 0.725 |
| YGLVTYATYPK_638.3_843.4 | CFAB_HUMAN | 0.724 |
| TLEAQLTPR_514.8_685.4 | HEP2_HUMAN | 0.724 |
| DAQYAPGYDK_564.3_315.1 | CFAB_HUMAN | 0.724 |
| QGHNSVFLIK_381.6_260.2 | HEMO_HUMAN | 0.722 |
| HELTDEELQSLFTNFANVVDK_817.1_854.4 | AFAM_HUMAN | 0.722 |
| TLEAQLTPR_514.8_814.4 | HEP2_HUMAN | 0.721 |
| IEEIAAK_387.2_660.4 | CO5_HUMAN | 0.721 |
| HFQNLGK_422.2_527.2 | AFAM_HUMAN | 0.721 |
| IAPQLSTEELVSLGEK_857.5_333.2 | AFAM_HUMAN | 0.721 |
| DALSSVQESQVAQQAR_573.0_502.3 | APOC3_HUMAN | 0.720 |
| ALNHLPLEYNSALYSR_621.0_696.4 | CO6_HUMAN | 0.719 |
| IAIDLFK_410.3_706.4 | HEP2_HUMAN | 0.719 |
| FLQEQGHR_338.8_369.2 | CO8G_HUMAN | 0.719 |
| ALQDQLVLVAAK_634.9_956.6 | ANGT_HUMAN | 0.718 |
| IEGNLIFDPNNYLPK_874.0_414.2 | APOB_HUMAN | 0.717 |
| YEFLNGR_449.7_293.1 | PLMN_HUMAN | 0.717 |
| TASDFITK_441.7_710.4 | GELS_HUMAN | 0.716 |
| DADPDTFFAK_563.8_825.4 | AFAM_HUMAN | 0.716 |
| TLLPVSKPEIR_418.3_514.3 | CO5_HUMAN | 0.716 |
| NADYSYSVWK_616.8_333.2 | CO5_HUMAN | 0.715 |
| YGLVTYATYPK_638.3_334.2 | CFAB_HUMAN | 0.715 |
| VNHVTLSQPK_374.9_459.3 | B2MG_HUMAN | 0.715 |
| HYGGLTGLNK_530.3_759.4 | PGAM1_HUMAN | 0.714 |
| DFHINLFQVLPWLK_885.5_400.2 | CFAB_HUMAN | 0.714 |
| NCSFSIIYPVVIK_770.4_555.4 | CRHBP_HUMAN | 0.714 |
| HPWIVHWDQLPQYQLNR_744.0_918.5 | KS6A3_HUMAN | 0.712 |
| AQPVQVAEGSEPDGFWEALGGK_758.0_574.3 | GELS_HUMAN | 0.711 |
| ALDLSLK_380.2_185.1 | ITIH3_HUMAN | 0.711 |
| ALDLSLK_380.2_575.3 | ITIH3_HUMAN | 0.710 |
| LDFHFSSDR_375.2_611.3 | INHBC_HUMAN | 0.709 |
| TLNAYDHR_330.5_312.2 | PAR3_HUMAN | 0.707 |
| EVFSKPISWEELLQ_852.9_260.2 | FA40A_HUMAN | 0.706 |
| IAPQLSTEELVSLGEK_857.5_533.3 | AFAM_HUMAN | 0.704 |
| LIENGYFHPVK_439.6_343.2 | F13B_HUMAN | 0.703 |
| NFPSPVDAAFR_610.8_775.4 | HEMO_HUMAN | 0.703 |
| QLYGDTGVLGR_589.8_345.2 | CO8G_HUMAN | 0.702 |
| LYYGDDEK_501.7_563.2 | CO8A_HUMAN | 0.702 |
| FQLSETNR_497.8_476.3 | PSG2_HUMAN | 0.701 |
| TGVAVNKPAEFTVDAK_549.6_977.5 | FLNA_HUMAN | 0.700 |
| IPGIFELGISSQSDR_809.9_679.3 | CO8B_HUMAN | 0.700 |
| TLFIFGVTK_513.3_215.1 | PSG4_HUMAN | 0.699 |
| YYGYTGAFR_549.3_450.3 | TRFL_HUMAN | 0.699 |
| QVFAVQR_424.2_473.3 | ELNE_HUMAN | 0.699 |
| AQPVQVAEGSEPDGFWEALGGK_758.0_623.4 | GELS_HUMAN | 0.699 |
| DFNQFSSGEK_386.8_189.1 | FETA_HUMAN | 0.699 |
| SVSLPSLDPASAK_636.4_473.3 | APOB_HUMAN | 0.699 |
| GNGLTWAEK_488.3_634.3 | C163B_HUMAN | 0.698 |
| LYYGDDEK_501.7_726.3 | CO8A_HUMAN | 0.698 |
| NFPSPVDAAFR_610.8_959.5 | HEMO_HUMAN | 0.698 |
| FAFNLYR_465.8_565.3 | HEP2 _HUMAN | 0.697 |
| SGFSFGFK_438.7_585.3 | CO8B_HUMAN | 0.696 |
| DFHINLFQVLPWLK_885.5_543.3 | CFAB_HUMAN | 0.696 |
| LQGTLPVEAR_542.3_842.5 | CO5_HUMAN | 0.694 |
| GAVHVVVAETDYQSFAVLYLER_822.8_863.5 | CO8G_HUMAN | 0.694 |
| TSESTGSLPSPFLR_739.9_716.4 | PSMG1_HUMAN | 0.694 |
| YISPDQLADLYK_713.4_277.2 | ENOA_HUMAN | 0.694 |
| ESDTSYVSLK_564.8_347.2 | CRP_HUMAN | 0.693 |
| ILDDLSPR_464.8_587.3 | ITIH4_HUMAN | 0.693 |
| VQEAHLTEDQIFYFPK_655.7_391.2 | CO8G_HUMAN | 0.692 |
| SGVDLADSNQK_567.3_662.3 | VGFR3_HUMAN | 0.692 |
| DTDTGALLFIGK_625.8_217.1 | PEDF_HUMAN | 0.692 |
| HFQNLGK_422.2_285.1 | AFAM_HUMAN | 0.691 |
| NNQLVAGYLQGPNVNLEEK_700.7_999.5 | IL1RA_HUMAN | 0.691 |
| IPGIFELGISSQSDR_809.9_849.4 | CO8B_HUMAN | 0.691 |
| ESDTSYVSLK_564.8_696.4 | CRP_HUMAN | 0.690 |
| GAVHVVVAETDYQSFAVLYLER_822.8_580.3 | CO8G_HUMAN | 0.690 |
| DADPDTFFAK_563.8_302.1 | AFAM_HUMAN | 0.690 |
| LDFHFSSDR_375.2_464.2 | INHBC_HUMAN | 0.689 |
| TLFIFGVTK_513.3_811.5 | PSG4_HUMAN | 0.688 |
| DFNQFSSGEK_386.8_333.2 | FETA_HUMAN | 0.687 |
| IQTHSTTYR_369.5_627.3 | F13B_HUMAN | 0.686 |
| HYFIAAVER_553.3_658.4 | FA8_HUMAN | 0.686 |
| VNHVTLSQPK_374.9_244.2 | B2MG_HUMAN | 0.686 |
| DLHLSDVFLK_396.2_366.2 | CO6_HUMAN | 0.685 |
| DPTFIPAPIQAK_433.2_556.3 | ANGT_HUMAN | 0.684 |
| AGITIPR_364.2_272.2 | IL17_HUMAN | 0.684 |
| IAQYYYTFK_598.8_884.4 | F13B_HUMAN | 0.684 |
| SGVDLADSNQK_567.3_591.3 | VGFR3_HUMAN | 0.683 |
| VEPLYELVTATDFAYSSTVR_754.4_549.3 | CO8B_HUMAN | 0.682 |
| AGITIPR_364.2_486.3 | IL17_HUMAN | 0.682 |
| YEVQGEVFTKPQLWP_911.0_293.1 | CRP_HUMAN | 0.681 |
| APLTKPLK_289.9_357.2 | CRP_HUMAN | 0.681 |
| YNSQLLSFVR_613.8_508.3 | TFR1_HUMAN | 0.681 |
| ANDQYLTAAALHNLDEAVK_686.4_301.1 | IL1A_HUMAN | 0.681 |
| IQTHSTTYR_369.5_540.3 | F13B_HUMAN | 0.681 |
| IHPSYTNYR_575.8_598.3 | PSG2_HUMAN | 0.681 |
| TEFLSNYLTNVDDITLVPGTLGR_846.8_699.4 | ENPP2_HUMAN | 0.681 |
| DPTFIPAPIQAK_433.2_461.2 | ANGT_HUMAN | 0.679 |
| FQSVFTVTR_542.8_623.4 | C1QC_HUMAN | 0.679 |
| LQVNTPLVGASLLR_741.0_925.6 | BPIA1_HUMAN | 0.679 |
| DEIPHNDIALLK_459.9_510.8 | HABP2_HUMAN | 0.678 |
| HATLSLSIPR_ 365.6_272.2 | VGFR3_HUMAN | 0.678 |
| EDTPNSVWEPAK_686.8_315.2 | C1S_HUMAN | 0.678 |
| TGISPLALIK_506.8_741.5 | APOB_HUMAN | 0.678 |
| ILPSVPK_377.2_244.2 | PGH1_HUMAN | 0.676 |
| HATLSLSIPR_ 365.6_472.3 | VGFR3_HUMAN | 0.676 |
| QGHNSVFLIK_381.6_520.4 | HEMO_HUMAN | 0.676 |
| LPATEKPVLLSK_432.6_460.3 | HYOU1_HUMAN | 0.675 |
| APLTKPLK_289.9_398.8 | CRP_HUMAN | 0.674 |
| GVTGYFTFNLYLK_508.3_683.9 | PSG5_HUMAN | 0.673 |
| TFLTVYWTPER_706.9_401.2 | ICAM1_HUMAN | 0.673 |
| GDTYPAELYITGSILR_885.0_274.1 | F13B_HUMAN | 0.672 |
| EDTPNSVWEPAK_686.8_630.3 | C1S_HUMAN | 0.672 |
| SLDFTELDVAAEK_719.4_316.2 | ANGT_HUMAN | 0.672 |
| VELAPLPSWQPVGK_760.9_342.2 | ICAM1_HUMAN | 0.671 |
| GPGEDFR_389.2_322.2 | PTGDS_HUMAN | 0.670 |
| TDAPDLPEENQAR_728.3_843.4 | CO5_HUMAN | 0.670 |
| GVTGYFTFNLYLK_508.3_260.2 | PSG5_HUMAN | 0.669 |
| FAFNLYR_465.8_712.4 | HEP2_HUMAN | 0.669 |
| ITENDIQJALDDAK_779.9_873.5 | APOB_HUMAN | 0.669 |
| ILNIFGVIK_508.8_790.5 | TFR1_HUMAN | 0.669 |
| ISQGEADINIAFYQR_575.6_684.4 | MMP8_HUMAN | 0.668 |
| GDTYPAELYITGSILR_885.0_1332.8 | F13B_HUMAN | 0.668 |
| ELLESYIDGR_597.8_710.4 | THRB_HUMAN | 0.668 |
| FTITAGSK_412.7_576.3 | FABPL_HUMAN | 0.667 |
| ILDGGNK_358.7_490.2 | CXCL5_HUMAN | 0.667 |
| GWVTDGFSSLK_598.8_854.4 | APOC3_HUMAN | 0.667 |
| FSLVSGWGQLLDR_493.3_403.2 | FA7_HUMAN | 0.665 |
| IHPSYTNYR_575.8_813.4 | PSG2_HUMAN | 0.665 |
| ELLESYIDGR_597.8_839.4 | THRB_HUMAN | 0.665 |
| SDGAKPGPR_442.7_213.6 | COLI_HUMAN | 0.664 |
| IAQYYYTFK_598.8_395.2 | F13B_HUMAN | 0.664 |
| SILFLGK_389.2_201.1 | THBG_HUMAN | 0.664 |
| IEVNESGTVASSSTAVIVSAR_693.0_545.3 | PAI1_HUMAN | 0.664 |
| VSAPSGTGHLPGLNPL_ 506.3_300.7 | PSG3_HUMAN | 0.664 |
| LLAPSDSPEWLSFDVTGVVR_730.1_430.3 | TGFB1_HUMAN | 0.664 |
| YYGYTGAFR_549.3_771.4 | TRFL_HUMAN | 0.663 |
| TDAPDLPEENQAR_728.3_613.3 | CO5_HUMAN | 0.663 |
| IEVIITLK_464.8_815.5 | CXL11_HUMAN | 0.662 |
| ILPSVPK_377.2_227.2 | PGH1_HUMAN | 0.662 |
| FGFGGSTDSGPIR_ 649.3_745.4 | ADA12_HUMAN | 0.661 |
| DYWSTVK_449.7_347.2 | APOC3_HUMAN | 0.661 |
| IEGNLIFDPNNYLPK_874.0_845.5 | APOB_HUMAN | 0.661 |
| WILTAAHTLYPK_ 471.9_407.2 | C1R_HUMAN | 0.661 |
| WNFAYWAAHQPWSR_607.3_545.3 | PRG2_HUMAN | 0.661 |
| SILFLGK_389.2_577.4 | THBG_HUMAN | 0.661 |
| FSLVSGWGQLLDR_493.3_516.3 | FA7_HUMAN | 0.661 |
| DTDTGALLFIGK_625.8_818.5 | PEDF_HUMAN | 0.661 |
| SEYGAALAWEK_612.8_845.5 | CO6_HUMAN | 0.660 |
| LWAYLTIQELLAK_781.5_371.2 | ITIH1_HUMAN | 0.660 |
| LLEVPEGR_456.8_356.2 | C1S_HUMAN | 0.659 |
| ITENDIQIALDDAK_779.9_632.3 | APOB_HUMAN | 0.659 |
| LTTVDIVTLR_565.8_716.4 | IL2RB_HUMAN | 0.658 |
| IEVIITLK_464.8_587.4 | CXL11_HUMAN | 0.658 |
| QLGLPGPPDVPDHAAYHPF_676.7_299.2 | ITIH4_HUMAN | 0.658 |
| TLAFVR_353.7_492.3 | FA7_HUMAN | 0.656 |
| NSDQEIDFK_548.3_294.2 | S10A5_HUMAN | 0.656 |
| YHFEALADTGISSEFYDNANDLLSK_940.8_874.5 | C08A_HUMAN | 0.656 |
| SEPRPGVLLR_375.2_454.3 | FA7_HUMAN | 0.655 |
| FLPCENK_454.2_390.2 | IL10_HUMAN | 0.654 |
| NCSFSIIYPVVIK_770.4_831.5 | CRHBP_HUMAN | 0.654 |
| SLDFTELDVAAEK_719.4_874.5 | ANGT_HUMAN | 0.654 |
| ILLLGTAVESAWGDEQSAFR_721.7_909.4 | CXA1_HUMAN | 0.653 |
| SVSLPSLDPASAK_636.4_885.5 | APOB_HUMAN | 0.653 |
| TGISPLALIK_506.8_654.5 | APOB_HUMAN | 0.653 |
| YNQLLR_403.7_288.2 | ENOA_HUMAN | 0.653 |
| YEVQGEVFTKPQLWP_911.0_392.2 | CRP_HUMAN | 0.652 |
| VPGLYYFTYHASSR_554.3_720.3 | C1QB_HUMAN | 0.650 |
| SLQNASAIESILK_687.4_589.4 | IL3_HUMAN | 0.650 |
| WILTAAHTLYPK_471.9_621.4 | C1R_HUMAN | 0.650 |
| GWVTDGFSSLK_598.8_953.5 | APOC3_HUMAN | 0.650 |
| YGIEEHGK_311.5_599.3 | CXA1_HUMAN | 0.649 |
| QDLGWK_373.7_503.3 | TGFB3_HUMAN | 0.649 |
| DYWSTVK_449.7_620.3 | APOC3_HUMAN | 0.648 |
| ALVLELAK_428.8_331.2 | INHBE_HUMAN | 0.647 |
| QLGLPGPPDVPDHAAYHPF_676.7_263.1 | ITIH4_HUMAN | 0.646 |
| SEYGAALAWEK_612.8_788.4 | CO6_HUMAN | 0.645 |
| TFLTVYWTPER_706.9_502.3 | ICAM1_HUMAN | 0.644 |
| FQSVFTVTR_542.8_722.4 | C1QC_HUMAN | 0.643 |
| DPNGLPPEAQK_583.3_669.4 | RET4_HUMAN | 0.642 |
| ETLLQDFR_511.3_322.2 | AMBP_HUMAN | 0.642 |
| IIEVEEEQEDPYLNDR_996.0_777.4 | FBLN1_HUMAN | 0.641 |
| ELCLDPK_437.7_359.2 | IL8_HUMAN | 0.641 |
| TPSAAYLWVGTGASEAEK_919.5_849.4 | GELS_HUMAN | 0.641 |
| NQSPVLEPVGR_598.3_866.5 | KS6A3_HUMAN | 0.641 |
| FNAVLTNPQGDYDTSTGK_964.5_333.2 | C1QC_HUMAN | 0.641 |
| LLEVPEGR_456.8_686.4 | C1S_HUMAN | 0.641 |
| FFQYDTWK_567.8_840.4 | IGF2_HUMAN | 0.640 |
| SPEAEDPLGVER_649.8_670.4 | Z512B_HUMAN | 0.639 |
| SEPRPGVLLR_375.2_654.4 | FA7_HUMAN | 0.639 |
| SGAQATWTELPWPHEK_613.3_793.4 | HEMO_HUMAN | 0.638 |
| YSHYNER_323.5_581.3 | HABP2_HUMAN | 0.638 |
| YHFEALADTG!SSEFYDNANDLLSK_940.8_301.1 | CO8A_HUMAN | 0.637 |
| DLHLSDVFLK_396.2_260.2 | CO6_HUMAN | 0.637 |
| YSHYNER_323.5_418.2 | HABP2_HUMAN | 0.637 |
| YYLQGAK_421.7_327.1 | ITIH4_HUMAN | 0.636 |
| EVPLSALTNILSAQLISHWK_740.8_996.6 | PAI1_HUMAN | 0.636 |
| VPGLYYFTYHASSR_554.3_420.2 | C1QB_HUMAN | 0.636 |
| AALAAFNAQNNGSNFQLEEISR_789.1_746.4 | FETUA_HUMAN | 0.636 |
| ETLLQDFR_511.3_565.3 | AMBP_HUMAN | 0.635 |
| IVLSLDVPIGLLQILLEQAR_735.1_503.3 | UCN2_HUMAN | 0.635 |
| ENPAVIDFELAPIVDLVR_670.7_811.5 | CO6_HUMAN | 0.635 |
| LQLSETNR_480.8_355.2 | PSG8_HUMAN | 0.635 |
| DPDQTDGLGLSYLSSHIANVER_796.4_456.2 | GELS_HUMAN | 0.635 |
| NVNQSLLELHK_432.2_656.3 | FRIH_HUMAN | 0.634 |
| EIGELYLPK_531.3_633.4 | AACT_HUMAN | 0.634 |
| SPEQQETVLDGNLIIR_906.5_699.3 | ITIH4_HUMAN | 0.634 |
| NKPGVYTDVAYYLAWIR_ 677.0_545.3 | FA12_HUMAN | 0.632 |
| QNYHQDSEAAINR_515.9_544.3 | FRIH_HUMAN | 0.632 |
| EKPAGGIPVLGSLVNTVLK_631.4_930.6 | BPIB1_HUMAN | 0.632 |
| VTFEYR_407.7_614.3 | CRHBP_HUMAN | 0.630 |
| DLPHITVDR_533.3_490.3 | MMP7_HUMAN | 0.630 |
| VEHSDLSFSK_383.5_234.1 | B2MG_HUMAN | 0.630 |
| ENPAVIDFELAPIVDLVR_670.7_601.4 | CO6_HUMAN | 0.630 |
| YGFYTHVFR_397.2_659.4 | THRB_HUMAN | 0.629 |
| ILDDLSPR_464.8_702.3 | ITIH4_HUMAN | 0.629 |
| DPNGLPPEAQK_583.3_497.2 | RET4_HUMAN | 0.629 |
| GSLVQASEANLQAAQDFVR_668.7_806.4 | ITIH1_HUMAN | 0.629 |
| FLYHK_354.2_447.2 | AMBP_HUMAN | 0.627 |
| FNAVLTNPQGDYDTSTGK_964.5_262.1 | C1QC_HUMAN | 0.627 |
| LQDAGVYR_461.2_680.3 | PD1L1_HUMAN | 0.627 |
| INPASLDK_429.2_630.4 | C163A_HUMAN | 0.626 |
| LEEHYELR_363.5_580.3 | PAI2_HUMAN | 0.625 |
| VEHSDLSFSK_383.5_468.2 | B2MG_HUMAN | 0.624 |
| TSDQIHFFFAK_447.6_659.4 | ANT3_HUMAN | 0.624 |
| ATLSAAPSNPR_542.8_570.3 | CXCL2_HUMAN | 0.624 |
| YGFYTHVFR_397.2_421.3 | THRB_HUMAN | 0.624 |
| EANQSTLENFLER_775.9_678.4 | IL4_HUMAN | 0.623 |
| GQQPADVTGTALPR_705.9_314.2 | CSF1_HUMAN | 0.623 |
| VELAPLPSWQPVGK_760.9_400.3 | ICAM1_HUMAN | 0.622 |
| GEVTYTTSQVSK_650.3_750.4 | EGLN_HUMAN | 0.622 |
| SLQAFVAVAAR_566.8_487.3 | IL23A_HUMAN | 0.622 |
| HYGGLTGLNK_530.3_301.1 | PGAM1_HUMAN | 0.622 |
| GPEDQDISISFAWDK_854.4_753.4 | DEF4_HUMAN | 0.622 |
| YVVISQGLDKPR_458.9_400.3 | LRP1_HUMAN | 0.621 |
| LWAYLTIQELLAK_781.5_300.2 | ITIH1_HUMAN | 0.621 |
| SGAQATWTELPWPHEK_613.3_510.3 | HEMO_HUMAN | 0.621 |
| GTAEWLSFDVTDTVR_848.9_952.5 | TGFB3_HUMAN | 0.621 |
| FFQYDTWK_567.8_712.3 | IGF2_HUMAN | 0.621 |
| AHQLAIDTYQEFEETYIPK_766.0_634.4 | CSH_HUMAN | 0.620 |
| LPATEKPVLLSK_432.6_347.2 | HYOU1_HUMAN | 0.620 |
| NIQSVNVK_451.3_546.3 | GROA_HUMAN | 0.620 |
| TAVTANLDIR_537.3_288.2 | CHL1_HUMAN | 0.619 |
| WSAGLTSSQVDLYIPK_883.0_515.3 | CBG_HUMAN | 0.616 |
| QINSYVK_426.2_496.3 | CBG_HUMAN | 0.616 |
| GFQALGDAADIR_617.3_288.2 | TIMP1_HUMAN | 0.615 |
| WNFAYWAAHQPWSR_607.3_673.3 | PRG2_HUMAN | 0.615 |
| NEIWYR_440.7_357.2 | FA12_HUMAN | 0.615 |
| VLEPTLK_400.3_587.3 | VTDB_HUMAN | 0.614 |
| YYLQGAK_421.7_516.3 | ITIH4_HUMAN | 0.614 |
| ALNSIIDVYHK_424.9_774.4 | S10A8_HUMAN | 0.614 |
| ETPEGAEAKPWYEPIYLGGVFQLEK_951.1_877.5 | TNFA_HUMAN | 0.614 |
| LNIGYIEDLK_589.3_837.4 | PAI2_HUMAN | 0.614 |
| NVNQSLLELHK_432.2_543.3 | FRIH_HUMAN | 0.613 |
| ILLLGTAVESAWGDEQSAFR_721.7_910.6 | CXA1_HUMAN | 0.613 |
| AALAAFNAQNNGSNFQLEEISR_789.1_633.3 | FETUA_HUMAN | 0.613 |
| VLEPTLK_400.3_458.3 | VTDB_HUMAN | 0.613 |
| VGEYSLYIGR_578.8_708.4 | SAMP_HUMAN | 0.613 |
| DIPHWLNPTR_416.9_373.2 | PAPP1_HUMAN | 0.612 |
| NEIVFPAGILQAPFYTR_968.5_357.2 | ECE1_HUMAN | 0.612 |
| AEHPTWGDEQLFQTTR_639.3_765.4 | PGH1_HUMAN | 0.612 |
| VEPLYELVTATDFAYSSTVR_754.4_712.4 | CO8B_HUMAN | 0.611 |
| DEIPHNDIALLK_459.9_260.2 | HABP2_HUMAN | 0.611 |
| QINSYVK_426.2_610.3 | CBG_HUMAN | 0.610 |
| SWNEPLYHLVTEVR_581.6_614.3 | PRL_HUMAN | 0.610 |
| YGIEEHGK_311.5_341.2 | CXA1_HUMAN | 0.610 |
| FGFGGSTDSGPIR_ 649.3_946.5 | ADA12_HUMAN | 0.610 |
| ANDQYLTAAALHNLDEAVK_686.4_317.2 | IL1A_HUMAN | 0.610 |
| VRPQQLVK_484.3_609.4 | ITIH4_HUMAN | 0.609 |
| IPKPEASFSPR_410.2_506.3 | ITIH4_HUMAN | 0.609 |
| SPEQQETVLDGNLIIR_906.5_685.4 | ITIH4_HUMAN | 0.609 |
| DDLYVSDAFHK_655.3_704.3 | ANT3_HUMAN | 0.609 |
| ELPEHTVK_476.8_347.2 | VTDB_HUMAN | 0.609 |
| FLYHK_354.2_284.2 | AMBP_HUMAN | 0.608 |
| QRPPDLDTSSNAVDLLFFTDESGDSR_961.5_262.2 | C1R_HUMAN | 0.608 |
| DPDQTDGLGLSYLSSHIANVER_796.4_328.1 | GELS_HUMAN | 0.608 |
| NEIWYR_440.7_637.4 | FA12_HUMAN | 0.607 |
| LQLSETN R_480.8_672.4 | PSG8_HUMAN | 0.606 |
| GQVPENEANVVITTLK_571.3_462.3 | CADH1_HUMAN | 0.606 |
| FTGSQPFGQGVEHATANK_626.0_521.2 | TSP1_HUMAN | 0.605 |
| LEPLYSASGPGLRPLVIK_637.4_260.2 | CAA60698 | 0.605 |
| QRPPDLDTSSNAVDLLFFTDESGDSR_961.5_866.3 | C1R_HUMAN | 0.604 |
| LTTVDIVTLR_565.8_815.5 | IL2RB_HUMAN | 0.604 |
| TSDQ!HFFFAK_447.6_512.3 | ANT3_HUMAN | 0.604 |
| IQHPFTVEEFVLPK_562.0_861.5 | PZP_HUMAN | 0.603 |
| NKPGVYTDVAYYLAWIR_ 677.0_821.5 | FA12_HUMAN | 0.603 |
| TEQAAVAR_423.2_615.4 | FA12_HUMAN | 0.603 |
| EIGELYLPK_531.3_819.5 | AACT_HUMAN | 0.602 |
| LFYADHPFIFLVR_546.6_647.4 | SERPH_HUMAN | 0.602 |
| AEHPTWGDEQLFQTTR_639.3_569.3 | PGH1_HUMAN | 0.601 |
| TSYQVYSK_488.2_787.4 | C163A_HUMAN | 0.601 |
| YTTEIIK_434.2_704.4 | C1R_HUMAN | 0.601 |
| NVIQISNDLENLR_509.9_402.3 | LEP_HUMAN | 0.600 |
| AFLEVNEEGSEAAASTAVVIAGR_764.4_685.4 | ANT3_HUMAN | 0.600 |

**Table 13. Middle Window Individual Stats**

| **Transition** | **Protein** | **AUC** |
|---|---|---|
| SEYGAALAWEK_612.8_788.4 | CO6_HUMAN | 0.738 |
| VFQFLEK_455.8_811.4 | CO5_HUMAN | 0.709 |
| ALNHLPLEYNSALYSR_621.0_696.4 | CO6_HUMAN | 0.705 |
| SLDFTELDVAAEK_719.4_316.2 | ANGT_HUMAN | 0.692 |
| VEHSDLSFSK_383.5_234.1 | B2MG_HUMAN | 0.686 |
| LLAPSDSPEWLSFDVTGVVR_730.1_430.3 | TGFB1_HUMAN | 0.683 |
| ALNHLPLEYNSALYSR_621.0_538.3 | CO6_HUMAN | 0.683 |
| VLEPTLK_400.3_458.3 | VTDB_HUMAN | 0.681 |
| LHEAFSPVSYQHDLALLR_699.4_251.2 | FA12_HUMAN | 0.681 |
| SEYGAALAWEK_612.8_845.5 | CO6_HUMAN | 0.679 |
| YGIEEHGK_311.5_599.3 | CXA1_HUMAN | 0.677 |
| ALQDQLVLVAAK_634.9_289.2 | ANGT_HUMAN | 0.675 |
| VLEPTLK_400.3_587.3 | VTDB_HUMAN | 0.667 |
| VNHVTLSQPK_374.9_244.2 | B2MG_HUMAN | 0.665 |
| IEEIAAK_387.2_660.4 | CO5_HUMAN | 0.664 |
| DALSSVQESQVAQQAR_573.0_502.3 | APOC3_HUMAN | 0.664 |
| TLLPVSKPEIR_418.3_514.3 | CO5_HUMAN | 0.662 |
| ALQDQLVLVAAK_634.9_956.6 | ANGT_HUMAN | 0.661 |
| TLAFVR_353.7_492.3 | FA7_HUMAN | 0.661 |
| SEPRPGVLLR_375.2_654.4 | FA7_HUMAN | 0.658 |
| VEHSDLSFSK_383.5_468.2 | B2MG_HUMAN | 0.653 |
| DPTFIPAPIQAK_433.2_461.2 | ANGT_HUMAN | 0.653 |
| QGHNSVFLIK_381.6_260.2 | HEMO_HUMAN | 0.650 |
| SLDFTELDVAAEK_719.4_874.5 | ANGT_HUMAN | 0.650 |
| ELPQSIVYK_538.8_417.7 | FBLN3_HUMAN | 0.649 |
| TYLHTYESEI_628.3_515.3 | ENPP2_HUMAN | 0.647 |
| SLQAFVAVAAR_566.8_804.5 | IL23A_HUMAN | 0.646 |
| AQPVQVAEGSEPDGFWEALGGK_758.0_574.3 | GELS_HUMAN | 0.644 |
| QGHNSVFLIK_381.6_520.4 | HEMO_HUMAN | 0.644 |
| VNHVTLSQPK_374.9_459.3 | B2MG_HUMAN | 0.643 |
| DLHLSDVFLK_396.2_260.2 | CO6_HUMAN | 0.643 |
| TEQAAVAR_423.2_615.4 | FA12_HUMAN | 0.643 |
| GPITSAAELNDPQSILLR_632.4_826.5 | EGLN_HUMAN | 0.643 |
| HFQNLGK_422.2_527.2 | AFAM_HUMAN | 0.642 |
| TEQAAVAR_423.2_487.3 | FA12_HUMAN | 0.642 |
| AVDIPGLEAATPYR_736.9_399.2 | TENA_HUMAN | 0.642 |
| TLFIFGVTK_513.3_811.5 | PSG4_HUMAN | 0.642 |
| DLHLSDVFLK_396.2_366.2 | CO6_HUMAN | 0.641 |
| AFTECCVVASQLR_770.9_574.3 | CO5_HUMAN | 0.640 |
| EVFSKPISWEELLQ_852.9_376.2 | FA40A_HUMAN | 0.639 |
| DPTFIPAPIQAK_433.2_556.3 | ANGT_HUMAN | 0.639 |
| FSLVSGWGQLLDR_493.3_403.2 | FA7_HUMAN | 0.638 |
| HYINLITR_515.3_301.1 | NPY_HUMAN | 0.637 |
| HFQNLGK_422.2_285.1 | AFAM_HUMAN | 0.637 |
| VPLALFALNR_ 557.3_620.4 | PEPD_HUMAN | 0.636 |
| IHPSYTNYR_575.8_813.4 | PSG2_HUMAN | 0.635 |
| IEEIAAK_387.2_531.3 | CO5_HUMAN | 0.635 |
| GEVTYTTSQVSK_650.3_750.4 | EGLN_HUMAN | 0.634 |
| DFNQFSSGEK_386.8_333.2 | FETA_HUMAN | 0.634 |
| VVGGLVALR_442.3_784.5 | FA12_HUMAN | 0.634 |
| SDGAKPGPR_442.7_459.2 | COLI_HUMAN | 0.634 |
| DVLLLVHNLPQNLTGHIWYK_791.8_310.2 | PSG7_HUMAN | 0.634 |
| TLLPVSKPEIR_418.3_288.2 | CO5_HUMAN | 0.633 |
| NKPGVYTDVAYYLAWIR_ 677.0_821.5 | FA12_HUMAN | 0.630 |
| QVFAVQR_424.2_473.3 | ELNE_HUMAN | 0.630 |
| NHYTESISVAK_624.8_415.2 | NEUR1_HUMAN | 0.630 |
| IAPQLSTEELVSLGEK_857.5_333.2 | AFAM_HUMAN | 0.629 |
| IHPSYTNYR_575.8_598.3 | PSG2_HUMAN | 0.627 |
| EVFSKPISWEELLQ_852.9_260.2 | FA40A_HUMAN | 0.627 |
| SILFLGK_389.2_201.1 | THBG_HUMAN | 0.626 |
| IEVIITLK_464.8_587.4 | CXL11_HUMAN | 0.625 |
| VVGGLVALR_442.3_685.4 | FA12_HUMAN | 0.624 |
| VVLSSGSGPGLDLPLVLGLPLQLK_791.5_598.4 | SHBG_HUMAN | 0.624 |
| FGFGGSTDSGPIR_ 649.3_946.5 | ADA12_HUMAN | 0.623 |
| VVLSSGSGPGLDLPLVLGLPLQLK_791.5_768.5 | SHBG_HUMAN | 0.622 |
| YGIEEHGK_311.5_341.2 | CXA1_HUMAN | 0.621 |
| LHEAFSPVSYQHDLALLR_699.4_380.2 | FA12_HUMAN | 0.621 |
| AHYDLR_387.7_566.3 | FETUA_HUMAN | 0.620 |
| FSVVYAK_407.2_381.2 | FETUA_HUMAN | 0.618 |
| ALALPPLGLAPLLNLWAKPQGR_770.5_256.2 | SHBG_HUMAN | 0.618 |
| YENYTSSFFIR_713.8_293.1 | IL12B_HUMAN | 0.617 |
| VELAPLPSWQPVGK_760.9_342.2 | ICAM1_HUMAN | 0.617 |
| S!LFLGK_389.2_577.4 | THBG_HUMAN | 0.616 |
| ILPSVPK_377.2_227.2 | PGH1_HUMAN | 0.615 |
| IPSNPSHR_303.2_496.3 | FBLN3_HUMAN | 0.615 |
| HYFIAAVER_553.3_301.1 | FA8_HUMAN | 0.615 |
| FSVVYAK_407.2_579.4 | FETUA_HUMAN | 0.613 |
| VFQFLEK_455.8_276.2 | CO5_HUMAN | 0.613 |
| IAPQLSTEELVSLGEK_857.5_533.3 | AFAM_HUMAN | 0.613 |
| ILPSVPK_377.2_244.2 | PGH1_HUMAN | 0.613 |
| NKPGVYTDVAYYLAWIR_ 677.0_545.3 | FA12_HUMAN | 0.613 |
| WSAGLTSSQVDLYIPK_883.0_515.3 | CBG_HUMAN | 0.612 |
| TPSAAYLWVGTGASEAEK_919.5_849.4 | GELS_HUMAN | 0.612 |
| ALALPPLGLAPLLNLWAKPQGR_770.5_457.3 | SHBG_HUMAN | 0.612 |
| QLGLPGPPDVPDHAAYHPF_676.7_299.2 | ITIH4_HUMAN | 0.612 |
| ILDDLSPR_464.8_587.3 | ITIH4_HUMAN | 0.611 |
| VELAPLPSWQPVGK_760.9_400.3 | ICAM1_HUMAN | 0.611 |
| DADPDTFFAK_563.8_825.4 | AFAM_HUMAN | 0.611 |
| NHYTESISVAK_624.8_252.1 | NEUR1_HUMAN | 0.611 |
| SEPRPGVLLR_375.2_454.3 | FA7_HUMAN | 0.611 |
| LNIGYIEDLK_589.3_950.5 | PAI2_HUMAN | 0.611 |
| ANLINNIFELAGLGK_793.9_299.2 | LCAP_HUMAN | 0.609 |
| LTTVDIVTLR_565.8_716.4 | IL2RB_HUMAN | 0.608 |
| TQILEWAAER_608.8_761.4 | EGLN_HUMAN | 0.608 |
| NEPEETPSIEK_ 636.8_573.3 | SOX5_HUMAN | 0.608 |
| AQPVQVAEGSEPDGFWEALGGK_758.0_623.4 | GELS_HUMAN | 0.607 |
| LQVNTPLVGASLLR_741.0_925.6 | BPIA1_HUMAN | 0.607 |
| VPSHAVVAR_312.5_345.2 | TRFL_HUMAN | 0.607 |
| SLQNASAIESILK_687.4_860.5 | IL3_HUMAN | 0.607 |
| GVTGYFTFNLYLK_508.3_260.2 | PSG5_HUMAN | 0.605 |
| DFNQFSSGEK_386.8_189.1 | FETA_HUMAN | 0.605 |
| QLGLPGPPDVPDHAAYHPF_676.7_263.1 | ITIH4_HUMAN | 0.605 |
| TLEAQLTPR_514.8_814.4 | HEP2_HUMAN | 0.604 |
| AFTECCVVASQLR_770.9_673.4 | CO5_HUMAN | 0.604 |
| LTTVDIVTLR_565.8_815.5 | IL2RB_HUMAN | 0.604 |
| TLNAYDHR_330.5_312.2 | PAR3_HUMAN | 0.603 |
| LWAYLTIQELLAK_781.5_300.2 | ITIH1_HUMAN | 0.603 |
| GGLFADIASHPWQAAIFAK_667.4_375.2 | TPA_HUMAN | 0.603 |
| IPSNPSHR_303.2_610.3 | FBLN3_HUMAN | 0.603 |
| TDAPDLPEENQAR_728.3_843.4 | CO5_HUMAN | 0.603 |
| SPQAFYR_434.7_684.4 | REL3_HUMAN | 0.602 |
| SSNNPHSPIVEEFQVPYNK_729.4_261.2 | C1S_HUMAN | 0.601 |
| AHYDLR_387.7_288.2 | FETUA_HUMAN | 0.600 |
| DGSPDVTTADIGANTPDATK_973.5_844.4 | PGRP2_HUMAN | 0.600 |
| SPQAFYR_434.7_556.3 | REL3_HUMAN | 0.600 |

**Table 14. Middle Late Individual Stats**

| **Transition** | **Protein** | **AUC** |
|---|---|---|
| ALNHLPLEYNSALYSR_621.0_696.4 | CO6_HUMAN | 0.656 |
| VPLALFALNR_ 557.3_620.4 | PEPD_HUMAN | 0.655 |
| ALNHLPLEYNSALYSR_621.0_538.3 | CO6_HUMAN | 0.652 |
| AVYEAVLR_460.8_587.4 | PEPD_HUMAN | 0.649 |
| SEPRPGVLLR_375.2_654.4 | FA7_HUMAN | 0.644 |
| VFQFLEK_455.8_811.4 | CO5_HUMAN | 0.643 |
| AQPVQVAEGSEPDGFWEALGGK_758.0_574.3 | GELS_HUMAN | 0.640 |
| TLAFVR_353.7_492.3 | FA7_HUMAN | 0.639 |
| TEQAAVAR_423.2_615.4 | FA12_HUMAN | 0.637 |
| YGIEEHGK_311.5_599.3 | CXA1_HUMAN | 0.637 |
| TEQAAVAR_423.2_487.3 | FA12_HUMAN | 0.633 |
| QINSYVK_426.2 496.3 | CBG_HUMAN | 0.633 |
| LIEIANHVDK_384.6_683.4 | ADA12_HUMAN | 0.633 |
| SEYGAALAWEK_612.8_845.5 | CO6_HUMAN | 0.633 |
| ALQDQLVLVAAK_634.9_956.6 | ANGT_HUMAN | 0.628 |
| VLEPTLK_400.3_587.3 | VTDB_HUMAN | 0.628 |
| DFNQFSSGEK_386.8_333.2 | FETA_HUMAN | 0.628 |
| TYLHTYESEI_628.3_515.3 | ENPP2_HUMAN | 0.628 |
| LIEIANHVDK_384.6_498.3 | ADA12_HUMAN | 0.626 |
| QINSYVK_426.2_610.3 | CBG_HUMAN | 0.625 |
| SLDFTELDVAAEK_719.4_316.2 | ANGT_HUMAN | 0.625 |
| DPTFIPAPIQAK_433.2_461.2 | ANGT_HUMAN | 0.625 |
| AVYEAVLR_460.8_750.4 | PEPD_HUMAN | 0.623 |
| YENYTSSFFIR_713.8_756.4 | IL12B_HUMAN | 0.623 |
| SEYGAALAWEK_612.8_788.4 | CO6_HUMAN | 0.623 |
| WSAGLTSSQVDLYIPK_883.0_515.3 | CBG_HUMAN | 0.622 |
| DALSSVQESQVAQQAR_573.0_502.3 | APOC3_HUMAN | 0.622 |
| ALQDQLVLVAAK_634.9_289.2 | ANGT_HUMAN | 0.621 |
| SLQAFVAVAAR_566.8_804.5 | IL23A_HUMAN | 0.621 |
| DPTFIPAPIQAK_433.2_556.3 | ANGT_HUMAN | 0.620 |
| FGFGGSTDSGPIR_ 649.3_946.5 | ADA12_HUMAN | 0.619 |
| VLEPTLK_400.3_458.3 | VTDB_HUMAN | 0.619 |
| SLDFTELDVAAEK_719.4_874.5 | ANGT_HUMAN | 0.618 |
| EVFSKPISWEELLQ_852.9_376.2 | FA40A_HUMAN | 0.618 |
| FGFGGSTDSGPIR_ 649.3_745.4 | ADA12_HUMAN | 0.618 |
| TPSAAYLWVGTGASEAEK_919.5_849.4 | GELS_HUMAN | 0.615 |
| LHEAFSPVSYQHDLALLR_699.4_251.2 | FA12_HUMAN | 0.615 |
| TLEAQLTPR_514.8_685.4 | HEP2_HUMAN | 0.613 |
| ELPQSIVYK_538.8_417.7 | FBLN3_HUMAN | 0.612 |
| GYQELLEK_490.3_631.4 | FETA_HUMAN | 0.612 |
| VPLALFALNR_ 557.3_917.6 | PEPD_HUMAN | 0.611 |
| DLHLSDVFLK_396.2_260.2 | CO6_HUMAN | 0.611 |
| LTTVDIVTLR_565.8_815.5 | IL2RB_HUMAN | 0.608 |
| WSAGLTSSQVDLYIPK_883.0_357.2 | CBG_HUMAN | 0.608 |
| ITQDAQLK_458.8_702.4 | CBG_HUMAN | 0.608 |
| NIQSVNVK_451.3_674.4 | GROA_HUMAN | 0.607 |
| ALEQDLPVNIK_620.4_570.4 | CNDP1_HUMAN | 0.607 |
| TLNAYDHR_330.5_312.2 | PAR3_HUMAN | 0.606 |
| LWAYLTIQELLAK_781.5_300.2 | ITIH1_HUMAN | 0.606 |
| VVGGLVALR_442.3_784.5 | FA12_HUMAN | 0.605 |
| AQPVQVAEGSEPDGFWEALGGK_758.0_623.4 | GELS_HUMAN | 0.603 |
| SVVLIPLGAVDDGEHSQNEK_703.0_798.4 | CNDP1_HUMAN | 0.603 |
| SETEIHQGFQHLHQLFAK_717.4_318.1 | CBG_HUMAN | 0.603 |
| LLAPSDSPEWLSFDVTGVVR_730.1_430.3 | TGFB1_HUMAN | 0.603 |
| IEVIITLK_464.8_587.4 | CXL11_HUMAN | 0.602 |
| ITQDAQLK_458.8_803.4 | CBG_HUMAN | 0.602 |
| AEIEYLEK_497.8_552.3 | LYAM1_HUMAN | 0.601 |
| AVDIPGLEAATPYR_736.9_399.2 | TENA_HUMAN | 0.601 |
| LTTVDIVTLR_565.8_716.4 | IL2RB_HUMAN | 0.600 |
| WWGGQPLWITATK_772.4_929.5 | ENPP2_HUMAN | 0.600 |

**Table 15. Late Window Individual Stats**

| **Transition** | **Protein** | **AUC** |
|---|---|---|
| AVYEAVLR_460.8_587.4 | PEPD_HUMAN | 0.724 |
| AEIEYLEK_497.8_552.3 | LYAM1_HUMAN | 0.703 |
| QJNSYVK_426.2_496.3 | CBG_HUMAN | 0.695 |
| AVYEAVLR_460.8_750.4 | PEPD_HUMAN | 0.693 |
| AALAAFNAQNNGSNFQLEEISR_789.1_746.4 | FETUA_HUMAN | 0.684 |
| QINSYVK_426.2_610.3 | CBG_HUMAN | 0.681 |
| VPLALFALNR_ 557.3_620.4 | PEPD_HUMAN | 0.678 |
| VGVISFAQK_474.8_580.3 | TFR2_HUMAN | 0.674 |
| TGVAVNKPAEFTVDAK_549.6_258.1 | FLNA_HUMAN | 0.670 |
| LIEIANHVDK_384.6_683.4 | ADA12_HUMAN | 0.670 |
| LIEIANHVDK_384.6_498.3 | ADA12_HUMAN | 0.660 |
| SGVDLADSNQK_567.3_662.3 | VGFR3_HUMAN | 0.660 |
| TSYQVYSK_488.2_787.4 | C163A_HUMAN | 0.657 |
| ITQDAQLK_458.8_702.4 | CBG_HUMAN | 0.652 |
| YYGYTGAFR_549.3_450.3 | TRFL_HUMAN | 0.650 |
| ALEQDLPVNIK_620.4_798.5 | CNDP1_HUMAN | 0.650 |
| VFQYIDLHQDEFVQTLK_708.4_375.2 | CNDP1_HUMAN | 0.650 |
| SGVDLADSNQK_567.3_591.3 | VGFR3_HUMAN | 0.648 |
| YENYTSSFFIR_713.8_756.4 | IL12B_HUMAN | 0.647 |
| VLSSIEQK_452.3_691.4 | 1433S_HUMAN | 0.647 |
| YSHYNER_323.5_418.2 | HABP2_HUMAN | 0.646 |
| ILDGGNK_358.7_603.3 | CXCL5_HUMAN | 0.645 |
| GTYLYNDCPGPGQDTDCR_697.0_666.3 | TNR1A_HUMAN | 0.645 |
| AEIEYLEK_497.8_389.2 | LYAM1_HUMAN | 0.645 |
| TLPFSR_360.7_506.3 | LYAM1_HUMAN | 0.645 |
| DEIPHNDIALLK_459.9_510.8 | HABP2_HUMAN | 0.644 |
| ALEQDLPVNIK_620.4_570.4 | CNDP1_HUMAN | 0.644 |
| SPEAEDPLGVER_649.8_314.1 | Z512B_HUMAN | 0.644 |
| FGFGGSTDSGPIR_ 649.3_745.4 | ADA12_HUMAN | 0.642 |
| TASDFITK_441.7_781.4 | GELS_HUMAN | 0.641 |
| SETEIHQGFQHLHQLFAK_717.4_447.2 | CBG_HUMAN | 0.640 |
| SPQAFYR_434.7_556.3 | REL3_HUMAN | 0.639 |
| TAVTANLDIR_537.3_288.2 | CHL1_HUMAN | 0.636 |
| VPLALFALNR_ 557.3_917.6 | PEPD_HUMAN | 0.636 |
| YISPDQLADLYK_713.4_277.2 | ENOA_HUMAN | 0.633 |
| SETEIHQGFQHLHQLFAK_717.4_318.1 | CBG_HUMAN | 0.633 |
| SEPRPGVLLR_375.2_654.4 | FA7_HUMAN | 0.633 |
| GYQELLEK_490.3_631.4 | FETA_HUMAN | 0.633 |
| AYSDLSR_406.2_375.2 | SAMP_HUMAN | 0.633 |
| SVVLIPLGAVDDGEHSQNEK_703.0_798.4 | CNDP1_HUMAN | 0.632 |
| TLEAQLTPR_514.8_685.4 | HEP2_HUMAN | 0.631 |
| WSAGLTSSQVDLYIPK_883.0_515.3 | CBG_HUMAN | 0.631 |
| TEQAAVAR_423.2_615.4 | FA12_HUMAN | 0.628 |
| AQPVQVAEGSEPDGFWEALGGK_758.0_574.3 | GELS_HUMAN | 0.626 |
| AGITIPR_364.2_486.3 | IL17_HUMAN | 0.626 |
| AEVIWTSSDHQVLSGK_586.3_300.2 | PD1L1_HUMAN | 0.625 |
| TEQAAVAR_423.2_487.3 | FA12_HUMAN | 0.625 |
| NHYTESISVAK_624.8_415.2 | NEUR1_HUMAN | 0.625 |
| WSAGLTSSQVDLYIPK_883.0_357.2 | CBG_HUMAN | 0.623 |
| YSHYNER_323.5_581.3 | HABP2_HUMAN | 0.623 |
| DFNQFSSGEK_386.8_333.2 | FETA_HUMAN | 0.621 |
| NIQSVNVK_451.3_674.4 | GROA_HUMAN | 0.620 |
| SVVLIPLGAVDDGEHSQNEK_703.0_286.2 | CNDP1_HUMAN | 0.620 |
| TLAFVR_353.7_492.3 | FA7_HUMAN | 0.619 |
| AVDIPGLEAATPYR_736.9_286.1 | TENA_HUMAN | 0.619 |
| TEFLSNYLTNVDDITLVPGTLGR_846.8_600.3 | ENPP2_HUMAN | 0.618 |
| YWGVASFLQK_599.8_849.5 | RET4_HUMAN | 0.618 |
| TPSAAYLWVGTGASEAEK_919.5_428.2 | GELS_HUMAN | 0.618 |
| DPNGLPPEAQK_583.3_669.4 | RET4_HUMAN | 0.617 |
| TYLHTYESEI_628.3_908.4 | ENPP2_HUMAN | 0.616 |
| SPQAFYR_434.7_684.4 | REL3_HUMAN | 0.616 |
| TPSAAYLWVGTGASEAEK_919.5_849.4 | GELS_HUMAN | 0.615 |
| ALNHLPLEYNSALYSR_621.0_538.3 | CO6_HUMAN | 0.615 |
| IEVNESGTVASSSTAVIVSAR_693.0_545.3 | PAI1_HUMAN | 0.615 |
| LTTVDIVTLR_565.8_815.5 | IL2RB_HUMAN | 0.615 |
| LWAYLTIQELLAK_781.5_371.2 | ITIH1_HUMAN | 0.613 |
| SYTITGLQPGTDYK_772.4_352.2 | FINC_HUMAN | 0.612 |
| GAVHVVVAETDYQSFAVLYLER_822.8_863.5 | CO8G_HUMAN | 0.612 |
| FQLPGQK_409.2_276.1 | PSG1_HUMAN | 0.612 |
| ILDGGNK_358.7_490.2 | CXCL5_HUMAN | 0.611 |
| DYWSTVK_449.7_620.3 | APOC3_HUMAN | 0.611 |
| AGLLRPDYALLGHR_518.0_595.4 | PGRP2_HUMAN | 0.611 |
| ALNFGGIGVVVGHELTHAFDDQGR_837.1_360.2 | ECE1_HUMAN | 0.611 |
| GYQELLEK_490.3_502.3 | FETA_HUMAN | 0.611 |
| HATLSLSIPR_ 365.6_472.3 | VGFR3_HUMAN | 0.610 |
| SVPVTKPVPVTKPITVTK_631.1_658.4 | Z512B_HUMAN | 0.610 |
| FQLPGQK_409.2_429.2 | PSG1_HUMAN | 0.610 |
| IYLQPGR_423.7_329.2 | ITIH2_HUMAN | 0.610 |
| TLNAYDHR_330.5_312.2 | PAR3_HUMAN | 0.609 |
| DPNGLPPEAQK_583.3_497.2 | RET4_HUMAN | 0.609 |
| FGFGGSTDSGPIR_ 649.3_946.5 | ADA12_HUMAN | 0.609 |
| TYLHTYESEI_628.3_515.3 | ENPP2_HUMAN | 0.608 |
| GAVHVVVAETDYQSFAVLYLER_822.8_580.3 | CO8G_HUMAN | 0.608 |
| VPSHAVVAR_312.5_515.3 | TRFL_HUMAN | 0.608 |
| YWGVASFLQK_599.8_350.2 | RET4_HUMAN | 0.608 |
| EWVAIESDSVQPVPR_856.4_468.3 | CNDP1_HUMAN | 0.607 |
| LQDAGVYR_461.2_680.3 | PD1L1_HUMAN | 0.607 |
| DLYHYITSYVVDGEIIIYGPAYSGR_955.5_650.3 | PSG1_HUMAN | 0.607 |
| LWAYLTIQELLAK_781.5_300.2 | ITIH1_HUMAN | 0.606 |
| ITENDIQIALDDAK_779.9_632.3 | APOB_HUMAN | 0.606 |
| SYTITGLQPGTDYK_772.4_680.3 | FINC_HUMAN | 0.606 |
| FFQYDTWK_567.8_712.3 | IGF2_HUMAN | 0.605 |
| IYLQPGR_423.7_570.3 | ITIH2_HUMAN | 0.605 |
| YNQLLR_403.7_529.4 | ENOA_HUMAN | 0.605 |
| WWGGQPLWITATK_772.4_929.5 | ENPP2_HUMAN | 0.605 |
| WWGGQPLWITATK_772.4_373.2 | ENPP2_HUMAN | 0.605 |
| TASDFITK_441.7_710.4 | GELS_HUMAN | 0.605 |
| EWVAIESDSVQPVPR_856.4_486.2 | CNDP1_HUMAN | 0.605 |
| YEFLNGR_449.7_606.3 | PLMN_HUMAN | 0.604 |
| SNPVTLNVLYGPDLPR_585.7_654.4 | PSG6_HUMAN | 0.604 |
| ITQDAQLK_458.8_803.4 | CBG_HUMAN | 0.603 |
| LTTVDIVTLR_565.8_716.4 | IL2RB_HUMAN | 0.602 |
| FNAVLTNPQGDYDTSTGK_964.5_262.1 | C1QC_HUMAN | 0.602 |
| ITGFLKPGK_320.9_301.2 | LBP_HUMAN | 0.601 |
| DYWSTVK_449.7_347.2 | APOC3_HUMAN | 0.601 |
| DPTFIPAPIQAK_433.2_556.3 | ANGT_HUMAN | 0.601 |
| GWVTDGFSSLK_598.8_953.5 | APOC3_HUMAN | 0.601 |
| YYGYTGAFR_549.3_771.4 | TRFL_HUMAN | 0.601 |
| ELPEHTVK_476.8_347.2 | VTDB_HUMAN | 0.601 |
| FTFTLHLETPKPSISSSNLNPR_829.4_874.4 | PSG1_HUMAN | 0.601 |
| DLYHYITSYVVDGEIIIYGPAYSGR_955.5_707.3 | PSG1_HUMAN | 0.601 |
| SPQAFYR_434.7_684.4 | REL3_HUMAN | 0.616 |
| TPSAAYLWVGTGASEAEK_919.5_849.4 | GELS_HUMAN | 0.615 |
| ALNHLPLEYNSALYSR_621.0_538.3 | CO6_HUMAN | 0.615 |
| IEVNESGTVASSSTAVIVSAR_693.0_545.3 | PAI1_HUMAN | 0.615 |
| LTTVDIVTLR_565.8_815.5 | IL2RB_HUMAN | 0.615 |
| LWAYLTIQELLAK_781.5_371.2 | ITIH1_HUMAN | 0.613 |
| SYTITGLQPGTDYK_772.4_352.2 | FINC_HUMAN | 0.612 |
| GAVHVVVAETDYQSFAVLYLER_822.8_863.5 | CO8G_HUMAN | 0.612 |
| FQLPGQK_409.2_276.1 | PSG1_HUMAN | 0.612 |
| DLYHYITSYVVDGEIIIYGPAYSGR_955.5_707.3 | PSG1_HUMAN | 0.601 |

**Table 16. Lasso Early 32**

| **Variable** | **Protein** | **Coefficient** |
|---|---|---|
| LIQDAVTGLTVNGQITGDK_972.0_798.4 | ITIH3_HUMAN | 9.53 |
| VQTAHFK_277.5_431.2 | CO8A_HUMAN | 9.09 |
| FLNWIK_410.7_560.3 | HABP2_HUMAN | 6.15 |
| ITGFLKPGK_320.9_429.3 | LBP_HUMAN | 5.29 |
| ELIEELVNITQNQK_557.6_517.3 | IL13_HUMAN | 3.83 |
| ALNHLPLEYNSALYSR_621.0_538.3 | CO6_HUMAN | 3.41 |
| DISEVVTPR_508.3_787.4 | CFAB_HUMAN | 0.44 |
| AHYDLR_387.7_288.2 | FETUA_HUMAN | 0.1 |

**Table 17. Lasso Early 100**

| **Variable** | **Protein** | **Coefficient** |
|---|---|---|
| LIQDAVTGLTVNGQITGDK_972.0_798.4 | ITIH3_HUMAN | 6.56 |
| ALNHLPLEYNSALYSR_621.0_538.3 | CO6_HUMAN | 6.51 |
| VQTAHFK_277.5_431.2 | CO8A_HUMAN | 4.51 |
| NIQSVNVK_451.3_674.4 | GROA_HUMAN | 3.12 |
| TYLHTYESEI_628.3_908.4 | ENPP2_HUMAN | 2.68 |
| LIENGYFHPVK_439.6_627.4 | F13B_HUMAN | 2.56 |
| AVLHIGEK_289.5_292.2 | THBG_HUMAN | 2.11 |
| FLNWIK_410.7_560.3 | HABP2_HUMAN | 1.85 |
| ITGFLKPGK_320.9_429.3 | LBP_HUMAN | 1.36 |
| DALSSVQESQVAQQAR_573.0_672.4 | APOC3_HUMAN | 1.3 |
| DALSSVQESQVAQQAR_573.0_502.3 | APOC3_HUMAN | 0.83 |
| FLPCENK_454.2_550.2 | IL10_HUMAN | 0.39 |
| ELIEELVNITQNQK_557.6_517.3 | IL13_HUMAN | 0.3 |
| TEFLSNYLTNVDDITLVPGTLGR_846.8_600.3 | ENPP2_HUMAN | 0.29 |
| VSEADSSNADWVTK_754.9_347.2 | CFAB_HUMAN | 0.27 |
| ITLPDFTGDLR_624.3_28.2 | LBP_HUMAN | 0.13 |
| TGVAVNKPAEFTVDAK_549.6_258.1 | FLNA_HUMAN | 0.04 |
| TASDFITK_41.7_781.4 | GELS_HUMAN | -5.91 |
| LIQDAVTGLTVNGQITGDK_972.0_798.4 | ITIH3_HUMAN | 6.56 |

**Table 18. Lasso Protein Early Window**

| **Variable** | **Protein** | **Coefficient** |
|---|---|---|
| ALNHLPLEYNSALYSR_621.0_538.3 | CO6_HUMAN | 7.17 |
| LIQDAVTGLTVNGQITGDK_972.0_798.4 | ITIH3_HUMAN | 6.06 |
| LIENGYFHPVK_439.6_627.4 | F13B_HUMAN | 3.23 |
| WWGGQPLWITATK_772.4_929.5 | ENPP2_HUMAN | 2.8 |
| QALEEFQK_496.8_680.3 | CO8B_HUMAN | 2.73 |
| NIQSVNVK_451.3_674.4 | GROA_HUMAN | 2.53 |
| DALSSVQESQVAQQAR_573.0_672.4 | APOC3_HUMAN | 2.51 |
| AVLHIGEK_289.5_348.7 | THBG_HUMAN | 2.33 |
| FLNWIK_410.7_560.3 | HABP2_HUMAN | 1.05 |
| FLPCENK_454.2_550.2 | IL10_HUMAN | 0.74 |
| ITLPDFTGDLR_624.3_288.2 | LBP_HUMAN | 0.7 |
| DISEVVTPR_508.3_787.4 | CFAB_HUMAN | 0.45 |
| EVFSKPISWEELLQ_852.9_260.2 | FA40A_HUMAN | 0.17 |
| YYGYTGAFR_549.3_450.3 | TRFL_HUMAN | 0.06 |
| TASDFITK_441.7_781.4 | GELS_HUMAN | -7.65 |

**Table 19. Lasso All Early Window**

| **Variable** | **Protein** | **Coefficient** |
|---|---|---|
| FLNWIK_410.7_560.3 | HABP2_HUMAN | 3.74 |
| AHYDLR_387.7_288.2 | FETUA_HUMAN | 0.07 |
| ALNHLPLEYNSALYSR_621.0_538.3 | CO6_HUMAN | 6.07 |
| LIQDAVTGLTVNGQITGDK_972.0_798.4 | ITIH3_HUMAN | 8.85 |
| TYLHTYESEI_628.3_908.4 | ENPP2_HUMAN | 2.97 |
| VQTAHFK_277.5_431.2 | CO8A_HUMAN | 3.36 |
| ELIEELVNITQNQK_557.6_618.3 | IL13_HUMAN | 11.24 |
| VSEADSSNADWVTK_754.9_347.2 | CFAB_HUMAN | 0.63 |
| AVLHIGEK_289.5_292.2 | THBG_HUMAN | 0.51 |
| TGVAVNKPAEFTVDAK_549.6_977.5 | FLNA_HUMAN | 0.17 |
| LIENGYFHPVK_439.6_343.2 | F13B_HUMAN | 1.7 |
| AQPVQVAEGSEPDGFWEALGGK_758.0_574.3 | GELS_HUMAN | -0.93 |
| YYGYTGAFR_549.3_450.3 | TRFL_HUMAN | 1.4 |
| TASDFITK_441.7_781.4 | GELS_HUMAN | -0.07 |
| NIQSVNVK_451.3_674.4 | GROA_HUMAN | 2.12 |
| DALSSVQESQVAQQAR_573.0_672.4 | APOC3_HUMAN | 1.15 |
| DALSSVQESQVAQQAR_573.0_502.3 | APOC3_HUMAN | 0.09 |
| FGFGGSTDSGPIR_ 649.3_745.4 | ADA12_HUMAN | 2.45 |
| ALDLSLK_380.2_575.3 | ITIH3_HUMAN | 2.51 |
| TLFIFGVTK_513.3_811.5 | PSG4_HUMAN | 4.12 |
| ISQGEADINIAFYQR_575.6_684.4 | MMP8_HUMAN | 1.29 |
| SGVDLADSNQK_567.3_591.3 | VGFR3_HUMAN | 0.55 |
| GPGEDFR_389.2_322.2 | PTGDS_HUMAN | 0.07 |
| DPNGLPPEAQK_583.3_669.4 | RET4_HUMAN | 1.36 |
| WNFAYWAAHQPWSR_607.3_545.3 | PRG2_HUMAN | -1.27 |
| ELCLDPK_437.7_359.2 | IL8_HUMAN | 0.3 |
| FFQYDTWK_567.8_840.4 | IGF2_HUMAN | 1.83 |
| IIEVEEEQEDPYLNDR_996.0_777.4 | FBLN1_HUMAN | 1.14 |
| ECEELEEK_533.2_405.2 | IL15_HUMAN | 1.78 |
| LEEHYELR_363.5_580.3 | PAI2_HUMAN | 0.15 |
| LNIGYIEDLK_589.3_837.4 | PAI2_HUMAN | 0.32 |
| TAVTANLDIR_537.3_288.2 | CHL1_HUMAN | -0.98 |
| SWNEPLYHLVTEVR_581.6_716.4 | PRL_HUMAN | 1.88 |
| ILNIFGVIK_508.8_790.5 | TFR1_HUMAN | 0.05 |
| TPSAAYLWVGTGASEAEK_919.5_849.4 | GELS_HUMAN | -2.69 |
| VGVISFAQK_474.8_693.4 | TFR2_HUMAN | -5.68 |
| LNIGYIEDLK_589.3_950.5 | PAI2_HUMAN | -1.43 |
| GQVPENEANVVITTLK_571.3_462.3 | CADH1_HUMAN | -0.55 |
| STPSLTTK_417.7_549.3 | IL6RA_HUMAN | -0.59 |
| ALLLGWVPTR_563.3_373.2 | PAR4_HUMAN | -0.97 |

**Table 20: Lasso SummedCoef Early Window**

| Transition | Protein | SumBestCoefs |
|---|---|---|
| LIQDAVTGLTVNGQITGDK_972.0_798.4 | ITIH3_HUMAN | 1173.723955 |
| ALNHLPLEYNSALYSR_621.0_538.3 | CO6_HUMAN | 811.0150364 |
| ELIEELVNITQNQK_557.6_618.3 | IL13_HUMAN | 621.9659363 |
| VQTAHFK_277.5_431.2 | CO8A_HUMAN | 454.178544 |
| NIQSVNVK_451.3_674.4 | GROA_HUMAN | 355.9550674 |
| TLFIFGVTK_513.3_811.5 | PSG4_HUMAN | 331.8629189 |
| GPGEDFR_389.2_322.2 | PTGDS_HUMAN | 305.9079494 |
| FLPCENK_454.2_550.2 | IL10_HUMAN | 296.9473975 |
| FLNWIK_410.7_560.3 | HABP2_HUMAN | 282.9841332 |
| LIENGYFHPVK_439.6_627.4 | F13B_HUMAN | 237.5320227 |
| ECEELEEK_533.2_405.2 | IL15_HUMAN | 200.38281 |
| FGFGGSTDSGPIR_ 649.3_745.4 | ADA12_HUMAN | 194.6252869 |
| QALEEFQK_496.8_680.3 | CO8B_HUMAN | 179.2518843 |
| IIEVEEEQEDPYLNDR_996.0_777.4 | FBLN1_HUMAN | 177.7534111 |
| TYLHTYESEI_628.3_908.4 | ENPP2_HUMAN | 164.9735228 |
| ELIEELVNITQNQK_557.6_517.3 | IL13_HUMAN | 162.2414693 |
| LEEHYELR_363.5_580.3 | PAI2_HUMAN | 152.9262386 |
| ISQGEADINIAFYQR_575.6_684.4 | MMP8_HUMAN | 144.2445011 |
| HPWIVHWDQLPQYQLNR_744.0_918.5 | KS6A3_HUMAN | 140.2287926 |
| AHYDLR_387.7_288.2 | FETUA_HUMAN | 137.9737525 |
| GFQALGDAADIR_617.3_288.2 | TIMP1_HUMAN | 130.4945567 |
| SWNEPLYHLVTEVR_581.6_716.4 | PRL_HUMAN | 127.442646 |
| SGVDLADSNQK_567.3_591.3 | VGFR3_HUMAN | 120.5149446 |
| YENYTSSFFIR_713.8_293.1 | IL12B_HUMAN | 117.0947487 |
| FFQYDTWK_567.8_840.4 | IGF2_HUMAN | 109.8569617 |
| HYFIAAVER_553.3_658.4 | FA8_HUMAN | 106.9426543 |
| ITGFLKPGK_320.9_429.3 | LBP_HUMAN | 103.8056505 |
| DALSSVQESQVAQQAR_573.0_502.3 | APOC3_HUMAN | 98.50490812 |
| SGVDLADSNQK_567.3_662.3 | VGFR3_HUMAN | 97.19989285 |
| ALDLSLK_380.2_575.3 | ITIH3_HUMAN | 94.84900337 |
| TGVAVNKPAEFTVDAK_549.6_258.1 | FLNA_HUMAN | 92.52335783 |
| HPWIVHWDQLPQYQLNR_744.0_1047.0 | KS6A3_HUMAN | 91.77547608 |
| LIQDAVTGLTVNGQITGDK_972.0_640.4 | ITIH3_HUMAN | 83.6483639 |
| LNIGYIEDLK_589.3_837.4 | PAI2_HUMAN | 83.50221521 |
| IALGGLLFPASNLR_481.3_657.4 | SHBG_HUMAN | 79.33146741 |
| LPATEKPVLLSK_ 432.6_460.3 | HYOU1_HUMAN | 78.89429168 |
| FQLSETNR_497.8_605.3 | PSG2_HUMAN | 78.13445824 |
| NEIVFPAGILQAPFYTR_968.5_357.2 | ECE1_HUMAN | 75.12145257 |
| ALDLSLK_380.2_185.1 | ITIH3_HUMAN | 63.05454715 |
| DLHLSDVFLK_396.2_366.2 | CO6_HUMAN | 58.26831142 |
| TQILEWAAER_608.8_761.4 | EGLN_HUMAN | 57.29461621 |
| FSVVYAK_407.2_381.2 | FETUA_HUMAN | 54.78436389 |
| VSEADSSNADWVTK_754.9_347.2 | CFAB_HUMAN | 54.40003244 |
| DPNGLPPEAQK_583.3_669.4 | RET4_HUMAN | 53.89169348 |
| VQEAHLTEDQIFYFPK_655.7_701.4 | CO8G_HUMAN | 53.33747599 |
| LSSPAVITDK_ 515.8_830.5 | PLMN_HUMAN | 53.22513181 |
| ITLPDFTGDLR_624.3_288.2 | LBP_HUMAN | 51.5477235 |
| AVLHIGEK_289.5_292.2 | THBG_HUMAN | 49.73092632 |
| GEVTYTTSQVSK_650.3_750.4 | EGLN_HUMAN | 45.14743629 |
| GYVIIKPLVWV_643.9_854.6 | SAMP_HUMAN | 44.05164273 |
| TGVAVNKPAEFTVDAK_549.6_977.5 | FLNA_HUMAN | 42.99898046 |
| YYGYTGAFR_549.3_450.3 | TRFL_HUMAN | 42.90897411 |
| ILDGGNK_358.7_490.2 | CXCL5_HUMAN | 42.60771281 |
| FLPCENK_454.2_390.2 | IL10_HUMAN | 42.56799651 |
| GFQALGDAADIR_617.3_717.4 | TIMP1_HUMAN | 38.68456017 |
| SDGAKPGPR_442.7_213.6 | COLI_HUMAN | 38.47800265 |
| NTGVISVVTTGLDR_716.4_662.4 | CADH1_HUMAN | 32.62953675 |
| SERPPIFEIR_415.2_288.2 | LRP1_HUMAN | 31.48248968 |
| DFHINLFQVLPWLK_885.5_400.2 | CFAB_HUMAN | 31.27286268 |
| DALSSVQESQVAQQAR_573.0_672.4 | APOC3_HUMAN | 31.26972354 |
| ELCLDPK_437.7_359.2 | IL8_HUMAN | 29.91108737 |
| ILNIFGVIK_508.8_790.5 | TFR1_HUMAN | 29.88784921 |
| TEFLSNYLTNVDDITLVPGTLGR_846.8_600.3 | ENPP2_HUMAN | 29.42327998 |
| GAVHVVVAETDYQSFAVLYLER_822.8_863.5 | CO8G_HUMAN | 26.70286929 |
| AVLHIGEK_289.5_348.7 | THBG_HUMAN | 25.78703299 |
| TFLTVYWTPER_706.9_401.2 | ICAM1_HUMAN | 24.73090242 |
| AGITIPR_364.2_486.3 | IL17_HUMAN | 23.84580477 |
| GAVHVVVAETDYQSFAVLYLER_822.8_580.3 | CO8G_HUMAN | 23.81167843 |
| SLQAFVAVAAR_566.8_487.3 | IL23A_HUMAN | 23.61468839 |
| SWNEPLYHLVTEVR_581.6_614.3 | PRL_HUMAN | 23.2538221 |
| TYLHTYESEI_628.3_515.3 | ENPP2_HUMAN | 22.70115313 |
| TAHISGLPPSTDFIVYLSGLAPSIR_871.5_800.5 | TENA_HUMAN | 22.42695892 |
| QNYHQDSEAAINR_515.9_544.3 | FRIH_HUMAN | 21.96827269 |
| AHQLAIDTYQEFEETYIPK_766.0_634.4 | CSH_HUMAN | 21.75765717 |
| GDTYPAELYITGSILR_885.0_274.1 | F13B_HUMAN | 20.89751398 |
| AHYDLR_387.7_566.3 | FETUA_HUMAN | 20.67629529 |
| IALGGLLFPASNLR_481.3_412.3 | SHBG_HUMAN | 19.28973033 |
| ATNATLDPR_479.8_272.2 | PAR1_HUMAN | 18.77604574 |
| FSVVYAK_407.2_579.4 | FETUA_HUMAN | 17.81136564 |
| HTLNQIDEVK_598.8_951.5 | FETUA_HUMAN | 17.29763288 |
| DIPHWLNPTR_416.9_373.2 | PAPP1_HUMAN | 17.00562521 |
| LYYGDDEK_501.7_563.2 | CO8A_HUMAN | 16.78897272 |
| AALAAFNAQNNGSNFQLEEISR_789.1_633.3 | FETUA_HUMAN | 16.41986569 |
| IQTHSTTYR_369.5_627.3 | F13B_HUMAN | 15.78335174 |
| GPITSAAELNDPQSILLR_632.4_826.5 | EGLN_HUMAN | 15.3936876 |
| QTLSWTVTPK_580.8_818.4 | PZP_HUMAN | 14.92509259 |
| AVGYLITGYQR_620.8_737.4 | PZP_HUMAN | 13.9795325 |
| DIIKPDPPK_511.8_342.2 | IL12B_HUMAN | 13.76508282 |
| YNQLLR_403.7_288.2 | ENOA_HUMAN | 12.61733711 |
| GNGLTWAEK_488.3_634.3 | C163B_HUMAN | 12.5891421 |
| QVFAVQR_424.2_473.3 | ELNE_HUMAN | 12.57709327 |
| FLQEQGHR_338.8_497.3 | CO8G_HUMAN | 12.51843475 |
| HVVQLR_376.2_515.3 | IL6RA_HUMAN | 11.83747559 |
| DVLLLVHNLPQNLTGHIWYK_791.8_883.0 | PSG7_HUMAN | 11.69074708 |
| TFLTVYWTPER_706.9_502.3 | ICAM1_HUMAN | 11.63709776 |
| VELAPLPSWQPVGK_760.9_400.3 | ICAM1_HUMAN | 10.79897269 |
| TLFIFGVTK_513.3_215.1 | PSG4_HUMAN | 10.2831751 |
| AYSDLSR_406.2_375.2 | SAMP_HUMAN | 10.00461148 |
| HATLSLSIPR_ 365.6_472.3 | VGFR3_HUMAN | 9.967933028 |
| LQGTLPVEAR_542.3_571.3 | CO5_HUMAN | 9.963760572 |
| NTVISVNPSTK_580.3_732.4 | VCAM1_HUMAN | 9.124228658 |
| EVFSKPISWEELLQ_852.9_260.2 | FA40A_HUMAN | 8.527980294 |
| SLQNASAIESILK_687.4_860.5 | IL3_HUMAN | 8.429061621 |
| IQHPFTVEEFVLPK_562.0_861.5 | PZP_HUMAN | 7.996504258 |
| GVTGYFTFNLYLK_508.3_683.9 | PSG5_HUMAN | 7.94396229 |
| VFQYIDLHQDEFVQTLK_708.4_361.2 | CNDP1_HUMAN | 7.860590049 |
| ILDDLSPR_464.8_587.3 | ITIH4_HUMAN | 7.593889262 |
| LIENGYFHPVK_439.6_343.2 | F13B_HUMAN | 7.05838337 |
| VFQFLEK_455.8_811.4 | CO5_HUMAN | 6.976884759 |
| AFTECCVVASQLR_770.9_574.3 | CO5_HUMAN | 6.847474286 |
| WWGGQPLWITATK_772.4_929.5 | ENPP2_HUMAN | 6.744837357 |
| IQTHSTTYR_369.5_540.3 | F13B_HUMAN | 6.71464509 |
| IAQYYYTFK_598.8_395.2 | F13B_HUMAN | 6.540497911 |
| YGFYTHVFR_397.2_421.3 | THRB_HUMAN | 6.326347548 |
| YHFEALADTGISSEFYDNANDLLSK_940.8_874. 5 | CO8A_HUMAN | 6.261787525 |
| ANDQYLTAAALHNLDEAVK_686.4_301.1 | IL1A_HUMAN | 6.217191651 |
| FSLVSGWGQLLDR_493.3_403.2 | FA7_HUMAN | 6.1038295 |
| GWVTDGFSSLK_598.8_854.4 | APOC3_HUMAN | 6.053494609 |
| TLEAQLTPR_514.8_814.4 | HEP2_HUMAN | 5.855967278 |
| VSAPSGTGHLPGLNPL_ 506.3_300.7 | PSG3_HUMAN | 5.625944609 |
| EAQLPVIENK_570.8_699.4 | PLMN_HUMAN | 5.407703773 |
| SPEAEDPLGVER_649.8_670.4 | Z512B_HUMAN | 5.341420139 |
| IAIDLFK_410.3_635.4 | HEP2_HUMAN | 4.698739039 |
| YEFLNGR_449.7_293.1 | PLMN_HUMAN | 4.658286706 |
| VQTAHFK_277.5_502.3 | CO8A_HUMAN | 4.628247194 |
| IEVIITLK_464.8_815.5 | CXL11_HUMAN | 4.57198762 |
| ILTPEVR_414.3_601.3 | GDF15_HUMAN | 4.452884608 |
| LEEHYELR_363.5_288.2 | PAI2_HUMAN | 4.411983862 |
| HATLSLSIPR_ 365.6_272.2 | VGFR3_HUMAN | 4.334242077 |
| NSDQEIDFK_548.3_294.2 | S10A5_HUMAN | 4.25302369 |
| LPNNVLQEK_527.8_844.5 | AFAM_HUMAN | 4.183602548 |
| ELANTIK_394.7_475.3 | S10AC_HUMAN | 4.13558153 |
| LSIPQITTK_500.8_687.4 | PSG5_HUMAN | 3.966238797 |
| TLNAYDHR_330.5_312.2 | PAR3_HUMAN | 3.961140111 |
| WWGGQPLWITATK_772.4_373.2 | ENPP2_HUMAN | 3.941476057 |
| ELLESYIDGR_597.8_710.4 | THRB_HUMAN | 3.832723338 |
| ATLSAAPSNPR_542.8_570.3 | CXCL2_HUMAN | 3.82834767 |
| VVLSSGSGPGLDLPLVLGLPLQLK_791.5_598.4 | SHBG_HUMAN | 3.80737887 |
| NADYSYSVWK_616.8_333.2 | CO5_HUMAN | 3.56404167 |
| ILILPSVTR_506.3_559.3 | PSGx_HUMAN | 3.526998593 |
| ALEQDLPVNIK_620.4_798.5 | CNDP1_HUMAN | 3.410412424 |
| QVCADPSEEWVQK_788.4_275.2 | CCL3_HUMAN | 3.30795151 |
| SVQNDSQAIAEVLNQLK_619.7_914.5 | DESP_HUMAN | 3.259270741 |
| QVFAVQR_424.2_620.4 | ELNE_HUMAN | 3.211482663 |
| ALPGEQQPLHALTR_511.0_807.5 | IBP1_HUMAN | 3.211207158 |
| LEPLYSASGPGLRPLVIK_637.4_260.2 | CAA60698 | 3.203088951 |
| GTYLYNDCPGPGQDTDCR_697.0_666.3 | TNR1A_HUMAN | 3.139418139 |
| DAGLSWGSAR_510.2_576.3 | NEUR4_HUMAN | 3.005197927 |
| YGFYTHVFR_397.2_659.4 | THRB_HUMAN | 2.985663918 |
| NNQLVAGYLQGPNVNLEEK_700.7_357.2 | IL1RA_HUMAN | 2.866983196 |
| EKPAGGIPVLGSLVNTVLK_631.4_930.6 | BPIB1_HUMAN | 2.798965142 |
| FGSDDEGR_441.7_735.3 | PTHR_HUMAN | 2.743283546 |
| IEVNESGTVASSSTAVIVSAR_693.0_545.3 | PAI1_HUMAN | 2.699725572 |
| FATTFYQHLADSK_510.3_533.3 | ANT3_HUMAN | 2.615073729 |
| DYWSTVK_449.7_347.2 | APOC3_HUMAN | 2.525459346 |
| QLGLPGPPDVPDHAAYHPF_676.7_263.1 | ITIH4_HUMAN | 2.525383799 |
| LSSPAVITDK_ 515.8_743.4 | PLMN_HUMAN | 2.522306831 |
| TEFLSNYLTNVDDITLVPGTLGR_846.8_699.4 | ENPP2_HUMAN | 2.473366805 |
| SILFLGK_389.2_201.1 | THBG_HUMAN | 2.472413913 |
| VTFEYR_407.7_614.3 | CRHBP_HUMAN | 2.425338167 |
| SVVLIPLGAVDDGEHSQNEK_703.0_798.4 | CNDP1_HUMAN | 2.421340244 |
| HTLNQIDEVK_598.8_958.5 | FETUA_HUMAN | 2.419851187 |
| ALNSIIDVYHK_424.9_661.3 | S10A8_HUMAN | 2.367904596 |
| ETLALLSTHR_ 570.8_500.3 | IL5_HUMAN | 2.230076769 |
| GLQYAAQEGLLALQSELLR_1037.1_858.5 | LBP_HUMAN | 2.205949216 |
| TYNVDK_370.2_262.1 | PPB1_HUMAN | 2.11849772 |
| FTITAGSK_412.7_576.3 | FABPL_HUMAN | 2.098589805 |
| GIVEECCFR_585.3_900.3 | IGF2_HUMAN | 2.059942995 |
| YGIEEHGK_311.5_599.3 | CXA1_HUMAN | 2.033828589 |
| ALVLELAK_428.8_331.2 | INHBE_HUMAN | 1.993820617 |
| ITLPDFTGDLR_624.3_920.5 | LBP_HUMAN | 1.968753183 |
| HELTDEELQSLFTNFANVVDK_817.1_906.5 | AFAM_HUMAN | 1.916438806 |
| EANQSTLENFLER_775.9_678.4 | IL4_HUMAN | 1.902033355 |
| DADPDTFFAK_563.8_825.4 | AFAM_HUMAN | 1.882254674 |
| LFIPQITR_494.3_727.4 | PSG9_HUMAN | 1.860649392 |
| DPNGLPPEAQK_583.3_497.2 | RET4_HUMAN | 1.847702127 |
| VEPLYELVTATDFAYSSTVR_754.4_549.3 | CO8B_HUMAN | 1.842159131 |
| FQLSETNR_497.8_476.3 | PSG2_HUMAN | 1.834693717 |
| FSLVSGWGQLLDR_493.3_516.3 | FA7_HUMAN | 1.790582748 |
| NKPGVYTDVAYYLAWIR_ 677.0_545.3 | FA12_HUMAN | 1.777303353 |
| FTGSQPFGQGVEHATANK_626.0_521.2 | TSP1_HUMAN | 1.736517431 |
| DDLYVSDAFHK_655.3_704.3 | ANT3_HUMAN | 1.717534082 |
| AFLEVNEEGSEAAASTAVVIAGR_764.4_685.4 | ANT3_HUMAN | 1.679420475 |
| LPNNVLQEK_527.8_730.4 | AFAM_HUMAN | 1.66321148 |
| IVLSLDVPIGLLQILLEQAR_735.1_503.3 | UCN2_HUMAN | 1.644983604 |
| DPTFIPAPIQAK_433.2_556.3 | ANGT_HUMAN | 1.625411496 |
| SDLEVAHYK_531.3_617.3 | CO8B_HUMAN | 1.543640117 |
| QLYGDTGVLGR_589.8_501.3 | CO8G_HUMAN | 1.505242962 |
| VNHVTLSQPK_374.9_459.3 | B2MG_HUMAN | 1.48233058 |
| TLLPVSKPEIR_418.3_288.2 | CO5_HUMAN | 1.439531341 |
| SEYGAALAWEK_612.8_845.5 | CO6_HUMAN | 1.424401638 |
| YGIEEHGK_311.5_341.2 | CXA1_HUMAN | 1.379872204 |
| DAGLSWGSAR_510.3_390.2 | NEUR4_HUMAN | 1.334272677 |
| AEHPTWGDEQLFQTTR_639.3_569.3 | PGH1_HUMAN | 1.30549273 |
| FQSVFTVTR_542.8_623.4 | C1QC_HUMAN | 1.302847429 |
| VPGLYYFTYHASSR_554.3_420.2 | C1QB_HUMAN | 1.245565877 |
| AYSDLSR_406.2_577.3 | SAMP_HUMAN | 1.220777002 |
| ALEQDLPVNIK_620.4_570.4 | CNDP1_HUMAN | 1.216612522 |
| NAVVQGLEQPHGLVVHPLR_688.4_890.6 | LRP1_HUMAN | 1.212935735 |
| TSDQIHFFFAK_447.6_659.4 | ANT3_HUMAN | 1.176238265 |
| GTYLYNDCPGPGQDTDCR_697.0_335.2 | TNR1A_HUMAN | 1.1455649 |
| TSYQVYSK_488.2_787.4 | C163A_HUMAN | 1.048896429 |
| ALNSIIDVYHK_424.9_774.4 | S10A8_HUMAN | 1.028522516 |
| VELAPLPSWQPVGK_760.9_342.2 | ICAM1_HUMAN | 0.995831393 |
| LSETNR_360.2_330.2 | PSG1_HUMAN | 0.976094717 |
| HFQNLGK_422.2_527.2 | AFAM_HUMAN | 0.956286531 |
| ELPQSIVYK_538.8_417.7 | FBLN3_HUMAN | 0.947931674 |
| LPATEKPVLLSK_ 432.6_347.2 | HYOU1_HUMAN | 0.932537153 |
| SPEAEDPLGVER_649.8_314.1 | Z512B_HUMAN | 0.905955419 |
| DEIPHNDIALLK_459.9_510.8 | HABP2_HUMAN | 0.9032484 |
| FFQYDTWK_567.8_712.3 | IGF2_HUMAN | 0.884340285 |
| LIEIANHVDK_384.6_498.3 | ADA12_HUMAN | 0.881493383 |
| AGFAGDDAPR_488.7_701.3 | ACTB_HUMAN | 0.814836556 |
| YEFLNGR_449.7_606.3 | PLMN_HUMAN | 0.767373087 |
| VIAVNEVGR_478.8_284.2 | CHL1_HUMAN | 0.721519592 |
| SLSQQIENIR_594.3_531.3 | CO1A1_HUMAN | 0.712051082 |
| EWVAIESDSVQPVPR_856.4_486.2 | CNDP1_HUMAN | 0.647712421 |
| YGLVTYATYPK_638.3_843.4 | CFAB_HUMAN | 0.618499569 |
| SVVLIPLGAVDDGEHSQNEK_703.0_286.2 | CNDP1_HUMAN | 0.606626346 |
| NSDQEIDFK_548.3_409.2 | S10A5_HUMAN | 0.601928175 |
| NVNQSLLELHK_432.2_543.3 | FRIH_HUMAN | 0.572008792 |
| IAQYYYTFK_598.8_884.4 | F13B_HUMAN | 0.495062844 |
| GPITSAAELNDPQSILLR_632.4_601.4 | EGLN_HUMAN | 0.47565795 |
| YTTEIIK_434.2_704.4 | C1R_HUMAN | 0.433318952 |
| GYVIIKPLVWV_643.9_304.2 | SAMP_HUMAN | 0.427905264 |
| LDFHFSSDR_375.2_464.2 | INHBC_HUMAN | 0.411898116 |
| IPSNPSHR_303.2_496.3 | FBLN3_HUMAN | 0.390037291 |
| APLTKPLK_289.9_357.2 | CRP_HUMAN | 0.38859469 |
| EVFSKPISWEELLQ_852.9_376.2 | FA40A_HUMAN | 0.371359974 |
| YENYTSSFFIR_713.8_756.4 | IL12B_HUMAN | 0.346336267 |
| SPQAFYR_434.7_556.3 | REL3_HUMAN | 0.345901234 |
| SVDEALR_395.2_488.3 | PRDX2_HUMAN | 0.307518869 |
| FVFGTTPEDILR_ 697.9_742.4 | TSP1_HUMAN | 0.302313589 |
| FTFTLHLETPKPSISSSNLNPR_829.4_787.4 | PSG1_HUMAN | 0.269826678 |
| VGEYSLYIGR_578.8_708.4 | SAMP_HUMAN | 0.226573173 |
| ILPSVPK_377.2_244.2 | PGH1_HUMAN | 0.225429414 |
| LFIPQITR_494.3_614.4 | PSG9_HUMAN | 0.18285533 |
| TGYYFDGISR_589.8_857.4 | FBLN1_HUMAN | 0.182474114 |
| HYGGLTGLNK_530.3_759.4 | PGAM1_HUMAN | 0.152397007 |
| NQSPVLEPVGR_598.3_866.5 | KS6A3_HUMAN | 0.128963949 |
| IGKPAPDFK_324.9_294.2 | PRDX2_HUMAN | 0.113383235 |
| TSESTGSLPSPFLR_739.9_716.4 | PSMG1_HUMAN | 0.108159874 |
| ESDTSYVSLK_ 564.8_347.2 | CRP_HUMAN | 0.08569303 |
| ETPEGAEAKPWYEPIYLGGVFQLEK_951.1_877. 5 | TNFA_HUMAN | 0.039781728 |
| TSDQIHFFFAK_447.6_512.3 | ANT3_HUMAN | 0.008064465 |

**Table 21. Lasso32 Middle Window**

| **Variable** | **UniProt_ID** | **Coefficient** |
|---|---|---|
| SEYGAALAWEK_612.8_788.4 | CO6_HUMAN | 6.99 |
| VFQFLEK_455.8_811.4 | CO5_HUMAN | 6.43 |
| VLEPTLK_400.3_458.3 | VTDB_HUMAN | 3.99 |
| SLDFTELDVAAEK_719.4_316.2 | ANGT_HUMAN | 3.33 |
| TLAFVR_353.7_492.3 | FA7_HUMAN | 2.44 |
| YGIEEHGK_311.5_599.3 | CXA1_HUMAN | 2.27 |
| LHEAFSPVSYQHDLALLR_699.4_251.2 | FA12_HUMAN | 2.14 |
| QGHNSVFLIK_381.6_520.4 | HEMO_HUMAN | 0.25 |
| LLAPSDSPEWLSFDVTGVVR_730.1_430.3 | TGFB1_HUMAN | -2.81 |
| ELPQSIVYK_538.8_417.7 | FBLN3_HUMAN | -3.46 |
| VNHVTLSQPK_374.9_244.2 | B2MG_HUMAN | -6.61 |

**Table 22. Lasso 100 Middle Window**

| **Variable** | **UniProt_ID** | **Coefficient** |
|---|---|---|
| VFQFLEK_455.8_811.4 | CO5_HUMAN | 6.89 |
| SEYGAALAWEK_612.8_788.4 | CO6_HUMAN | 4.67 |
| GEVTYTTSQVSK_650.3_750.4 | EGLN_HUMAN | 3.4 |
| QVFAVQR_424.2_473.3 | ELNE_HUMAN | 1.94 |
| VELAPLPSWQPVGK_760.9_342.2 | ICAM1_HUMAN | 1.91 |
| LHEAFSPVSYQHDLALLR_699.4_251.2 | FA12_HUMAN | 1.8 |
| SLDFTELDVAAEK_719.4_316.2 | ANGT_HUMAN | 1.67 |
| YGIEEHGK_311.5_599.3 | CXA1_HUMAN | 1.53 |
| YGIEEHGK_311.5_341.2 | CXA1_HUMAN | 1.51 |
| HYINLITR_515.3_301.1 | NPY_HUMAN | 1.47 |
| TLAFVR_353.7_492.3 | FA7_HUMAN | 1.46 |
| GVTGYFTFNLYLK_508.3_260.2 | PSG5_HUMAN | 1.28 |
| FSLVSGWGQLLDR_493.3_403.2 | FA7_HUMAN | 0.84 |
| DALSSVQESQVAQQAR_573.0_502.3 | APOC3_HUMAN | 0.41 |
| VELAPLPSWQPVGK_760.9_400.3 | ICAM1_HUMAN | 0.3 |
| AVDIPGLEAATPYR_736.9_399.2 | TENA_HUMAN | -0.95 |
| ELPQSIVYK_538.8_417.7 | FBLN3_HUMAN | -1.54 |
| DVLLLVHNLPQNLTGHIWYK_791.8_310.2 | PSG7_HUMAN | -1.54 |
| VPLALFALNR_ 557.3_620.4 | PEPD_HUMAN | -1.91 |
| LLAPSDSPEWLSFDVTGVVR_730.1_430.3 | TGFB1_HUMAN | -2.3 |
| VNHVTLSQPK_374.9_244.2 | B2MG_HUMAN | -3.6 |
| EVFSKPISWEELLQ_852.9_376.2 | FA40A_HUMAN | -3.96 |

**Table 23. Lasso Protein Middle Window**

| **Variable** | **UniProt_ID** | **Coefficient** |
|---|---|---|
| SEYGAALAWEK_612.8_788.4 | CO6_HUMAN | 5.84 |
| VFQFLEK_455.8_811.4 | CO5_HUMAN | 5.58 |
| SLDFTELDVAAEK_719.4_316.2 | ANGT_HUMAN | 2.11 |
| TLAFVR_353.7_492.3 | FA7_HUMAN | 1.83 |
| LHEAFSPVSYQHDLALLR_699.4_251.2 | FA12_HUMAN | 1.62 |
| HYINLITR_515.3_301.1 | NPY_HUMAN | 1.39 |
| VLEPTLK_400.3_458.3 | VTDB_HUMAN | 1.37 |
| YGIEEHGK_311.5_599.3 | CXA1_HUMAN | 1.17 |
| VELAPLPSWQPVGK_760.9_342.2 | ICAM1_HUMAN | 1.13 |
| QVFAVQR_424.2_473.3 | ELNE_HUMAN | 0.79 |
| ANLINNIFELAGLGK_793.9_299.2 | LCAP_HUMAN | 0.23 |
| DVLLLVHNLPQNLTGHIWYK_791.8_310.2 | PSG7_HUMAN | -0.61 |
| VEHSDLSFSK_383.5_234.1 | B2MG_HUMAN | -0.69 |
| AVDIPGLEAATPYR_736.9_399.2 | TENA_HUMAN | -0.85 |
| VPLALFALNR_ 557.3_620.4 | PEPD_HUMAN | -1.45 |
| ELPQSIVYK_538.8- 417.7 | FBLN3_HUMAN | -1.9 |
| LLAPSDSPEWLSFDVTGVVR_730.1_430.3 | TGFB1_HUMAN | -2.07 |
| EVFSKPISWEELLQ_852.9_376.2 | FA40A_HUMAN | -2.32 |

**Table 24. Lasso All Middle Window**

| **Variable** | **UniProt_ID** | **Coefficient** |
|---|---|---|
| SEYGAALAWEK_612.8_788.4 | CO6_HUMAN | 2.48 |
| VFQFLEK_455.8_811.4 | CO5_HUMAN | 2.41 |
| SLDFTELDVAAEK_719.4_316.2 | ANGT_HUMAN | 1.07 |
| YGIEEHGK_311.5_599.3 | CXA1_HUMAN | 0.64 |
| VLEPTLK_400.3_458.3 | VTDB_HUMAN | 0.58 |
| LHEAFSPVSYQHDLALLR_699.4_251.2 | FA12_HUMAN | 0.21 |
| LLAPSDSPEWLSFDVTGVVR_730.1_430.3 | TGFB1_HUMAN | -0.62 |
| VNHVTLSQPK_374.9_244.2 | B2MG_HUMAN | -1.28 |

**Table 25. Lasso32 Middle-Late Window**

| **Variable** | **UniProt_ID** | **Coefficient** |
|---|---|---|
| SEYGAALAWEK_612.8_845.5 | CO6_HUMAN | 4.35 |
| TLAFVR_353.7_492.3 | FA7_HUMAN | 2.42 |
| YGIEEHGK_311.5_599.3 | CXA1_HUMAN | 1.46 |
| DFNQFSSGEK_386.8_333.2 | FETA_HUMAN | 1.37 |
| VFQFLEK_455.8_811.4 | CO5_HUMAN | 0.89 |
| LIEIANHVDK_384.6_683.4 | ADA12_HUMAN | 0.85 |
| QINSYVK_426.2_496.3 | CBG_HUMAN | 0.56 |
| TYLHTYESEI_628.3_515.3 | ENPP2_HUMAN | 0.53 |
| SLQAFVAVAAR_566.8_804.5 | IL23A_HUMAN | 0.39 |
| TEQAAVAR_423.2_615.4 | FA12_HUMAN | 0.26 |
| VLEPTLK_400.3_587.3 | VTDB_HUMAN | 0.24 |
| AQPVQVAEGSEPDGFWEALGGK_758.0_574.3 | GELS_HUMAN | -2.08 |
| VPLALFALNR_ 557.3_620.4 | PEPD_HUMAN | -2.09 |
| AVYEAVLR_460.8_587.4 | PEPD_HUMAN | -3.37 |

**Table 26. Lasso 100 Middle-Late Window**

| **Variable** | **UniProt_ID** | **Coefficient** |
|---|---|---|
| VFQFLEK_455.8_811.4 | CO5_HUMAN | 3.82 |
| SEYGAALAWEK_612.8_845.5 | CO6_HUMAN _ | 2.94 |
| YGIEEHGK_311.5_599.3 | CXA1_HUMAN | 2.39 |
| DPTFIPAPIQAK_433.2_556.3 | ANGT_HUMAN | 2.05 |
| TLAFVR_353.7_492.3 | FA7_HUMAN | 1.9 |
| NQSPVLEPVGR_598.3_866.5 | KS6A3_HUMAN | 1.87 |
| ALNHLPLEYNSALYSR_621.0_538.3 | CO6_HUMAN | 1.4 |
| TQILEWAAER_608.8_761.4 | EGLN_HUMAN | 1.29 |
| VVGGLVALR_442.3_784.5 | FA12_HUMAN | 1.24 |
| QINSYVK_426.2_496.3 | CBG_HUMAN | 1.14 |
| YGIEEHGK_311.5_341.2 | CXA1_HUMAN | 0.84 |
| ALEQDLPVNIK_620.4_570.4 | CNDP1_HUMAN | 0.74 |
| GTYLYNDCPGPGQDTDCR_697.0_666.3 | TNR1A_HUMAN | 0.51 |
| SLQNASAIESILK_687.4_860.5 | IL3_HUMAN | 0.44 |
| DLHLSDVFLK_396.2_260.2 | CO6_HUMAN | 0.38 |
| LIEIANHVDK_384.6_683.4 | ADA12 _HUMAN | 0.37 |
| NIQSVNVK_451.3_674.4 | GROA_HUMAN | 0.3 |
| FFQYDTWK_567.8_712.3 | IGF2_HUMAN | 0.19 |
| ANLINNIFELAGLGK_793.9_299.2 | LCAP_HUMAN | 0.19 |
| TYLHTYESEI_628.3_515.3 | ENPP2_HUMAN | 0.15 |
| AALAAFNAQNNGSNFQLEEISR_789.1_746.4 | FETUA_HUMAN | -0.09 |
| AQPVQVAEGSEPDGFWEALGGK_758.0_574.3 | GELS_HUMAN | -0.52 |
| TSYQVYSK_488.2_787.4 | C163A_HUMAN | -0.62 |
| AVDIPGLEAATPYR_736.9_399.2 | TENA_HUMAN | -1.29 |
| TAHISGLPPSTDFIVYLSGLAPSIR_871.5_472.3 | TENA_HUMAN | -1.53 |
| AEIEYLEK_497.8_552.3 | LYAM1_HUMAN | -1.73 |
| LLAPSDSPEWLSFDVTGVVR_730.1_430.3 | TGFB1_HUMAN | -1.95 |
| VPLALFALNR_ 557.3_620.4 | PEPD_HUMAN | -2.9 |
| AVYEAVLR_460.8_587.4 | PEPD_HUMAN | -3.04 |
| ELPQSIVYK_538.8_417.7 | FBLN3_HUMAN | -3.49 |
| EVFSKPISWEELLQ_852.9_376.2 | FA40A_HUMAN | -3.71 |

**Table 27. Lasso Protein Middle-LateWindow**

| **Variable** | **UniProt_ID** | **Coefficient** |
|---|---|---|
| VFQFLEK_455.8_811.4 | CO5_HUMAN | 4.25 |
| ALNHLPLEYNSALYSR_621.0_696.4 | CO6_HUMAN | 3.06 |
| YGIEEHGK_311.5_599.3 | CXA1_HUMAN | 2.36 |
| SEPRPGVLLR_375.2_654.4 | FA7_HUMAN | 2.11 |
| TQILEWAAER_608.8_761.4 | EGLN_HUMAN | 1.81 |
| NQSPVLEPVGR_598.3_866.5 | KS6A3_HUMAN | 1.79 |
| TEQAAVAR_423.2_615.4 | FA12_HUMAN | 1.72 |
| QINSYVK_426.2_496.3 | CBG_HUMAN | 0.98 |
| ALEQDLPVNIK_620.4_570.4 | CNDP1_HUMAN | 0.98 |
| NCSFSIIYPVVIK_770.4_555.4 | CRHBP_HUMAN | 0.76 |
| LIEIANHVDK_384.6_683.4 | ADA12_HUMAN | 0.63 |
| SLQNASAIESILK_687.4_860.5 | IL3_HUMAN | 0.59 |
| ANLINNIFELAGLGK_793.9_299.2 | LCAP_HUMAN | 0.55 |
| GTYLYNDCPGPGQDTDCR_697.0_666.3 | TNR1A_HUMAN | 0.55 |
| TYLHTYESEI_628.3_515.3 | ENPP2_HUMAN | 0.46 |
| NIQSVNVK_451.3_674.4 | GROA_HUMAN | 0.22 |
| LTTVDIVTLR_565.8_815.5 | IL2RB_HUMAN | 0.11 |
| FFQYDTWK_567.8_712.3 | IGF2_HUMAN | 0.01 |
| TSYQVYSK_488.2_787.4 | C163A_HUMAN | -0.76 |
| AQPVQVAEGSEPDGFWEALGGK_758.0_574.3 | GELS_HUMAN | -1.31 |
| AEIEYLEK_497.8_552.3 | LYAM1_HUMAN | -1.59 |
| LLAPSDSPEWLSFDVTGVVR_730.1_430.3 | TGFB1_HUMAN | -1.73 |
| AVDIPGLEAATPYR_736.9_399.2 | TENA_HUMAN | -2.02 |
| EVFSKPISWEELLQ_852.9_376.2 | | -3 |
| TGVAVNKPAEFTVDAK_549.6_258.1 | FLNA_HUMAN | -3.15 |
| ELPQSIVYK_538.8_417.7 | FBLN3_HUMAN | -3.49 |
| VNHVTLSQPK_374.9_244.2 | B2MG_HUMAN | -3.82 |
| VPLALFALNR_ 557.3_620.4 | PEPD_HUMAN | -4.94 |

**Table 28. Lasso All Middle-LateWindow**

| **Variable** | **UniProt_ID** | **Coefficient** |
|---|---|---|
| ALNHLPLEYNSALYSR_621.0_538.3 | CO6_HUMAN | 2.38 |
| TLAFVR_353.7_492.3 | FA7_HUMAN | 0.96 |
| YGIEEHGK_311.5_599.3 | CXA1_HUMAN | 0.34 |
| DPTFIPAPIQAK_433.2_461.2 | ANGT_HUMAN | 0.33 |
| DFNQFSSGEK_386.8_333.2 | FETA_HUMAN | 0.13 |
| QINSYVK_426.2_496.3 | CBG_HUMAN | 0.03 |
| TYLHTYESEI_628.3_515.3 | ENPP2_HUMAN | 0 |
| AQPVQVAEGSEPDGFWEALGGK_758.0_574.3 | GELS_HUMAN | -0.02 |
| AEIEYLEK_497.8_552.3 | LYAM1_HUMAN | -0.05 |
| VNHVTLSQPK_374.9_244.2 | B2MG_HUMAN | -0.12 |
| LLAPSDSPEWLSFDVTGVVR_730.1_430.3 | TGFB1_HUMAN | -0.17 |
| EVFSKPISWEELLQ_852.9_376.2 | | -0.31 |
| AVDIPGLEAATPYR_736.9_399.2 | TENA_HUMAN | -0.35 |
| VPLALFALNR_ 557.3_620.4 | PEPD_HUMAN | -0.43 |
| AVYEAVLR_460.8_587.4 | PEPD_HUMAN | -2.33 |

**Table 29. Lasso 32 LateWindow**

| **Variable** | **UniProt_ID** | **Coefficient** |
|---|---|---|
| QINSYVK_426.2_610.3 | CBG_HUMAN | 3.24 |
| ILDGGNK_358.7_603.3 | CXCL5_HUMAN | 2.65 |
| VFQYIDLHQDEFVQTLK_708.4_375.2 | CNDP1_HUMAN | 2.55 |
| SGVDLADSNQK_567.3_662.3 | VGFR3_HUMAN | 2.12 |
| YSHYNER_323.5_418.2 | HABP2_HUMAN | 1.63 |
| DEIPHNDIALLK_459.9_510.8 | HABP2_HUMAN | 1.22 |
| SGVDLADSNQK_567.3_591.3 | VGFR3_HUMAN | 0.96 |
| FGFGGSTDSGPIR_ 649.3_745.4 | ADA12_HUMAN | 0.86 |
| GTYLYNDCPGPGQDTDCR_697.0_666.3 | TNR1A_HUMAN | 0.45 |
| TSYQVYSK_488.2_787.4 | C163A_HUMAN | -1.73 |
| TGVAVNKPAEFTVDAK_549.6_258.1 | FLNA HUMAN | -2.56 |
| SPEAEDPLGVER_649.8_314.1 | Z512B_HUMAN | -3.04 |
| VPLALFALNR_ 557.3_620.4 | PEPD_HUMAN | -3.33 |
| YYGYTGAFR_549.3_450.3 | TRFL_HUMAN | -4.24 |
| AVYEAVLR_460.8_587.4 | PEPD_HUMAN | -5.83 |
| AEIEYLEK_497.8_552.3 | LYAM1_HUMAN | -6.52 |
| AALAAFNAQNNGSNFQLEE!SR_789.1_746.4 | FETUA_HUMAN | -6.55 |

**Table 30: Lasso 100 Late Window**

| **Variable** | **UniProt_ID** | **Coefficient** |
|---|---|---|
| SGVDLADSNQK_567.3_662.3 | VGFR3_HUMAN | 4.13 |
| ILDGGNK_358.7_603.3 | CXCL5_HUMAN | 3.57 |
| QINSYVK_426.2_610.3 | CBG_HUMAN | 3.41 |
| DEIPHNDIALLK_459.9_510.8 | HABP2_HUMAN | 1.64 |
| VFQYIDLHQDEFVQTLK_708.4_375.2 | CNDP1_HUMAN | 1.57 |
| FGFGGSTDSGPIR_ 649.3_745.4 | ADA12_HUMAN | 1.45 |
| LTTVDIVTLR_565.8_815.5 | IL2RB_HUMAN | 0.71 |
| YSHYNER_323.5_418.2 | HABP2_HUMAN | 0.68 |
| FFQYDTWK_567.8_712.3 | IGF2_HUMAN | 0.42 |
| IEVNESGTVASSSTAVIVSAR_693.0_545.3 | PAI1_HUMAN | 0.36 |
| GTYLYNDCPGPGQDTDCR_697.0_666.3 | TNR1A_HUMAN | 0.21 |
| LIEIANHVDK_384.6_683.4 | ADA12_HUMAN | 0.1 |
| VGVISFAQK_474.8_580.3 | TFR2_HUMAN | 0.08 |
| TSYQVYSK_488.2_787.4 | C163A_HUMAN | -0.36 |
| ALNFGG!GVVVGHELTHAFDDQGR_837.1_3 60.2 | ECE1_HUMAN | -0.65 |
| AYSDLSR_406.2_375.2 | SAMP_HUMAN | -1.23 |
| TGVAVNKPAEFTVDAK_549.6_258.1 | FLNA_HUMAN | -1.63 |
| SPEAEDPLGVER_649.8_314.1 | Z512B_HUMAN | -2.29 |
| YYGYTGAFR_549.3_450.3 | TRFL_HUMAN | -2.58 |
| VPLALFALNR_ 557.3_620.4 | PEPD_HUMAN | -2.73 |
| YISPDQLADLYK_713.4_277.2 | ENOA_HUMAN | -2.87 |
| AVDIPGLEAATPYR_736.9_286.1 | TENA_HUMAN | -3.9 |
| AEIEYLEK_497.8_552.3 | LYAM1_HUMAN | -5.29 |
| AVYEAVLR_460.8_587.4 | PEPD_HUMAN | -5.51 |
| AALAAFNAQNNGSNFQLEEISR_789.1_746.4 | FETUA_HUMAN | -6.49 |

**Table 31: Lasso Protein Late Window**

| **Variable** | **UniProt_ID** | **Coefficient** |
|---|---|---|
| SGVDLADSNQK_567.3_662.3 | VGFR3_HUMAN | 3.33 |
| ILDGGNK_358.7_603.3 | CXCL5_HUMAN | 3.25 |
| QINSYVK_426.2_496.3 | CBG_HUMAN | 2.41 |
| YSHYNER_323.5_418.2 | HABP2_HUMAN | 1.82 |
| ALEQDLPVNIK_620.4_798.5 | CNDP1_HUMAN | 1.32 |
| LIEIANHVDK_384.6_683.4 | ADA12_HUMAN | 1.27 |
| GTYLYNDCPGPGQDTDCR_697.0_666.3 | TNR1A_HUMAN | 0.26 |
| IEVNESGTVASSSTAVIVSAR_693.0_545.3 | PAI1_HUMAN | 0.18 |
| LTTVDIVTLR_565.8_815.5 | IL2RB_HUMAN | 0.18 |
| TSYQVYSK_488.2_787.4 | C163A_HUMAN | -0.11 |
| TGVAVNKPAEFTVDAK_549.6_258.1 | FLNA_HUMAN | -0.89 |
| AYSDLSR_406.2_375.2 | SAMP_HUMAN | -1.47 |
| SPEAEDPLGVER_649.8_314.1 | Z512B_HUMAN | -1.79 |
| YYGYTGAFR_549.3_450.3 | TRFL_HUMAN | -2.22 |
| YISPDQLADLYK_713.4_277.2 | ENOA_HUMAN | -2.41 |
| AVDIPGLEAATPYR_736.9_286.1 | TENA_HUMAN | -2.94 |
| AEIEYLEK_497.8_552.3 | LYAM1_HUMAN | -5.18 |
| AALAAFNAQNNGSNFQLEEISR_789.1_746.4 | FETUA_HUMAN | -5.71 |
| AVYEAVLR_460.8_587.4 | PEPD_HUMAN | -7.33 |

**Table 32: Lasso All Late Window**

| **Variable** | **UniProt_ID** | **Coefficient** |
|---|---|---|
| QINSYVK_426.2_496.3 | CBG_HUMAN | 0.5 |
| DEIPHNDIALLK_459.9_510.8 | HABP2_HUMAN | 0.15 |
| ALEQDLPVNIK_620.4_570.4 | CNDP1_HUMAN | 0.11 |
| ILDGGNK_358.7_603.3 | CXCL5_HUMAN | 0.08 |
| LIEIANHVDK_384.6_683.4 | ADA12_HUMAN | 0.06 |
| YYGYTGAFR_549.3_450.3 | TRFL_HUMAN | -0.39 |
| AALAAFNAQNNGSNFQLEEISR_789.1_746.4 | FETUA_HUMAN | -1.57 |
| AEIEYLEK_497.8_552.3 | LYAM1_HUMAN | -2.46 |
| AVYEAVLR_460.8_587.4 | PEPD_HUMAN | -2.92 |

**Table 33 : Random Forest 32 Early Window**

| **Variable** | **Protein** | **MeanDecreaseGini** |
|---|---|---|
| ELIEELVNITQNQK_557.6_517.3 | IL13_HUMAN | 3.224369171 |
| AHYDLR_387.7_288.2 | FETUA_HUMAN | 1.869007658 |
| FSVVYAK_407.2_381.2 | FETUA_HUMAN | 1.770198171 |
| ITLPDFTGDLR_624.3_288.2 | LBP_HUMAN | 1.710936472 |
| ITGFLKPGK_320.9_301.2 | LBP_HUMAN | 1.623922439 |
| ITGFLKPGK_320.9_429.3 | LBP_HUMAN | 1.408035272 |
| ELIEELVNITQNQK_557.6_618.3 | IL13_HUMAN | 1.345412168 |
| VFQFLEK_455.8_811.4 | CO5_HUMAN | 1.311332013 |
| VQTAHFK_277.5_431.2 | CO8A_HUMAN | 1.308902373 |
| FLNWIK_410.7_560.3 | HABP2_HUMAN | 1.308093745 |
| DAGLSWGSAR_510.3_390.2 | NEUR4_HUMAN | 1.297033607 |
| TLLPVSKPEIR_418.3_288.2 | CO5_HUMAN | 1.291280928 |
| LIQDAVTGLTVNGQITGDK_972.0_798.4 | ITIH3_HUMAN | 1.28622301 |
| QALEEFQK_496.8_680.3 | CO8B_HUMAN | 1.191731825 |
| FSVVYAK_407.2_579.4 | FETUA_HUMAN | 1.078909138 |
| ITLPDFTGDLR_624.3_920.5 | LBP_HUMAN | 1.072613747 |
| AHYDLR_387.7_566.3 | FETUA_HUMAN | 1.029562263 |
| ALNHLPLEYNSALYSR_621.0_538.3 | CO6_HUMAN | 1.00992071 |
| DVLLLVHNLPQNLPGYFWYK_810.4_967.5 | PSG9_HUMAN | 1.007095529 |
| SFRPFVPR_335.9_635.3 | LBP_HUMAN | 0.970312536 |
| SDLEVAHYK_531.3_617.3 | CO8B_HUMAN | 0.967904893 |
| VQEAHLTEDQIFYFPK_655.7_701.4 | CO8G_HUMAN | 0.960398254 |
| VFQFLEK_455.8_276.2 | CO5_HUMAN | 0.931652095 |
| SLLQPNK_400.2_599.4 | CO8A_HUMAN | 0.926470249 |
| SFRPFVPR_335.9_272.2 | LBP_HUMAN | 0.911599611 |
| FLNWIK_410.7_561.3 | HABP2_HUMAN | 0.852022868 |
| LSSPAVITDK_ 515.8_743.4 | PLMN_HUMAN | 0.825455824 |
| DVLLLVHNLPQNLPGYFWYK_810.4_594.3 | PSG9_HUMAN | 0.756797142 |
| ALVLELAK_428.8_672.4 | INHBE_HUMAN | 0.748802555 |
| DISEVVTPR_508.3_787.4 | CFAB_HUMAN | 0.733731518 |

**Table 34. Random Forest 100 Early Window**

| **Variable** | **Protein** | **MeanDecreaseGini** |
|---|---|---|
| ELIEELVNITQNQK_557.6_517.3 | IL13_HUMAN | 1.709778508 |
| LPNNVLQEK_527.8_844.5 | AFAM_HUMAN | 0.961692716 |
| AHYDLR_387.7_288.2 | FETUA_HUMAN | 0.901586746 |
| ITLPDFTGDLR_624.3_288.2 | LBP_HUMAN | 0.879119498 |
| IEGNLIFDPNNYLPK_874.0_414.2 | APOB_HUMAN | 0.842483095 |
| ITGFLKPGK_320.9_301.2 | LBP_HUMAN | 0.806905233 |
| FSVVYAK_407.2_381.2 | FETUA_HUMAN | 0.790429706 |
| ITGFLKPGK_320.9_429.3 | LBP_HUMAN | 0.710312386 |
| VFQFLEK_455.8_811.4 | CO5_HUMAN | 0.709531553 |
| LIQDAVTGLTVNGQITGDK_972.0_798.4 | ITIH3_HUMAN | 0.624325189 |
| DADPDTFFAK_563.8_825.4 | AFAM_HUMAN | 0.618684313 |
| FLNWIK_410.7_560.3 | HABP2_HUMAN | 0.617501242 |
| TASDFITK_441.7_781.4 | GELS_HUMAN | 0.609275999 |
| DAGLSWGSAR_510.3_390.2 | NEUR4_HUMAN | 0.588718595 |
| VQTAHFK_277.5_431.2 | CO8A_HUMAN | 0.58669845 |
| TLLPVSKPEIR_418.3_288.2 | CO5_HUMAN | 0.5670608 |
| ELIEELVNITQNQK_557.6_618.3 | IL13_HUMAN | 0.555624783 |
| TYLHTYESEI_628.3_908.4 | ENPP2_HUMAN | 0.537678415 |
| HFQNLGK_422.2_527.2 | AFAM_HUMAN | 0.535543137 |
| TASDFITK_441.7_710.4 | GELS_HUMAN | 0.532743323 |
| ITLPDFTGDLR_624.3_920.5 | LBP_HUMAN | 0.51667902 |
| QALEEFQK_496.8_680.3 | CO8B_HUMAN | 0.511314017 |
| AVLHIGEK_289.5_348.7 | THBG_HUMAN | 0.510284122 |
| FSVVYAK_407.2_579.4 | FETUA_HUMAN | 0.503907813 |
| LPNNVLQEK_527.8_730.4 | AFAM_HUMAN | 0.501281631 |
| AHYDLR_387.7_566.3 | FETUA_HUMAN | 0.474166711 |
| IAPQLSTEELVSLGEK_857.5_333.2 | AFAM_HUMAN | 0.459595701 |
| WWGGQPLWITATK_772.4_929.5 | ENPP2_HUMAN | 0.44680777 |
| TYLHTYESEI_628.3_515.3 | ENPP2_HUMAN | 0.434157773 |
| DALSSVQESQVAQQAR_573.0_502.3 | APOC3_HUMAN | 0.432484862 |

**Table 35. Random Forest Protein Early Window**

| **Variable** | **Protein** | **MeanDecreaseGini** |
|---|---|---|
| ELIEELVNITQNQK_557.6_517.3 | IL13_HUMAN | 2.881452809 |
| LPNNVLQEK_527.8_844.5 | AFAM_HUMAN | 1.833987752 |
| ITLPDFTGDLR_624.3_288.2 | LBP_HUMAN | 1.608843881 |
| IEGNLIFDPNNYLPK_874.0_414.2 | APOB_HUMAN | 1.594658208 |
| VFQFLEK_455.8_811.4 | CO5_HUMAN | 1.290134412 |
| LIQDAVTGLTVNGQITGDK_972.0_798.4 | ITIH3_HUMAN | 1.167981736 |
| TASDFITK_441.7_781.4 | GELS_HUMAN | 1.152847453 |
| DAGLSWGSAR_510.3_390.2 | NEUR4_HUMAN | 1.146752656 |
| FSVVYAK_407.2_579.4 | FETUA_HUMAN | 1.060168583 |
| AVLHIGEK_289.5_348.7 | THBG_HUMAN | 1.033625773 |
| FLNWIK_410.7_560.3 | HABP2_HUMAN | 1.022356789 |
| QALEEFQK_496.8_680.3 | CO8B_HUMAN | 0.990074129 |
| DVLLLVHNLPQNLPGYFWYK_810.4_967.5 | PSG9_HUMAN | 0.929633865 |
| WWGGQPLWITATK_772.4_929.5 | ENPP2_HUMAN | 0.905895642 |
| VQEAHLTEDQIFYFPK_655.7_701.4 | CO8G_HUMAN | 0.883887371 |
| NNQLVAGYLQGPNVNLEEK_700.7_999.5 | IL1RA_HUMAN | 0.806472085 |
| SLLQPNK_400.2_599.4 | CD8A_HUMAN | 0.783623222 |
| DALSSVQESQVAQQAR_573.0_672.4 | APOC3_HUMAN | 0.774365756 |
| NIQSVNVK_451.3_674.4 | GROA_HUMAN | 0.767963386 |
| HPWIVHWDQLPQYQLNR_744.0_1047.0 | KS6A3_HUMAN | 0.759960139 |
| TTSDGGYSFK_531.7_860.4 | INHA_HUMAN | 0.732813448 |
| ALNHLPLEYNSALYSR_621.0_538.3 | CO6_HUMAN | 0.718779092 |
| LSSPAVITDK_ 515.8_743.4 | PLMN_HUMAN | 0.699547739 |
| TGVAVNKPAEFTVDAK_549.6_258.1 | FLNA_HUMAN | 0.693159192 |
| TLNAYDHR_330.5_312.2 | PAR3_HUMAN | 0.647300964 |
| DISEVVTPR_508.3_787.4 | CFAB_HUMAN | 0.609165621 |
| LIENGYFHPVK_439.6_627.4 | F13B_HUMAN | 0.60043345 |
| SGVDLADSNQK_567.3_662.3 | VGFR3_HUMAN | 0.596079858 |
| ALQDQLVLVAAK_634.9_289.2 | ANGT_HUMAN | 0.579034994 |
| ALVLELAK_428.8_672.4 | INHBE_HUMAN | 0.573458483 |

**Table 36. Random Forest All Early Window**

| **Variable** | **Protein** | **MeanDecreaseGini** |
|---|---|---|
| ELIEELVNITQNQK_557.6_517.3 | IL13_HUMAN | 0.730972421 |
| ITLPDFTGDLR_624.3_288.2 | LBP_HUMAN | 0.409808774 |
| AHYDLR_387.7_288.2 | FETUA_HUMAN | 0.409298983 |
| FSVVYAK_407.2_381.2 | FETUA_HUMAN | 0.367730833 |
| ITGFLKPGK_320.9_301.2 | LBP_HUMAN | 0.350485117 |
| VFQFLEK_455.8_811.4 | CO5_HUMAN | 0.339289475 |
| ELIEELVNITQNQK_557.6_618.3 | IL13_HUMAN | 0.334303166 |
| LPNNVLQEK_527.8_844.5 | AFAM_HUMAN | 0.329800706 |
| IEGNLIFDPNNYLPK_874.0_414.2 | APOB_HUMAN | 0.325596677 |
| ITGFLKPGK_320.9_429.3 | LBP_HUMAN | 0.31473104 |
| FLNWIK_410.7_560.3 | HABP2_HUMAN | 0.299810081 |
| LIQDAVTGLTVNGQITGDK_972.0_798.4 | ITIH3_HUMAN | 0.295613448 |
| ITLPDFTGDLR_624.3_920.5 | LBP_HUMAN | 0.292212699 |
| DAGLSWGSAR_510.3_390.2 | NEUR4_HUMAN | 0.285812225 |
| TLLPVSKPEIR_418.3_288.2 | CO5_HUMAN | 0.280857718 |
| FSVVYAK_407.2_579.4 | FETUA_HUMAN | 0.278531322 |
| DADPDTFFAK_563.8_825.4 | AFAM_HUMAN | 0.258938798 |
| AHYDLR_387.7_566.3 | FETUA_HUMAN | 0.256160046 |
| QALEEFQK_496.8_680.3 | CO8B_HUMAN | 0.245543641 |
| HTLNQIDEVK_598.8_951.5 | FETUA_HUMAN | 0.239528081 |
| TASDFITK_441.7_781.4 | GELS_HUMAN | 0.227485958 |
| VFQFLEK_455.8_276.2 | CO5_HUMAN | 0.226172392 |
| DVLLLVHNLPQNLPGYFWYK_810.4_967.5 | PSG9_HUMAN | 0.218613384 |
| VQTAHFK_277.5_431.2 | CO8A_HUMAN | 0.217171548 |
| SFRPFVPR_335.9_635.3 | LBP_HUMAN | 0.214798112 |
| HFQNLGK_422.2_527.2 | AFAM_HUMAN | 0.211756476 |
| SVSLPSLDPASAK_636.4_473.3 | APOB_HUMAN | 0.211319422 |
| FGFGGSTDSGPIR_ 649.3_745.4 | ADA12_HUMAN | 0.206574494 |
| HFQNLGK_422.2_285.1 | AFAM_HUMAN | 0.204024196 |
| AVLHIGEK_289.5_348.7 | THBG_HUMAN | 0.201102917 |

**Table 37. Random Forest SummedGini Early Window**

| **Transition** | **Protein** | **SumBestGini** |
|---|---|---|
| ELIEELVNITQNQK_557.6_517.3 | IL13_HUMAN | 242.5373659 |
| VFQFLEK_455.8_811.4 | CO5_HUMAN | 115.1113943 |
| FLNWIK_410.7_560.3 | HABP2_HUMAN | 107.4572447 |
| ITLPDFTGDLR_624.3_288.2 | LBP_HUMAN | 104.0742727 |
| LIQDAVTGLTVNGQITGDK_972.0_798.4 | ITIH3_HUMAN | 103.3238077 |
| DAGLSWGSAR_510.3_390.2 | NEUR4_HUMAN | 70.4151533 |
| AHYDLR_387.7_288.2 | FETUA_HUMAN | 140.2670822 |
| FSVVYAK_407.2_381.2 | FETUA_HUMAN | 121.3664352 |
| LPNNVLQEK_527.8_844.5 | AFAM_HUMAN | 115.5211679 |
| ITGFLKPGK_320.9_429.3 | LBP_HUMAN | 114.9512704 |
| ITGFLKPGK_320.9_301.2 | LBP_HUMAN | 112.916627 |
| IEGNLIFDPNNYLPK_874.0_414.2 | APOB_HUMAN | 52.21169288 |
| VQTAHFK_277.5_431.2 | CO8A_HUMAN | 144.5237215 |
| TLLPVSKPEIR_418.3_288.2 | CO5_HUMAN | 96.16982897 |
| QALEEFQK_496.8_680.3 | CO8B_HUMAN | 85.35050759 |
| FSVVYAK_407.2_579.4 | FETUA_HUMAN | 73.23969945 |
| ELIEELVNITQNQK_557.6_618.3 | IL13_HUMAN | 61.61450671 |
| TASDFITK_441.7_781.4 | GELS_HUMAN | 61.32155633 |
| DVLLLVHNLPQNLPGYFWYK_810.4_967.5 | PSG9_HUMAN | 99.68404123 |
| AVLHIGEK_289.5_348.7 | THBG_HUMAN | 69.96748485 |
| ITLPDFTGDLR_624.3_920.5 | LBP_HUMAN | 56.66810872 |
| WWGGQPLWITATK_772.4_929.5 | ENPP2_HUMAN | 56.54173176 |
| VQEAHLTEDQIFYFPK_655.7_701.4 | CO8G_HUMAN | 47.92505575 |
| DADPDTFFAK_563.8_825.4 | AFAM_HUMAN | 40.34147696 |
| DALSSVQESQVAQQAR_573.0_502.3 | APOC3_HUMAN | 145.0311483 |
| FGFGGSTDSGPIR_ 649.3_745.4 | ADA12_HUMAN | 109.4072996 |
| FLPCENK_454.2_550.2 | IL10_HUMAN | 105.7756691 |
| VQTAHFK_277.5_502.3 | CO8A_HUMAN | 101.5877845 |
| VFQFLEK_455.8_276.2 | CO5_HUMAN | 95.71159157 |
| TYLHTYESEI_628.3_908.4 | ENPP2_HUMAN | 94.92157517 |
| ALNHLPLEYNSALYSR_621.0_538.3 | CO6_HUMAN | 90.67568777 |
| NKPGVYTDVAYYLAWIR_ 677.0_545.3 | FA12_HUMAN | 90.35890105 |
| LEEHYELR_363.5_580.3 | PAI2_HUMAN | 88.44833508 |
| HPWIVHWDQLPQYQLNR_744.0_1047.0 | KS6A3_HUMAN | 88.37680942 |
| HTLNQIDEVK_598.8_951.5 | FETUA_HUMAN | 87.63064143 |
| LPNNVLQEK_527.8_730.4 | AFAM_HUMAN | 86.64484642 |
| ALDLSLK_380.2_575.3 | ITIH3_HUMAN | 83.51201287 |
| YGIEEHGK_311.5_599.3 | CXA1_HUMAN | 82.47620831 |
| LSSPAVITDK_ 515.8_830.5 | PLMN_HUMAN | 81.5433587 |
| LEEHYELR_363.5_288.2 | PAI2_HUMAN | 79.01571985 |
| NVIQISNDLENLR_509.9_402.3 | LEP_HUMAN | 78.86670236 |
| SGFSFGFK_438.7_732.4 | CO8B_HUMAN | 78.71961929 |
| SDLEVAHYK_531.3_617.3 | CO8B_HUMAN | 78.24005567 |
| NADYSYSVWK_616.8_333.2 | CO5_HUMAN | 76.07974354 |
| AHYDLR_387.7_566.3 | FETUA_HUMAN | 74.68253347 |
| GAVHVVVAETDYQSFAVLYLER_822.8_580.3 | CO8G_HUMAN | 73.75860248 |
| LIENGYFHPVK_439.6_627.4 | F13B_HUMAN | 73.74965194 |
| ALDLSLK_380.2_185.1 | ITIH3_HUMAN | 72.760739 |
| WWGGQPLWITATK_772.4_373.2 | ENPP2_HUMAN | 72.51936706 |
| FGFGGSTDSGPIR_ 649.3_946.5 | ADA12_HUMAN | 72.49183198 |
| GLQYAAQEGLLALQSELLR_1037.1_929.5 | LBP_HUMAN | 67.17588648 |
| HFQNLGK_422.2_527.2 | AFAM_HUMAN | 66.11702719 |
| YSHYNER_323.5_581.3 | HABP2_HUMAN | 65.56238612 |
| ISQGEADINIAFYQR_575.6_684.4 | MMP8_HUMAN | 65.50301246 |
| TGVAVNKPAEFTVDAK_549.6_258.1 | FLNA_HUMAN | 64.85259525 |
| NIQSVNVK_451.3_674.4 | GROA_HUMAN | 64.53010225 |
| DALSSVQESQVAQQAR_573.0_672.4 | APOC3_HUMAN | 64.12149927 |
| SLLQPNK_400.2_599.4 | CO8A_HUMAN | 62.68167847 |
| SFRPFVPR_335.9_635.3 | LBP_HUMAN | 61.90157662 |
| NNQLVAGYLQGPNVNLEEK_700.7_999.5 | IL1RA_HUMAN | 61.54435815 |
| LYYGDDEK_501.7_563.2 | CO8A_HUMAN | 60.16700473 |
| SWNEPLYHLVTEVR_581.6_716.4 | PRL_HUMAN | 59.78209065 |
| SGVDLADSNQK_567.3_662.3 | VGFR3_HUMAN | 58.93982896 |
| GTYLYNDCPGPGQDTDCR_697.0_335.2 | TNR1A_HUMAN | 58.72963941 |
| HATLSLSIPR_ 365.6_472.3 | VGFR3_HUMAN | 57.98669834 |
| FIVGFTR_420.2_261.2 | CCL20_HUMAN | 57.23165578 |
| QNYHQDSEAAINR_515.9_544.3 | FRIH_HUMAN | 57.21116697 |
| DVLLLVHNLPQNLPGYFWYK_810.4_594.3 | PSG9_HUMAN | 56.84150484 |
| FLNWIK_410.7_561.3 | HABP2_HUMAN | 56.37258274 |
| SLQAFVAVAAR_566.8_487.3 | IL23A_HUMAN | 56.09012981 |
| HFQNLGK_422.2_285.1 | AFAM_HUMAN | 56.04480022 |
| GPGEDFR_389.2_322.2 | PTGDS_HUMAN | 55.7583763 |
| NKPGVYTDVAYYLAWIR_ 677.0_821.5 | FA12_HUMAN | 55.53857645 |
| LIQDAVTGLTVNGQITGDK_972.0_640.4 | ITIH3_HUMAN | 55.52577583 |
| YYGYTGAFR_549.3_450.3 | TRFL_HUMAN | 54.27147366 |
| TLNAYDHR_330.5_312.2 | PAR3_HUMAN | 54.19190934 |
| IQTHSTTYR_369.5_627.3 | F13B_HUMAN | 54.18950583 |
| TASDFITK_441.7_710.4 | GELS_HUMAN | 54.1056456 |
| ALNHLPLEYNSALYSR_621.0_696.4 | CO6_HUMAN | 53.8997252 |
| DADPDTFFAK_563.8_302.1 | AFAM_HUMAN | 53.85914848 |
| SVSLPSLDPASAK_636.4_473.3 | APOB_HUMAN | 53.41996191 |
| TTSDGGYSFK_531.7_860.4 | INHA_HUMAN | 52.24655536 |
| AFTECCVVASQLR_770.9_574.3 | CO5_HUMAN | 51.67853429 |
| ELPQSIVYK_538.8_409.2 | FBLN3_HUMAN | 51.35853002 |
| TYLHTYESEI_628.3_515.3 | ENPP2_HUMAN | 51.23842124 |
| FQLSETNR_497.8_605.3 | PSG2_HUMAN | 51.01576848 |
| GSLVQASEANLQAAQDFVR_668.7_806.4 | ITIH1_HUMAN | 50.81923338 |
| FSLVSGWGQLLDR_493.3_403.2 | FA7_HUMAN | 50.54425114 |
| ECEELEEK_533.2_405.2 | IL15_HUMAN | 50.41977421 |
| NADYSYSVWK_616.8_769.4 | CO5_HUMAN | 50.36434595 |
| SLLQPNK_400.2_358.2 | CO8A_HUMAN | 49.75593162 |
| LIEIANHVDK_384.6_683.4 | ADA12 _ HUMAN | 49.43389721 |
| DISEVVTPR_508.3_787.4 | CFAB _ HUMAN | 49.00234897 |
| AEVIWTSSDHQVLSGK_586.3_300.2 | PD1L1_HUMAN | 48.79028835 |
| SGVDLADSNQK_567.3_591.3 | VGFR3_HUMAN | 48.70665587 |
| SILFLGK_389.2_201.1 | THBG_HUMAN | 48.5997957 |
| AVLHIGEK_289.5_292.2 | THBG_HUMAN | 48.4605866 |
| QLYGDTGVLGR_589.8_501.3 | CO8G_HUMAN | 48.11414904 |
| FSLVSGWGQLLDR_493.3_516.3 | FA7_HUMAN | 47.59635333 |
| DSPVLIDFFEDTER_841.9_399.2 | HRG_HUMAN | 46.83840473 |
| INPASLDK_429.2_630.4 | C163A_HUMAN | 46.78947931 |
| GAVHVVVAETDYQSFAVLYLER_822.8_863.5 | CO8G_HUMAN | 46.66185339 |
| FLQEQGHR_338.8_497.3 | CO8G_HUMAN | 46.64415952 |
| LNIGYIEDLK_589.3_837.4 | PAI2_HUMAN | 46.5879123 |
| LSSPAVITDK_ 515.8_743.4 | PLMN _ HUMAN | 46.2857838 |
| GLQYAAQEGLLALQSELLR_1037.1_858.5 | LBP_HUMAN | 45.7427767 |
| SDGAKPGPR_442.7_213.6 | COLI _ HUMAN | 45.27828366 |
| GYQELLEK_490.3_502.3 | FETA_HUMAN | 43.52928868 |
| GGEGTGYFVDFSVR_745.9_869.5 | HRG_HUMAN | 43.24514327 |
| ADLFYDVEALDLESPK_913.0_447.2 | HRG_HUMAN | 42.56268679 |
| ADLFYDVEALDLESPK_913.0_331.2 | HRG_HUMAN | 42.48967422 |
| EAQLPVIENK_570.8_699.4 | PLMN_HUMAN | 42.21213429 |
| SILFLGK_389.2_577.4 | THBG_HUMAN | 42.03379581 |
| HTLNQIDEVK_598.8_958.5 | FETUA_HUMAN | 41.98377176 |
| AQPVQVAEGSEPDGFWEALGGK_758.0_574.3 | GELS_HUMAN | 41.89547273 |
| FLPCENK_454.2_390.2 | IL10_HUMAN | 41.66612478 |
| LIEIANHVDK_384.6_498.3 | ADA12_HUMAN | 41.50878046 |
| DEIPHNDIALLK_459.9_510.8 | HABP2_HUMAN | 41.27830935 |
| SLQAFVAVAAR_566.8_804.5 | IL23A_HUMAN | 41.00430596 |
| YISPDQLADLYK_713.4_277.2 | ENOA_HUMAN | 40.90053801 |
| SLPVSDSVLSGFEQR_810.9_836.4 | CO8G_HUMAN | 40.62020941 |
| DGSPDVTTADIGANTPDATK_973.5_531.3 | PGRP2_HUMAN | 40.33913091 |
| NTGVISVVTTGLDR_ 716.4_662.4 | CADH1_HUMAN | 40.05291612 |
| ALVLELAK_428.8_672.4 | INHBE_HUMAN | 40.01646465 |
| YEFLNGR_449.7_293.1 | PLMN_HUMAN | 39.83344278 |
| WGAAPYR_410.7_577.3 | PGRP2_HUMAN | 39.52766213 |
| TFLTVYWTPER_706.9_401.2 | ICAM1_HUMAN | 39.13662034 |
| SEYGAALAWEK_612.8_845.5 | CO6_HUMAN | 38.77511119 |
| VGVISFAQK_474.8_693.4 | TFR2_HUMAN | 38.5823457 |
| IIEVEEEQEDPYLNDR_996.0_777.4 | FBLN1_HUMAN | 38.30913304 |
| TGYYFDGISR_589.8_694.4 | FBLN1_HUMAN | 38.30617106 |
| LQGTLPVEAR_542.3_571.3 | CO5_HUMAN | 37.93064544 |
| DSPVLIDFFEDTER_841.9_512.3 | HRG_HUMAN | 37.4447737 |
| AALAAFNAQNNGSNFQLEEISR_789.1_746.4 | FETUA_HUMAN | 37.02483715 |
| DGSPDVTTADIGANTPDATK_973.5_844.4 | PGRP2_HUMAN | 36.59864788 |
| ILILPSVTR_506.3_785.5 | PSGx_HUMAN | 36.43814815 |
| SVSLPSLDPASAK_636.4_885.5 | APOB_HUMAN | 36.27689491 |
| TLAFVR_353.7_492.3 | FA7_HUMAN | 36.18771771 |
| VAPGVANPGTPLA_ 582.3_555.3 | A6NIT4_HUMAN | 35.70677357 |
| HELTDEELQSLFTNFANVVDK_817.1_906.5 | AFAM_HUMAN | 35.14441609 |
| AGLLRPDYALLGHR_518.0_369.2 | PGRP2_HUMAN | 35.13047098 |
| GDTYPAELYITGSILR_885.0_1332.8 | F13B_HUMAN | 34.97832404 |
| LFIPQITR_494.3_727.4 | PSG9_HUMAN | 34.76811249 |
| GYQELLEK_490.3_631.4 | FETA_HUMAN | 34.76117605 |
| VSEADSSNADWVTK_754.9_533.3 | CFAB_HUMAN | 34.49787512 |
| LNIGYIEDLK_589.3_950.5 | PAI2_HUMAN | 34.48448691 |
| SFRPFVPR_335.9_272.2 | LBP_HUMAN | 34.27529415 |
| ILDGGNK_358.7_490.2 | CXCL5_HUMAN | 34.2331388 |
| EANQSTLENFLER_775.9_678.4 | IL4_HUMAN | 34.14295797 |
| DFNQFSSGEK_386.8_189.1 | FETA_HUMAN | 34.05459951 |
| IEEIAAK_387.2_660.4 | CO5_HUMAN | 33.93778148 |
| TEFLSNYLTNVDDITLVPGTLGR_846.8_600.3 | ENPP2_HUMAN | 33.87864446 |
| LPATEKPVLLSK_432.6_347.2 | HYOU1_HUMAN | 33.69005522 |
| FLQEQGHR_338.8_369.2 | CO8G_HUMAN | 33.61179024 |
| APLTKPLK_289.9_357.2 | CRP_HUMAN | 33.59900279 |
| YSHYNER_323.5_418.2 | HABP2_HUMAN | 33.50888447 |
| TSYQVYSK_488.2_787.4 | C163A_HUMAN | 33.11650018 |
| IALGGLLFPASNLR_481.3_657.4 | SHBG_HUMAN | 33.02974341 |
| TGISPLALIK_506.8_741.5 | APOB_HUMAN | 32.64471573 |
| LYYGDDEK_501.7_726.3 | CO8A_HUMAN | 32.60782458 |
| IVLSLDVPIGLLQILLEQAR_735.1_503.3 | UCN2_HUMAN | 32.37907686 |
| EAQLPVIENK_570.8_329.2 | PLMN_HUMAN | 32.34049256 |
| TGYYFDGISR_589.8_857.4 | FBLN1_HUMAN | 32.14526507 |
| VGVISFAQK_474.8_580.3 | TFR2_HUMAN | 32.11753213 |
| FQSVFTVTR_542.8_623.4 | C1QC_HUMAN | 32.11360444 |
| TSDQIHFFFAK_447.6_659.4 | ANT3_HUMAN | 31.95867038 |
| IAPQLSTEELVSLGEK_857.5_333.2 | AFAM_HUMAN | 31.81531364 |
| EVFSKPISWEELLQ_852.9_260.2 | FA40A_HUMAN | 31.36698726 |
| DEIPHNDIALLK_459.9_260.2 | HABP2_HUMAN | 31.1839869 |
| NYFTSVAHPNLFIATK_608.3_319.2 | IL1A_HUMAN | 31.09867061 |
| ITENDIQIALDDAK_779.9_632.3 | APOB_HUMAN | 30.77026845 |
| DTYVSSFPR_357.8_272.2 | TCEA1_HUMAN | 30.67784731 |
| TDAPDLPEENQAR_728.3_843.4 | CO5_HUMAN | 30.66251941 |
| LFYADHPFIFLVR_546.6_647.4 | SERPH_HUMAN | 30.65831566 |
| TEQAAVAR_423.2_487.3 | FA12_HUMAN | 30.44356842 |
| AVGYLITGYQR_620.8_737.4 | PZP_HUMAN | 30.36425528 |
| HSHESQDLR_370.2_288.2 | HRG_HUMAN | 30.34684703 |
| IALGGLLFPASNLR_481.3_412.3 | SHBG_HUMAN | 30.34101643 |
| IAQYYYTFK_598.8_884.4 | F13B_HUMAN | 30.23453833 |
| SLPVSDSVLSGFEQR_810.9_723.3 | CO8G_HUMAN | 30.11396489 |
| IIGGSDADIK_494.8_762.4 | C1S HUMAN | 30.06572687 |
| QTLSWTVTPK_580.8_545.3 | PZP_HUMAN | 30.04139865 |
| HYFIAAVER_553.3_658.4 | FA8_HUMAN | 29.80239884 |
| QVCADPSEEWVQK_788.4_374.2 | CCL3_HUMAN | 29.61435573 |
| DLHLSDVFLK_396.2_366.2 | CO6_HUMAN | 29.60077507 |
| NIQSVNVK_451.3_546.3 | GROA_HUMAN | 29.47619619 |
| QTLSWTVTPK_580.8_818.4 | PZP_HUMAN | 29.40047934 |
| HSHESQDLR_370.2_403.2 | HRG_HUMAN | 29.32242262 |
| LLEVPEGR_456.8_356.2 | C1S_HUMAN | 29.14169137 |
| LIENGYFHPVK_439.6_343.2 | F13B_HUMAN | 28.63056809 |
| EDTPNSVWEPAK_686.8_630.3 | C1S_HUMAN | 28.61352686 |
| AFTECCVVASQLR_770.9_673.4 | CO5_HUMAN | 28.57830281 |
| VNHVTLSQPK_374.9_459.3 | B2MG_HUMAN | 28.27203693 |
| VSFSSPLVAISGVALR_802.0_715.4 | PAPP1_HUMAN | 28.13008712 |
| DPDQTDGLGLSYLSSHIANVER_796.4_456.2 | GELS_HUMAN | 28.06549895 |
| VVGGLVALR_442.3_784.5 | FA12_HUMAN | 28.00684006 |
| NEIVFPAGILQAPFYTR_968.5_357.2 | ECE1_HUMAN | 27.97758456 |
| QVCADPSEEWVQK_788.4_275.2 | CCL3_HUMAN | 27.94276837 |
| LQDAGVYR_461.2_680.3 | PD1L1_HUMAN | 27.88063261 |
| IQTHSTTYR_369.5_540.3 | F13B_HUMAN | 27.68873826 |
| TPSAAYLWVGTGASEAEK_919.5_849.4 | GELS_HUMAN | 27.66889639 |
| ALALPPLGLAPLLNLWAKPQGR_770.5_256.2 | SHBG_HUMAN | 27.63105727 |
| ALQDQLVLVAAK_634.9_289.2 | ANGT_HUMAN | 27.63097319 |
| IEEIAAK_387.2_531.3 | CO5_HUMAN | 27.52427934 |
| TAVTANLDIR_537.3_288.2 | CHL1_HUMAN | 27.44246841 |
| VSEADSSNADWVTK_754.9_347.2 | CFAB_HUMAN | 27.43976782 |
| ITENDIQIALDDAK_779.9_873.5 | APOB_HUMAN | 27.39263522 |
| SSNNPHSPIVEEFQVPYNK_729.4_521.3 | C1S_HUMAN | 27.34493617 |
| HPWIVHWDQLPQYQLNR_744.0_918.5 | KS6A3_HUMAN | 27.19681613 |
| TPSAAYLWVGTGASEAEK_919.5_428.2 | GELS_HUMAN | 27.17319953 |
| AFLEVNEEGSEAAASTAVVIAGR_764.4_614.4 | ANT3_HUMAN | 27.10487351 |
| WGAAPYR_410.7_634.3 | PGRP2 HUMAN | 27.09930054 |
| IEVNESGTVASSSTAVIVSAR_693.0_545.3 | PAI1_HUMAN | 27.02567296 |
| AEAQAQYSAAVAK_654.3_908.5 | ITIH4_HUMAN | 26.98305259 |
| VPLALFALNR_ 557.3_917.6 | PEPD_HUMAN | 26.96988826 |
| TLEAQLTPR_514.8_685.4 | HEP2_HUMAN | 26.94672621 |
| QALEEFQK_496.8_551.3 | CO8B_HUMAN | 26.67037155 |
| WNFAYWAAHQPWSR_607.3_545.3 | PRG2_HUMAN | 26.62600679 |
| IYLQPGR_423.7_570.3 | ITIH2_HUMAN | 26.58752589 |
| FFQYDTWK_567.8_840.4 | IGF2_HUMAN | 26.39942037 |
| NEIWYR_440.7_357.2 | FA12_HUMAN | 26.35177282 |
| GGEGTGYFVDFSVR_745.9_722.4 | HRG_HUMAN | 26.31688167 |
| VGEYSLYIGR_578.8_708.4 | SAMP_HUMAN | 26.17367498 |
| TAHISGLPPSTDFIVYLSGLAPSIR_871.5_800.5 | TENA_HUMAN | 26.13688183 |
| GVTGYFTFNLYLK_508.3_260.2 | PSG5_HUMAN | 26.06007032 |
| DYWSTVK_449.7_620.3 | APOC3_HUMAN | 26.03765187 |
| YENYTSSFFIR_713.8_756.4 | IL12B _ HUMAN | 25.9096605 |
| YGLVTYATYPK_638.3_334.2 | CFAB_HUMAN | 25.84440452 |
| LFIPQITR_494.3_614.4 | PSG9_HUMAN | 25.78081129 |
| YEFLNGR_449.7_606.3 | PLMN_HUMAN | 25.17159874 |
| SEPRPGVLLR_375.2_454.3 | FA7_HUMAN | 25.16444381 |
| NSDQEIDFK_548.3_294.2 | S10A5_HUMAN | 25.12266401 |
| YEVQGEVFTKPQLWP_911.0_293.1 | CRP_HUMAN | 24.77595195 |
| GVTGYFTFNLYLK_508.3_683.9 | PSG5_HUMAN | 24.75289081 |
| ISLLLIESWLEPVR_834.5_371.2 | CSH_HUMAN | 24.72379326 |
| ALLLGWVPTR_563.3_373.2 | PAR4_HUMAN | 24.68096599 |
| VNHVTLSQPK_374.9_244.2 | B2MG_HUMAN | 24.53420489 |
| SGAQATWTELPWPHEK_613.3_793.4 | HEMO_HUMAN | 24.25610995 |
| AQPVQVAEGSEPDGFWEALGGK_758.0_623.4 | GELS_HUMAN | 24.18769142 |
| DLPHITVDR_533.3_490.3 | MMP7_HUMAN | 24.02606052 |
| SEYGAALAWEK_612.8_788.4 | CO6_HUMAN | 24.00163743 |
| AVGYLITGYQR_620.8_523.3 | PZP _ HUMAN | 23.93958524 |
| GFQALGDAADIR_617.3_717.4 | TIMP1_HUMAN | 23.69249513 |
| YEVQGEVFTKPQLWP_911.0_392.2 | CRP_HUMAN | 23.67764212 |
| SDGAKPGPR_442.7_459.2 | COLI_HUMAN | 23.63551614 |
| GFQALGDAADIR_617.3_288.2 | TIMP1_HUMAN | 23.55832742 |
| IAPQLSTEELVSLGEK_857.5_533.3 | AFAM_HUMAN | 23.38139357 |
| DTDTGALLFIGK_625.8_217.1 | PEDF_HUMAN | 23.33375418 |
| LHEAFSPVSYQHDLALLR_699.4_380.2 | FA12_HUMAN | 23.27455931 |
| IYLQPGR_423.7_329.2 | ITIH2_HUMAN | 23.19122626 |

**Table 38. Random Forest 32 Middle Window**

| **Variable** | **UniProt_ID** | **MeanDecreaseGini** |
|---|---|---|
| SEYGAALAWEK_612.8_788.4 | CO6_HUMAN | 2.27812193 |
| LLAPSDSPEWLSFDVTGVVR_730.1_430.3 | TGFB1_HUMAN | 2.080133179 |
| ALNHLPLEYNSALYSR_621.0_696.4 | CO6_HUMAN | 1.952233942 |
| ELPQSIVYK_538.8_417.7 | FBLN3_HUMAN | 1.518833357 |
| VEHSDLSFSK_383.5_234.1 | B2MG_HUMAN | 1.482593086 |
| VFQFLEK_455.8_811.4 | CO5_HUMAN | 1.448810425 |
| VNHVTLSQPK_374.9_244.2 | B2MG_HUMAN | 1.389922815 |
| YGIEEHGK_311.5_599.3 | CXA1_HUMAN | 1.386794676 |
| TLAFVR_353.7_492.3 | FA7_HUMAN | 1.371530925 |
| VLEPTLK_400.3_587.3 | VTDB_HUMAN | 1.368583173 |
| VLEPTLK_400.3_458.3 | VTDB HUMAN | 1.336029064 |
| DALSSVQESQVAQQAR_573.0_502.3 | APOC3_HUMAN | 1.307024357 |
| AQPVQVAEGSEPDGFWEALGGK_758.0_574.3 | GELS_HUMAN | 1.282930911 |
| LHEAFSPVSYQHDLALLR_699.4_251.2 | FA12_HUMAN | 1.25362163 |
| SEPRPGVLLR_375.2_654.4 | FA7_HUMAN | 1.205539225 |
| VEHSDLSFSK_383.5_468.2 | B2MG_HUMAN | 1.201047302 |
| SLDFTELDVAAEK_719.4_316.2 | ANGT_HUMAN | 1.189617326 |
| SEYGAALAWEK_612.8_845.5 | CO6_HUMAN | 1.120706696 |
| TYLHTYESEI_628.3_515.3 | ENPP2_HUMAN | 1.107036657 |
| VNHVTLSQPK_374.9_459.3 | B2MG_HUMAN | 1.083264902 |
| IEEIAAK_387.2_660.4 | CO5_HUMAN | 1.043635292 |
| ALNHLPLEYNSALYSR_621.0_538.3 | CO6_HUMAN | 0.962643698 |
| TLLPVSKPEIR_418.3_514.3 | CO5_HUMAN | 0.933440467 |
| TEQAAVAR_423.2_615.4 | FA12_HUMAN | 0.878933553 |
| DLHLSDVFLK_396.2_260.2 | CO6_HUMAN | 0.816855601 |
| ALQDQLVLVAAK_634.9_289.2 | ANGT_HUMAN | 0.812620232 |
| SLQAFVAVAAR_566.8_804.5 | IL23A_HUMAN | 0.792274782 |
| QGH_NSVFLIK_381.6_260.2 | HEMO_HUMAN | 0.770830031 |
| ALQDQLVLVAAK_634.9_956.6 | ANGT_HUMAN | 0.767468246 |
| SLDFTELDVAAEK_719.4_874.5 | ANGT_HUMAN | 0.745827911 |

**Table 39. Random Forest 100 Middle Window**

| **Variable** | **UniProt_ID** | **MeanDecreaseGini** |
|---|---|---|
| SEYGAALAWEK_612.8_788.4 | CO6_HUMAN | 1.241568411 |
| ALNHLPLEYNSALYSR_621.0_696.4 | CO6_HUMAN | 0.903126414 |
| LLAPSDSPEWLSFDVTGVVR_730.1_430.3 | TGFB1_HUMAN | 0.846216563 |
| ANLINNIFELAGLGK_793.9_299.2 | LCAP_HUMAN | 0.748261193 |
| VFQFLEK_455.8_811.4 | CO5_HUMAN | 0.717545171 |
| VEHSDLSFSK_383.5_234.1 | B2MG_HUMAN | 0.683219617 |
| ELPQSIVYK_538.8_417.7 | FBLN3_HUMAN | 0.671091545 |
| LNIGYIEDLK_589.3_950.5 | PAI2_HUMAN | 0.652293621 |
| VLEPTLK_400.3_587.3 | VTDB_HUMAN | 0.627095631 |
| VNHVTLSQPK_374.9_244.2 | B2MG_HUMAN | 0.625773888 |
| VLEPTLK_400.3_458.3 | VTDB_HUMAN | 0.613655529 |
| AQPVQVAEGSEPDGFWEALGGK_758.0_574.3 | GELS_HUMAN | 0.576305627 |
| TLFIFGVTK_513.3_811.5 | PSG4_HUMAN | 0.574056825 |
| YGIEEHGK_311.5_599.3 | CXA1_HUMAN | 0.570270447 |
| VPLALFALNR_ 557.3_620.4 | PEPD_HUMAN | 0.556087614 |
| EVFSKPISWEELLQ_852.9_376.2 | FA40A_HUMAN | 0.531461012 |
| VEHSDLSFSK_383.5_468.2 | B2MG_HUMAN | 0.531214597 |
| TLAFVR_353.7_492.3 | FA7_HUMAN | 0.53070743 |
| DALSSVQESQVAQQAR_573.0_502.3 | APOC3_HUMAN | 0.521633041 |
| SEYGAALAWEK_612.8_845.5 | CO6_HUMAN | 0.514509661 |
| SLDFTELDVAAEK_719.4_316.2 | ANGT_HUMAN | 0.50489698 |
| SEPRPGVLLR_375.2_654.4 | FA7_HUMAN | 0.4824926 |
| LHEAFSPVSYQHDLALLR_699.4_251.2 | FA12_HUMAN | 0.48217238 |
| TYLHTYESEI_628.3_515.3 | ENPP2_HUMAN | 0.472286273 |
| AVDIPGLEAATPYR_736.9_399.2 | TENA_HUMAN | 0.470892051 |
| FSLVSGWGQLLDR_493.3_403.2 | FA7_HUMAN | 0.465839813 |
| GEVTYTTSQVSK_650.3_750.4 | EGLN_HUMAN | 0.458736205 |
| VNHVTLSQPK_374.9_459.3 | B2MG_HUMAN | 0.454348892 |
| HFQNLGK_422.2_527.2 | AFAM_HUMAN | 0.45127405 |
| YGIEEHGK_311.5_341.2 | CXA1_HUMAN | 0.430641646 |

**Table 40. Random Forest Protein Middle Window**

| **Variable** | **UniProt_ID** | **MeanDecreaseGini** |
|---|---|---|
| SEYGAALAWEK_612.8_788.4 | CO6_HUMAN | 2.09649626 |
| LLAPSDSPEWLSFDVTGVVR_730.1_430.3 | TGFB1_HUMAN | 1.27664656 |
| VFQFLEK_455.8_811.4 | CO5_HUMAN | 1.243884833 |
| ANLINNIFELAGLGK_793.9_299.2 | LCAP_HUMAN | 1.231814882 |
| VEHSDLSFSK_383.5_234.1 | B2MG_HUMAN | 1.188808078 |
| ELPQSIVYK_538.8_417.7 | FBLN3_HUMAN | 1.185075445 |
| LNIGYIEDLK_589.3_950.5 | PAI2_HUMAN | 1.122351536 |
| VLEPTLK_400.3_458.3 | VTDB_HUMAN | 1.062664798 |
| VPLALFALNR_ 557.3_620.4 | PEPD_HUMAN | 1.019466776 |
| TLAFVR_353.7_492.3 | FA7_HUMAN | 0.98797064 |
| TLFIFGVTK_513.3_811.5 | PSG4_HUMAN | 0.980159531 |
| AQPVQVAEGSEPDGFWEALGGK_758.0_574.3 | GELS_HUMAN | 0.960286027 |
| DALSSVQESQVAQQAR_573.0_502.3 | APOC3_HUMAN | 0.947091926 |
| YGIEEHGK_311.5_599.3 | CXA1_HUMAN | 0.946937719 |
| EVFSKPISWEELLQ_852.9_376.2 | FA40A_HUMAN | 0.916262164 |
| LHEAFSPVSYQHDLALLR_699.4_251.2 | FA12_HUMAN | 0.891310053 |
| SLDFTELDVAAEK_719.4_316.2 | ANGT_HUMAN | 0.884498494 |
| TYLHTYESEI_628.3_515.3 | ENPP2_HUMAN | 0.869043942 |
| HFQNLGK_422.2_527.2 | AFAM_HUMAN | 0.865435217 |
| AVDIPGLEAATPYR_736.9_399.2 | TENA_HUMAN | 0.844842109 |
| TLNAYDHR_330.5_312.2 | PAR3_HUMAN | 0.792615068 |
| DVLLLVHNLPQNLTGHIWYK_791.8_310.2 | PSG7_HUMAN | 0.763629346 |
| GPITSAAELNDPQSILLR_632.4_826.5 | EGLN_HUMAN | 0.762305265 |
| VVLSSGSGPGLDLPLVLGLPLQLK_791.5_598.4 | SHBG_HUMAN | 0.706312721 |
| SLQNASAIESILK_687.4_860.5 | IL3_HUMAN | 0.645503581 |
| HYINLITR_515.3_301.1 | NPY_HUMAN | 0.62631682 |
| VELAPLPSWQPVGK_760.9_342.2 | ICAM1_HUMAN | 0.608991877 |
| LQVNTPLVGASLLR_741.0_925.6 | BPIA1_HUMAN | 0.607801279 |
| TLEAQLTPR_514.8_814.4 | HEP2_HUMAN | 0.597771074 |
| SDGAKPGPR_442.7_459.2 | COLI_HUMAN | 0.582773073 |

**Table 41. Random Forest All Middle Window**

| **Variable** | **UniProt_ID** | **MeanDecreaseGini** |
|---|---|---|
| SEYGAALAWEK_612.8_788.4 | CO6_HUMAN | 0.493373282 |
| ALNHLPLEYNSALYSR_621.0_696.4 | CO6_HUMAN | 0.382180772 |
| VFQFLEK_455.8_811.4 | CO5_HUMAN | 0.260292083 |
| LLAPSDSPEWLSFDVTGVVR_730.1_430.3 | TGFB1_HUMAN | 0.243156718 |
| NADYSYSVWK_616.8_769.4 | CO5_HUMAN | 0.242388196 |
| VLEPTLK_400.3_458.3 | VTDB_HUMAN | 0.238171849 |
| VEHSDLSFSK_383.5_234.1 | B2MG_HUMAN | 0.236873731 |
| ELPQSIVYK_538.8_417.7 | FBLN3_HUMAN | 0.224727161 |
| VLEPTLK_400.3_587.3 | VTDB_HUMAN | 0.222105614 |
| TLFIFGVTK_513.3_811.5 | PSG4_HUMAN | 0.210807574 |
| ANLINNIFELAGLGK_793.9_299.2 | LCAP_HUMAN | 0.208714978 |
| LNIGYIEDLK_589.3_950.5 | PAI2_HUMAN | 0.208027555 |
| SEYGAALAWEK_612.8_845.5 | CO6_HUMAN | 0.197362212 |
| VNHVTLSQPK_374.9_244.2 | B2MG_HUMAN | 0.195728091 |
| YGIEEHGK_311.5_599.3 | CXA1_HUMAN | 0.189969499 |
| HFQNLGK_422.2_527.2 | AFAM_HUMAN | 0.189572857 |
| AGITIPR_364.2_486.3 | IL17_HUMAN | 0.188351054 |
| AQPVQVAEGSEPDGFWEALGGK_758.0_574.3 | GELS_HUMAN | 0.185069517 |
| SLDFTELDVAAEK_719.4_316.2 | ANGT_HUMAN | 0.173688295 |
| TLAFVR_353.7_492.3 | FA7_HUMAN | 0.170636045 |
| SEPRPGVLLR_375.2_654.4 | FA7_HUMAN | 0.170608352 |
| TLLIANETLR_572.3_703.4 | IL5_HUMAN | 0.16745571 |
| ALNHLPLEYNSALYSR_621.0_538.3 | CO6_HUMAN | 0.161514946 |
| LHEAFSPVSYQHDLALLR_699.4_251.2 | FA12_HUMAN | 0.15852146 |
| DGSPDVTTADIGANTPDATK_973.5_844.4 | PGRP2_HUMAN | 0.154028378 |
| VPLALFALNR_ 557.3_620.4 | PEPD_HUMAN | 0.153725879 |
| AVDIPGLEAATPYR_736.9_399.2 | TENA_HUMAN | 0.150920884 |
| YGIEEHGK_311.5_341.2 | CXA1_HUMAN | 0.150319671 |
| FSLVSGWGQLLDR_493.3_403.2 | FA7_HUMAN | 0.144781622 |
| IEEIAAK_387.2_660.4 | CO5_HUMAN | 0.141983196 |

**Table 42. Random Forest 32 Middle-Late Window**

| **Variable** | **UniProt_ID** | **MeanDecreaseGini** |
|---|---|---|
| VPLALFALNR_ 557.3_620.4 | PEPD_HUMAN | 4.566619475 |
| VFQFLEK_455.8_811.4 | CO5_HUMAN | 3.062474666 |
| AQPVQVAEGSEPDGFWEALGGK_758.0_574.3 | GELS_HUMAN | 3.033740627 |
| LIEIANHVDK_384.6_498.3 | ADA12_HUMAN | 2.825082394 |
| DALSSVQESQVAQQAR_573.0_502.3 | APOC3_HUMAN | 2.787777983 |
| TLAFVR_353.7_492.3 | FA7_HUMAN | 2.730532075 |
| ALNHLPLEYNSALYSR_621.0_696.4 | CO6_HUMAN | 2.671290375 |
| AVYEAVLR_460.8_587.4 | PEPD_HUMAN | 2.621357053 |
| SEPRPGVLLR_375.2_654.4 | FA7_HUMAN | 2.57568964 |
| TYLHTYESEI_628.3_515.3 | ENPP2_HUMAN | 2.516708906 |
| ALNHLPLEYNSALYSR_621.0_538.3 | CO6_HUMAN | 2.497348374 |
| LIEIANHVDK_384.6_683.4 | ADA12_HUMAN | 2.457401462 |
| YGIEEHGK_311.5_599.3 | CXA1_HUMAN | 2.396824268 |
| VLEPTLK_400.3_587.3 | VTDB_HUMAN | 2.388105564 |
| SEYGAALAWEK_612.8_788.4 | CO6_HUMAN | 2.340473883 |
| WSAGLTSSQVDLYIPK_883.0_515.3 | CBG_HUMAN | 2.332007976 |
| FGFGGSTDSGPIR_ 649.3_946.5 | ADA12_HUMAN | 2.325669514 |
| SEYGAALAWEK_612.8_845.5 | CO6_HUMAN | 2.31761671 |
| QINSYVK_426.2_496.3 | CBG_HUMAN | 2.245221163 |
| QINSYVK_426.2_610.3 | CBG_HUMAN | 2.212307699 |
| TEQAAVAR_423.2_615.4 | FA12_HUMAN | 2.105860336 |
| AVYEAVLR_460.8_750.4 | PEPD_HUMAN | 2.098321893 |
| TEQAAVAR_423.2_487.3 | FA12_HUMAN | 2.062684763 |
| DFNQFSSGEK_386.8_333.2 | FETA_HUMAN | 2.05160689 |
| SLQAFVAVAAR_566.8_804.5 | IL23A_HUMAN | 1.989521006 |
| SLDFTELDVAAEK _ 719.4 _ 316.2 | ANGT_HUMAN | 1.820628782 |
| DPTFIPAPIQAK_433.2_556.3 | ANGT_HUMAN | 1.763514326 |
| DPTFIPAPIQAK_433.2_461.2 | ANGT_HUMAN | 1.760870392 |
| VLEPTLK_400.3_458.3 | VTDB_HUMAN | 1.723389354 |
| YENYTSSFFIR_713.8_756.4 | IL12B_HUMAN | 1.63355187 |

**Table 43. Random Forest 100 Middle-Late Window**

| **Variable** | **UniProt_ID** | **MeanDecreaseGini** |
|---|---|---|
| VPLALFALNR_ 557.3_620.4 | PEPD_HUMAN | 1.995805024 |
| VFQFLEK_455.8_811.4 | CO5_HUMAN | 1.235926416 |
| DALSSVQESQVAQQAR_573.0_502.3 | APOC3_HUMAN | 1.187464899 |
| EVFSKPISWEELLQ_852.9_376.2 | FA40A_HUMAN | 1.166642578 |
| AQPVQVAEGSEPDGFWEALGGK_758.0_574.3 | GELS_HUMAN | 1.146077071 |
| TLAFVR_353.7_492.3 | FA7_HUMAN | 1.143038275 |
| ANLINNIFELAGLGK_793.9_299.2 | LCAP_HUMAN | 1.130656591 |
| ALNHLPLEYNSALYSR_621.0_538.3 | CO6_HUMAN | 1.098305298 |
| ELPQSIVYK_538.8_417.7 | FBLN3_HUMAN | 1.096715712 |
| LLAPSDSPEWLSFDVTGVVR_730.1_430.3 | TGFB1_HUMAN | 1.086171713 |
| YGIEEHGK_311.5_341.2 | CXA1_HUMAN | 1.071880823 |
| ALNHLPLEYNSALYSR_621.0_696.4 | CO6_HUMAN | 1.062278869 |
| TQILEWAAER_608.8_761.4 | EGLN_HUMAN | 1.059019017 |
| AVYEAVLR_460.8_587.4 | PEPD_HUMAN | 1.057920661 |
| AEIEYLEK_497.8_552.3 | LYAM1_HUMAN | 1.038388955 |
| SEPRPGVLLR_375.2_654.4 | FA7_HUMAN | 1.028275728 |
| AVDIPGLEAATPYR_736.9_399.2 | TENA_HUMAN | 1.026032369 |
| LIEIANHVDK_384.6_498.3 | ADA12_HUMAN | 1.015065282 |
| YGIEEHGK_311.5_599.3 | CXA1_HUMAN | 0.98667651 |
| VLEPTLK_400.3_587.3 | VTDB_HUMAN | 0.970330675 |
| DVLLLVHNLPQNLTGHIWYK_791.8_883.0 | PSG7_HUMAN | 0.934747674 |
| TAHISGLPPSTDFIVYLSGLAPSIR_871.5_472.3 | TENA_HUMAN | 0.889111923 |
| TLNAYDHR_330.5_312.2 | PAR3_HUMAN | 0.887605636 |
| FGFGGSTDSGPIR_ 649.3_946.5 | ADA12_HUMAN | 0.884305889 |
| LIEIANHVDK_384.6_683.4 | ADA12_HUMAN | 0.880889836 |
| SEYGAALAWEK_612.8_788.4 | CO6_HUMAN | 0.863585472 |
| TYLHTYESEI_628.3_515.3 | ENPP2_HUMAN | 0.849232356 |
| FGFGGSTDSGPIR_ 649.3_745.4 | ADA12_HUMAN | 0.843334824 |
| SEYGAALAWEK_612.8_845.5 | CO6_HUMAN | 0.842319271 |
| TPSAAYLWVGTGASEAEK_919.5_849.4 | GELS_HUMAN | 0.828959173 |

**Table 44. Random Forest Protein Middle-Late Window**

| **Variable** | **UniProt_ID** | **MeanDecreaseGini** |
|---|---|---|
| VPLALFALNR_ 557.3_620.4 | PEPD_HUMAN | 3.202123047 |
| ANLINNIFELAGLGK_793.9_299.2 | LCAP_HUMAN | 2.100447309 |
| VFQ.FLEK_455.8_811.4 | CO5_HUMAN | 2.096157529 |
| AQPVQVAEGSEPDGFWEALGGK_758.0_574.3 | GELS_HUMAN | 2.052960939 |
| ALNHLPLEYNSALYSR_621.0_696.4 | CO6_HUMAN | 2.046139797 |
| TQILEWAAER_608.8_761.4 | EGLN_HUMAN | 1.99287941 |
| ELPQSIVYK_538.8_417.7 | FBLN3_HUMAN | 1.920894959 |
| TGVAVNKPAEFTVDAK_549.6_258.1 | FLNA_HUMAN | 1.917665697 |
| SEPRPGVLLR_375.2_654.4 | FA7_HUMAN | 1.883557705 |
| DALSSVQESQVAQQAR_573.0_502.3 | APOC3_HUMAN | 1.870232155 |
| EVFSKPISWEELLQ_852.9_376.2 | FA40A_HUMAN | 1.869000136 |
| LIEIANHVDK_384.6_683.4 | ADA12_HUMAN | 1.825457092 |
| VLEPTLK_400.3_587.3 | VTDB_HUMAN | 1.695327774 |
| TEQAAVAR_423.2_615.4 | FA12_HUMAN | 1.685013152 |
| LLAPSDSPEWLSFDVTGVVR_730.1_430.3 | TGFB1_HUMAN | 1.684068039 |
| TLNAYDHR_330.5_312.2 | PAR3_HUMAN | 1.673758239 |
| AVDIPGLEAATPYR_736.9_399.2 | TENA_HUMAN | 1.648896853 |
| DVLLLVHNLPQNLTGHIWYK_791.8_883.0 | PSG7_HUMAN | 1.648146088 |
| AEIEYLEK_497.8_552.3 | LYAM1_HUMAN | 1.645833005 |
| TYLHTYESEI_628.3_515.3 | ENPP2_HUMAN | 1.639121965 |
| AGLLRPDYALLGHR_518.0_595.4 | PGRP2_HUMAN | 1.610227875 |
| YGIEEHGK_311.5_599.3 | CXA1_HUMAN | 1.606978339 |
| QINSYVK_426.2_496.3 | CBG_HUMAN | 1.554905578 |
| LTTVDIVTLR_565.8_815.5 | IL2RB_HUMAN | 1.484081016 |
| AALAAFNAQNNGSNFQLEEISR_789.1_746.4 | FETUA_HUMAN | 1.43173022 |
| AEVIWTSSDHQVLSGK_586.3_300.2 | PD1L1_HUMAN | 1.394857397 |
| ALEQDLPVNIK_620.4_570.4 | CNDP1_HUMAN | 1.393464547 |
| DFNQFSSGEK_386.8_333.2 | FETA_HUMAN | 1.374296237 |
| TSYQVYSK_488.2_787.4 | C163A_HUMAN | 1.36141387 |
| TLEAQLTPR_514.8_685.4 | HEP2_HUMAN | 1.311118611 |

**Table 45. Random Forest All Middle-Late Window**

| **Variable** | **UniProt_ID** | **MeanDecreaseGini** |
|---|---|---|
| VPLALFALNR_ 557.3_620.4 | PEPD_HUMAN | 0.685165163 |
| VFQFLEK_455.8_811.4 | CO5_HUMAN | 0.426827804 |
| ALNHLPLEYNSALYSR_621.0_538.3 | CO6_HUMAN | 0.409942379 |
| YGIEEHGK_311.5_341.2 | CXA1_HUMAN | 0.406589512 |
| ALNHLPLEYNSALYSR_621.0_696.4 | CO6_HUMAN | 0.402152062 |
| AQPVQVAEGSEPDGFWEALGGK_758.0_574.3 | GELS_HUMAN | 0.374861014 |
| ANLINNIFELAGLGK_793.9_299.2 | LCAP_HUMAN | 0.367089422 |
| TQILEWAAER_608.8_761.4 | EGLN_HUMAN | 0.353757524 |
| AVYEAVLR_460.8_587.4 | PEPD_HUMAN | 0.350518668 |
| TLAFVR_353.7_492.3 | FA7_HUMAN | 0.344669505 |
| SEPRPGVLLR_375.2_654.4 | FA7_HUMAN | 0.338752336 |
| LIEIANHVDK_384.6_683.4 | ADA12_HUMAN | 0.321850027 |
| ELPQSIVYK_538.8_417.7 | FBLN3_HUMAN | 0.301819017 |
| EVFSKPISWEELLQ_852.9_376.2 | FA40A_HUMAN | 0.299561811 |
| LIEIANHVDK_384.6_498.3 | ADA12_HUMAN | 0.298253589 |
| VLEPTLK_400.3_587.3 | VTDB_HUMAN | 0.296206088 |
| YGIEEHGK_311.5_599.3 | CXA1_HUMAN | 0.295621408 |
| DVLLLVHNLPQNLTGHIWYK_791.8_883.0 | PSG7_HUMAN | 0.292937475 |
| TYLHTYESEI_628.3_515.3 | ENPP2_HUMAN | 0.275902848 |
| DALSSVQESQVAQQAR_573.0_502.3 | APOC3_HUMAN | 0.275664578 |
| FGFGGSTDSGPIR_649.3_745.4 | ADA12_HUMAN | 0.27120436 |
| AVDIPGLEAATPYR_736.9_399.2 | TENA_HUMAN | 0.266568271 |
| TGVAVNKPAEFTVDAK_549.6_258.1 | FLNA_HUMAN | 0.262537889 |
| TLNAYDHR_330.5_312.2 | PAR3 _ HUMAN | 0.259901193 |
| IYLQPGR_423.7_329.2 | ITIH2_HUMAN | 0.259086112 |
| AEVIWTSSDHQVLSGK_586.3_300.2 | PD1L1_HUMAN | 0.25722354 |
| VPSHAVVAR_312.5_515.3 | TRFL_HUMAN | 0.256151812 |
| SEYGAALAWEK_612.8_845.5 | CO6_HUMAN | 0.251704855 |
| FGFGGSTDSGPIR_ 649.3_946.5 | ADA12_HUMAN | 0.249400642 |
| SEYGAALAWEK_612.8_788.4 | CO6_HUMAN | 0.245930393 |

**Table 46. Random Forest 32 Late Window**

| **Variable** | **UniProt_ID** | **MeanDecreaseGini** |
|---|---|---|
| AVYEAVLR_460.8_587.4 | PEPD_HUMAN | 1.889521223 |
| AEIEYLEK_497.8_552.3 | LYAM1_HUMAN | 1.75233545 |
| AALAAFNAQNNGSNFQLEEISR_789.1_746.4 | FETUA_HUMAN | 1.676813493 |
| TGVAVNKPAEFTVDAK_549.6_258.1 | FLNA_HUMAN | 1.600684153 |
| AVYEAVLR_460.8_750.4 | PEPD_HUMAN | 1.462889662 |
| LIEIANHVDK_384.6_683.4 | ADA12_HUMAN | 1.364115361 |
| VPLALFALNR_ 557.3_620.4 | PEPD_HUMAN | 1.324317148 |
| QINSYVK_426.2_610.3 | CBG_HUMAN | 1.305932064 |
| ITQDAQLK_458.8_702.4 | CBG_HUMAN | 1.263533228 |
| FGFGGSTDSGPIR_ 649.3_745.4 | ADA12_HUMAN | 1.245153376 |
| LIEIANHVDK_384.6_498.3 | ADA12_HUMAN | 1.236529173 |
| QINSYVK_426.2_496.3 | CBG_HUMAN | 1.221866266 |
| YSHYNER_323.5_418.2 | HABP2_HUMAN | 1.169575572 |
| YYGYTGAFR_549.3_450.3 | TRFL_HUMAN | 1.126684146 |
| VGVISFAQK_474.8_580.3 | TFR2_HUMAN | 1.075283855 |
| VFQYIDLHQDEFVQTLK_708.4_375.2 | CNDP1_HUMAN | 1.07279097 |
| SPEAEDPLGVER_649.8_314.1 | Z512B_HUMAN | 1.05759256 |
| DEIPHNDIALLK_459.9_510.8 | HABP2_HUMAN | 1.028933332 |
| ALEQDLPVNIK_620.4_798.5 | CNDP1_HUMAN | 1.014443799 |
| ALEQDLPVNIK_620.4_570.4 | CNDP1_HUMAN | 1.010573267 |
| ILDGGNK_358.7_603.3 | CXCL5_HUMAN | 0.992175141 |
| TSYQVYSK_488.2_787.4 | C163A_HUMAN | 0.95649585 |
| YENYTSSFFIR_713.8_756.4 | IL12B_HUMAN | 0.955085198 |
| SETEIHQGFQHLHQLFAK_717.4_447.2 | CBG_HUMAN | 0.944726739 |
| TLPFSR_360.7_506.3 | LYAM1_HUMAN | 0.944426109 |
| VLSSIEQK_452.3_691.4 | 1433S_HUMAN | 0.933902495 |
| AEIEYLEK_497.8_389.2 | LYAM1_HUMAN | 0.891235263 |
| GTYLYNDCPGPGQDTDCR_697.0_666.3 | TNR1A_HUMAN | 0.87187037 |
| SGVDLADSNQK_567.3_662.3 | VGFR3_HUMAN | 0.869821307 |
| SGVDLADSNQK_567.3_591.3 | VGFR3_HUMAN | 0.839946466 |

**Table 47. Random Forest 100 Late Window**

| **Variable** | **UniProt_ID** | **MeanDecreaseGini** |
|---|---|---|
| AVYEAVLR_460.8_587.4 | PEPD_HUMAN | 0.971695767 |
| AEIEYLEK_497.8_552.3 | LYAM1_HUMAN | 0.920098693 |
| TGVAVNKPAEFTVDAK_549.6_258.1 | FLNA_HUMAN | 0.786924487 |
| AVYEAVLR_460.8_750.4 | PEPD_HUMAN | 0.772867983 |
| AALAAFNAQNNGSNFQLEEISR_789.1_746.4 | FETUA_HUMAN | 0.744138513 |
| AYSDLSR_406.2_375.2 | SAMP_HUMAN | 0.736078079 |
| VPLALFALNR_ 557.3_620.4 | PEPD_HUMAN | 0.681784822 |
| QINSYVK_426.2_610.3 | CBG_HUMAN | 0.585819307 |
| LIEIANHVDK_384.6_498.3 | ADA12_HUMAN | 0.577161158 |
| FGFGGSTDSGPIR_ 649.3_745.4 | ADA12_HUMAN | 0.573055613 |
| WSAGLTSSQVDLYIPK_883.0_515.3 | CBG_HUMAN | 0.569156128 |
| ITQDAQLK_458.8_702.4 | CBG_HUMAN | 0.551017844 |
| LIEIANHVDK_384.6_683.4 | ADA12_ HUMAN | 0.539330047 |
| YYGYTGAFR_549.3_450.3 | TRFL_ HUMAN | 0.527652175 |
| VFQYIDLHQDEFVQTLK_708.4_375.2 | CNDP1_HUMAN | 0.484155289 |
| FQLPGQK_409.2_429.2 | PSG1_HUMAN | 0.480394031 |
| AVDIPGLEAATPYR_736.9_286.1 | TENA_HUMAN | 0.475252565 |
| QINSYVK_426.2_496.3 | CBG_HUMAN | 0.4728541 |
| YISPDQLADLYK_713.4_277.2 | ENOA_HUMAN | 0.470079977 |
| TLPFSR_360.7_506.3 | LYAM1_HUMAN | 0.46881451 |
| SPEAEDPLGVER_649.8_314.1 | Z512B_HUMAN | 0.4658941 |
| ALEQDLPVNIK_620.4_798.5 | CNDP1_HUMAN | 0.463604174 |
| YSHYNER_323.5_418.2 | HABP2_HUMAN | 0.453076307 |
| VGVISFAQK_474.8_580.3 | TFR2_HUMAN | 0.437768219 |
| LQDAGVYR_461.2_680.3 | PD1L1_HUMAN | 0.428524689 |
| AEIEYLEK_497.8_389.2 | LYAM1_HUMAN | 0.42041448 |
| TSYQVYSK_488.2_787.4 | C163A_HUMAN | 0.419411932 |
| SVVLIPLGAVDDGEHSQNEK_703.0_798.4 | CNDP1_HUMAN | 0.415325735 |
| ALEQDLPVNIK_620.4_570.4 | CNDP1_HUMAN | 0.407951733 |
| ILDGGNK_358.7_603.3 | CXCL5_HUMAN | 0.401059572 |

**Table 48. Random Forest Protein Late Window**

| **Variable** | **UniProt_ID** | **MeanDecreaseGini** |
|---|---|---|
| AVYEAVLR_460.8_587.4 | PEPD_HUMAN | 1.836010146 |
| AEIEYLEK_497.8_552.3 | LYAM1_HUMAN | 1.739802548 |
| AALAAFNAQNNGSNFQLEEISR_789.1_746.4 | FETUA_HUMAN | 1.455337749 |
| TGVAVNKPAEFTVDAK_549.6_258.1 | FLNA_HUMAN | 1.395043941 |
| AYSDLSR_406.2_375.2 | SAMP_HUMAN | 1.177349958 |
| LIEIANHVDK_384.6_683.4 | ADA12_HUMAN | 1.14243936 |
| QINSYVK_426.2_496.3 | CBG_HUMAN | 1.05284482 |
| ALEQDLPVNIK_620.4_798.5 | CNDP1_HUMAN | 0.971678206 |
| YISPDQLADLYK_713.4_277.2 | ENOA_HUMAN | 0.902293734 |
| AVDIPGLEAATPYR_736.9_286.1 | TENA_HUMAN | 0.893163413 |
| SPEAEDPLGVER_649.8_314.1 | Z512B_HUMAN | 0.856551531 |
| ILDGGNK_358.7_603.3 | CXCL5_HUMAN | 0.841485153 |
| VGVISFAQK_474.8_580.3 | TFR2_HUMAN | 0.835256078 |
| YYGYTGAFR_549.3_450.3 | TRFL_HUMAN | 0.831195917 |
| YSHYNER_323.5_418.2 | HABP2_HUMAN | 0.814479968 |
| FQLPGQK_409.2_276.1 | PSG1_HUMAN | 0.77635168 |
| YENYTSSFFIR_713.8_756.4 | IL12B_HUMAN | 0.761241391 |
| TEQAAVAR_423.2_615.4 | FA12_HUMAN | 0.73195592 |
| SGVDLADSNQK_567.3_662.3 | VGFR3_HUMAN | 0.72504131 |
| VLSSIEQK_452.3_691.4 | 1433S_HUMAN | 0.713380314 |
| GTYLYNDCPGPGQDTDCR_697.0_666.3 | TNR1A_HUMAN | 0.704248586 |
| TSYQVYSK_488.2_787.4 | C163A_HUMAN | 0.69026345 |
| TLEAQLTPR_514.8_685.4 | HEP2_HUMAN | 0.654641588 |
| AEVIWTSSDHQVLSGK_586.3_300.2 | PD1L1_HUMAN | 0.634751081 |
| TAVTANLDIR_537.3_288.2 | CHL1_HUMAN | 0.619871203 |
| ITENDIQIALDDAK_779.9_632.3 | APOB_HUMAN | 0.606313398 |
| TASDFITK_441.7_781.4 | GELS_HUMAN | 0.593535076 |
| SPQAFYR_434.7_556.3 | REL3_HUMAN | 0.592004045 |
| NHYTESISVAK_624.8_415.2 | NEUR1_HUMAN | 0.588383911 |
| LTTVDIVTLR_565.8_815.5 | IL2RB_HUMAN | 0.587343951 |

**Table 49. Random Forest All Late Window**

| **Variable** | **UniProt_ID** | **MeanDecreaseGini** |
|---|---|---|
| AVYEAVLR_460.8_587.4 | PEPD_HUMAN | 0.437300283 |
| AEIEYLEK_497.8_552.3 | LYAM1_HUMAN | 0.371624293 |
| AALAAFNAQNNGSNFQLEEISR_789.1_746.4 | FETUA_HUMAN | 0.304039734 |
| TGVAVNKPAEFTVDAK_549.6_258.1 | FLNA_HUMAN | 0.280588526 |
| AVYEAVLR_460.8_750.4 | PEPD_HUMAN | 0.266788699 |
| AYSDLSR_406.2_375.2 | SAMP_HUMAN | 0.247412666 |
| VPLALFALNR_ 557.3_620.4 | PEPD_HUMAN | 0.229955358 |
| LIEIANHVDK_384.6_683.4 | ADA12_HUMAN | 0.218186524 |
| ITQDAQLK_458.8_702.4 | CBG_HUMAN | 0.217646659 |
| WSAGLTSSQVDLYIPK_883.0_515.3 | CBG_HUMAN | 0.213840705 |
| FGFGGSTDSGPIR_ 649.3_745.4 | ADA12_HUMAN | 0.212794469 |
| LIEIANHVDK_384.6_498.3 | ADA12_HUMAN | 0.208620264 |
| QINSYVK_426.2_610.3 | CBG_HUMAN | 0.202054546 |
| QINSYVK_426.2_496.3 | CBG_HUMAN | 0.197235139 |
| FQLPGQK_409.2_429.2 | PSG1_HUMAN | 0.188311102 |
| VFQYIDLHQDEFVQTLK_708.4_375.2 | CNDP1_HUMAN | 0.180534913 |
| ALEQDLPVNIK_620.4_798.5 | CNDP1_HUMAN | 0.178464358 |
| YYGYTGAFR_549.3_450.3 | TRFL_HUMAN | 0.176050092 |
| ALFLDALGPPAVTR_720.9_640.4 | INHA_HUMAN | 0.171492975 |
| FQLPGQK_409.2_276.1 | PSG1_HUMAN | 0.167576198 |
| SETEIHQGFQHLHQLFAK_717.4_447.2 | CBG_HUMAN | 0.162231844 |
| ALEQDLPVNIK_620.4_570.4 | CNDP1_HUMAN | 0.162165399 |
| VPSHAVVAR_312.5_515.3 | TRFL_HUMAN | 0.156742065 |
| AVDIPGLEAATPYR_736.9_286.1 | TENA_HUMAN | 0.153681405 |
| FTFTLHLETPKPSISSSNLNPR_829.4_874.4 | PSG1_HUMAN | 0.152042057 |
| VGVISFAQK_474.8_580.3 | TFR2_HUMAN | 0.149034355 |
| TLPFSR_360.7_506.3 | LYAM1_HUMAN | 0.143223501 |
| SLDFTELDVAAEK_719.4_874.5 | ANGT_HUMAN | 0.141216186 |
| SPEAEDPLGVER_649.8_314.1 | Z512B_HUMAN | 0.139843479 |
| YGIEEHGK_311.5_341.2 | CXA1_HUMAN | 0.135236953 |

**Table 50. Selected Transitions for Early Window**

| **Transition** | **Parent Protein** |
|---|---|
| LIQDAVTGLTVNGQITGDK_ 972.0_798.4 | ITIH3_HUMAN |
| VQTAHFK_ 277.5_431.2 | CO8A_ HUMAN |
| FLNWIK_ 410.7_560.3 | HABP2_HUMAN |
| ITGFLKPGK_ 320.9_429.3 | LBP_HUMAN |
| ALNHLPLEYNSALYSR_ 621.0_538.3 | CO6_ HUMAN |
| TYLHTYESEI_ 628.3_908.4 | ENPP2_HUMAN |
| LIENGYFHPVK_ 439.6_627.4 | F13B_HUMAN |
| AVLHIGEK_ 289.5_292.2 | THBG_HUMAN |
| QALEEFQK_496.8_680.3 | CO8B_ HUMAN |
| TEFLSNYLTNVDDITLVPGTLGR_ 846.8_600.3 | ENPP2_HUMAN |
| TASDFITK_ 441.7_781.4 | GELS_ HUMAN |
| LPNNVLQEK_ 527.8_844.5 | AFAM_HUMAN |
| AHYDLR_ 387.7_288.2 | FETUA_HUMAN |
| ITLPDFTGDLR_ 624.3_288.2 | LBP_HUMAN |
| IEGNLIFDPNNYLPK_ 874.0_414.2 | APOB_HUMAN |
| ITGFLKPGK_ 320.9_301.2 | LBP_HUMAN |
| FSVVYAK_ 407.2_381.2 | FETUA_HUMAN |
| ITGFLKPGK_ 320.9_429.3 | LBP_HUMAN |
| VFQFLEK_ 455.8_811.4 | CO5_ HUMAN |
| LIQDAVTGLTVNGQITGDK_ 972.0_798.4 | ITIH3_HUMAN |
| DADPDTFFAK_ 563.8_825.4 | AFAM_HUMAN |

**Table 51. Selected Proteins for Early Window**

| **Protein** | |
|---|---|
| complement component C6 precursor | CO6_HUMAN |
| inter-alpha-trypsin inhibitor heavy chain H3 preproprotein | ITIH3_HUMAN |
| Coagulation factor XIII B chain | F13B_HUMAN |
| Ectonucleotide pyrophosphatase/phosphodiesterase family member 2 | ENPP2_HUMAN |
| Complement component C8 beta chain | CO8B_HUMAN |
| thyroxine-binding globulin precursor | THBG_HUMAN |
| Hyaluronan-binding protein 2 | HABP2_HUMAN |
| lipopolysaccharide-binding protein | LBP_HUMAN |
| Complement factor B | CFAB_HUMAN |
| Gelsolin | GELS_HUMAN |
| afamin precursor | AFAM_HUMAN |
| apolipoprotein B-100 precursor | APOB_HUMAN |
| complement component C5 | CO5_HUMAN |
| Alpha-2-HS-glycoprotein | FETUA_HUMAN |
| complement component C8 gamma chain | CO8G_HUMAN |

**Table 52. Selected Transitions for Middle-Late Window**

| **Transition** | **Patent Protein** |
|---|---|
| VPLALFALNR_ 557.3_620.4 | PEPD_HUMAN |
| VFQFLEK_455.8_811.4 | CO5_HUMAN |
| AQPVQVAEGSEPDGFWEALGGK_758.0_574.3 | GELS_HUMAN |
| LIEIANHVDK_384.6_498.3 | ADA12_HUMAN |
| TLAFVR_353.7_492.3 | FA7_HUMAN |
| ALNHLPLEYNSALYSR_621.0_696.4 | CO6_HUMAN |
| AVYEAVLR_460.8_587.4 | PEPD_HUMAN |
| SEPRPGVLLR_375.2_654.4 | FA7_HUMAN |
| TYLHTYESEI_628.3_515.3 | ENPP2_HUMAN |
| ALNHLPLEYNSALYSR_621.0_538.3 | CO6_HUMAN |

**Table 53. Selected Proteins for Middle-Late Window**

| **Protein** | |
|---|---|
| Xaa-Pro dipeptidase | PEPD_HUMAN |
| Leucyl-cystinyl aminopeptidase | LCAP_HUMAN |
| complement component C5 | CO5_HUMAN |
| Gelsolin | GELS_HUMAN |
| complement component C6 precursor | CO6_HUMAN |
| Endoglin precursor | EGLN_HUMAN |
| EGF-containing fibulin-like extracellular matrix protein 1 | FBLN3_HUMAN |
| coagulation factor VII isoform a | FA7_HUMAN |
| Disintegrin and metalloproteinase domain-containing protein 12 | ADA12_HUMAN |
| vitamin D-binding protein isoform 1 precursor | VTDB_HUMAN |
| coagulation factor XII precursor | FA12_HUMAN |
| Corticosteroid-binding globulin | CBG_HUMAN |

### Example 6. Study V to Further Refine Preterm Birth Biomarkers

A additional hypothesis-dependent discovery study was performed with a further refined scheduled MRM assay. Less robust transitions were again removed to improve analytical performance and make room for the inclusion of stable-isotope labeled standards (SIS) corresponding to 79 analytes of interest identified in previous studies. SIS peptides have identical amino acid sequence, chromatographic and MS fragmentation behaviour as their endogenous peptide counterparts, but differ in mass. Therefore they can be used to reduce LC-MS analytical variability and confirm analyte identity. Samples included approximately 60 spontaneous PTB cases (delivery at less than 37 weeks, 0 days), and 180 term controls (delivery at greater than or equal to 37 weeks, 0 days). Each case was designated a "matched" control to within one day of blood draw and two "random" controls matched to the same 3 week blood draw window (17-19, 20-22 or 23-25 weeks gestation). For the purposes of analysis these three blood draw windows were combined. Samples were processed essentially as described previously, except that in this study, tryptic digests were reconstituted in a solution containing SIS standards. Raw analyte peak areas were Box-Cox transformed, corrected for run order and batch effects by regression and used for univariate and multivariate statistical analyses. Univariate analysis included determination of p-values for adjusted peak areas for all analytes from t-tests considering cases vs controls defined as either deliveries at >37 weeks (Table 54) or deliveries at >40 weeks (Table 55). Univariate analysis also included the determination of p-values for a linear model that evaluates the dependence of each analyte's adjusted peak area on the time to birth (gestational age at birth minus the gestational age at blood draw) (Table 56) and the gestational age at birth (Table 57). Additionally raw peak area ratios were calculated for endogenous analytes and their corresponding SIS counterparts, Box-Cox transformed and then used for univariate and multivariate statistical analyses. The above univariate analysis was repeated for analyte/SIS peak area ratio values, summarized in Tables 58-61, respectively.

Multivariate random forest regression models were built using analyte values and clinical variables (e.g. Maternal age, (MAGE), Body mass index, (BMI)) to predict Gestational Age at Birth (GAB). The accuracy of the random forest was evaluated with respect to correlation of the predicted and actual GAB, and with respect to the mean absolute deviation (MAD) of the predicted from actual GAB. The accuracy was further evaluated by determining the area under the receiver operating characteristic curve (AUC) when using the predicted GAB as a quantitative variable to classify subjects as full term or pre-term. Random Forest Importance Values were fit to an Empirical Cumulative Disribution Function and probabilities (P) were calculated. We report the analytes by importance ranking (P>0.7) in the random forest models, using adjusted analyte peak area values (Table 62) and analyte/SIS peak area ratio values (Table 63).

The probability of pre-term birth, p(PTB), may be estimated using the predicted gestational age at birth (GAB) as follows. The estimate will be based on women enrolled in the Sera PAPR clinical trial, which provided the subjects used to develop the PTB prediction methods.

Among women with a predicted GAB of j days plus or minus k days, p(PTB) was estimated as the proportion of women in the PAPR clinical trial with a predicted GAB of j days plus or minus k days who actually deliver before 37 weeks gestational age.

More generally, for women with a predicted GAB of j days plus or minus k days, the probability that the actual gestational age at birth will be less than a specified gestational age, p(actual GAB < specified GAB), was estimated as the proportion of women in the PAPR clinical trial with a predicted GAB of j days plus or minus k days who actually deliver before the specified gestational age. Figure 1 depicts a scatterplot of actual gestational age at birth versus predicted gestational age from random forest regression model. Firgure 2 shows the distribution of predicted gestational age from random forest regression model versus actual gestational age at birth (GAB), where actual GAB was given in categories of (i) less than 37 weeks, (ii) 37 to 39 weeks, and (iii) 40 weeks or greater.

**Table 54. Univariate p-values for Adjusted Peak Areas (<37 vs >37 weeks)**

| Transition | Protein | pvalue |
|---|---|---|
| SPELQAEAK 486.8 659.4 | APOA2 HUMAN | 0.00246566 |
| ALALPPLGLAPLLNLWAKPQGR_ 770.5_457.3 | SHBG_ HUMAN | 0.002623332 |
| ALALPPLGLAPLLNLWAKPQGR_ 770.5_256.2 | SHBG_ HUMAN | 0.002822593 |
| SPELQAEAK_486.8_788.4 | APOA2_HUMAN | 0.003183869 |
| VVLSSGSGPGLDLPLVLGLPLQLK_ 791.5_768.5 | SHBG_ HUMAN | 0.004936049 |
| VVLSSGSGPGLDLPLVLGLPLQLK_ 791.5_598.4 | SHBG_ HUMAN | 0.005598977 |
| DYWSTVK_ 449.7_347.2 | APOC3_HUMAN | 0.005680405 |
| DYWSTVK_ 449.7_620.3 | APOC3_HUMAN | 0.006288693 |
| WGAAPYR_ 410.7_634.3 | PGRP2_HUMAN | 0.006505238 |
| DALSSVQESQVAQQAR_ 573.0_502.3 | APOC3_HUMAN | 0.007626246 |
| DALSSVQESQVAQQAR_ 573.0_672.4 | APOC3_HUMAN | 0.008149335 |
| LSIPQITTK_ 500.8_687.4 | PSG5_HUMAN | 0.009943955 |
| GWVTDGFSSLK_598.8_854.4 | APOC3_HUMAN | 0.010175055 |
| IALGGLLFPASNLR_ 481.3_657.4 | SHBG_ HUMAN | 0.010784167 |
| AKPALEDLR_ 506.8_813.5 | APOA1_HUMAN | 0.011331968 |
| WGAAPYR_ 410.7_577.3 | PGRP2_HUMAN | 0.011761088 |
| VPLALFALNR_ 557.3_620.4 | PEPD HUMAN | 0.014050395 |
| FSLVSGWGQLLDR_ 493.3_447.3 | FA7_HUMAN | 0.014271151 |
| LSIPQITTK_ 500.8_800.5 | PSG5_HUMAN | 0.014339942 |
| TLAFVR_ 353.7_274.2 | FA7_HUMAN | 0.014459876 |
| DVLLLVHNLPQNLPGYFWYK_ 810.4_960.5 | PSG9_HUMAN | 0.016720007 |
| FSVVYAK_ 407.2_381.2 | FETUA_HUMAN | 0.016792786 |
| DVLLLVHNLPQNLPGYFWYK_ 810.4_215.1 | PSG9_HUMAN | 0.017335929 |
| SEPRPGVLLR_375.2_654.4 | FA7_HUMAN | 0.018147773 |
| ALNHLPLEYNSALYSR_ 621.0_538.3 | CO6_ HUMAN | 0.019056484 |
| WNFAYWAAHQPWSR_ 607.3_545.3 | PRG2_HUMAN | 0.019190043 |
| ALNHLPLEYNSALYSR_ 621.0_696.4 | CO6_ HUMAN | 0.020218682 |
| AQPVQVAEGSEPDGFWEALGGK_ 758.0_623.4 | GELS_ HUMAN | 0.020226218 |
| GWVTDGFSSLK_598.8_953.5 | APOC3_HUMAN | 0.023192703 |
| IALGGLLFPASNLR_ 481.3_412.3 | SHBG_ HUMAN | 0.023916911 |
| WNFAYWAAHQPWSR_ 607.3_673.3 | PRG2_HUMAN | 0.026026975 |
| FGFGGSTDSGPIR_ 649.3_745.4 | ADA12_HUMAN | 0.027731407 |
| SEYGAALAWEK_612.8_788.4 | CO6_ HUMAN | 0.031865281 |
| DADPDTFFAK_ 563.8_302.1 | AFAM_HUMAN | 0.0335897 |
| LFIPQITR_ 494.3_614.4 | PSG9_HUMAN | 0.034140767 |
| DVLLLVHNLPQNLPGYFWYK_ 810.4_328.2 | PSG9_HUMAN | 0.034653304 |
| TLAFVR_ 353.7_492.3 | FA7_HUMAN | 0.036441189 |
| AVLHIGEK_ 289.5_292.2 | THBG_HUMAN | 0.038539433 |
| IHPSYTNYR_ 384.2_452.2 | PSG2_HUMAN | 0.039733019 |
| AGLLRPDYALLGHR_ 518.0_369.2 | PGRP2_HUMAN | 0.040916226 |
| ILILPSVTR_ 506.3_559.3 | PSGx_HUMAN | 0.042460036 |
| YYLQGAK_ 421.7_516.3 | ITIH4_HUMAN | 0.044511962 |
| TPSAAYLWVGTGASEAEK_ 919.5_849.4 | GELS_ HUMAN | 0.046362381 |
| AGLLRPDYALLGHR_ 518.0_595.4 | PGRP2_HUMAN | 0.046572355 |
| TYLHTYESEI_ 628.3_908.4 | ENPP2_HUMAN | 0.04754503 |
| FSLVSGWGQLLDR_ 493.3_403.2 | FA7_HUMAN | 0.048642964 |
| VNFTEIQK_ 489.8_765.4 | FETA_HUMAN | 0.04871392 |
| LFIPQITR_ 494.3_727.4 | PSG9_HUMAN | 0.049288923 |
| DISEVVTPR_ 508.3_787.4 | CFAB_ HUMAN | 0.049458374 |
| SEPRPGVLLR_375.2_454.3 | FA7_HUMAN | 0.049567047 |

**Table 55. Univariate p-values for Adjusted Peak Areas (<37 vs >40 weeks)**

| Transition | Protein | pvalue |
|---|---|---|
| SPELQAEAK_486.8_659.4 | APOA2_HUMAN | 0.001457796 |
| DYWSTVK_ 449.7_347.2 | APOC3_HUMAN | 0.001619622 |
| DYWSTVK_ 449.7_620.3 | APOC3_HUMAN | 0.002068704 |
| DALSSVQESQVAQQAR_ 573.0_502.3 | APOC3_HUMAN | 0.00250563 |
| GWVTDGFSSLK_598.8_854.4 | APOC3_HUMAN | 0.002543943 |
| SPELQAEAK_486.8_788.4 | APOA2_HUMAN | 0.003108814 |
| SEPRPGVLLR_375.2_654.4 | FA7_HUMAN | 0.004035832 |
| DALSSVQESQVAQQAR_ 573.0_672.4 | APOC3_HUMAN | 0.00434652 |
| SEYGAALAWEK_612.8_788.4 | CO6_ HUMAN | 0.005306924 |
| GWVTDGFSSLK_598.8_953.5 | APOC3_HUMAN | 0.005685534 |
| ALNHLPLEYNSALYSR_ 621.0_696.4 | CO6_ HUMAN | 0.005770384 |
| TYLHTYESEI_ 628.3_515.3 | ENPP2_HUMAN | 0.005798991 |
| ENPAVIDFELAPIVDLVR_ 670.7_601.4 | CO6_ HUMAN | 0.006248095 |
| ALNHLPLEYNSALYSR_ 621.0_538.3 | CO6_ HUMAN | 0.006735817 |
| TYLHTYESEI_ 628.3_908.4 | ENPP2_HUMAN | 0.007351774 |
| AGLLRPDYALLGHR_ 518.0_369.2 | PGRP2_HUMAN | 0.009541521 |
| AKPALEDLR_ 506.8_813.5 | APOA1_HUMAN | 0.009780371 |
| SEYGAALAWEK_612.8_845.5 | CO6_ HUMAN | 0.010085363 |
| FSLVSGWGQLLDR_ 493.3_447.3 | FA7_HUMAN | 0.010401836 |
| WGAAPYR_ 410.7_634.3 | PGRP2_HUMAN | 0.011233623 |
| ENPAVIDFELAPIVDLVR_ 670.7_811.5 | CO6_ HUMAN | 0.012029564 |
| DVLLLVHNLPQNLPGYFWYK_ 810.4_215.1 | PSG9_HUMAN | 0.014808277 |
| LFIPQITR_ 494.3_614.4 | PSG9_HUMAN | 0.015879755 |
| WGAAPYR_ 410.7_577.3 | PGRP2_HUMAN | 0.016562435 |
| AGLLRPDYALLGHR_ 518.0_595.4 | PGRP2_HUMAN | 0.016793521 |
| TLAFVR_ 353.7_492.3 | FA7_HUMAN | 0.016919708 |
| FSLVSGWGQLLDR_ 493.3_403.2 | FA7_HUMAN | 0.016937583 |
| WWGGQPLWITATK_ 772.4_373.2 | ENPP2_HUMAN | 0.019050115 |
| GYVIIKPLVWV_643.9_304.2 | SAMP_ HUMAN | 0.019675317 |
| DVLLLVHNLPQNLPGYFWYK_ 810.4_960.5 | PSG9_HUMAN | 0.020387647 |
| FGFGGSTDSGPIR_ 649.3_745.4 | ADA12_HUMAN | 0.020458335 |
| DVLLLVHNLPQNLPGYFWYK_ 810.4_328.2 | PSG9_HUMAN | 0.021488084 |
| WWGGQPLWITATK_ 772.4_929.5 | ENPP2_HUMAN | 0.021709354 |
| LDFHFSSDR_ 375.2_448.2 | INHBC_HUMAN | 0.022403383 |
| LFIPQITR_ 494.3_727.4 | PSG9_HUMAN | 0.025561103 |
| TEFLSNYLTNVDDITLVPGTLGR_ 846.8_600.3 | ENPP2_HUMAN | 0.029344366 |
| LSIPQITTK_ 500.8_800.5 | PSG5_HUMAN | 0.031361776 |
| ALVLELAK_ 428.8_672.4 | INHBE_HUMAN | 0.031690737 |
| SEPRPGVLLR_375.2_454.3 | FA7_HUMAN | 0.033067953 |
| LSIPQITTK_ 500.8_687.4 | PSG5_HUMAN | 0.033972449 |
| LDFHFSSDR_ 375.2_611.3 | INHBC_HUMAN | 0.034500249 |
| LDFHFSSDR_ 375.2_464.2 | INHBC_HUMAN | 0.035166664 |
| GAVHVVVAETDYQSFAVLYLER_822.8_580.3 | CO8G_ HUMAN | 0.037334975 |
| HELTDEELQSLFTNFANVVDK_ 817.1_854.4 | AFAM_HUMAN | 0.039258528 |
| AYSDLSR_ 406.2_375.2 | SAMP_ HUMAN | 0.04036485 |
| YYLQGAK_ 421.7_516.3 | ITIH4_HUMAN | 0.042204165 |
| ILPSVPK_ 377.2_264.2 | PGH1_HUMAN | 0.042397885 |
| ELLESYIDGR_ 597.8_710.4 | THRB_HUMAN | 0.043053589 |
| ALALPPLGLAPLLNLWAKPQGR_ 770.5_256.2 | SHBG_ HUMAN | 0.045692283 |
| VGEYSLYIGR_ 578.8_871.5 | SAMP_ HUMAN | 0.04765767 |
| ANDQYLTAAALHNLDEAVK_ 686.4_317.2 | IL1A_HUMAN | 0.048928376 |
| YYGYTGAFR 549.3_551.3 | TRFL_HUMAN | 0.049568351 |

**Table 56. Univariate p-values for Adjusted Peak Areas in Time to Birth Linear Model**

| Protein | pvalue |
|---|---|
| ADA12_HUMAN | 0.003412707 |
| ENPP2_HUMAN | 0.003767393 |
| ADA12_HUMAN | 0.004194234 |
| ENPP2_HUMAN | 0.004298493 |
| ADA12_HUMAN | 0.004627197 |
| ADA12_HUMAN | 0.004918852 |
| ENPP2_HUMAN | 0.005792374 |
| CO6_ HUMAN | 0.005858282 |
| ENPP2_HUMAN | 0.007123606 |
| CO6_ HUMAN | 0.007162317 |
| ENPP2_HUMAN | 0.008228726 |
| ENPP2_HUMAN | 0.009168492 |
| PSG9_HUMAN | 0.011531192 |
| PSG9_HUMAN | 0.019389627 |
| PSG9_HUMAN | 0.023680865 |
| INHBE_HUMAN | 0.02581564 |
| B2MG_HUMAN | 0.026544689 |
| LBP_HUMAN | 0.031068274 |
| PSG9_HUMAN | 0.031091843 |
| APOA2_HUMAN | 0.033130498 |
| INHBC_HUMAN | 0.03395215 |
| CBG_ HUMAN | 0.034710348 |
| PSGx_HUMAN | 0.035719227 |
| CBG_ HUMAN | 0.036331871 |
| CSH_ HUMAN | 0.039896611 |
| CSH_ HUMAN | 0.04244001 |
| SAMP_ HUMAN | 0.047112128 |
| LBP_HUMAN | 0.048141371 |
| LBP_HUMAN | 0.048433174 |
| CO6_ HUMAN | 0.04850949 |
| PSGx_HUMAN | 0.049640167 |

**Table 57. Univariate p-values for Adjusted Peak Areas in Gestation Age at Birth Linear Model**

| Transition | Protein | pvalue |
|---|---|---|
| ENPAVIDFELAPIVDLVR_ 670.7_811.5 | CO6_ HUMAN | 0.000117239 |
| ENPAVIDFELAPIVDLVR_ 670.7_601.4 | CO6_ HUMAN | 0.000130113 |
| TYLHTYESEI_ 628.3_908.4 | ENPP2_HUMAN | 0.000160472 |
| TYLHTYESEI_ 628.3_515.3 | ENPP2_HUMAN | 0.000175167 |
| TEFLSNYLTNVDDITLVPGTLGR_ 846.8_600.3 | ENPP2_HUMAN | 0.000219886 |
| TEFLSNYLTNVDDITLVPGTLGR_ 846.8_699.4 | ENPP2_HUMAN | 0.000328416 |
| WWGGQPLWITATK_ 772.4_373.2 | ENPP2_HUMAN | 0.000354644 |
| WWGGQPLWITATK_ 772.4_929.5 | ENPP2_HUMAN | 0.000390821 |
| SEYGAALAWEK_612.8_788.4 | CO6_ HUMAN | 0.000511882 |
| LDFHFSSDR_ 375.2_448.2 | INHBC_HUMAN | 0.000600637 |
| ALVLELAK_ 428.8_672.4 | INHBE_HUMAN | 0.000732445 |
| GLQYAAQEGLLALQSELLR_1037.1_929.5 | LBP_HUMAN | 0.000743924 |
| DVLLLVHNLPQNLPGYFWYK_ 810.4_960.5 | PSG9_HUMAN | 0.000759173 |
| FGFGGSTDSGPIR_ 649.3_745.4 | ADA12_HUMAN | 0.001224347 |
| DVLLLVHNLPQNLPGYFWYK_ 810.4_328.2 | PSG9_HUMAN | 0.001241329 |
| GYVIIKPLVWV_643.9_304.2 | SAMP_ HUMAN | 0.001853785 |
| SPELQAEAK_486.8_659.4 | APOA2_HUMAN | 0.001856303 |
| GLQYAAQEGLLALQSELLR_1037.1_858.5 | LBP_HUMAN | 0.001978165 |
| LDFHFSSDR_ 375.2_611.3 | INHBC_HUMAN | 0.002098948 |
| LIEIANHVDK_ 384.6_683.4 | ADA12_HUMAN | 0.002212096 |
| SFRPFVPR_335.9_272.2 | LBP_HUMAN | 0.002545286 |
| SFRPFVPR_335.9_635.3 | LBP_HUMAN | 0.002620268 |
| WSAGLTSSQVDLYIPK_ 883.0_515.3 | CBG_ HUMAN | 0.002787272 |
| DLHLSDVFLK_ 396.2_260.2 | CO6_ HUMAN | 0.002954612 |
| LIEIANHVDK_ 384.6_498.3 | ADA12_HUMAN | 0.002955081 |
| DVLLLVHNLPQNLPGYFWYK_ 810.4_215.1 | PSG9_HUMAN | 0.003541011 |
| LFIPQITR_ 494.3_614.4 | PSG9_HUMAN | 0.003750666 |
| FGFGGSTDSGPIR_ 649.3_946.5 | ADA12_HUMAN | 0.003773696 |
| YYLQGAK_ 421.7_516.3 | ITIH4_HUMAN | 0.004064026 |
| SEYGAALAWEK_612.8_845.5 | CO6_ HUMAN | 0.004208136 |
| AITPPHPASQANIIFDITEGNLR_ 825.8_459.3 | FBLN1_HUMAN | 0.004709104 |
| LDFHFSSDR_ 375.2_464.2 | INHBC_HUMAN | 0.005355741 |
| HELTDEELQSLFTNFANVVDK_ 817.1_854.4 | AFAM_HUMAN | 0.005370567 |
| ALNHLPLEYNSALYSR_ 621.0_696.4 | CO6_ HUMAN | 0.005705922 |
| ITQDAQLK_ 458.8_702.4 | CBG_ HUMAN | 0.006762484 |
| ITLPDFTGDLR_ 624.3_920.5 | LBP_HUMAN | 0.006993268 |
| SILFLGK_389.2_577.4 | THBG_HUMAN | 0.007134146 |
| WSAGLTSSQVDLYIPK_ 883.0_357.2 | CBG_ HUMAN | 0.007670388 |
| GVTSVSQIFHSPDLAIR_609.7_472.3 | IC1_ HUMAN | 0.007742729 |
| VGEYSLYIGR_ 578.8_871.5 | SAMP_ HUMAN | 0.007778691 |
| ITLPDFTGDLR_ 624.3_288.2 | LBP_HUMAN | 0.008179918 |
| YYLQGAK_ 421.7_327.1 | ITIH4_HUMAN | 0.008404686 |
| ALNHLPLEYNSALYSR_ 621.0_538.3 | CO6_ HUMAN | 0.008601162 |
| DYWSTVK_ 449.7_620.3 | APOC3_HUMAN | 0.008626786 |
| TVQAVLTVPK_ 528.3_855.5 | PEDF_HUMAN | 0.008907523 |
| ITGFLKPGK_ 320.9_301.2 | LBP_HUMAN | 0.009155417 |
| LFIPQITR_ 494.3_727.4 | PSG9_HUMAN | 0.009571006 |
| SPELQAEAK_486.8_788.4 | APOA2_HUMAN | 0.009776508 |
| DYWSTVK_ 449.7_347.2 | APOC3_HUMAN | 0.00998356 |
| ITGFLKPGK_ 320.9_429.3 | LBP_HUMAN | 0.010050264 |
| FLNWIK_ 410.7_560.3 | HABP2_HUMAN | 0.010372454 |
| DLHLSDVFLK_ 396.2_366.2 | CO6_ HUMAN | 0.010806378 |
| GVTSVSQIFHSPDLAIR_609.7_908.5 | IC1_ HUMAN | 0.011035991 |
| VEHSDLSFSK_ 383.5_468.2 | B2MG_HUMAN | 0.011113172 |
| LLDSLPSDTR_ 558.8_276.2 | IC1_HUMAN | 0.011589013 |
| LLDSLPSDTR_ 558.8_890.4 | IC1_HUMAN | 0.011629438 |
| QALEEFQK_496.8_551.3 | CO8B_ HUMAN | 0.011693839 |
| LLDSLPSDTR_ 558.8_575.3 | IC1_HUMAN | 0.012159314 |
| IIGGSDADIK_ 494.8_762.4 | C1S_ HUMAN | 0.013080243 |
| AFIQLWAFDAVK_ 704.9_650.4 | AMBP_HUMAN | 0.013462234 |
| GFQALGDAADIR_617.3_717.4 | TIMP1_ HUMAN | 0.014370997 |
| LPNNVLQEK_ 527.8_730.4 | AFAM_HUMAN | 0.014424891 |
| DTDTGALLFIGK_ 625.8_217.1 | PEDF_HUMAN | 0.014967952 |
| VQTAHFK_ 277.5_502.3 | CO8A_ HUMAN | 0.01524844 |
| ILILPSVTR_ 506.3_559.3 | PSGx_HUMAN | 0.015263132 |
| SILFLGK_389.2_201.1 | THBG_HUMAN | 0.015265233 |
| TVQAVLTVPK_ 528.3_428.3 | PEDF_HUMAN | 0.015344052 |
| VEPLYELVTATDFAYSSTVR_ 754.4_712.4 | CO8B_ HUMAN | 0.015451068 |
| FSLVSGWGQLLDR_ 493.3_447.3 | FA7_HUMAN | 0.015510454 |
| GWVTDGFSSLK_598.8_854.4 | APOC3_HUMAN | 0.01610797 |
| LSETNR_ 360.2_519.3 | PSG1_HUMAN | 0.016433362 |
| TQILEWAAER_ 608.8_632.3 | EGLN_HUMAN | 0.01644844 |
| SETEIHQGFQHLHQLFAK_717.4_318.1 | CBG_ HUMAN | 0.016720367 |
| TNLESILSYPK_ 632.8_936.5 | IC1_HUMAN | 0.017314185 |
| TNLESILSYPK_ 632.8_807.5 | IC1_HUMAN | 0.017593786 |
| AYSDLSR_ 406.2_375.2 | SAMP_ HUMAN | 0.018531348 |
| YEVQGEVFTKPQLWP_ 911.0_392.2 | CRP_ HUMAN | 0.019111323 |
| AYSDLSR_ 406.2_577.3 | SAMP_ HUMAN | 0.019271266 |
| QALEEFQK_496.8_680.3 | CO8B_ HUMAN | 0.019429489 |
| APLTKPLK_ 289.9_398.8 | CRP_ HUMAN | 0.020110081 |
| FQPTLLTLPR_ 593.4_276.1 | IC1_HUMAN | 0.020114306 |
| ITQDAQLK_ 458.8_803.4 | CBG_ HUMAN | 0.020401782 |
| AVLHIGEK_ 289.5_292.2 | THBG_HUMAN | 0.02056597 |
| ANDQYLTAAALHNLDEAVK_ 686.4_317.2 | IL1A_HUMAN | 0.020770124 |
| VGEYSLYIGR_ 578.8_708.4 | SAMP_ HUMAN | 0.021126414 |
| TLYSSSPR_ 455.7_533.3 | IC1_HUMAN | 0.021306106 |
| VEHSDLSFSK_ 383.5_234.1 | B2MG_HUMAN | 0.021640643 |
| HELTDEELQSLFTNFANVVDK_ 817.1_906.5 | AFAM_HUMAN | 0.021921609 |
| TLYSSSPR_ 455.7_696.3 | IC1_HUMAN | 0.022196181 |
| GYVIIKPLVWV_643.9_854.6 | SAMP_ HUMAN | 0.023126336 |
| DEIPHNDIALLK_ 459.9_260.2 | HABP2_HUMAN | 0.023232158 |
| ILILPSVTR_ 506.3_785.5 | PSGx_HUMAN | 0.023519909 |
| WNFAYWAAHQPWSR_ 607.3_545.3 | PRG2_HUMAN | 0.023697087 |
| FQPTLLTLPR_ 593.4_712.5 | IC1_HUMAN | 0.023751959 |
| AQPVQVAEGSEPDGFWEALGGK_ 758.0_623.4 | GELS_ HUMAN | 0.024262721 |
| DEIPHNDIALLK_ 459.9_510.8 | HABP2_HUMAN | 0.024414348 |
| GDSGGAFAVQDPNDK_739.3_716.3 | C1S_ HUMAN | 0.025075028 |
| FLNWIK_ 410.7_561.3 | HABP2_HUMAN | 0.025649617 |
| APLTKPLK_ 289.9_357.2 | CRP_ HUMAN | 0.025961162 |
| ALDLSLK_ 380.2_185.1 | ITIH3_HUMAN | 0.026233504 |
| GWVTDGFSSLK_598.8_953.5 | APOC3_HUMAN | 0.026291884 |
| SETEIHQGFQHLHQLFAK_717.4_447.2 | CBG_ HUMAN | 0.026457136 |
| GDSGGAFAVQDPNDK_739.3_473.2 | C1S_ HUMAN | 0.02727457 |
| YEVQGEVFTKPQLWP_ 911.0_293.1 | CRP_ HUMAN | 0.028244448 |
| HVVQLR_ 376.2_614.4 | IL6RA_HUMAN | 0.028428028 |
| DTDTGALLFIGK_ 625.8_818.5 | PEDF_HUMAN | 0.028773557 |
| EVPLSALTNILSAQLISHWK_ 740.8_996.6 | PAI1_HUMAN | 0.029150774 |
| AFTECCVVASQLR_ 770.9_574.3 | CO5_ HUMAN | 0.029993325 |
| TLAFVR_ 353.7_492.3 | FA7_HUMAN | 0.030064307 |
| LWAYLTIQELLAK_ 781.5_300.2 | ITIH1_ HUMAN | 0.030368674 |
| DEIPHNDIALLK_ 459.9_245.1 | HABP2_HUMAN | 0.031972082 |
| AGLLRPDYALLGHR_ 518.0_369.2 | PGRP2_HUMAN | 0.032057409 |
| AVYEAVLR_ 460.8_587.4 | PEPD_HUMAN | 0.032527521 |
| LPNNVLQEK_ 527.8_844.5 | AFAM_HUMAN | 0.033807082 |
| GAVHVVVAETDYQSFAVLYLER_822.8_580.3 | CO8G_ HUMAN | 0.034370139 |
| WNFAYWAAHQPWSR_ 607.3_673.3 | PRG2_HUMAN | 0.0349737 |
| EAQLPVIENK_ 570.8_329.2 | PLMN_HUMAN | 0.035304322 |
| VQEAHLTEDQIFYFPK_ 655.7_701.4 | CO8G_ HUMAN | 0.035704382 |
| AFIQLWAFDAVK_ 704.9_836.4 | AMBP_HUMAN | 0.035914532 |
| SGFSFGFK_438.7_585.3 | CO8B_ HUMAN | 0.037168221 |
| SGFSFGFK_438.7_732.4 | CO8B_ HUMAN | 0.040182596 |
| DADPDTFFAK_ 563.8_302.1 | AFAM_HUMAN | 0.041439744 |
| EAQLPVIENK_ 570.8_699.4 | PLMN_HUMAN | 0.041447675 |
| IIGGSDADIK_ 494.8_260.2 | C1S_ HUMAN | 0.041683256 |
| AVLTIDEK_ 444.8_718.4 | A1AT_HUMAN | 0.043221658 |
| SEPRPGVLLR_375.2_654.4 | FA7_HUMAN | 0.044079127 |
| YHFEALADTGISSEFYDNANDLLSK_ 940.8_874.5 | CO8A_ HUMAN | 0.045313634 |
| HFQNLGK_ 422.2_527.2 | AFAM_HUMAN | 0.047118971 |
| LEQGENVFLQATDK_ 796.4_822.4 | C1QB_ HUMAN | 0.047818928 |
| NTVISVNPSTK_ 580.3_732.4 | VCAM1_HUMAN | 0.048102262 |
| YYGYTGAFR_ 549.3_551.3 | TRFL_HUMAN | 0.048331316 |
| ISLLLIESWLEPVR_ 834.5_500.3 | CSH_ HUMAN | 0.049561581 |
| LQVLGK_ 329.2_416.3 | A2GL_HUMAN | 0.049738493 |

**Table 58. Univariate p-values for Peak Area Ratios (<37 vs >37 weeks)**

| UniProt_ID | Transition | pvalue |
|---|---|---|
| SHBG_ HUMAN | IALGGLLFPASNLR_ 481.3_657.4 | 0.006134652 |
| SHBG_ HUMAN | IALGGLLFPASNLR_ 481.3_412.3 | 0.019049498 |
| APOC3_HUMAN | DALSSVQESQVAQQAR_ 573.0_672.4 | 0.020688543 |
| THBG_HUMAN | AVLHIGEK_ 289.5_292.2 | 0.0291698 |
| PSG9_HUMAN | DVLLLVHNLPQNLPGYFWYK_ 810.4_960.5 | 0.033518454 |
| APOC3_HUMAN | DALSSVQESQVAQQAR_ 573.0_502.3 | 0.043103265 |
| PSG9_HUMAN | LFIPQITR_ 494.3_614.4 | 0.04655948 |

**Table 59. Univariate p-values for Peak Area Ratios (<37 vs >40 weeks)**

| UniProt ID | Transition | pvalue |
|---|---|---|
| APOC3_HUMAN | DALSSVQESQVAQQAR_ 573.0_672.4 | 0.011174438 |
| APOC3_HUMAN | DALSSVQESQVAQQAR_ 573.0_502.3 | 0.015231617 |
| PSG9_HUMAN | LFIPQITR_ 494.3_614.4 | 0.018308413 |
| PSG9_HUMAN | LFIPQITR_ 494.3_727.4 | 0.027616871 |
| PSG9_HUMAN | DVLLLVHNLPQNLPGYFWYK_ 810.4_960.5 | 0.028117582 |
| THBG_HUMAN | AVLHIGEK_ 289.5_292.2 | 0.038899107 |
| CO6_ HUMAN | ALNHLPLEYNSALYSR_ 621.0_696.4 | 0.040662269 |
| ENPP2_HUMAN | TYLHTYESEI_ 628.3_908.4 | 0.044545826 |

**Table 60. Univariate p-values for Peak Area Ratios in Time to Birth Linear**

| UniProt_ID | Transition | pvalue |
|---|---|---|
| ADA12_HUMAN | FGFGGSTDSGPIR_ 649.3_946.5 | 5.85E-27 |
| ADA12_HUMAN | FGFGGSTDSGPIR_ 649.3_745.4 | 2.65E-24 |
| PSG4_HUMAN | TLFIFGVTK_ 513.3_215.1 | 1.07E-20 |
| PSG4_HUMAN | TLFIFGVTK_ 513.3_811.5 | 2.32E-20 |
| PSGx_HUMAN | ILILPSVTR_ 506.3_785.5 | 8.25E-16 |
| PSGx_HUMAN | ILILPSVTR_ 506.3_559.3 | 9.72E-16 |
| PSG1_HUMAN | FQLPGQK_ 409.2_429.2 | 1.29E-12 |
| PSG11_HUMAN | LFIPQITPK_ 528.8_261.2 | 2.11E-12 |
| PSG1_HUMAN | FQLPGQK_ 409.2_276.1 | 2.33E-12 |
| PSG11_HUMAN | LFIPQITPK_ 528.8_683.4 | 3.90E-12 |

| UniProt ID | Transition | pvalue |
|---|---|---|
| PSG6_HUMAN | SNPVTLNVLYGPDLPR_585.7_817.4 | 5.71E-12 |
| PSG6_HUMAN | SNPVTLNVLYGPDLPR_585.7_654.4 | 1.82E-11 |
| VGFR3_HUMAN | SGVDLADSNQK_567.3_662.3 | 4.57E-11 |
| INHBE_HUMAN | ALVLELAK_ 428.8_331.2 | 1.04E-08 |
| PSG2_HUMAN | IHPSYTNYR_ 384.2_452.2 | 6.27E-08 |
| PSG9_HUMAN | LFIPQITR_ 494.3_727.4 | 1.50E-07 |
| VGFR3_HUMAN | SGVDLADSNQK_567.3_591.3 | 2.09E-07 |
| PSG9_HUMAN | LFIPQITR_ 494.3_614.4 | 2.71E-07 |
| PSG9_HUMAN | DVLLLVHNLPQNLPGYFWYK_ 810.4_960.5 | 3.10E-07 |
| PSG2_HUMAN | IHPSYTNYR_ 384.2_338.2 | 2.55E-06 |
| ITIH3_HUMAN | LIQDAVTGLTVNGQITGDK_ 972.0_640.4 | 2.76E-06 |
| ENPP2_HUMAN | TYLHTYESEI_ 628.3_908.4 | 2.82E-06 |
| ENPP2_HUMAN | WWGGQPLWITATK_ 772.4_373.2 | 3.75E-06 |
| PSG9_HUMAN | DVLLLVHNLPQNLPGYFWYK_ 810.4_328.2 | 3.94E-06 |
| B2MG_HUMAN | VEHSDLSFSK_ 383.5_468.2 | 5.42E-06 |
| ENPP2_HUMAN | WWGGQPLWITATK_ 772.4_929.5 | 7.93E-06 |
| ANGT_HUMAN | ALQDQLVLVAAK_ 634.9_289.2 | 1.04E-05 |
| B2MG_HUMAN | VNHVTLSQPK_ 374.9_244.2 | 1.46E-05 |
| AFAM_HUMAN | LPNNVLQEK_ 527.8_730.4 | 1.50E-05 |
| AFAM_HUMAN | LPNNVLQEK_ 527.8_844.5 | 1.98E-05 |
| THBG_HUMAN | AVLHIGEK_ 289.5_292.2 | 2.15E-05 |
| ENPP2_HUMAN | TYLHTYESEI_ 628.3_515.3 | 2.17E-05 |
| IL12B_HUMAN | DIIKPDPPK_ 511.8_342.2 | 3.31E-05 |
| AFAM_HUMAN | DADPDTFFAK_ 563.8_302.1 | 6.16E-05 |
| THBG_HUMAN | AVLHIGEK_ 289.5_348.7 | 8.34E-05 |
| PSG9_HUMAN | DVLLLVHNLPQNLPGYFWYK_ 810.4_215.1 | 0.000104442 |
| B2MG_HUMAN | VEHSDLSFSK_ 383.5_234.1 | 0.000140786 |
| TRFL_HUMAN | YYGYTGAFR_ 549.3_450.3 | 0.000156543 |
| HEMO_HUMAN | QGHNSVFLIK_381.6_260.2 | 0.000164578 |
| A1BG_HUMAN | LLELTGPK_ 435.8_227.2 | 0.000171113 |
| CO6_ HUMAN | ALNHLPLEYNSALYSR_ 621.0_696.4 | 0.000242116 |
| CO6_ HUMAN | ALNHLPLEYNSALYSR_ 621.0_538.3 | 0.00024681 |
| ALS_HUMAN | IRPHTFTGLSGLR_ 485.6_432.3 | 0.000314359 |
| ITIH2_HUMAN | LSNENHGIAQR_ 413.5_544.3 | 0.0004877 |
| PEDF_HUMAN | TVQAVLTVPK_ 528.3_855.5 | 0.000508174 |
| AFAM_HUMAN | HFQNLGK_ 422.2_527.2 | 0.000522139 |
| FLNA_HUMAN | TGVAVNKPAEFTVDAK_ 549.6_258.1 | 0.000594403 |
| ANGT_HUMAN | ALQDQLVLVAAK_ 634.9_956.6 | 0.000640673 |
| AFAM_HUMAN | HFQNLGK_ 422.2_285.1 | 0.000718763 |
| HGFA_HUMAN | LHKPGVYTR_ 357.5_692.4 | 0.000753293 |
| HGFA_HUMAN | LHKPGVYTR_ 357.5_479.3 | 0.000909298 |
| HABP2_HUMAN | FLNWIK_ 410.7_561.3 | 0.001282014 |
| FETUA_HUMAN | HTLNQIDEVK_ 598.8_951.5 | 0.001389792 |
| AFAM_HUMAN | DADPDTFFAK_ 563.8_825.4 | 0.001498237 |
| B2MG_HUMAN | VNHVTLSQPK_ 374.9_459.3 | 0.001559862 |
| ALS_HUMAN | IRPHTFTGLSGLR_ 485.6_545.3 | 0.001612361 |
| A1BG_HUMAN | LLELTGPK_ 435.8_644.4 | 0.002012656 |
| F13B_HUMAN | LIENGYFHPVK_ 439.6_343.2 | 0.00275216 |
| ITIH2_HUMAN | LSNENHGIAQR_ 413.5_519.8 | 0.00356561 |
| APOC3_HUMAN | DALSSVQESQVAQQAR_ 573.0_672.4 | 0.00392745 |
| F13B_HUMAN | LIENGYFHPVK_ 439.6_627.4 | 0.00434836 |
| PEDF_HUMAN | TVQAVLTVPK_ 528.3_428.3 | 0.00482765 |
| PLMN_HUMAN | YEFLNGR_ 449.7_293.1 | 0.007325436 |
| HEMO_HUMAN | QGHNSVFLIK_381.6_520.4 | 0.009508516 |
| FETUA_HUMAN | HTLNQIDEVK_ 598.8_958.5 | 0.010018936 |
| CO5_ HUMAN | LQGTLPVEAR_ 542.3_842.5 | 0.011140661 |
| PLMN_HUMAN | YEFLNGR_ 449.7_606.3 | 0.01135322 |
| CO5_ HUMAN | TLLPVSKPEIR_ 418.3_288.2 | 0.015045275 |
| HABP2_HUMAN | FLNWIK_ 410.7_560.3 | 0.01523134 |
| APOC3_HUMAN | DALSSVQESQVAQQAR_ 573.0_502.3 | 0.01584708 |
| CO5_ HUMAN | LQGTLPVEAR_ 542.3_571.3 | 0.017298064 |
| CFAB_ HUMAN | DISEVVTPR_ 508.3_472.3 | 0.021743221 |
| CERU_ HUMAN | TTIEKPVWLGFLGPIIK_ 638.0_640.4 | 0.02376225 |
| CO8G_ HUMAN | SLPVSDSVLSGFEQR_810.9_723.3 | 0.041150397 |
| CO8G_ HUMAN | FLQEQGHR_ 338.8_497.3 | 0.042038143 |
| CO5_ HUMAN | VFQFLEK_ 455.8_811.4 | 0.043651929 |
| CO8B_ HUMAN | QALEEFQK_496.8_680.3 | 0.04761631 |

**Table 61. Univariate p-values for Peak Area Ratios in Gestation Age at Birth Linear Model**

| UniProt ID | Transition | pvalue |
|---|---|---|
| PSG9_HUMAN | DVLLLVHNLPQNLPGYFWYK_ 810.4_960.5 | 0.000431547 |
| B2MG_HUMAN | VEHSDLSFSK_ 383.5_468.2 | 0.000561148 |
| PSG9_HUMAN | DVLLLVHNLPQNLPGYFWYK_ 810.4_328.2 | 0.000957509 |
| ENPP2_HUMAN | TYLHTYESEI_ 628.3_908.4 | 0.001058809 |
| THBG_HUMAN | AVLHIGEK_ 289.5_292.2 | 0.001180484 |
| ENPP2_HUMAN | WWGGQPLWITATK_ 772.4_373.2 | 0.001524983 |
| PSG9_HUMAN | LFIPQITR_ 494.3_614.4 | 0.001542932 |
| ENPP2_HUMAN | WWGGQPLWITATK_ 772.4_929.5 | 0.002047607 |
| ENPP2_HUMAN | TYLHTYESEI_ 628.3_515.3 | 0.003087492 |
| PSG9_HUMAN | LFIPQITR_ 494.3_727.4 | 0.00477154 |
| PSG9_HUMAN | DVLLLVHNLPQNLPGYFWYK_ 810.4_215.1 | 0.004824351 |
| THBG_HUMAN | AVLHIGEK_ 289.5_348.7 | 0.006668084 |
| AFAM_HUMAN | LPNNVLQEK_ 527.8_730.4 | 0.006877647 |
| ADA12_HUMAN | FGFGGSTDSGPIR_ 649.3_745.4 | 0.011738104 |
| PEDF_HUMAN | TVQAVLTVPK_ 528.3_855.5 | 0.013349511 |
| A1BG_HUMAN | LLELTGPK_ 435.8_227.2 | 0.015793885 |
| ITIH3_HUMAN | ALDLSLK_ 380.2_185.1 | 0.016080436 |
| ADA12_HUMAN | FGFGGSTDSGPIR_ 649.3_946.5 | 0.017037089 |
| B2MG_HUMAN | VEHSDLSFSK_ 383.5_234.1 | 0.017072093 |
| CO6_ HUMAN | ALNHLPLEYNSALYSR_ 621.0_696.4 | 0.024592775 |
| TRFL_HUMAN | YYGYTGAFR_ 549.3_450.3 | 0.030890831 |

| UniProt_ID | Transition | pvalue |
|---|---|---|
| AFAM_HUMAN | DADPDTFFAK_ 563.8_302.1 | 0.033791429 |
| CO6_ HUMAN | ALNHLPLEYNSALYSR_ 621.0_538.3 | 0.034865341 |
| AFAM_HUMAN | LPNNVLQEK_ 527.8_844.5 | 0.039880594 |
| PEDF_HUMAN | TVQAVLTVPK_ 528.3_428.3 | 0.040854402 |
| PLMN_HUMAN | EAQLPVIENK_ 570.8_329.2 | 0.041023812 |
| LBP_HUMAN | ITLPDFTGDLR_ 624.3_920.5 | 0.042276813 |
| CO8G_ HUMAN | VQEAHLTEDQIFYFPK_ 655.7_701.4 | 0.042353851 |
| PLMN_HUMAN | YEFLNGR_ 449.7_606.3 | 0.04416504 |
| B2MG_HUMAN | VNHVTLSQPK_ 374.9_459.3 | 0.045458409 |
| CFAB_ HUMAN | DISEVVTPR_ 508.3_472.3 | 0.046493405 |
| INHBE_HUMAN | ALVLELAK_ 428.8_331.2 | 0.04789353 |

**Table 62. Random Forest Importance Values Using Adjusted Peak Areas**

| Transition | Rank | Importance |
|---|---|---|
| INHBE_ALVLELAK 428.8 672.4 | 1 | 2964.951571 |
| EGLN_TQILEWAAER 608.8_761.4 | 2 | 1218.3406 |
| FA7_ SEPRPGVLLR_375.2 654.4 | 3 | 998.92897 |
| CBG_ ITQDAQLK_ 458.8_702.4 | 4 | 930.9931102 |
| ITIH3_ALDLSLK_ 380.2_185.1 | 5 | 869.6315408 |
| ENPP2_WWGGQPLWITATK_ 772.4_929.5 | 6 | 768.9182114 |
| CBG_ ITQDAQLK_ 458.8_803.4 | 7 | 767.8940452 |
| PSG1_LSETNR_ 360.2_519.3 | 8 | 714.6160065 |
| CAA60698_ LEPLYSASGPGLRPLVIK_ 637.4_834.5 | 9 | 713.4086612 |
| INHBC_LDFHFSSDR_ 375.2_611.3 | 11 | 681.2442909 |
| CBG_QINSYVK_426.2_610.3 | 12 | 674.3363415 |
| LBP_ GLQYAAQEGLLALQSELLR_1037.1_858.5 | 13 | 603.197751 |
| A1BG_LLELTGPK_ 435.8_644.4 | 14 | 600.9902818 |
| CO6_ DLHLSDVFLK_ 396.2_366.2 | 15 | 598.8214342 |
| VCAM1_TQIDSPLSGK_ 523.3_816.5 | 16 | 597.4038769 |
| LRP1_NAVVQGLEQPHGLVVHPLR_ 688.4_285.2 | 17 | 532.0500081 |
| CBG_QINSYVK_426.2_496.3 | 18 | 516.5575201 |
| CO6_ ENPAVIDFELAPIVDLVR_ 670.7_811.5 | 19 | 501.4669261 |
| ADA12_FGFGGSTDSGPIR_ 649.3_745.4 | 20 | 473.5510333 |
| CO6_ DLHLSDVFLK_ 396.2_260.2 | 21 | 470.5473702 |
| ENPP2_TYLHTYESEI_ 628.3_908.4 | 22 | 444.7580726 |
| A1BG_LLELTGPK_ 435.8_227.2 | 23 | 444.696292 |
| FRIH_ QNYHQDSEAAINR_515.9_544.3 | 24 | 439.2648872 |
| ENPP2_TEFLSNYLTNVDDITLVPGTLGR_ 846.8_600.3 | 25 | 389.3769604 |
| CBG_ WSAGLTSSQVDLYIPK_ 883.0_515.3 | 26 | 374.0749768 |
| C1QC_ FQSVFTVTR_ 542.8_623.4 | 27 | 370.6957977 |
| GELS_ DPDQTDGLGLSYLSSHIANVER_ 796.4_456.2 | 28 | 353.1176588 |
| A1BG_ATWSGAVLAGR_ 544.8_643.4 | 29 | 337.4580124 |
| APOA1_AKPALEDLR_ 506.8_813.5 | 30 | 333.5742035 |
| ENPP2_TYLHTYESEI_ 628.3_515.3 | 31 | 322.6339162 |
| PEPD_AVYEAVLR_ 460.8_750.4 | 32 | 321.4377907 |
| TIMP1_ GFQALGDAADIR_617.3_717.4 | 33 | 310.0997949 |
| ADA12_LIEIANHVDK_ 384.6_498.3 | 34 | 305.8803542 |
| PGRP2_WGAAPYR_ 410.7_577.3 | 35 | 303.5539874 |
| PSG9_LFIPQITR_ 494.3_614.4 | 36 | 300.7877317 |
| HABP2_FLNWIK_ 410.7_560.3 | 37 | 298.3363186 |
| CBG_ WSAGLTSSQVDLYIPK_ 883.0_357.2 | 38 | 297.2474385 |
| PSG2_IHPSYTNYR_ 384.2_452.2 | 39 | 292.6203405 |
| PSG5_LSIPQITTK_ 500.8_800.5 | 40 | 290.2023364 |
| HABP2_FLNWIK_ 410.7_561.3 | 41 | 289.5092933 |
| CO6_SEYGAALAWEK_612.8_788.4 | 42 | 287.7634114 |
| ADA12_LIEIANHVDK_ 384.6_683.4 | 43 | 286.5047372 |
| EGLN_TQILEWAAER_ 608.8_632.3 | 44 | 284.5138846 |
| CO6_ ENPAVIDFELAPIVDLVR_ 670.7_601.4 | 45 | 273.5146272 |
| FA7_FSLVSGWGQLLDR_ 493.3_447.3 | 46 | 271.7850098 |
| ITIH3_ALDLSLK_ 380.2_575.3 | 47 | 269.9425709 |
| ADA12_FGFGGSTDSGPIR_ 649.3_946.5 | 48 | 264.5698225 |
| FETUA_AALAAFNAQNNGSNFQLEEISR_ 789.1_746.4 | 49 | 247.4728828 |
| FBLN1_AITPPHPASQANIIFDITEGNLR 825.8_459.3 | 50 | 246.572102 |
| TSP1_FVFGTTPEDILR_ 697.9_843.5 | 51 | 245.0459575 |
| VCAM1_NTVISVNPSTK_ 580.3_732.4 | 52 | 240.576729 |
| ENPP2_TEFLSNYLTNVDDITLVPGTLGR_ 846.8_699.4 | 53 | 240.1949512 |
| FBLN3_ELPQSIVYK_ 538.8_409.2 | 55 | 233.6825304 |
| ACTB_VAPEEHPVLLTEAPLNPK_ 652.0_892.5 | 56 | 226.9772749 |
| TSP1_FVFGTTPEDILR_ 697.9_742.4 | 57 | 224.4627393 |
| PLMN_EAQLPVIENK_ 570.8_699.4 | 58 | 221.4663735 |
| C1S_ IIGGSDADIK_ 494.8_260.2 | 59 | 218.069476 |
| ILIA_ANDQYLTAAALHNLDEAVK_ 686.4_317.2 | 60 | 216.5531949 |
| PGRP2_WGAAPYR_ 410.7_634.3 | 61 | 211.0918302 |
| PSG5_LSIPQITTK_ 500.8_687.4 | 62 | 208.7871461 |
| PSG6_ SNPVTLNVLYGPDLPR_585.7_654.4 | 63 | 207.9294937 |
| PRG2_WNFAYWAAHQPWSR_ 607.3_545.3 | 64 | 202.9494031 |
| CXCL2_CQCLQTLQGIHLK_13p8RT_533.6_567.4 | 65 | 202.9051326 |
| CXCL2_CQCLQTLQGIHLK_13p48RT_533.6_695.4 | 66 | 202.6561548 |
| G6PE_ LLDFEFSSGR_ 585.8_553.3 | 67 | 201.004611 |
| GELS_ TASDFITK_ 441.7_710.4 | 68 | 200.2704809 |
| B2MG_VEHSDLSFSK_ 383.5_468.2 | 69 | 199.880987 |
| CO8B_ IPGIFELGISSQSDR_ 809.9_849.4 | 70 | 198.7563875 |
| PSG8_LQLSETNR_ 480.8_606.3 | 71 | 197.6739966 |
| LBP_ GLQYAAQEGLLALQSELLR_1037.1_929.5 | 72 | 197.4094851 |
| AFAM_LPNNVLQEK_ 527.8_844.5 | 73 | 196.8123228 |
| MAGE | 74 | 196.2410502 |
| PSG2_IHPSYTNYR_ 384.2_338.2 | 75 | 196.2410458 |
| PSG9_LFIPQITR_ 494.3_727.4 | 76 | 193.5329266 |
| TFR1_YNSQLLSFVR_ 613.8_734.5 | 77 | 193.2711994 |
| C1R_QRPPDLDTSSNAVDLLFFTDESGDSR_961.5_866.3 | 78 | 193.0625419 |
| PGH1_ILPSVPK_ 377.2_264.2 | 79 | 190.0504508 |
| FA7_ SEPRPGVLLR_375.2_454.3 | 80 | 188.2718422 |
| FA7_TLAFVR_ 353.7_274.2 | 81 | 187.6895294 |
| PGRP2_DGSPDVTTADIGANTPDATK_ 973.5_844.4 | 82 | 185.6017519 |
| C1S_ IIGGSDADIK_ 494.8_762.4 | 83 | 184.5985543 |
| PEPD_VPLALFALNR_ 557.3_620.4 | 84 | 184.3962957 |
| C1S_ EDTPNSVWEPAK_ 686.8_630.3 | 85 | 179.2043504 |
| CHL1_ TAVTANLDIR_ 537.3_802.4 | 86 | 174.9866792 |
| CHL1_ VIAVNEVGR_ 478.8_744.4 | 88 | 172.2053147 |
| SDF1_ ILNTPNCALQIVAR_ 791.9_341.2 | 89 | 171.4604557 |
| PAI1_EVPLSALTNILSAQLISHWK_ 740.8_996.6 | 90 | 169.5635635 |
| AMBP_AFIQLWAFDAVK_ 704.9_650.4 | 91 | 169.2124477 |
| G6PE_ LLDFEFSSGR_ 585.8_944.4 | 92 | 168.2398598 |
| THBG_ SILFLGK_389.2_577.4 | 93 | 166.3110206 |
| PRDX2_ GLFIIDGK_431.8_545.3 | 94 | 164.3125132 |
| ENPP2_WWGGQPLWITATK_ 772.4_373.2 | 95 | 163.4011689 |
| VGFR3_ SGVDLADSNQK_567.3_662.3 | 96 | 162.8822352 |
| C1S_ EDTPNSVWEPAK_ 686.8_315.2 | 97 | 161.6140915 |
| AFAM_DADPDTFFAK_ 563.8_302.1 | 98 | 159.5917449 |
| CBG_SETEIHQGFQHLHQLFAK_717.4_447.2 | 99 | 156.1357404 |
| C1S_ LLEVPEGR_ 456.8_686.4 | 100 | 155.1763293 |
| PTGDS_ GPGEDFR_389.2_623.3 | 101 | 154.9205208 |
| ITIH2_IYLQPGR_ 423.7_329.2 | 102 | 154.6552717 |
| FA7_TLAFVR_ 353.7_492.3 | 103 | 152.5009422 |
| FA7_FSLVSGWGQLLDR_ 493.3_403.2 | 104 | 151.9971204 |
| SAMP_ VGEYSLYIGR_ 578.8_871.5 | 105 | 151.4738449 |
| APOH_EHSSLAFWK_ 552.8_267.1 | 106 | 151.0052645 |
| PGRP2_AGLLRPDYALLGHR_ 518.0_595.4 | 107 | 150.4149907 |
| C1QC_ FNAVLTNPQGDYDTSTGK_ 964.5_333.2 | 108 | 149.2592827 |
| PGRP2_AGLLRPDYALLGHR_ 518.0_369.2 | 109 | 147.3609354 |
| PGRP2_TFTLLDPK_ 467.8_686.4 | 111 | 145.2145223 |
| CO5_ TDAPDLPEENQAR_ 728.3_843.4 | 112 | 144.5213118 |
| THRB_ELLESYIDGR_ 597.8_839.4 | 113 | 143.924639 |
| GELS_ DPDQTDGLGLSYLSSHIANVER_ 796.4_328.1 | 114 | 142.8936101 |
| TRFL_YYGYTGAFR_ 549.3_450.3 | 115 | 142.8651352 |
| HEMO_ QGHNSVFLIK_381.6_260.2 | 116 | 142.703845 |
| C1S_GDSGGAFAVQDPNDK_739.3_716.3 | 117 | 142.2799122 |
| B1A4H9_AHQLAIDTYQEFR_ 531.3_450.3 | 118 | 138.196407 |
| C1S_SSNNPHSPIVEEFQVPYNK_729.4_261.2 | 119 | 136.7868935 |
| HYOU1_LPATEKPVLLSK_ 432.6_347.2 | 120 | 136.1146437 |
| FETA_ GYQELLEK_490.3_502.3 | 121 | 135.2890322 |
| LRP1_ SERPPIFEIR_415.2_288.2 | 122 | 134.6569527 |
| CO6_SEYGAALAWEK_612.8_845.5 | 124 | 132.8634704 |
| CERU_ TTIEKPVWLGFLGPIIK_ 638.0_844.5 | 125 | 132.1047746 |
| IBP1_AQETSGEEISK_ 589.8_850.4 | 126 | 130.934446 |
| SHBG_ VVLSSGSGPGLDLPLVLGLPLQLK_ 791.5_768.5 | 127 | 128.2052287 |
| CBG_SETEIHQGFQHLHQLFAK_717.4_318.1 | 128 | 127.9873837 |
| A1AT_LSITGTYDLK_ 555.8_696.4 | 129 | 127.658818 |
| PGRP2_DGSPDVTTADIGANTPDATK_ 973.5_531.3 | 130 | 126.5775806 |
| C1QB_ LEQGENVFLQATDK_ 796.4_675.4 | 131 | 126.1762726 |
| EGLN_ GPITSAAELNDPQSILLR_632.4_826.5 | 132 | 125.7658253 |
| IL12B_YENYTSSFFIR_ 713.8_293.1 | 133 | 125.0476631 |
| B2MG_VEHSDLSFSK_ 383.5_234.1 | 134 | 124.9154706 |
| PGH1_AEHPTWGDEQLFQTTR_ 639.3_765.4 | 135 | 124.8913193 |
| INHBE_ALVLELAK_ 428.8_331.2 | 136 | 124.0109276 |
| HYOU1_LPATEKPVLLSK_ 432.6_460.3 | 137 | 123.1900369 |
| CXCL2_CQCLQTLQGIHLK_13p48RT_533.6_567.4 | 138 | 122.8800873 |
| PZP_AVGYLITGYQR_ 620.8_523.3 | 139 | 122.4733204 |
| AFAM_IAPQLSTEELVSLGEK_ 857.5_333.2 | 140 | 122.4707849 |
| ICAM1_VELAPLPSWQPVGK_ 760.9_400.3 | 141 | 121.5494206 |
| CHL1_ VIAVNEVGR_ 478.8_284.2 | 142 | 119.0877137 |
| APOB_ITENDIQIALDDAK_ 779.9_632.3 | 143 | 118.0222045 |
| SAMP_ AYSDLSR_ 406.2_577.3 | 144 | 116.409429 |
| AMBP_AFIQLWAFDAVK_ 704.9_836.4 | 145 | 116.1900846 |
| EGLN_ GPITSAAELNDPQSILLR_632.4_601.4 | 146 | 115.8438804 |
| LRP1_NAVVQGLEQPHGLVVHPLR_ 688.4_890.6 | 147 | 114.539707 |
| SHBG_ VVLSSGSGPGLDLPLVLGLPLQLK_ 791.5_598.4 | 148 | 113.1931134 |
| IBP1_AQETSGEEISK_ 589.8_979.5 | 149 | 112.9902709 |
| PSG6_ SNPVTLNVLYGPDLPR_585.7_817.4 | 150 | 112.7910917 |
| APOC3_DYWSTVK 449.7_347.2 | 151 | 112.544736 |
| C1R_WILTAAHTLYPK_ 471.9_621.4 | 152 | 112.2199708 |
| ANGT_ADSQAQLLLSTVVGVFTAPGLHLK_ 822.5_983.6 | 153 | 111.9634671 |
| PSG9_DVLLLVHNLPQNLPGYFWYK_ 810.4_328.2 | 154 | 111.5743214 |
| A1AT_AVLTIDEK_ 444.8_605.3 | 155 | 111.216651 |
| PSGx_ILILPSVTR_ 506.3_785.5 | 156 | 110.8482935 |
| THRB_ELLESYIDGR_ 597.8_710.4 | 157 | 110.7496103 |
| SHBG_ ALALPPLGLAPLLNLWAKPQGR_ 770.5_256.2 | 158 | 110.5091269 |
| PZP_ QTLSWTVTPK_580.8_545.3 | 159 | 110.4675104 |
| SHBG_ ALALPPLGLAPLLNLWAKPQGR_ 770.5_457.3 | 160 | 110.089808 |
| PSG4_TLFIFGVTK_ 513.3_811.5 | 161 | 109.9039967 |
| PLMN_YEFLNGR_ 449.7_293.1 | 162 | 109.6880397 |
| PEPD_AVYEAVLR_ 460.8_587.4 | 163 | 109.3697285 |
| PLMN_LSSPAVITDK_ 515.8_830.5 | 164 | 108.963353 |
| FINC_ SYTITGLQPGTDYK_772.4_352.2 | 165 | 108.452612 |
| C1R_WILTAAHTLYPK_ 471.9_407.2 | 166 | 107.8348417 |
| CHL1_ TAVTANLDIR_ 537.3_288.2 | 167 | 107.7278897 |
| TENA_AVDIPGLEAATPYR_ 736.9_286.1 | 168 | 107.6166195 |
| CRP_ YEVQGEVFTKPQLWP_ 911.0_293.1 | 169 | 106.9739589 |
| APOB_ SVSLPSLDPASAK_636.4_885.5 | 170 | 106.5901668 |
| PRDX2_ SVDEALR_395.2_488.3 | 171 | 106.2325046 |
| CO8A_ YHFEALADTGISSEFYDNANDLLSK_ 940.8_301.1 | 172 | 105.8963287 |
| C1QC_ FQSVFTVTR_ 542.8_722.4 | 173 | 105.4338742 |
| PSGx_ILILPSVTR_ 506.3_559.3 | 174 | 105.1942655 |
| VCAM1_TQIDSPLSGK_ 523.3_703.4 | 175 | 105.0091767 |
| VCAM1_NTVISVNPSTK_ 580.3_845.5 | 176 | 104.8754444 |
| CSH_ ISLLLIESWLEPVR_ 834.5_500.3 | 177 | 104.6158295 |
| HGFA_EALVPLVADHK_ 397.9_439.8 | 178 | 104.3383142 |
| CGB1_CRPINATLAVEK_457.9_660.4 | 179 | 104.3378072 |
| APOB_IEGNLIFDPNNYLPK_ 874.0_414.2 | 180 | 103.9849346 |
| C1QB_ LEQGENVFLQATDK_ 796.4_822.4 | 181 | 103.9153207 |
| APOH_EHSSLAFWK_ 552.8_838.4 | 182 | 103.9052103 |
| CO5_ LQGTLPVEAR_ 542.3_842.5 | 183 | 103.1061869 |
| SHBG_ IALGGLLFPASNLR_ 481.3_412.3 | 184 | 102.2490294 |
| B2MG_VNHVTLSQPK_ 374.9_459.3 | 185 | 102.1204362 |
| APOA2_ SPELQAEAK_486.8_659.4 | 186 | 101.9166647 |
| FLNA_TGVAVNKPAEFTVDAK_ 549.6_258.1 | 187 | 101.5207852 |
| PLMN_YEFLNGR_ 449.7_606.3 | 188 | 101.2531011 |

**Table 63. Random Forest Importance Values Using Peak Area Ratios**

| Variable | Rank | Importance |
|---|---|---|
| HABP2_FLNWIK_ 410.7_561.3 | 1 | 3501.905733 |
| ADA12_FGFGGSTDSGPIR_ 649.3_946.5 | 2 | 3136.589992 |
| A1BG_LLELTGPK_ 435.8_227.2 | 3 | 2387.891934 |
| B2MG_VEHSDLSFSK_ 383.5_234.1 | 4 | 1431.31771 |
| ADA12_FGFGGSTDSGPIR_ 649.3_745.4 | 5 | 1400.917331 |
| B2MG_VEHSDLSFSK_ 383.5_468.2 | 6 | 1374.453629 |
| APOB_IEGNLIFDPNNYLPK_ 874.0_414.2 | 7 | 1357.812445 |
| PSG9_DVLLLVHNLPQNLPGYFWYK_ 810.4_960.5 | 8 | 1291.934596 |
| A1BG_LLELTGPK_ 435.8_644.4 | 9 | 1138.712941 |
| ITIH3_ALDLSLK_ 380.2_185.1 | 10 | 1137.127027 |
| ENPP2_TYLHTYESEI_ 628.3_908.4 | 11 | 1041.036693 |
| IL12B_YENYTSSFFIR_ 713.8_293.1 | 12 | 970.1662913 |
| ENPP2_WWGGQPLWITATK_ 772.4_373.2 | 13 | 953.0631062 |
| ENPP2_TYLHTYESEI_ 628.3_515.3 | 14 | 927.3512901 |
| PSG9_LFIPQITR_ 494.3_614.4 | 15 | 813.9965357 |
| MAGE | 16 | 742.2425022 |
| ENPP2_WWGGQPLWITATK_ 772.4_929.5 | 17 | 731.5206413 |
| CERU_ TTIEKPVWLGFLGPIIK_ 638.0_640.4 | 18 | 724.7745695 |
| ITIH3_ALDLSLK_ 380.2_575.3 | 19 | 710.1982467 |
| PSG2_IHPSYTNYR_ 384.2_452.2 | 20 | 697.4750893 |
| ITIH1_LWAYLTIQELLAK_ 781.5_371.2 | 21 | 644.7416886 |
| INHBE_ALVLELAK_ 428.8_331.2 | 22 | 643.008853 |
| HGFA_LHKPGVYTR_ 357.5_692.4 | 23 | 630.8698445 |
| TRFL_YYGYTGAFR_ 549.3_450.3 | 24 | 609.5866675 |
| THBG_AVLHIGEK_ 289.5_348.7 | 25 | 573.9320948 |
| GELS_ TASDFITK_ 441.7_710.4 | 26 | 564.3288862 |
| PSG9_LFIPQITR_ 494.3_727.4 | 27 | 564.1749327 |
| VGFR3_ SGVDLADSNQK_567.3_662.3 | 28 | 563.8087791 |
| INHA_TTSDGGYSFK_ 531.7_860.4 | 29 | 554.210214 |
| PSG9_DVLLLVHNLPQNLPGYFWYK_ 810.4_328.2 | 30 | 545.1743627 |
| HYOU1_LPATEKPVLLSK_ 432.6_347.2 | 31 | 541.6208032 |
| CO8G_ VQEAHLTEDQIFYFPK_ 655.7_701.4 | 32 | 541.3193428 |
| BMI | 33 | 540.5028818 |
| HGFA_LHKPGVYTR_ 357.5_479.3 | 34 | 536.6051948 |
| PSG2_IHPSYTNYR_ 384.2_338.2 | 35 | 536.5363489 |
| GELS_ AQPVQVAEGSEPDGFWEALGGK_ 758.0_623.4 | 36 | 536.524931 |
| PSG6_ SNPVTLNVLYGPDLPR_585.7_654.4 | 37 | 520.108646 |
| HABP2_FLNWIK_ 410.7_560.3 | 38 | 509.0707814 |
| PGH1_ILPSVPK_ 377.2_527.3 | 39 | 503.593718 |
| HYOU1_LPATEKPVLLSK_ 432.6_460.3 | 40 | 484.047422 |
| CO6_ ALNHLPLEYNSALYSR_ 621.0_696.4 | 41 | 477.8773179 |
| INHBE_ALVLELAK_ 428.8_672.4 | 42 | 459.1998276 |
| PLMN_LSSPAVITDK_ 515.8_743.4 | 43 | 452.9466414 |
| PSG9_DVLLLVHNLPQNLPGYFWYK_ 810.4_215.1 | 44 | 431.8528248 |
| BGH3_LTLLAPLNSVFK_ 658.4_875.5 | 45 | 424.2540315 |
| AFAM_LPNNVLQEK_ 527.8_730.4 | 46 | 421.4953221 |
| ITIH2_LSNENHGIAQR_ 413.5_519.8 | 47 | 413.1231437 |
| GELS_ TASDFITK_ 441.7_781.4 | 48 | 404.2679723 |
| FETUA_AHYDLR_ 387.7_566.3 | 49 | 400.4711207 |
| CERU_ TTIEKPVWLGFLGPIIK_ 638.0_844.5 | 50 | 396.2873451 |
| PSGx_ILILPSVTR_ 506.3_785.5 | 51 | 374.5672526 |
| APOB_ SVSLPSLDPASAK_636.4_885.5 | 52 | 371.1416438 |
| FLNA_TGVAVNKPAEFTVDAK_ 549.6_258.1 | 53 | 370.4175588 |
| PLMN_YEFLNGR_ 449.7_606.3 | 54 | 367.2768078 |
| PSGx_ILILPSVTR_ 506.3_559.3 | 55 | 365.7704321 |

### SEQUENCE LISTING

<110> SERA PROGNOSTICS, INC.
<120> BIOMARKERS AND METHODS FOR PREDICTING PRETERM BIRTH
<130>
<140>
   <141>
<150> 61/919,586
   <151> 2013-12-20
<150> 61/798,504
   <151> 2013-03-15
<160>
<170>
<210> 1
   <211> 13
   <212> PRT
   <213>
<400> 1
   AFTECCVVASQLR
<210> 2
   <211> 10
   <212> PRT
   <213>
<400> 2
   ELLESYIDGR
<210> 3
   <211> 11
   <212> PRT
   <213>
<400> 3
   ITLPDFTGDLR
<210> 4
   <211> 13
   <212> PRT
   <213>
<400> 4
   TDAPDLPEENQAR
<210> 5
   <211> 8
   <212> PRT
   <213>
<400> 5
   SFRPFVPR
   ITLPDFTCDLR_624.34_920.4
   ELLESYIDGR_597.8_710.3
   TDAPDLPEENQAR_728.34_613.3
   AFTECCWASQLR_770.87_574.3
   SFRPFVPR_335.86_272.2
   ITLPDFTGDLR_624.34_288.2
   SFRPFVPR_335.86_635.3
   ELLESYIDGR_597.8_839.4
   LEQGENVFLQATDK_796.4_822.4
   ETAASLLQAGYK_626.33_679.4
   VTGWGNLK_437.74_617.3
   EAQLPVIENK_570.82_699.4
   EAQLPVIENK_570.82_329.1
   FLQEQGHR_338.84_497.3
   IRPFFPQQ_516.79_661.4
   ETAASLLQAGYK_626.33_879.5
   AFTECCWASQLR_770.87_673.4
   TLLPVSKPEIR_418.26_288.2
   LSSPAVITDK_515.79_743.4
   YEVQGEVFTKPQLWP_910.96_392.2
   LQGTLPVEAR_542.31_571.3
   VRPQQLVK_484.31_609.3
   IEEIAAK_387.22_531.3
   TLLPVSKPEIR_418.26_514.3
   VQEAHLTEDQIFYFPK_655.66_701.4
   ISLLLIESWLEPVR_834.49_371.2
   ALQDQLVLVAAK_634.88_289.2
   YEFLNGR_449.72_293.1
   ITLPDFTCDLR_624.34_920.4
   TPSAAYLWVGTGASEAEK_919.45_849.4
   TATSEYQTFFNPR_781.37_386.2
   TASDFITK_441.73_781.4
   IITGLLEFEVYLEYLQNR_738.4_530.3
   IIGGSDADIK_494.77_762.4
   YTTEIIK_434.25_603.4
   EDTPNSVWEPAK_686.82_315.2
   LYYGDDEK_501.72_726.3
   VRPQQLVK_484.31_609.3
   GGEIEGFR_432.71_379.2
   DGSPDVTTADIGANTPDATK_973.45_844.4
   VQEAHLTEDQIFYFPK_655.66_391.2
   VEIDTK_352.7_476.3
   AVLTIDEK_444.76_605.3
   FSVVYAK_407.23_579.4
   YYLQGAK_421.72_516.3
   EENFYVDETTWK_786.88_259.1
   YGFYTHVFR_397.2_421.3
   HTLNQIDEVK_598.82_951.5
   AFIQLWAFDAVK_704.89_836.4
   SGFSFGFK_438.72_585.3
   GWVTDGFSSLK_598.8_854.4
   ITENDIQIALDDAK_779.9_632.3
   AFTECCWASQLR_770.87_574.3
   ELLESYIDGR_597.8_710.3
   ITLPDFTCDLR_624.34_920.4
   ELLESYIDGR_597.8_710.3
   AFTECCWASQLR_770.87_574.3
   ITLPDFTCDLR_624.34_920.4
   IRPFFPQQ_516.79_661.4
   TDAPDLPEENQAR_728.34_613.3
   ITLPDFTGDLR_624.34_288.2
   ELLESYIDGR_597.8_839.4
   SFRPFVPR_335.86_635.3
   ETAASLLQAGYK_626.33_879.5
   TLLPVSKPEIR_418.26_288.2
   ETAASLLQAGYK_626.33_679.4
   SFRPFVPR_335.86_272.2
   LQGTLPVEAR_542.31_571.3
   VEPLYELVTATDFAYSSTVR_754.38_712.4
   DPDQTDGLGLSYLSSHIANVER_796.39_328.1
   VTGWGNLK_437.74_617.3
   ALQDQLVLVAAK_634.88_289.2
   EAQLPVIENK_570.82_329.1
   VRPQQLVK_484.31_609.3
   AFTECCWASQLR_770.87_673.4
   YEFLNGR_449.72_293.1
   VGEYSLYIGR_578.8_871.5
   EAQLPVIENK_570.82_699.4
   TLLPVSKPEIR_418.26_514.3
   IEEIAAK_387.22_531.3
   LEQGENVFLQATDK_796.4_822.4
   LQGTLPVEAR_542.31_842.5
   FLQEQGHR_338.84_497.3
   ISLLLIESWLEPVR_834.49_371.2
   IITGLLEFEVYLEYLQNR_738.4_530.3
   LSSPAVITDK_515.79_743.4
   VRPQQLVK_484.31_722.4
   SLPVSDSVLSGFEQR_810.92_723.3
   VQEAHLTEDQIFYFPK_655.66_701.4
   NADYSYSVWK_616.78_333.2
   DAQYAPGYDK_564.25_813.4
   FQLPGQK_409.2 3_2 76.1
   TASDFITK_441.73_781.4
   YGLVTYATYPK_638.33_334.2
   GSFALSFPVESDVAPIAR_931.99_363.2
   TLLIANETLR_572.34_703.4
   VILGAHQEVNLEPHVQEIEVSR_832.78_860.4
   TATSEYQTFFNPR_781.37_386.2
   YEVQGEVFTKPQLWP_910.96_392.2
   DISEVVTPR_508.27_472.3
   GSFALSFPVESDVAPIAR_931.99_456.3
   YGFYTHVFR_397.2_421.3
   TLEAQLTPR_514.79_685.4
   YGFYTHVFR_397.2_659.4
   AVGYLITGYQR_620.84_737.4
   DPDQTDGLGLSYLSSHIANVER_796.39_456.2
   FNAVLTNPQGDYDTSTGK_964.46_262.1
   SPEQQETVLDGNLIIR_906.48_685.4
   ALNHLPLEYNSALYSR_620.99_538.3
   GGEIEGFR_432.71_508.3
   GIVEECCFR_585.26_900.3
   DAQYAPGYDK_564.25_315.1
   FAFNLYR_465.75_712.4
   YTTEIIK_434.25_603.4
   AVLTIDEK_444.76_605.3
   AITPPHPASQANIIFDITEGNLR_825.77_459.3
   EPGLCTWQSLR_673.83_790.4
   AVYEAVLR_460.76_587.4
   ALQDQLVLVAAK_634.88_956.6
   AWVAWR_394.71_531.3
   TNLESILSYPK_632.84_807.5
   HLSLLTTLSNR_418.91_376.2
   FTFTLHLETPKPSISSSNLNPR_829.44_787.4
   AVGYLITGYQR_620.84_523.3
   FQLPGQK_409.23_429.2
   YGLVTYATYPK_638.33_843.4
   TELRPGETLNVNFLLR_624.68_662.4
   LSSPAVITDK_515.79_830.5
   TATSEYQTFFNPR_781.37_272.2
   LPTAVVPLR_483.31_385.3
   APLTKPLK_289.86_260.2
   ELLESYIDGR_597.8_710.3
   TATSEYQTFFNPR_781.37_386.2
   ITLPDFTCDLR_624.34_920.4
   AFTECCWASQLR_770.87_574.3
   VEPLYELVTATDFAYSSTVR_754.38_712.4
   GSFALSFPVESDVAPIAR_931.99_363.2
   VGEYSLYIGR_578.8_871.5
   SFRPFVPR_335.86_635.3
   ALQDQLVLVAAK_634.88_289.2
   EDTPNSVWEPAK_686.82_315.2
   YGFYTHVFR_397.2_421.3
   DPDQTDGLGLSYLSSHIANVER_796.39_328.1
   LEQGENVFLQATDK_796.4_822.4
   LQGTLPVEAR_542.31_571.3
   SFRPFVPR_335.86_272.2
   AFTECCWASQLR_770.87_574.3
   DPDQTDGLGLSYLSSHIANVER_796.39_328.1
   ELLESYIDGR_597.8_710.3
   TATSEYQTFFNPR_781.37_386.2
   ITLPDFTCDLR_624.34_920.4
   GGEIEGFR_432.71_379.2
   ALQDQLVLVAAK_634.88_289.2
   VGEYSLYIGR_578.8_871.5
   VEPLYELVTATDFAYSSTVR_754.38_712.4
   PEQQETVLDGNLIIR_906.48_685.4
   YEFLNGR_449.72_293.1
   LEQGENVFLQATDK_796.4_822.4
   LQGTLPVEAR_542.31_571.3
   ISLLLIESWLEPVR_834.49_371.2
   TASDFITK_441.73_781.4
   AFTECCWASQLR_770.87_574.3
   ISLLLIESWLEPVR_834.49_371.2
   LPTAVVPLR_483.31_385.3
   ALQDQLVLVAAK_634.88_289.2
   ETAASLLQAGYK_626.33_679.4
   IITGLLEFEVYLEYLQNR_738. 4_530.3
   ADSQAQLLLSTVVGVFTAPGLHLK_822.46_983.6
   SLPVSDSVLSGFEQR_810.92_723.3
   SFRPFVPR_335.86_272.2
   IIGGSDADIK_494.77_260.2
   NADYSYSVWK_616.78_333.2
   GSFALSFPVESDVAPIAR_931.99_456.3
   LSSPAVITDK_515.79_743.4
   ELPEHTVK_476.76_347.2
   EAQLPVIENK_570.82_699.4
   ALQDQLVLVAAK_634.88_289.2
   AVLTIDEK_444.76_605.3
   Collection.Window.GA.in.Days
   AHYDLR_387.7_566.3
   AEAQAQYSAAVAK_654.33_908.5
   AEAQAQYSAAVAK_654.33_709.4
   ADSQAQLLLSTWGVFTAPGLHLK_822.46_983.6
   AITPPHPASQANIIFDITEGNLR_825.77_459.3
   ADSQAQLLLSTWGVFTAPGLHLK_822.46_664.4
   AYSDLSR_406.2_375.2
   DALSSVQESQVAQQAR_572.96_672.4
   ANRPFLVFIR_411.58_435.3
   DALSSVQESQVAQQAR_572.96_502.3
   ALEQDLPVNIK_620.35_570.4
   AVLTIDEK_444.76_718.4
   DFHINLFQVLPWLK
   ITLPDFTGDLR
   WWGGQPLWITATK
   TASDFITK
   AGLLRPDYALLGHR
   FLQEQGHR
   FLNWIK
   EKPAGGIPVLGSLVNTVLK
   ITGFLKPGK
   YGLVTYATYPK
   SLLQPNK
   DISEVVTPR
   VQEAHLTEDQIFYFPK
   SPELQAEAK
   TYLHTYESEI
   DSPSVWAAVPGK
   HYINLITR
   SLPVSDSVLSGFEQR
   IPGIFELGISSQSDR
   IQTHSTTYR
   DGSPDVTTADIGANTPDATK
   QLGLPGPPDVPDHAAYHPF
   FPLGSYTIQNIVAGSTYLFSTK
   AHYDLR
   SFRPFVPR
   K.LVIFDTM*LEIK.K
   K.FIEDNIEYITIIAFAQYVQEATFEEMEK.L
   K.IAPQLSTEELVSLGEK.M
   K.LKHELTDEELQSLFTNFANWDK.C
   K.LPNNVLQEK.I
   K.SDVGFLPPFPTLDPEEK.C
   K.VMNHICSK.Q
   R.ESLLNHFLYEVAR.R
   R.LCFFYNKK.S
   K.AVLDVFEEGTEASAATAVK.I
   K.EQLSLLDR.F
   K.EQLSLLDRFTEDAK.R
   K.EQLSLLDRFTEDAKR.L
   K.ITDLIKDLDSQTMM*VLVNYIFFK.A
   K.ITLLSALVETR.T
   K.RLYGSEAFATDFQDSAAAK.K
   R.EIGELYLPK.F
   R.CEGPIPDVTFELLR.E
   R.FALVR.E
   K.SPPGVCSR.D
   R.DSFHLDEQFTVPVEMMQAR.T
   K.CNLLAEK.Q
   K.EHAVEGDCDFQLLK.L
   K.HTLNQIDEVKVWPQQPSGELFEIEIDTLETTCHVLDPTPVAR.C
   K.MVSGFIPLKPTVK.M
   R.AFQPFFVELTM*PYSVIR.G
   R.AFQPFFVELTMPYSVIR.G
   R.NQGNTWLTAFVLK.T
   K.IDRFMQAVTGWK.T
   K.LDTEDKLR.A
   K.TGCSLMGASVDSTLAFNTYVHFQGK.M
   R.AAMVGMLANFLGFR.I
   K.NDNDNIFLSPLSISTAFAMTK.L
   K.SKLPGIVAEGRDDLYVSDAFHK.A
   R.EVPLNTIIFMGR.V
   R.FATTFYQHLADSKNDNDNIFLSPLSISTAFAMTK.L
   R.ITDVIPSEAINELTVLVLVNTIYFK.G
   R.RVWELSK.A
   R.VAEGTQVLELPFKGDDITM*VLILPKPEK.S
   R.VAEGTQVLELPFKGDDITMVLILPKPEK.S
   K.AGTELVNFLSYFVELGTQPATQ.-
   K.EPCVESLVSQYFQTVTDYGK.D
   K.ALVQQMEQLR.Q
   K.LGPHAGDVEGHLSFLEK.D
   K.SELTQQLNALFQDK.L
   K.SLAELGGHLDQQVEEFRR.R
   K.VKIDQTVEELRR.S
   K.VNSFFSTFK.E
   K.ATFQTPDFIVPLTDLR.I
   K.AVSM*PSFSILGSDVR.V
   K.AVSMPSFSILGSDVR.V
   K.EQHLFLPFSYK.N
   K.KIISDYHQQFR.Y
   K.QVFLYPEKDEPTYILNIK.R
   K.SPAFTDLHLR.Y
   K.TILGTMPAFEVSLQALQK.A
   K.VLADKFIIPGLK.L
   K.YSQPEDSLIPFFEITVPESQLTVSQFTLPK.S
   R.DLKVEDIPLAR.I
   R.GIISALLVPPETEEAK.Q
   R.ILGEELGFASLHDLQLLGK.L
   R.LELELRPTGEIEQYSVSATYELQR.E
   R.NIQEYLSILTDPDGK.G
   R.TFQIPGYTVPVVNVEVSPFTIEMSAFGYVFPK.A
   R.TIDQMLNSELQWPVPDIYLR.D
   K.MREWFSETFQK.V
   K.STAAMSTYTGIFTDQVLSVLKGEE.-
   R.GWVTDGFSSLK.D
   R.AATVGSLAGQPLQER.A
   R.LKSWFEPLVEDMQR.Q
   R.WVQTLSEQVQEELLSSQVTQELR.A
   K.LCGGGRWELM*R.I
   K.LPGLLK.R
   K.EHSSLAFWK.T
   R.TCPKPDDLPFSTWPLK.T
   R.VCPFAGILENGAVR.Y
   K.LFAAFFLEMAQLH.-
   K.SHLIIAQVAK.N
   K.NAIWIDCGIHAR.E
   R.EALIQFLEQVHQGIK.G
   R.LLNIQTYCAGPAYLK.G
   R.LCENIAGHLKDAQIFIQK.K
   K.AETGDKVYVHLK.N
   K.AGLQAFFQVQECNK.S
   K.DIASGLIGPLIICK.K
   K.DIFTGLIGPM*K.I
   K.DIFTGLIGPMK.I
   K.M*YYSAVDPTKDIFTGLIGPMK.I
   K.MYYSAVDPTKDIFTGLIGPM*K.I
   K.PVWLGFLGPIIK.A
   R.ADDKVYPGEQYTYMLLATEEQSPGEGDGNCVTR.I
   R.DTANLFPQTSLTLHM*WPDTEGTFNVECLTTDHYTGGMK.Q
   R.DTANLFPQTSLTLHMWPDTEGTFNVECLTTDHYTGGMK.Q
   R.FNKNNEGTYYSPNYNPQSR.S
   R.IDTINLFPATLFDAYM*VAQNPGEWM*LSCQNLNHLK.A
   R.IDTINLFPATLFDAYM*VAQNPGEWMLSCQNLNHLK.A
   R.IDTINLFPATLFDAYMVAQNPGEWM*LSCQNLNHLK.A
   R.KAEEEHLGILGPQLHADVGDKVK.I
   R.TTIEKPVWLGFLGPIIK.A
   R.FWTSFFPK.V
   K.LFDSDPITVTVPVEVSR.K
   R.ASSIIDELFQDR.F
   K.WIVTAAHCVETGVK.I
   R.FSLVSGWGQLLDR.G
   K.ETYDFDIAVLR.L
   K.VRQLEMEIGQLNVHYLR.N
   R.PAFSAIR.R
   K.WTFCDYAYNTFQVTTGGMVLK.L
   K.FQSVFTVTR.Q
   K.TLDEFTIIQNLQPQYQFR.D
   R.MDVFSQNMFCAGHPSLK.Q
   R.WILTAAHTLYPK.E
   K.FYAAGLVSWGPQCGTYGLYTR.V
   K.GFQVVVTLR.R
   R.GALISDQWVLTAAHCFR.D
   R.PICLPCTMEANLALR.R
   R.YYGGGYGSTQATFMVFQALAQYQK.D
   K.GLCVATPVQLR.V
   K.M*RPSTDTITVM*VENSHGLR.V
   K.MRPSTDTITVM*VENSHGLR.V
   K.VGLSGM*AIADVTLLSGFHALR.A
   K.VLSLAQEQVGGSPEK.L
   R.EMSGSPASGIPVK.V
   R.GCGEQTM*IYLAPTLAASR.Y
   R.GLQDEDGYR.M
   R. GQIVFMNREPK. R
   R.KKEVYM*PSSIFQDDFVIPDISEPGTWK.I
   R.LPMSVR.R
   R.LTVAAPPSGGPGFLSIER.P
   R.NFLVR.A
   R.NGESVKLHLETDSLALVALGALDTALYAAGSK.S
   R.QGSFQGGFR.S
   R.TLEIPGNSDPNMIPDGDFNSYVR.V
   R.VTASDPLDTLGSEGALSPGGVASLLR.L
   R.YLDKTEQWSTLPPETK.D
   K.ADNFLLENTLPAQSTFTLAISAYALSLGDK.T
   K.ALVEGVDQLFTDYQIK.D
   K.DGHVILQLNSIPSSDFLCVR.F
   K.DVFLEMNIPYSWR.G
   K.EFPYRIPLDLVPK.T
   K.FQNSAILTIQPK.Q
   K.VFKDVFLEMNIPYSVVR.G
   R.VFQFLEK.S
   K.DLHLSDVFLK.A
   R.TECIKPVVQEVLTITPFQR.L
   K.SSGWHFVVK.F
   R.ILPLTVCK.M
   R.ALDQYLMEFNACR.C
   K.YGFCEAADQFHVLDEVR.R
   R.AIEDYINEFSVRK.C
   R.TAGYGINILGMDPLSTPFDNEFYNGLCNR.D
   K.ALFVSEEEKK.L
   K.CLVNLIEK.V
   K.EAGIPEFYDYDVALIK.L
   K.VSEADSSNADWVTK.Q
   K.YGQTIRPICLPCTEGTTR.A
   R.DLEIEVVLFHPNYNINGK.K
   R.FLCTGGVSPYADPNTCR.G
   K.DGWSAQPTCIK.S
   K.EGWIHTVCINGR.W
   K.TDCLSLPSFENAIPMGEK.K
   R.DTSCVNPPTVQNAYIVSR.Q
   K.CTSTGWIPAPR.C
   K.IIYKENER.F
   K.IVSSAM*EPDREYHFGQAVR.F
   K.IVSSAMEPDREYHFGQAVR.F
   R.CTLKPCDYPDIK.H
   R.KGEWVALNPLR.K
   R.KGEWVALNPLRK.C
   R.RPYFPVAVGK.Y
   R.EIMENYNIALR.W
   K.DASGITCGGIYIGGCWILTAAHCLR.A
   K.VANYFDWISYHVGR.P
   R.IIFHENYNAGTYQNDIALIEMK.K
   R.YQIWTTWDWIHPDLK.R
   K.ISNLLK.F
   R.WSAGLTSSQVDLYIPK.V
   K.YEVQGEVFTKPQLWP.-
   R.HVLAAWALGAK.A
   R.AGLRYVCLAEPAER.R
   R.FTQLCVKGGGGGGNGIR.D
   R.VQLGPYQPGRPAACDLR.E
   R.GGLGSLFYLTLDVLETDCHVLR.K
   R.ELLSQGATLSGWYHLCLPEGR.A
   K.KTTDM*ILNEIKQGK.F
   K.NWRDPDQTDGLGLSYLSSHIANVER.V
   K.TPSAAYLWVGTGASEAEK.T
   R.VEKFDLVPVPTNLYGDFFTGDAYVILK.T
   R.VPFDAATLHTSTAMAAQHGMDDDGTGQK.Q
   K.FYTFLK.N
   K.GDKVWVYPPEKK.E
   K.LLQDEFPGIPSPLDAAVECHR.G
   K.SGAQATWTELPWPHEK.V
   K.SGAQATWTELPWPHEKVDGALCMEK.S
   K.VDGALCMEK.S
   R.DYFMPCPGR.G
   R.EWFWDLATGTM*K.E
   R.QGHNSVFLIK.G
   K.HQGTITVNEEGTQATTVTTVGFMPLSTQVR.F
   K.YEITTIHNLFR.K
   R.LNILNAK.F
   R.NFGYTLR.S
   R.VLKDQVNTFDNIFIAPVGISTAMGM*ISLGLK.G
   K.PLLNDSRMLLSPDQK.V
   R.VQLSPDLLATLPEPASPGR.Q
   R.DGYLFQLLR.I
   K.FLNWIK.A
   K.LKPVDGHCALESK.Y
   K.RPGVYTQVTK.F
   K.AGADTHGRLLQGNICNDAVTK.A
   K.KIIEKM*ATFEIDEK.R
   R.SFEGLGQLEVLTLDHNQLQEVK.A
   K.ELAAQTIKK.S
   K.GSLVQASEANLQAAQDFVR.G
   K.QLVHHFEIDVDIFEPQGISK.L
   K.QYYEGSEIVVAGR.I
   R.EVAFDLEIPKTAFISDFAVTADGNAFIGDIK.D
   R.GMADQDGLKPTIDKPSEDSPPLEM*LGPR.R
   R.GMADQDGLKPTIDKPSEDSPPLEMLGPR.R
   K.FDPAKLDQIESVITATSANTQLVLETLAQM*DDLQDFLSK.D
   K.KFYNQVSTPLLR.N
   K.NILFVIDVSGSM*WGVK.M
   K.NILFVIDVSGSMWGVK.M
   R.KLGSYEHR.I
   R.LSNENHGIAQR.I
   R.MATTMIQSK.V
   R.SILQM*SLDHHIVTPLTSLVIENEAGDER.M
   R.SILQMSLDHHIVTPLTSLVIENEAGDER.M
   R.TEVNVLPGAK.V
   K.NVVFVIDK.S
   K.WKETLFSVMPGLK.M
   K.YIFHNFM*ER.L
   R.FAHTVVTSR.V
   R.FKPTLSQQQK.S
   R.IHEDSDSALQLQDFYQEVANPLLTAVTFEYPSNAVEEVTQNNFR.L
   R.MNFRPGVLSSR.Q
   R.NVHSAGAAGSR.M
   R.NVHSGSTFFK.Y
   R.RLGVYELLLK.V
   K.KLELHLPK.F
   R.EIEEVLTPEMLMR.W
   K.AATGECTATVGKR.S
   K.LGQSLDCNAEVYVVPWEK.K
   K.YNSQNQSNNQFVLYR.I
   R.QVVAGLNFR.I
   K.DLLLPQPDLR.Y
   R.LHLEGNKLQVLGK.D
   R.TLDLGENQLETLPPDLLR.G
   K.GLQYAAQEGLLALQSELLR.I
   K.LAEGFPLPLLK.R
   K.SLEYLDLSFNQIAR.L
   R.LKEDAVSAAFK.G
   R.IVFENPDPSDGFVLIPDLK.W
   R.VYFFK.G
   K.WFDYLR.E
   R.LTVGAAQVPAQLLVGALR.V
   R.VSFSSPLVAISGVALR.S
   K.GIVAAFYSGPSLSDKEK.L
   R.WYVPVKDLLGIYEK.L
   K.LQSLFDSPDFSK.I
   R.ALYYDLISSPDIHGTYK.E
   R.CLLFSFLPASSINDMEKR.F
   K.FQPTLLTLPR.I
   K.GVTSVSQIFHSPDLAIR.D
   K.VIPACLPSPNYVVADR.T
   R.FVTWIEGVMR.N
   R.HSIFTPETNPR.A
   K.GKEESLDSDLYAELR.C
   K.MVLLEQLFLDHNALR.G
   R.LVSLDSGLLNSLGALTELQFHR.N
   K.ALLAYAFSLLGK.Q
   K.DLFHCVSFTLPR.I
   K.MLQITNTGFEMK.L
   R.NELIPLIYLENPRR.N
   R.SYIFIDEAHITQSLTWLSQMQK.D
   R.SDPVTLNLLHGPDLPR.I
   R.TLFLFGVTK.Y
   R.ILILPSVTR.N
   R.SDPVTLNLLPK.L
   R.VLQLEK.Q
   R.WAQGVGIPEDSIFTMADR.G
   R.LTEREWADEWKHLDHALNCIMEMVEK.T
   R.ALCGGDGAAALREPGAGLR.L
   K.ALM*DLLAGKGSQGSQAPQALDR.T
   K.MGDHLALEDYLTTDLVETWLR.N
   K.SPQELLCGASLISDR.W
   R.LAVTTHGLPCLAWASAQAK.A
   R.SEGSSVNLSPPLEQCVPDR.G
   R. SGIECQLWR. S
   R.TATSEYQTFFNPR.T
   R.VTGWGNLKETWTANVGK.G
   R.IHLM*AGR.V
   R.IHLMAGR.V
   R.PAHPALR.L
   K.ISNIIK.Q
   K.M*KYWGVASFLQK.G
   R.FSGTWYAM*AK.K
   R.LLNLDGTCADSYSFVFSR.D
   R.LLNNWDVCADMVGTFTDTEDPAKFK.M
   R.LFLGALPGEDSSTSFCLNGLWAQGQR.L
   K.DDWFMLGLR.D
   R.SCDVESNPGIFLPPGTQAEFNLR.G
   R.TWDPEGVIFYGDTNPKDDWFM*LGLR.D
   R.TWDPEGVIFYGDTNPKDDWFMLGLR.D
   R.KFCRDIQDPTQLAEMIFNLLLEEK.R
   R.NELIRQEKLEQLAR.R
   R.KNLSER.W
   R.KWYNLMIQNK.D
   K.SRLDTLAQEVALLK.E
   K.RLDVNAAGIWEPKK.G
   R.SILFLGK.V
   R.ELISDLEHRLQGSVM*ELLQGVDGVIK.R
   K.EDFTSLSLVLYSR.K
   K.ELSSFIDKGQELCADYSENTFTEYK.K
   K.ELSSFIDKGQELCADYSENTFTEYKK.K
   K.EVVSLTEACCAEGADPDCYDTR.T
   K.TAMDVFVCTYFMPAAQLPELPDVELPTNKDVCDPGNTK.V
   R.RTHLPEVFLSK.V
   R.VCSQYAAYGEK.K
   K.LIRDVWGIEGPIDAAFTR.I
   R.DVWGIEGPIDAAFTR.I
   R.ERVYFFK.G
   R.FEDGVLDPDYPR.N
   R.IYISGM*APRPSLAK.K
   K.TRFLLR.T
   K.HELTDEELQSLFTNFANWDK.C
   R.NPFVFAPTLLTVAVHFEEVAK.S
   K.ADLSGITGAR.N
   K.MEEVEAMLLPETLKR.W
   K.WEMPFDPQDTHQSR.F
   R.LYGSEAFATDFQDSAAAK.K
   K.HQFLLTGDTQGR.Y
   K.NGVAQEPVHLDSPAIK.H
   K.SLPAPWLSM*APVSWITPGLK.T
   K.VTLTCVAPLSGVDFQLRR.G
   R.C*EGPIPDVTFELLR.E
   R.C*LAPLEGAR.F
   R.CLAPLEGAR.F
   R.GVTFLLR.R
   R.LHDNQNGWSGDSAPVELILSDETLPAPEFSPEPESGR.A
   R.TPGAAANLELIFVGPQHAGNYR.C
   K.HQM*DLVATLSQLGLQELFQAPDLR.G
   R.LCQDLGPGAFR.L
   R.WFLLEQPEIQVAHFPFK.N
   K.VWPQQPSGELFEIEIDTLETTCHVLDPTPVAR.C
   R.HTFMGVVSLGSPSGEVSHPR.K
   R.Q*PNCDDPETEEAALVAIDYINQNLPWGYK.H
   R.QPNCDDPETEEAALVAIDYINQNLPWGYK.H
   R.TVVQPSVGAAAGPVVPPCPGR.I
   K.QPFVQGLALYTPVVLPR.S
   R.AAM*VGM*LANFLGFR.I
   K.LVPFATELHER.L
   R.LLPHANEVSQK.I
   R.SLAPYAQDTQEKLNHQLEGLTFQMK.K
   K.FPEVDVLTK.Y
   K.HINIDQFVR.K
   K.LLSGGNTLHLVSTTK.T
   K.Q*VFLYPEKDEPTYILNIKR.G
   K.QVFLYPEKDEPTYILNIKR.G
   K.SLHMYANR.L
   K.SVSDGIAALDLNAVANK.I
   K.SVSLPSLDPASAKIEGNLIFDPNNYLPK.E
   K.TEVIPPLIENR.Q
   K.VLVDHFGYTK.D
   R.TSSFALNLPTLPEVKFPEVDVLTK.Y
   R.GWVTDGFSSLKDYWSTVK.D
   R.GEVQAMLGQSTEELR.V
   R.LAVYQAGAR.E
   R.LGPLVEQGR.V
   K.LTLTPWVGLR.K
   K.FICPLTGLWPINTLK.C
   K.TFYEPGEEITYSCKPGYVSR.G
   K.MWSMTLGLHPWIANIDDTQYLAAK.R
   K.VFQYIDLHQDEFVQTLK.E
   R.TSIYPFLDFM*PSPQVVR.W
   R.ELMLQLSEFLCEEFR.N
   K.AEEEHLGILGPQLHADVGDKVK.I
   K.ALYLQYTDETFR.T
   K.DVDKEFYLFPTVFDENESLLLEDNIR.M
   K.HYYIGIIETTWDYASDHGEK.K
   R.EYTDASFTNRK.E
   R.HYYIAAEEIIWNYAPSGIDIFTK.E
   R.IYHSHIDAPK.D
   R.Q*KDVDKEFYLFPTVFDENESLLLEDNIR.M
   R.QKDVDKEFYLFPTVFDENESLLLEDNIR.M
   R.TYYIAAVEVEWDYSPQR.E
   R.SALVLQYLR.V
   K.EFNPLVIVGLSK.D
   R.NPDNDIRPWCFVLNR.D
   R.VVGGLVALR.G
   K.NSLLGMEGANSIFSGFLLFPDMEA.-
   K.VPGLYYFTYHASSR.G
   R.Q*THQPPAPNSLIR.F
   R.LPVANPQACENWLR.G
   K.NQGILEFYGDDIALLK.L
   K.RNDYLDIYAIGVGK.L
   R.QPYSYDFPEDVAPALGTSFSHMLGATNPTQK.T
   R.IHWESASLLR.S
   K.FACYYPR.V
   K.LHLETDSLALVALGALDTALYAAGSK.S
   K.LVNGQSHISLSK.A
   K.M*RPSTDTITVMVENSHGLR.V
   K.MRPSTDTITVMVENSHGLR.V
   K.SCGLHQLLR.G
   K.VGLSGMAIADVTLLSGFHALR.A
   K.YVLPNFEVK.I
   R.ALEILQEEDLIDEDDIPVR.S
   R.ECVGFEAVQEVPVGLVQPASATLYDYYNPER.R
   R.EELVYELNPLDHR.G
   R.STQDTVIALDALSAYWIASHTTEER.G
   R.VGDTLNLNLR.A
   R.VHYTVCIWR.N
   K.GLCVATPVQLR.V
   K.KYVLPNFEVK.I
   K.VDFTLSSERDFALLSLQVPLKDAK.S
   R.EMSGSPASGIPVK.V
   R.GCGEQTM*IYLAPTLAASR.Y
   R.NGESVKLHLETDSLALVALGALDTALYAAGSK.S
   R.IPLDLVPK.T
   R.SYFPESWLWEVHLVPR.R
   R.YGGGFYSTQDTINAIEGLTEYSLLVK.Q
   K.ENPAVIDFELAPIVDLVR.N
   K.YNPVVIDFEMQPIHEVLR.H
   R.HTSLGPLEAK.R
   K.C*QHEMDQYWGIGSLASGINLFTNSFEGPVLDHR.Y
   K.SGFSFGFK.I
   R.DTMVEDLVVLVR.G
   K.ANFDAQQFAGTWLLVAVGSACR.F
   R.AEATTLHVAPQGTAMAVSTFR.K
   R.DVVLTTTFVDDIK.A
   R.RPWNVASLIYETK.G
   K.ISVIRPSK.G
   K.VASYGVKPR.Y
   R.DFHINLFQVLPWLK.E
   R.DLLYIGK.D
   R.GDSGGPLIVHK.R
   R.LEDSVTYHCSR.G
   R.LPPTTTCQQQK.E
   K.CLHPCVISR.E
   K.CTSTGWIPAPR.C
   K. IDVHLVPDR.K
   K.IVSSAMEPDREYHFGQAVR.F
   K.SIDVACHPGYALPK.A
   K.VSVLCQENYLIQEGEEITCKDGR.W
   K.WSSPPQCEGLPCK.S
   R.EIMENYNIALR.W
   R.RPYFPVAVGK.Y
   R.WQSIPLCVEK.I
   R.YQIWTTVVDWIHPDLKR.I
   K.AVLQLNEEGVDTAGSTGVTLNLTSKPIILR.F
   R.GLASANVDFAFSLYK.H
   K.TFPGFFSPMLGEFVSETESR.G
   K.FDLVPVPTNLYGDFFTGDAYVILK.T
   K.QTQVSVLPEGGETPLFK.Q
   K.TPSAAYLWVGTGASEAEK.T
   R.AQPVQVAEGSEPDGFWEALGGK.A
   R.IEGSNKVPVDPATYGQFYGGDSYIILYNYR.H
   R.VEKFDLVPVPTNLYGDFFTGDAYVILK.T
   R.VPFDAATLHTSTAMAAQHGMDDDGTGQK.Q
   K.FLVGPDGIPIMR.W
   K.ALPQPQNVTSLLGCTH.-
   K.SLGPNSCSANGPGLYLIHGPNLYCYSDVEK.L
   R.DGWHSWPIAHQWPQGPSAVDAAFSWEEK.L
   R.GECQAEGVLFFQGDR.E
   R.GECQAEGVLFFQGDREWFWDLATGTM*K.E
   R.LEKEVGTPHGIILDSVDAAFICPGSSR.L
   R.LWWLDLK.S
   R.WKNFPSPVDAAFR.Q
   K.DQVNTFDNIFIAPVGISTAMGMISLGLK.G
   K.ANVFVQLPR.L
   R.LEALPNSLLAPLGR.L
   R.LFQGLGK.L
   R.NLIAAVAPGAFLGLK.A
   R.TFTPQPPGLER.L
   K.Q*LVHHFEIDVDIFEPQGISK.L
   K.VTFQLTYEEVLK.R
   K.VTFQLTYEEVLKR.N
   R.GIEILNQVQESLPELSNHASILIMLTDGDPTEGVTDR.S
   R.GM*ADQDGLKPTIDKPSEDSPPLEM*LGPR.R
   R.KAAISGENAGLVR.A
   K.AGELEVFNGYFVHFFAPDNLDPIPK.N
   K.FYNQVSTPLLR.N
   K.VQFELHYQEVK.W
   R.ETAVDGELWLYDVK.R
   R.IYLQPGR.L
   R.LWAYLTIEQLLEK.R
   K.ITFELVYEELLK.R
   K.LQDRGPDVLTATVSGK.L
   K.TGLLLLSDPDKVTIGLLFWDGRGEGLR.L
   K.WKETLFSVM*PGLK.M
   R.AISGGSIQIENGYFVHYFAPEGLTTM*PK.N
   R.AISGGSIQIENGYFVHYFAPEGLTTMPK.N
   R.ANTVQEATFQMELPK.K
   R.SFAAGIQALGGTNINDAMLMAVQLLDSSNQEER.L
   R.VQGNDHSATR.E
   K.ITFELVYEELLKR.R
   K.VTIGLLFWDGR.G
   R.LWAYLTIQQLLEQTVSASDADQQALR.N
   K.LFHTNFYDTVGTIQLINDHVK.K
   K.ENFLFLTPDCK.S
   K.IYPTVNCQPLGMISLMK.R
   K.KIYPTVNCQPLGMISLMK.R
   K.SLWNGDTGECTDNAYIDIQLR.I
   K.ILGPLSYSK.I
   K.EYGVVIAPDGSTVAVEPLLAGLEAGLQGR.R
   R.EGKEYGVVIAPDGSTVAVEPLLAGLEAGLQGR.R
   R.Q*NGAALTSASILAQQVWGTLVLLQR.L
   K.IAQLPLTGSMSIIFFLPLK.V
   R.SSTSPTTNVLLSPLSVATALSALSLGAEQR.T
   K.VAEYMDWILEK.T
   R.C*LLFSFLPASSINDMEKR.F
   R.C*QFFSYATQTFHK.A
   R.CLLFSFLPASSINDMEK.R
   R.LVLLNAIYLSAK.W
   R.NALFCLESAWNVAK.E
   R.NQGNTWLTAFVLK.T
   R.SNPVILNVLYGPDLPR.I
   K.IAIIGAGIGGTSAAYYLR.Q
   K.WYNLAIGSTCPWLK.K
   R.TVAACNLPIVR.G
   R.IVEGSDAEIGMSPWQVMLFR.K
   R.RQECSIPVCGQDQVTVAMTPR.S
   R.TFGSGEADCGLRPLFEK.K
   R.FSGTWYAMAK.K
   R.LLNNWDVCADMVGTFTDTEDPAK.F
   R.GYVIIKPLVWV.-
   K.VVLSSGSGPGLDLPLVLGLPLQLK.L
   R.TWDPEGVIFYGDTNPKDDWFM*LGLR.D
   R.TWDPEGVIFYGDTNPKDDWFMLGLR.D
   K.GFLLLASLR.Q
   K.AVLHIGEK.G
   K.FSISATYDLGATLLK.M
   K.KELELQIGNALFIGK.H
   K.MSSINADFAFNLYR.R
   R.LTLLAPLNSVFK.D
   R.GSPAINVAVHVFR.K
   K.MPSHLMLAR.K
   K.ELPEHTVK.L
   K.EYANQFMWEYSTNYGQAPLSLLVSYTK.S
   K.HLSLLTTLSNR.V
   K.HQPQEFPTYVEPTNDEICEAFR.K
   K.LAQKVPTADLEDVLPLAEDITNILSK.C
   K.LCDNLSTK.N
   K.LCMAALK.H
   K.SCESNSPFPVHPGTAECCTK.E
   K.SYLSMVGSCCTSASPTVCFLK.E
   K.TAMDVFVCTYFM*PAAQLPELPDVELPTNK.D
   K.VLEPTLK.S
   R.KFPSGTFEQVSQLVK.E
   R.THLPEVFLSK.V
   R.TSALSAK.S
   R.GQYCYELDEK.A
   R.M*DWLVPATCEPIQSVFFFSGDK.Y
   R.Q*PQFISR.D
   K.HFQNLGK.D
   R.RHPDLSIPELLR.I
   R.TINPAVDHCCK.T
   K.ITDLIKDLDSQTMMVLVNYIFFK.A
   R.DYNLNDILLQLGIEEAFTSK.A
   R.GTHVDLGLASANVDFAFSLYK.Q
   K.SLPAPWLSMAPVSWITPGLK.T
   K.GFPIKEDFLEQSEQLFGAKPVSLTGK.Q
   K.HQMDLVATLSQLGLQELFQAPDLR.G
   R.QLTSGPNQEQVSPLTLLK.L
   R.AQLVPLPPSTYVEFTVSGTDCVAK.E
   K.DPTFIPAPIQAK.T
   R.FM*QAVTGWK.T
   K.ANRPFLVFIR.E
   K.GDDITMVLILPKPEK.S
   R.DIPMNPMCIYR.S
   K.KLVPFATELHER.L
   K.SLAELGGHLDQQVEEFR.R
   K.ALYWVNGQVPDGVSK.V
   K.FIIPGLK.L
   K.FSVPAGIVIPSFQALTAR.F
   K.IEGNLIFDPNNYLPK.E
   K.LNDLNSVLVMPTFHVPFTDLQVPSCK.L
   K.VELEVPQLCSFILK.T
   K.VNWEEEAASGLLTSLK.D
   R.ATLYALSHAVNNYHK.T
   R.TGISPLALIK.G
   R.TLQGIPQMIGEVIR.K
   K.DALSSVQESQVAQQAR.G
   R.DGWQWFWSPSTFR.G
   K.VQAAVGTSAAPVPSDNH.-
   R.WELALGR.F
   K.SNFLNCYVSGFHPSDIEVDLLK.N
   R.GGHCVALCTR.G
   R.LVDFYVMPWNVDGYDYSWK.K
   R.YTHGHGSETLYLAPGGGDDWIYDLGIK.Y
   K.LSNNALSGLPQGVFGK.L
   K.TLNLAQNLLAQLPEELFHPLTSLQTLK.L
   R.WLNVQLSPR.Q
   K.GDSVVWYLFSAGNEADVHGIYFSGNTYLWR.G
   K.MYYSAVDPTK.D
   R.GPEEEHLGILGPVIWAEVGDTIR.V
   R.IDTINLFPATLFDAYMVAQNPGEWMLSCQNLNHLK.A
   R.SGAGTEDSACIPWAYYSTVDQVKDLYSGLIGPLIVCR.R
   K.IFFPGVSEFGK.E
   R.AILQSGSFNAPWAVTSLYEAR.N
   R.VLQGVLPALPQVVCNYR.D
   R.ISLLLIESWLEPVR.F
   R.LHEAFSPVSYQHDLALLR.L
   R.TTLSGAPCQPWASEATYR.N
   K.GLFQVVSGGMVLQLQQGDQVWVEKDPK.K
   K.VLNYVDWIKK.E
   K.SNALDIIFQTDLTGQK.K
   K.EGAIHREELVYELNPLDHR.G
   K.ITQVLHFTK.D
   K.SHALQLNNR.Q
   R.AVGSGATFSHYYYM*ILSR.G
   R.EPFLSCCQFAESLR.K
   R.GHLFLQTDQPIYNPGQR.V
   R.GLEEELQFSLGSK.I
   R.GSFEFPVGDAVSK.V
   R.LLATLCSAEVCQCAEGK.C
   R.VQQPDCREPFLSCCQFAESLRK.K
   R.YIYGKPVQGVAYVR.F
   K.ITHYNYLILSK.G
   R.ENSLYLTAFTVIGIR.K
   R.KAFDICPLVK.I
   R.VDDGVASFVLNLPSGVTVLEFNVK.T
   K.TFSEWLESVKENPAVIDFELAPIVDLVR.N
   R.IFDDFGTHYFTSGSLGGVYDLLYQFSSEELK.N
   K.ELSHLPSLYDYSAYR.R
   R.RYSAWAESVTNLPQVIK.Q
   K.YNPVVIDFEM*QPIHEVLR.H
   K.VEPLYELVTATDFAYSSTVR.Q
   R.SLM*LHYEFLQR.V
   K.YGFCEAADQFHVLDEVRR.-
   R.FLQEQGHR.A
   R.KLDGICWQVR.Q
   R.SLPVSDSVLSGFEQR.V
   R.GTVIDVTDFVNWASSINDAPVLISQK.L
   K.NPREDYLDVYVFGVGPLVNQVNINALASK.K
   R.GDSGGPLIVHKR.S
   R.HVIILMTDGLHNM*GGDPITVIDEIR.D
   R.KNPREDYLDVYVFGVGPLVNQVNINALASK.K
   K.SCDIPVFMNAR.T
   K.SPPEISHGVVAHMSDSYQYGEEVTYK.C
   K.TDCLSLPSFENAIPMGEKK.D
   K.RAQLGDLPWQVAIK.D
   K.SLECLHPGTK.F
   R.TMGYQDFADVVCYTQK.A
   R.ELLALIQLER.E
   R.VPEARPNSMVVEHPEFLK.A
   R.LFWEPMK.V
   R.DVRDYFMPCPGR.G
   K.DALENIDPATQMMILNCIYFK.G
   K.GLIKDALENIDPATQMMILNCIYFK.G
   K.QFPILLDFK.T
   R.VLKDQVNTFDNIFIAPVGISTAMGMISLGLK.G
   R.AFWLDVSHNR.L
   K.ADVQAHGEGQEFSITCLVDEEEMKK.L
   K.ILGDM*QPGDYFDLVLFGTR.V
   K.ILGDMQPGDYFDLVLFGTR.V
   K.NVVFVIDISGSMR.G
   K.TAFISDFAVTADGNAFIGDIKDK.V
   R. GHMLENHVER. L
   R.GM*ADQDGLKPTIDKPSEDSPPLEMLGPR.R
   R.LWAYLTIQELLAK.R
   R.NHM*QYEIVIK.V
   K.AHVSFKPTVAQQR.I
   K.ENIQDNISLFSLGM*GFDVDYDFLKR.L
   K.ENIQDNISLFSLGMGFDVDYDFLKR.L
   K.HLEVDVWVIEPQGLR.F
   K.LWAYLTINQLLAER.S
   R.AEDHFSVIDFNQNIR.T
   R.FLHVPDTFEGHFDGVPVISK.G
   K.ILDDLSPR.D
   K. IPKPEASFSPR.R
   K.SPEQQETVLDGNLIIR.Y
   K.YIFHNFMER.L
   R.FSSHVGGTLGQFYQEVLWGSPAASDDGRR.T
   R.GPDVLTATVSGK.L
   R.NMEQFQVSVSVAPNAK.I
   R.RLDYQEGPPGVEISCWSVEL.-
   K.IVDLVSELKK.D
   R.VGSALFLSHNLK.F
   K.IYPTVNCQPLGM*ISLM*K.R
   K.TVGSDTFYSFK.Y
   R.DIPTNSPELEETLTHTITK.L
   R.VQWAGK.K
   R.FNALQYLR.L
   K.VIPGPPALTLVPAELVR.I
   K.ITGTMPPLPLEATGLALSSLR.L
   K.EFTEAFLGCPAIHPR.C
   R.RVINLPLDSMAAPWETGDTFPDVVAIAPDVR.A
   R.GVFFSVNSWTPDSMSFIYK.A
   K.EIPDEISILLLGVAHFK.G
   K.IAQLPLTGSM*SIIFFLPLK.V
   K. TVQAVLTVPK. L
   R.ALYYDLISSPDIHGTYKELLDTVTAPQK.N
   R.DTDTGALLFIGK.I
   R.ELRPWCFTTDPNKR.W
   R.TECFITGWGETQGTFGAGLLK.E
   K.GTHCNQVEVIATLK.D
   K.AVGYLITGYQR.Q
   R.AVDQSVLLM*KPEAELSVSSVYNLLTVK.D
   R.IQHPFTVEEFVLPK.F
   R.NELIPLIYLENPR.R
   R.AFIQLWAFDAVK.G
   K.GFGGLTGQIVAALSTAK.Y
   K.YGFYTHVFR.L
   R.IVEGSDAEIGM*SPWQVMLFR.K
   K.KDPEGLFLQDNIVAEFSVDETGQMSATAK.G
   K.AQWANPFDPSKTEDSSSFLIDK.T
   K.GWVDLFVPK.F
   R.SFM*LLILER.S
   R.SFMLLILER.S
   K.EFSHLGKEDFTSLSLVLYSR.K
   K.EYANQFM*WEYSTNYGQAPLSLLVSYTK.S
   K.HQPQEFPTYVEPTNDEICEAFRK.D
   K.SYLSM*VGSCCTSASPTVCFLK.E
   K.TAM*DVFVCTYFMPAAQLPELPDVELPTNK.D
   K.VPTADLEDVLPLAEDITNILSK.C
   K.AVRPGYPK.L
   R.MDWLVPATCEPIQSVFFFSGDK.Y
   K.EIPAWVPFDPAAQITK.Q
   K.ADLSGITGAR.N
   K.EQLSLLDR.F
   K.ITLLSALVETR.T
   R.EIGELYLPK.F
   R.GTHVDLGLASANVDFAFSLYK.Q
   R.NLAVSQVVHK.A
   K.AAISGENAGLVR.A
   K.GSLVQASEANLQAAQDFVR.G
   K.TAFISDFAVTADGNAFIGDIK.D
   K.VTYDVSR.D
   R.EVAFDLEIPK.T
   R.LWAYLTIQELLAK.R
   K.FYNQVSTPLLR.N
   K.HLEVDVWVIEPQGLR.F
   K.TAGLVR.S
   R.IYLQPGR.L
   R.LSNENHGIAQR.I
   R.SLAPTAAAKR.R
   K.EVSFDVELPK.T
   K. IQENVR.N
   R.ALDLSLK.Y
   R.LIQDAVTGLTVNGQITGDK.R
   K.SSFVAPLEK.S
   K.TVQAVLTVPK.L
   R.ALYYDLISSPDIHGTYK.E
   R.DTDTGALLFIGK.I
   K.ELLDTVTAPQK.N
   K.FQLPGQK.L
   R.DLYHYITSYVVDGEIIIYGPAYSGR.E
   K.LFIPQITPK.H
   NSATGEESSTSLTIR
   K.FQQSGQNLFIPQITTK.H
   IHPSYTNYR
   FQLSETNR
   VSAPSGTGHLPGLNPL
   EDAGSYTLHIVK
   R.TLFIFGVTK.Y
   NYTYIWWLNGQSLPVSPR
   GVTGYFTFNLYLK
   SNPVTLNVLYGPDLPR
   DVLLLVHNLPQNLTGHIWYK
   YGPAYSGR
   LQLSETNR
   K.LFIPQITR.N
   K.LPIPYITINNLNPR.E
   R.SNPVILNVLYGPDLPR.I
   DVLLLVHNLPQNLPGYFWYK
   SENYTYIWWLNGQSLPVSPGVK
   ILILPSVTR
   K.AQWANPFDPSK.T
   K.AVLHIGEK.G
   K.FLNDVK.T
   K.FSISATYDLGATLLK.M
   K.GWVDLFVPK.F
   K.NALALFVLPK.E
   R.SILFLGK.V
   R.DFNQFSSGEK.N
   K.GYQELLEK.C
   K.GEEELQK.Y
   K.FIYEIAR.R
   R.HPFLYAPTILLWAAR.Y
   R.TFQAITVTK.L
   K.LTTLER.G
   R.HPQLAVSVILR.V
   K.LGEYYLQNAFLVAYTK.K
   R.ILPSVPK.D
   K.AEHPTWGDEQLFQTTR.L
   R.LILIGETIK.I
   R.LQPFNEYR.K
   ELIEELVNITQNQK_557.6_517.3
   ITLPDFTGDLR_624.3_288.2
   FLNWIK_410.7_560.3
   ITLPDFTGDLR_624.3_920.5
   SFRPFVPR_335.9_635.3
   LIQDAVTGLTVNGQITGDK_972.0_798.4
   FSVVYAK_407.2_579.4
   ITGFLKPGK_320.9_429.3
   AHYDLR_387.7_288.2
   FSVVYAK_407.2_381.2
   SFRPFVPR_335.9_272.2
   DVLLLVHNLPQNLPGYFWYK_810.4_967.5
   ELIEELVNITQNQK_557.6_618.3
   QALEEFQK_496.8_680.3
   DAGLSWGSAR_510.3_390.2
   AHYDLR_387.7_566.3
   VFQFLEK_455.8_811.4
   ITGFLKPGK_320.9_301.2
   VFQFLEK_455.8_276.2
   SLLQPNK_400.2_599.4
   VQTAHFK_277.5_431.2
   SDLEVAHYK_531.3_617.3
   SLLQPNK_400.2_358.2
   TLLPVSKPEIR_418.3_288.2
   ALNHLPLEYNSALYSR_621.0_538.3
   DISEVVTPR_508.3_787.4
   VSEADSSNADWVTK_754.9_533.3
   LSSPAVITDK_515.8_743.4
   VQEAHLTEDQIFYFPK_655.7_701.4
   DVLLLVHNLPQNLPGYFWYK_810.4_594.3
   ALVLELAK_428.8_672.4
   FLNWIK_410.7_561.3
   LSSPAVITDK_515.8_830.5
   LPNNVLQEK_527.8_844.5
   VSEADSSNADWVTK_754. 9_347.2
   HTLNQIDEVK_598.8_951.5
   TTSDGGYSFK_531.7_860.4
   YENYTSSFFIR_713.8_756.4
   HTLNQIDEVK_598.8_958.5
   DISEVVTPR_508.3_472.3
   LIQDAVTGLTVNGQITGDK_972.0_640.4
   EAQLPVIENK_570.8_699.4
   SLPVSDSVLSGFEQR_810.9_836.4
   AVLHIGEK_289.5_348.7
   GLQYAAQEGLLALQSELLR_1037.1_929.5
   FLQEQGHR_338.8_497.3
   LPNNVLQEK_527.8_730.4
   AVLHIGEK_289.5_292.2
   QLYGDTGVLGR_589.8_501.3
   WWGGQPLWITATK_772.4_929.5
   NADYSYSVWK_616.8_769.4
   GLQYAAQEGLLALQSELLR_1037.1_858.5
   SLPVSDSVLSGFEQR_810.9_723.3
   IEEIAAK_387.2_531.3
   TYLHTYESEI_628.3_908.4
   WWGGQPLWITATK_772.4_373.2
   FQLSETNR_497.8_605.3
   NIQSVNVK_451.3_674.4
   TGVAVNKPAEFTVDAK_549.6_258.1
   LQGTLPVEAR_542.3_571.3
   SGFSFGFK_438.7_732.4
   HELTDEELQSLFTNFANWDK_817.1_906.5
   VQTAHFK_277.5_502.3
   YENYTSSFFIR_713.8_293.1
   AFTECCVVASQLR_770.9_574.3
   EAQLPVIENK_570.8_329.2
   QALEEFQK_496.8_551.3
   DAQYAPGYDK_564.3_813.4
   TEFLSNYLTNVDDITLVPGTLGR_846.8_600.3
   IAIDLFK_410.3_635.4
   TASDFITK_441.7_781.4
   YEFLNGR_449.7_606.3
   TVQAVLTVPK_528.3_428.3
   LIENGYFHPVK_439.6_627.4
   DALSSVQESQVAQQAR_573.0_672.4
   TVQAVLTVPK_528.3_855.5
   ALQDQLVLVAAK_634.9_289.2
   TYLHTYESEI_628.3_515.3
   SDLEVAHYK_531.3_746.4
   FLPCENK_454.2_550.2
   HPWIVHWDQLPQYQLNR_744.0_1047.0
   AFTECCVVASQLR_770.9_673.4
   YGLVTYATYPK_638.3_843.4
   TLEAQLTPR_514.8_685.4
   DAQYAPGYDK_564.3_315.1
   QGHNSVFLIK_381.6_260.2
   HELTDEELQSLFTNFANVVDK_817.1_854.4
   TLEAQLTPR_514.8_814.4
   IEEIAAK_387.2_660.4
   HFQNLGK_422.2_527.2
   IAPQLSTEELVSLGEK_857.5_333.2
   DALSSVQESQVAQQAR_573.0_502.3
   ALNHLPLEYNSALYSR_621.0_696.4
   IAIDLFK_410.3_706.4
   FLQEQGHR_338.8_369.2
   ALQDQLVLVAAK_634.9_956.6
   IEGNLIFDPNNYLPK_874.0_414.2
   YEFLNGR_449.7_293.1
   TASDFITK_441.7_710.4
   DADPDTFFAK_563.8_825.4
   TLLPVSKPEIR_418.3_514.3
   NADYSYSVWK_616.8_333.2
   YGLVTYATYPK_638.3_334.2
   VNHVTLSQPK_374.9_459.3
   HYGGLTGLNK_530.3_759.4
   DFHINLFQVLPWLK_885.5_400.2
   NCSFSIIYPVVIK_770.4_555.4
   HPWIVHWDQLPQYQLNR_744.0_918.5
   AQPVQVAEGSEPDGFWEALGGK_758.0_574.3
   ALDLSLK_380.2_185.1
   ALDLSLK_380.2_575.3
   LDFHFSSDR_375.2_611.3
   TLNAYDHR_330.5_312.2
   EVFSKPISWEELLQ_852.9_260.2
   IAPQLSTEELVSLGEK_857.5_533.3
   LIENGYFHPVK_439.6_343.2
   NFPSPVDAAFR_610.8_775.4
   QLYGDTGVLGR_589.8_345.2
   LYYGDDEK_501.7_563.2
   FQLSETNR_497.8_476.3
   TGVAVNKPAEFTVDAK_549.6_977.5
   IPGIFELGISSQSDR_809.9_679.3
   TLFIFGVTK_513.3_215.1
   YYGYTGAFR_549.3_450.3
   QVFAVQR_424.2_473.3
   AQPVQVAEGSEPDGFWEALGGK_758.0_623.4
   DFNQFSSGEK_386.8_189.1
   SVSLPSLDPASAK_636.4_473.3
   GNGLTWAEK_488.3_634.3
   LYYGDDEK_501.7_726.3
   NFPSPVDAAFR_610.8_959.5
   FAFNLYR_465.8_565.3
   SGFSFGFK_438.7_585.3
   DFHINLFQVLPWLK_885.5_543.3
   LQGTLPVEAR_542.3_842.5
   GAVHVVVAETDYQSFAVLYLER_822.8_863.5
   TSESTGSLPSPFLR_739.9_716.4
   YISPDQLADLYK_713.4_277.2
   ESDTSYVSLK_564.8_347.2
   ILDDLSPR_464.8_587.3
   VQEAHLTEDQIFYFPK_655.7_391.2
   SGVDLADSNQK_567.3_662.3
   DTDTGALLFIGK_625.8_217.1
   HFQNLGK_422.2_285.1
   NNQLVAGYLQGPNVNLEEK_700.7_999.5
   IPGIFELGISSQSDR_809.9_849.4
   ESDTSYVSLK_564.8_696.4
   GAVHVVVAETDYQSFAVLYLER_822.8_580.3
   DADPDTFFAK_563.8_302.1
   LDFHFSSDR_375.2_464.2
   TLFIFGVTK_513.3_811.5
   DFNQFSSGEK_386.8_333.2
   IQTHSTTYR_369.5_627.3
   HYFIAAVER_553.3_658.4
   VNHVTLSQPK_374.9_244.2
   DLHLSDVFLK_396.2_366.2
   DPTFIPAPIQAK_433.2_556.3
   AGITIPR_364.2_272.2
   IAQYYYTFK_598.8_884.4
   SGVDLADSNQK_567.3_591.3
   VEPLYELVTATDFAYSSTVR_754.4_549.3
   AGITIPR_364.2_486.3
   YEVQGEVFTKPQLWP_911.0_293.1
   APLTKPLK_289.9_357.2
   YNSQLLSFVR_613.8_508.3
   ANDQYLTAAALHNLDEAVK_686.4_301.1
   IQTHSTTYR_369.5_540.3
   IHPSYTNYR_575.8_598.3
   TEFLSNYLTNVDDITLVPGTLGR_846.8_699.4
   DPTFIPAPIQAK_433.2_461.2
   FQSVFTVTR_542.8_623.4
   LQVNTPLVGASLLR_741.0_925.6
   DEIPHNDIALLK_459.9_510.8
   HATLSLSIPR_365.6_272.2
   EDTPNSVWEPAK_686.8_315.2
   TGISPLALIK_506.8_741.5
   ILPSVPK_377.2_244.2
   HATLSLSIPR_365.6_472.3
   QGHNSVFLIK_381.6_520.4
   LPATEKPVLLSK_432.6_460.3
   APLTKPLK_289.9_398.8
   GVTGYFTFNLYLK_508.3_683.9
   TFLTVYWTPER_70 6.9_4 01.2
   GDTYPAELYITGSILR_885.0_274.1
   EDTPNSVWEPAK_686.8_630.3
   SLDFTELDVAAEK_719.4_316.2
   VELAPLPSWQPVGK_760.9_342.2
   GPGEDFR_389.2_322.2
   TDAPDLPEENQAR_728.3_843.4
   GVTGYFTFNLYLK_508.3_260.2
   FAFNLYR_465.8_712.4
   ITENDIQIALDDAK_779.9_873.5
   ILNIFGVIK_508.8_790.5
   ISQGEADINIAFYQR_575.6_684.4
   GDTYPAELYITGSILR_885.0_1332.8
   ELLESYIDGR_597.8_710.4
   FTITAGSK_412.7_576.3
   ILDGGNK_358.7_490.2
   GWVTDGFSSLK_598.8_854.4
   FSLVSGWGQLLDR_493.3_403.2
   IHPSYTNYR_575.8_813.4
   ELLESYIDGR_597.8_839.4
   SDGAKPGPR_442.7_213.6
   IAQYYYTFK_598.8_395.2
   SILFLGK_389.2_201.1
   IEVNESGTVASSSTAVIVSAR_693.0_545.3
   VSAPSGTGHLPGLNPL_506.3_300.7
   LLAPSDSPEWLSFDVTGVVR_730.1_430.3
   YYGYTGAFR_549.3_771.4
   TDAPDLPEENQAR_728.3_613.3
   IEVIITLK_464.8_815.5
   ILPSVPK_377.2_227.2
   FGFGGSTDSGPIR_649.3_745.4
   DYWSTVK_449.7_347.2
   IEGNLIFDPNNYLPK_874.0_845.5
   WILTAAHTLYPK_471.9_407.2
   WNFAYWAAHQPWSR_607.3_545.3
   SILFLGK_389.2_577.4
   FSLVSGWGQLLDR_493.3_516.3
   DTDTGALLFIGK_625.8_818.5
   SEYGAALAWEK_612.8_845.5
   LWAYLTIQELLAK_781.5_371.2
   LLEVPEGR_456.8_356.2
   ITENDIQIALDDAK_779.9_632.3
   LTTVDIVTLR_565.8_716.4
   IEVIITLK_464.8_587.4
   QLGLPGPPDVPDHAAYHPF_676.7_299.2
   TLAFVR_353.7_492.3
   NSDQEIDFK_548.3_294.2
   YHFEALADTGISSEFYDNANDLLSK_940.8_874.5
   SEPRPGVLLR_375.2_454.3
   FLPCENK_454.2_39o.2
   NCSFSIIYPVVIK_770.4_831.5
   SLDFTELDVAAEK_719.4_874.5
   ILLLGTAVESAWGDEQSAFR_721.7_909.4
   SVSLPSLDPASAK_636.4_885.5
   TGISPLALIK_506.8_654.5
   YNQLLR_403.7_288.2
   YEVQGEVFTKPQLWP_911.0_392.2
   VPGLYYFTYHASSR_554.3_720.3
   SLQNASAIESILK_687.4_589.4
   WILTAAHTLYPK_471.9_621.4
   GWVTDGFSSLK_598.8_953.5
   YGIEEHGK_311.5_599.3
   QDLGWK_373.7_503.3
   DYWSTVK_449.7_620.3
   ALVLELAK_428.8_331.2
   QLGLPGPPDVPDHAAYHPF_676.7_263.1
   SEYGAALAWEK_612.8_788.4
   TFLTVYWTPER_706.9_502.3
   FQSVFTVTR_542.8_722.4
   DPNGLPPEAQK_583.3_669.4
   ETLLQDFR_511.3_322.2
   IIEVEEEQEDPYLNDR_996.0_777.4
   ELCLDPK_437.7_359.2
   TPSAAYLWVGTGASEAEK_919.5_849.4
   NQSPVLEPVGR_598.3_866.5
   FNAVLTNPQGDYDTSTGK_964.5_333.2
   LLEVPEGR_456.8_686.4
   FFQYDTWK_567.8_840.4
   SPEAEDPLGVER_649.8_670.4
   SEPRPGVLLR_375.2_654.4
   SGAQATWTELPWPHEK_613.3_793.4
   YSHYNER_323.5_581.3
   YHFEALADTGISSEFYDNANDLLSK_940.8_301.1
   DLHLSDVFLK_396.2_260.2
   YSHYNER_323.5_418.2
   YYLQGAK_421.7_327.1
   EVPLSALTNILSAQLISHWK_740.8_996.6
   VPGLYYFTYHASSR_554.3_420.2
   AALAAFNAQNNGSNFQLEEISR_789.1_746.4
   ETLLQDFR_511.3_565.3
   IVLSLDVPIGLLQILLEQAR_735.1_503.3
   ENPAVIDFELAPIVDLVR_670.7_811.5
   LQLSETNR_480.8_355.2
   DPDQTDGLGLSYLSSHIANVER_796.4_456.2
   NVNQSLLELHK_432.2_656.3
   EIGELYLPK_531.3_633.4
   SPEQQETVLDGNLIIR_906.5_699.3
   NKPGVYTDVAYYLAWIR_677.0_545.3
   QNYHQDSEAAINR_515.9_544.3
   EKPAGGIPVLGSLVNTVLK_631.4_930.6
   VTFEYR_407.7_614.3
   DLPHITVDR_533.3_490.3
   VEHSDLSFSK_383.5_234.1
   ENPAVIDFELAPIVDLVR_670.7_601.4
   YGFYTHVFR_397.2_659.4
   ILDDLSPR_464.8_702.3
   DPNGLPPEAQK_583.3_497.2
   GSLVQASEANLQAAQDFVR_668.7_806.4
   FLYHK_354.2_447.2
   FNAVLTNPQGDYDTSTGK_964.5_262.1
   LQDAGVYR_461.2_680.3
   INPASLDK_429.2_630.4
   LEEHYELR_363.5_580.3
   VEHSDLSFSK_383.5_468.2
   TSDQIHFFFAK_447.6_659.4
   ATLSAAPSNPR_542.8_570.3
   YGFYTHVFR_397.2_421.3
   EANQSTLENFLER_775.9_678.4
   GQQPADVTGTALPR_705.9_314.2
   VELAPLPSWQPVGK_760.9_400.3
   GEVTYTTSQVSK_650.3_750.4
   SLQAFVAVAAR_566.8_487.3
   HYGGLTGLNK_530.3_301.1
   GPEDQDISISFAWDK_854.4_753.4
   YVVISQGLDKPR_458.9_400.3
   LWAYLTIQELLAK_781.5_300.2
   SGAQATWTELPWPHEK_613.3_510.3
   GTAEWLSFDVTDTVR_848.9_952.5
   FFQYDTWK_567.8_712.3
   AHQLAIDTYQEFEETYIPK_766.0_634.4
   LPATEKPVLLSK_432.6_347.2
   NIQSVNVK_451.3_546.3
   TAVTANLDIR_537.3_288.2
   WSAGLTSSQVDLYIPK_883.0_515.3
   QINSYVK_426.2_496.3
   GFQALGDAADIR_617.3_288.2
   WNFAYWAAHQPWSR_607.3_673.3
   NEIWYR_440.7_357.2
   VLEPTLK_400.3_587.3
   YYLQGAK_421.7_516.3
   ALNSIIDVYHK_424.9_774.4
   ETPEGAEAKPWYEPIYLGGVFQLEK_951.1_877.5
   LNIGYIEDLK_589.3_837.4
   NVNQSLLELHK_432.2_543.3
   ILLLGTAVESAWGDEQSAFR_721.7_910.6
   AALAAFNAQNNGSNFQLEEISR_789.1_633.3
   VLEPTLK_400.3_458.3
   VGEYSLYIGR_578.8_708.4
   DIPHWLNPTR_416.9_373.2
   NEIVFPAGILQAPFYTR_968.5_357.2
   AEHPTWGDEQLFQTTR_639.3_765.4
   VEPLYELVTATDFAYSSTVR_754.4_712.4
   DEIPHNDIALLK_459.9_260.2
   QINSYVK_426.2_610.3
   SWNEPLYHLVTEVR_581.6_614.3
   YGIEEHGK_311.5_341.2
   FGFGGSTDSGPIR_649.3_946.5
   ANDQYLTAAALHNLDEAVK_686.4_317.2
   VRPQQLVK_484.3_609.4
   IPKPEASFSPR_410.2_506.3
   SPEQQETVLDGNLIIR_906.5_685.4
   DDLYVSDAFHK_655.3_704.3
   ELPEHTVK_476.8_347.2
   FLYHK_354.2_284.2
   QRPPDLDTSSNAVDLLFFTDESGDSR_961.5_262.2
   DPDQTDGLGLSYLSSHIANVER_796.4_328.1
   NEIWYR_440.7_637.4
   LQLSETNR_480.8_672.4
   GQVPENEANVVITTLK_571.3_462.3
   FTGSQPFGQGVEHATANK_626.0_521.2
   LEPLYSASGPGLRPLVIK_637.4_260.2
   QRPPDLDTSSNAVDLLFFTDESGDSR_961.5_866.3
   LTTVDIVTLR_565.8_815.5
   TSDQIHFFFAK_447.6_512.3
   IQHPFTVEEFVLPK_562.0_861.5
   NKPGVYTDVAYYLAWIR_677.0_821.5
   TEQAAVAR_423.2_615.4
   EIGELYLPK_531.3_819.5
   LFYADHPFIFLVR_546.6_647.4
   AEHPTWGDEQLFQTTR_639.3_569.3
   TSYQVYSK_488.2_787.4
   YTTEIIK_434.2_704.4
   NVIQISNDLENLR_509.9_402.3
   AFLEVNEEGSEAAASTAVVIAGR_764.4_685.4
   SEYGAALAWEK_612.8_788.4
   VFQFLEK_455.8_811.4
   ALNHLPLEYNSALYSR_621.0_696.4
   SLDFTELDVAAEK_719.4_316.2
   VEHSDLSFSK_383.5_234.1
   LLAPSDSPEWLSFDVTGVVR_730.1_430.3
   ALNHLPLEYNSALYSR_621.0_538.3
   VLEPTLK_400.3_458.3
   LHEAFSPVSYQHDLALLR_699.4_251.2
   SEYGAALAWEK_612.8_845.5
   YGIEEHGK_311.5_599.3
   ALQDQLVLVAAK_634.9_289.2
   VLEPTLK_400.3_587.3
   VNHVTLSQPK_374.9_244.2
   IEEIAAK_387.2_660.4
   DALSSVQESQVAQQAR_573.0_502.3
   TLLPVSKPEIR_418.3_514.3
   ALQDQLVLVAAK_634.9_956.6
   TLAFVR_353.7_492.3
   SEPRPGVLLR_375.2_654.4
   VEHSDLSFSK_383.5_468.2
   DPTFIPAPIQAK_433.2_461.2
   QGHNSVFLIK_381.6_260.2
   SLDFTELDVAAEK_719.4_874.5
   ELPQSIVYK_538.8_417.7
   TYLHTYESEI_628.3_515.3
   SLQAFVAVAAR_566.8_804.5
   AQPVQVAEGSEPDGFWEALGGK_758.0_574.3
   QGHNSVFLIK_381.6_520.4
   VNHVTLSQPK_374.9_459.3
   DLHLSDVFLK_396.2_260.2
   TEQAAVAR_423.2_615.4
   GPITSAAELNDPQSILLR_632.4_826.5
   HFQNLGK_422.2_527.2
   TEQAAVAR_423.2_487.3
   AVDIPGLEAATPYR_736.9_399.2
   TLFIFGVTK_513.3_811.5
   DLHLSDVFLK_396.2_366.2
   AFTECCVVASQLR_770.9_574.3
   EVFSKPISWEELLQ_852.9_376.2
   DPTFIPAPIQAK_433.2_556.3
   FSLVSGWGQLLDR_493.3_403.2
   HYINLITR_515.3_301.1
   HFQNLGK_422.2_285.1
   VPLALFALNR_557.3_620.4
   IHPSYTNYR_575.8_813.4
   IEEIAAK_387.2_531.3
   GEVTYTTSQVSK_650.3_750.4
   DFNQFSSGEK_386.8_333.2
   VVGGLVALR_442.3_784.5
   SDGAKPGPR_442.7_459.2
   DVLLLVHNLPQNLTGHIWYK_791.8_310.2
   TLLPVSKPEIR_418.3_288.2
   NKPGVYTDVAYYLAWIR_677.0_821.5
   QVFAVQR_424.2_473.3
   NHYTESISVAK_624.8_415.2
   IAPQLSTEELVSLGEK_857.5_333.2
   IHPSYTNYR_575.8_598.3
   EVFSKPISWEELLQ_852.9_260.2
   SILFLGK_389.2_201.1
   IEVIITLK_464.8_587.4
   VVGGLVALR_442.3_685.4
   VVLSSGSGPGLDLPLVLGLPLQLK_791.5_598.4
   FGFGGSTDSGPIR_649.3_946.5
   VVLSSGSGPGLDLPLVLGLPLQLK_791.5_768.5
   YGIEEHGK_311.5_341.2
   LHEAFSPVSYQHDLALLR_699.4_380.2
   AHYDLR_387.7_566.3
   FSVVYAK_407.2_381.2
   ALALPPLGLAPLLNLWAKPQGR_770.5_256.2
   YENYTSSFFIR_713.8_293.1
   VELAPLPSWQPVGK_760.9_342.2
   SILFLGK_389.2_577.4
   ILPSVPK_377.2_227.2
   IPSNPSHR_303.2_496.3
   HYFIAAVER_553.3_301.1
   FSVVYAK_407.2_579.4
   VFQFLEK_455.8_276.2
   IAPQLSTEELVSLGEK_857.5_533.3
   ILPSVPK_377.2_244.2
   NKPGVYTDVAYYLAWIR_677.0_545.3
   WSAGLTSSQVDLYIPK_883.0_515.3
   TPSAAYLWVGTGASEAEK_919.5_849.4
   ALALPPLGLAPLLNLWAKPQGR_770.5_457.3
   QLGLPGPPDVPDHAAYHPF_676.7_299.2
   ILDDLSPR_464.8_587.3
   VELAPLPSWQPVGK_760.9_400.3
   DADPDTFFAK_563.8_825.4
   NHYTESISVAK_624.8_252.1
   SEPRPGVLLR_375.2_454.3
   LNIGYIEDLK_589.3_950.5
   ANLINNIFELAGLGK_793.9_299.2
   LTTVDIVTLR_565.8_716.4
   TQILEWAAER_608.8_761.4
   NEPEETPSIEK_636.8_573.3
   AQPVQVAEGSEPDGFWEALGGK_758.0_623.4
   LQVNTPLVGASLLR_741.0_925.6
   VPSHAVVAR_312.5_345.2
   SLQNASAIESILK_687.4_860.5
   GVTGYFTFNLYLK_508.3_260.2
   DFNQFSSGEK_386.8_189.1
   QLGLPGPPDVPDHAAYHPF_676.7_263.1
   TLEAQLTPR_514.8_814.4
   AFTECCVVASQLR_770.9_673.4
   LTTVDIVTLR_565.8_815.5
   TLNAYDHR_330.5_312.2
   LWAYLTIQELLAK_781.5_300.2
   GGLFADIASHPWQAAIFAK_667.4_375.2
   IPSNPSHR_303.2_610.3
   TDAPDLPEENQAR_728.3_843.4
   SPQAFYR_434.7_684.4
   SSNNPHSPIVEEFQVPYNK_729.4_261.2
   AHYDLR_387.7_288.2
   DGSPDVTTADIGANTPDATK_973.5_844.4
   SPQAFYR_434.7_556.3
   ALNHLPLEYNSALYSR_621.0_696.4
   VPLALFALNR_557.3_620.4
   ALNHLPLEYNSALYSR_621.0_538.3
   AVYEAVLR_460.8_587.4
   SEPRPGVLLR_375.2_654.4
   VFQFLEK_455.8_811.4
   AQPVQVAEGSEPDGFWEALGGK_758.0_574.3
   TLAFVR_353.7_492.3
   TEQAAVAR_423.2_615.4
   YGIEEHGK_311.5_599.3
   TEQAAVAR_423.2_487.3
   QINSYVK_426.2_496.3
   LIEIANHVDK_384.6_683.4
   SEYGAALAWEK_612.8_845.5
   ALQDQLVLVAAK_634.9_956.6
   VLEPTLK_400.3_587.3
   DFNQFSSGEK_386.8_333.2
   TYLHTYESEI_628.3_515.3
   LIEIANHVDK_384.6_498.3
   QINSYVK_426.2_610.3
   SLDFTELDVAAEK_719.4_316.2
   DPTFIPAPIQAK_433.2_461.2
   AVYEAVLR_460.8_750.4
   YENYTSSFFIR_713.8_756.4
   SEYGAALAWEK_612.8_788.4
   WSAGLTSSQVDLYIPK_883.0_515.3
   DALSSVQESQVAQQAR_573.0_502.3
   ALQDQLVLVAAK_634.9_289.2
   SLQAFVAVAAR_566.8_804.5
   DPTFIPAPIQAK_433.2_556.3
   FGFGGSTDSGPIR_649.3_946.5
   VLEPTLK_400.3_458.3
   SLDFTELDVAAEK_719.4_874.5
   EVFSKPISWEELLQ_852.9_376.2
   FGFGGSTDSGPIR_649.3_745.4
   TPSAAYLWVGTGASEAEK_919.5_849.4
   LHEAFSPVSYQHDLALLR_699.4_251.2
   TLEAQLTPR_514.8_685.4
   ELPQSIVYK_538.8_417.7
   GYQELLEK_490.3_631.4
   VPLALFALNR_557.3_917.6
   DLHLSDVFLK_396.2_260.2
   LTTVDIVTLR_565.8_815.5
   WSAGLTSSQVDLYIPK_883.0_357.2
   ITQDAQLK_458.8_702.4
   NIQSVNVK_451.3_674.4
   ALEQDLPVNIK_620.4_570.4
   TLNAYDHR_330.5_312.2
   LWAYLTIQELLAK_781.5_300.2
   VVGGLVALR_442.3_784.5
   AQPVQVAEGSEPDGFWEALGGK_758.0_623.4
   SVVLIPLGAVDDGEHSQNEK_703.0_798.4
   SETEIHQGFQHLHQLFAK_717.4_318.1
   LLAPSDSPEWLSFDVTGVVR_730.1_430.3
   IEVIITLK_464.8_587.4
   ITQDAQLK_458.8_803.4
   AEIEYLEK_497.8_552.3
   AVDIPGLEAATPYR_736.9_399.2
   LTTVDIVTLR_565.8_716.4
   WWGGQPLWITATK_772.4_929.5
   AVYEAVLR_460.8_587.4
   AEIEYLEK_497.8_552.3
   QINSYVK_426.2_496.3
   AVYEAVLR_460.8_750.4
   AALAAFNAQNNGSNFQLEEISR_789.1_746.4
   QINSYVK_426.2_610.3
   VPLALFALNR_557.3_620.4
   VGVISFAQK_474.8_580.3
   TGVAVNKPAEFTVDAK_549.6_258.1
   LIEIANHVDK_384.6_683.4
   LIEIANHVDK_384.6_498.3
   SGVDLADSNQK_567.3_662.3
   TSYQVYSK_488.2_787.4
   ITQDAQLK_458.8_702.4
   YYGYTGAFR_549.3_450.3
   ALEQDLPVNIK_620.4_798.5
   VFQYIDLHQDEFVQTLK_708.4_375.2
   SGVDLADSNQK_567.3_591.3
   YENYTSSFFIR_713.8_756.4
   VLSSIEQK_452.3_691.4
   YSHYNER_323.5_418.2
   ILDGGNK_358.7_603.3
   GTYLYNDCPGPGQDTDCR_697.0_666.3
   AEIEYLEK_497.8_389.2
   TLPFSR_360.7_506.3
   DEIPHNDIALLK_459.9_510.8
   ALEQDLPVNIK_620.4_570.4
   SPEAEDPLGVER_649.8_314.1
   FGFGGSTDSGPIR_649.3_745.4
   TASDFITK_441.7_781.4
   SETEIHQGFQHLHQLFAK_717.4_447.2
   SPQAFYR_434.7_556.3
   TAVTANLDIR_537.3_288.2
   VPLALFALNR_557.3_917.6
   YISPDQLADLYK_713.4_277.2
   SETEIHQGFQHLHQLFAK_717.4_318.1
   SEPRPGVLLR_375.2_654.4
   GYQELLEK_490.3_631.4
   AYSDLSR_406.2_375.2
   SVVLIPLGAVDDGEHSQNEK_703.0_798.4
   TLEAQLTPR_514.8_685.4
   WSAGLTSSQVDLYIPK_883.0_515.3
   TEQAAVAR_423.2_615.4
   AQPVQVAEGSEPDGFWEALGGK_758.0_574.3
   AGITIPR_364.2_486.3
   AEVIWTSSDHQVLSGK_586.3_300.2
   TEQAAVAR_423.2_487.3
   NHYTESISVAK_624.8_415.2
   WSAGLTSSQVDLYIPK_883.0_357.2
   YSHYNER_323.5_581.3
   DFNQFSSGEK_386.8_333.2
   NIQSVNVK_451.3_674.4
   SVVLIPLGAVDDGEHSQNEK_703.0_286.2
   TLAFVR_353.7_492.3
   AVDIPGLEAATPYR_736.9_286.1
   TEFLSNYLTNVDDITLVPGTLGR_846.8_600.3
   YWGVASFLQK_599.8_849.5
   TPSAAYLWVGTGASEAEK_919.5_428.2
   DPNGLPPEAQK_583.3_669.4
   TYLHTYESEI_628.3_908.4
   SPQAFYR_434.7_684.4
   TPSAAYLWVGTGASEAEK_919.5_849.4
   ALNHLPLEYNSALYSR_621.0_538.3
   IEVNESGTVASSSTAVIVSAR_693.0_545.3
   LTTVDIVTLR_565.8_815.5
   LWAYLTIQELLAK_781.5_371.2
   SYTITGLQPGTDYK_772.4_352.2
   GAVHVVVAETDYQSFAVLYLER_822.8_863.5
   FQLPGQK_409.2_276.1
   ILDGGNK_358.7_490.2
   DYWSTVK_449.7_620.3
   AGLLRPDYALLGHR_518.0_595.4
   ALNFGGIGVVVGHELTHAFDDQGR_837.1_360.2
   GYQELLEK_490.3_502.3
   HATLSLSIPR_365.6_472.3
   SVPVTKPVPVTKPITVTK_631.1_658.4
   FQLPGQK_409.2_429.2
   IYLQPGR_423.7_329.2
   TLNAYDHR_330.5_312.2
   DPNGLPPEAQK_583.3_497.2
   FGFGGSTDSGPIR_649.3_946.5
   TYLHTYESEI_628.3_515.3
   GAVHVVVAETDYQSFAVLYLER_822.8_580.3
   VPSHAVVAR_312.5_515.3
   YWGVASFLQK_599.8_350.2
   EWVAIESDSVQPVPR_856.4_468.3
   LQDAGVYR_461.2_680.3
   DLYHYITSYVVDGEIIIYGPAYSGR_955.5_650.3
   LWAYLTIQELLAK_781.5_300.2
   ITENDIQIALDDAK_779.9_632.3
   SYTITGLQPGTDYK_772.4_680.3
   FFQYDTWK_567.8_712.3
   IYLQPGR_423.7_570.3
   YNQLLR_403.7_529.4
   WWGGQPLWITATK_772.4_929.5
   WWGGQPLWITATK_772.4_373.2
   TASDFITK_441.7_710.4
   EWVAIESDSVQPVPR_856.4_486.2
   YEFLNGR_449.7_606.3
   SNPVTLNVLYGPDLPR_585.7_654.4
   ITQDAQLK_458.8_803.4
   LTTVDIVTLR_565.8_716.4
   FNAVLTNPQGDYDTSTGK_964.5_262.1
   ITGFLKPGK_320.9_301.2
   DYWSTVK_449.7_347.2
   DPTFIPAPIQAK_433.2_556.3
   GWVTDGFSSLK_598.8_953.5
   YYGYTGAFR_549.3_771.4
   ELPEHTVK_476.8_347.2
   FTFTLHLETPKPSISSSNLNPR_829.4_874.4
   DLYHYITSYVVDGEIIIYGPAYSGR_955.5_707.3
   SPQAFYR_434.7_684.4
   TPSAAYLWVGTGASEAEK_919.5_849.4
   ALNHLPLEYNSALYSR_621.0_538.3
   IEVNESGTVASSSTAVIVSAR_693.0_545.3
   LTTVDIVTLR_565.8_815.5
   LWAYLTIQELLAK_781.5_371.2
   SYTITGLQPGTDYK_772.4_352.2
   GAVHVVVAETDYQSFAVLYLER_822.8_863.5
   FQLPGQK_409.2_276.1
   DLYHYITSYVVDGEIIIYGPAYSGR_955.5_707.3
   LIQDAVTGLTVNGQITGDK_972.0_798.4
   VQTAHFK_277.5_431.2
   FLNWIK_410.7_560.3
   ITGFLKPGK_320.9_429.3
   ELIEELVNITQNQK_557.6_517.3
   ALNHLPLEYNSALYSR_621.0_538.3
   DISEWTPR_508.3_787.4
   AHYDLR_387.7_288.2
   LIQDAVTGLTVNGQITGDK_972.0_798.4
   ALNHLPLEYNSALYSR_621.0_538.3
   VQTAHFK_277.5_431.2
   NIQSVNVK_451.3_674.4
   TYLHTYESEI_628.3_908.4
   LIENGYFHPVK_439.6_627.4
   AVLHIGEK_289.5_292.2
   FLNWIK_410.7_560.3
   ITGFLKPGK_320.9_429.3
   DALSSVQESQVAQQAR_573.0_672.4
   DALSSVQESQVAQQAR_573.0_502.3
   FLPCENK_454.2_550.2
   ELIEELVNITQNQK_557.6_517.3
   TEFLSNYLTNVDDITLVPGTLGR_846.8_600.3
   VSEADSSNADWVTK_754.9_347.2
   ITLPDFTGDLR_624.3_288.2
   TGVAVNKPAEFTVDAK_549.6_258.1
   TASDFITK_441.7_781.4
   LIQDAVTGLTVNGQITGDK_972.0_798.4
   ALNHLPLEYNSALYSR_621.0_538.3
   LIQDAVTGLTVNGQITGDK_972.0_798.4
   LIENGYFHPVK_439.6_627.4
   WWGGQPLWITATK_772.4_929.5
   QALEEFQK_496.8_680.3
   NIQSVNVK_451.3_674.4
   DALSSVQESQVAQQAR_573.0_672.4
   AVLHIGEK_289.5_348.7
   FLNWIK_410.7_560.3
   FLPCENK_454.2_550.2
   ITLPDFTGDLR_624.3_288.2
   DISEVVTPR_508.3_787.4
   EVFSKPISWEELLQ_852.9_260.2
   YYGYTGAFR_549.3_450.3
   TASDFITK_441.7_781.4
   FLNWIK_410.7_560.3
   AHYDLR_387.7_288.2
   ALNHLPLEYNSALYSR_621.0_538.3
   LIQDAVTGLTVNGQITGDK_972.0_798.4
   TYLHTYESEI_628.3_908.4
   VQTAHFK_277.5_431.2
   ELIEELVNITQNQK_557.6_618.3
   VSEADSSNADWVTK_754.9_347.2
   AVLHIGEK_289.5_292.2
   TGVAVNKPAEFTVDAK_549.6_977.5
   LIENGYFHPVK_439.6_343.2
   AQPVQVAEGSEPDGFWEALGGK_758.0_574.3
   YYGYTGAFR_549.3_450.3
   TASDFITK_441.7_781.4
   NIQSVNVK_451.3_674.4
   DALSSVQESQVAQQAR_573.0_672.4
   DALSSVQESQVAQQAR_573.0_502.3
   FGFGGSTDSGPIR_649.3_745.4
   ALDLSLK_380.2_575.3
   TLFIFGVTK_513.3_811.5
   ISQGEADINIAFYQR_575.6_684.4
   SGVDLADSNQK_567.3_591.3
   GPGEDFR_389.2_322.2
   DPNGLPPEAQK_583.3_669.4
   WNFAYWAAHQPWSR_607.3_545.3
   ELCLDPK_437.7_359.2
   FFQYDTWK_567.8_840.4
   IIEVEEEQEDPYLNDR_996.0_777.4
   ECEELEEK_533.2_405.2
   LEEHYELR_363.5_580.3
   LNIGYIEDLK_589.3_837.4
   TAVTANLDIR_537.3_288.2
   SWNEPLYHLVTEVR_581.6_716.4
   ILNIFGVIK_508.8_790.5
   TPSAAYLWVGTGASEAEK_919.5_849.4
   VGVISFAQK_474.8_693.4
   LNIGYIEDLK_589.3_950.5
   GQVPENEANVVITTLK_571.3_462.3
   STPSLTTK_417.7_549.3
   ALLLGWVPTR_563.3_373.2
   LIQDAVTGLTVNGQITGDK_972.0_798.4
   ALNHLPLEYNSALYSR_621.0_538.3
   ELIEELVNITQNQK_557.6_618.3
   VQTAHFK_277.5_431.2
   NIQSVNVK_451.3_674.4
   TLFIFGVTK_513.3_811.5
   GPGEDFR_389.2_322.2
   FLPCENK_454.2_550.2
   FLNWIK_410.7_560.3
   LIENGYFHPVK_439.6_627.4
   ECEELEEK_533.2_405.2
   FGFGGSTDSGPIR_649.3_745.4
   QALEEFQK_496.8_680.3
   IIEVEEEQEDPYLNDR_996.0_777.4
   TYLHTYESEI_628.3_908.4
   ELIEELVNITQNQK_557.6_517.3
   LEEHYELR_363.5_580.3
   ISQGEADINIAFYQR_575.6_684.4
   HPWIVHWDQLPQYQLNR_744.0_918.5
   AHYDLR_387.7_288.2
   GFQALGDAADIR_617.3_288.2
   SWNEPLYHLVTEVR_581.6_716.4
   SGVDLADSNQK_567.3_591.3
   YENYTSSFFIR_713.8_293.1
   FFQYDTWK_567.8_840.4
   HYFIAAVER_553.3_658.4
   ITGFLKPGK_320.9_429.3
   DALSSVQESQVAQQAR_573.0_502.3
   SGVDLADSNQK_567.3_662.3
   ALDLSLK_380.2_575.3
   TGVAVNKPAEFTVDAK_549.6_258.1
   HPWIVHWDQLPQYQLNR_744.0_1047.0
   LIQDAVTGLTVNGQITGDK_972.0_640.4
   LNIGYIEDLK_589.3_837.4
   IALGGLLFPASNLR_481.3_657.4
   LPATEKPVLLSK_432.6_460.3
   FQLSETNR_497.8_605.3
   NEIVFPAGILQAPFYTR_968.5_357.2
   ALDLSLK_380.2_185.1
   DLHLSDVFLK_396.2_366.2
   TQILEWAAER_608.8_761.4
   FSVVYAK_407.2_381.2
   VSEADSSNADWVTK_754.9_347.2
   DPNGLPPEAQK_583.3_669.4
   VQEAHLTEDQIFYFPK_655.7_701.4
   LSSPAVITDK_515.8_830.5
   ITLPDFTGDLR_624.3_288.2
   AVLHIGEK_289.5_292.2
   GEVTYTTSQVSK_650.3_750.4
   GYVIIKPLVWV_643.9_854.6
   TGVAVNKPAEFTVDAK_549.6_977.5
   YYGYTGAFR_549.3_450.3
   ILDGGNK_358.7_490.2
   FLPCENK_454.2_390.2
   GFQALGDAADIR_617.3_717.4
   SDGAKPGPR_442.7_213.6
   NTGVISVVTTGLDR_716.4_662.4
   SERPPIFEIR_415.2_288.2
   DFHINLFQVLPWLK_885.5_400.2
   DALSSVQESQVAQQAR_573.0_672.4
   ELCLDPK_437.7_359.2
   ILNIFGVIK_508.8_790.5
   TEFLSNYLTNVDDITLVPGTLGR_846.8_600.3
   GAVHVVVAETDYQSFAVLYLER_822.8_863.5
   AVLHIGEK_289.5_348.7
   TFLTVYWTPER_706.9_401.2
   AGITIPR_364.2_486.3
   GAVHVVVAETDYQSFAVLYLER_822.8_580.3
   SLQAFVAVAAR_566.8_487.3
   SWNEPLYHLVTEVR_581.6_614.3
   TYLHTYESEI_628.3_515.3
   TAHISGLPPSTDFIVYLSGLAPSIR_871.5_800.5
   QNYHQDSEAAINR_515.9_544.3
   AHQLAIDTYQEFEETYIPK_766.0_634.4
   GDTYPAELYITGSILR_885.0_274.1
   AHYDLR_387.7_566.3
   IALGGLLFPASNLR_481.3_412.3
   ATNATLDPR_479.8_272.2
   FSVVYAK_407.2_579.4
   HTLNQIDEVK_598.8_951.5
   DIPHWLNPTR_416.9_373.2
   LYYGDDEK_501.7_563.2
   AALAAFNAQNNGSNFQLEEISR_789.1_633.3
   IQTHSTTYR_369.5_627.3
   GPITSAAELNDPQSILLR_632.4_826.5
   QTLSWTVTPK_580.8_818.4
   AVGYLITGYQR_620.8_737.4
   DIIKPDPPK_511.8_342.2
   YNQLLR_403.7_288.2
   GNGLTWAEK_488.3_634.3
   QVFAVQR_424.2_473.3
   FLQEQGHR_338.8_497.3
   HVVQLR_376.2_515.3
   DVLLLVHNLPQNLTGHIWYK_791.8_883.0
   TFLTVYWTPER_706.9_502.3
   VELAPLPSWQPVGK_760.9_400.3
   TLFIFGVTK_513.3_215.1
   AYSDLSR_406.2_375.2
   HATLSLSIPR_365.6_472.3
   LQGTLPVEAR_542.3_571.3
   NTVISVNPSTK_580.3_732.4
   EVFSKPISWEELLQ_852.9_260.2
   SLQNASAIESILK_687.4_860.5
   IQHPFTVEEFVLPK_562.0_861.5
   GVTGYFTFNLYLK_508.3_683.9
   VFQYIDLHQDEFVQTLK_708.4_361.2
   ILDDLSPR_464.8_587.3
   LIENGYFHPVK_439.6_343.2
   VFQFLEK_455.8_811.4
   AFTECCVVASQLR_770.9_574.3
   WWGGQPLWITATK_772.4_929.5
   IQTHSTTYR_369.5_540.3
   IAQYYYTFK_598.8_395.2
   YGFYTHVFR_397.2_421.3
   YHFEALADTGISSEFYDNANDLLSK_940.8_874.5
   ANDQYLTAAALHNLDEAVK_686.4_301.1
   FSLVSGWGQLLDR_493.3_403.2
   GWVTDGFSSLK_598.8_854.4
   TLEAQLTPR_514.8_814.4
   VSAPSGTGHLPGLNPL_506.3_300.7
   EAQLPVIENK_570.8_699.4
   SPEAEDPLGVER_649.8_670.4
   IAIDLFK_410.3_635.4
   YEFLNGR_449.7_293.1
   VQTAHFK_277.5_502.3
   IEVIITLK_464.8_815.5
   ILTPEVR_414.3_601.3
   LEEHYELR_363.5_288.2
   HATLSLSIPR_365.6_272.2
   NSDQEIDFK_548.3_294.2
   LPNNVLQEK_527.8_844.5
   ELANTIK_394.7_475.3
   LSIPQITTK_500.8_687.4
   TLNAYDHR_330.5_312.2
   WWGGQPLWITATK_772.4_373.2
   ELLESYIDGR_597.8_710.4
   ATLSAAPSNPR_542.8_570.3
   VVLSSGSGPGLDLPLVLGLPLQLK_791.5_598.4
   NADYSYSVWK_616.8_333.2
   ILILPSVTR_506.3_559.3
   ALEQDLPVNIK_620.4_798.5
   QVCADPSEEWVQK_788.4_275.2
   SVQNDSQAIAEVLNQLK_619.7_914.5
   QVFAVQR_424.2_620.4
   ALPGEQQPLHALTR_511.0_807.5
   LEPLYSASGPGLRPLVIK_637.4_260.2
   GTYLYNDCPGPGQDTDCR_697.0_666.3
   DAGLSWGSAR_510.2_576.3
   YGFYTHVFR_397.2_659.4
   NNQLVAGYLQGPNVNLEEK_700.7_357.2
   EKPAGGIPVLGSLVNTVLK_631.4_930.6
   FGSDDEGR_441.7_735.3
   IEVNESGTVASSSTAVIVSAR_693.0_545.3
   FATTFYQHLADSK_510.3_533.3
   DYWSTVK_449.7_347.2
   QLGLPGPPDVPDHAAYHPF_676.7_263.1
   LSSPAVITDK_515.8_743.4
   TEFLSNYLTNVDDITLVPGTLGR_846.8_699.4
   SILFLGK_389.2_201.1
   VTFEYR_407.7_614.3
   SVVLIPLGAVDDGEHSQNEK_703.0_798.4
   HTLNQIDEVK_598.8_958.5
   ALNSIIDVYHK_424.9_661.3
   ETLALLSTHR_570.8_500.3
   GLQYAAQEGLLALQSELLR_1037.1_858.5
   TYNVDK_370.2_262.1
   FTITAGSK_412.7_576.3
   GIVEECCFR_585.3_900.3
   YGIEEHGK_311.5_599.3
   ALVLELAK_428.8_331.2
   ITLPDFTGDLR_624.3_920.5
   HELTDEELQSLFTNFANVVDK_817.1_906.5
   EANQSTLENFLER_775.9_678.4
   DADPDTFFAK_563.8_825.4
   LFIPQITR_494.3_727.4
   DPNGLPPEAQK_583.3_497.2
   VEPLYELVTATDFAYSSTVR_754.4_549.3
   FQLSETNR_497.8_476.3
   FSLVSGWGQLLDR_493.3_516.3
   NKPGVYTDVAYYLAWIR_677.0_545.3
   FTGSQPFGQGVEHATANK_626.0_521.2
   DDLYVSDAFHK_655.3_704.3
   AFLEVNEEGSEAAASTAVVIAGR_764.4_685.4
   LPNNVLQEK_527.8_730.4
   IVLSLDVPIGLLQILLEQAR_735.1_503.3
   DPTFIPAPIQAK_433.2_556.3
   SDLEVAHYK_531.3_617.3
   QLYGDTGVLGR_589.8_501.3
   VNHVTLSQPK_374.9_459.3
   TLLPVSKPEIR_418.3_288.2
   SEYGAALAWEK_612.8_845.5
   YGIEEHGK_311.5_341.2
   DAGLSWGSAR_510.3_390.2
   AEHPTWGDEQLFQTTR_639.3_569.3
   FQSVFTVTR_542.8_623.4
   VPGLYYFTYHASSR_554.3_420.2
   AYSDLSR_406.2_577.3
   ALEQDLPVNIK_620.4_570.4
   NAVVQGLEQPHGLVVHPLR_688.4_890.6
   TSDQIHFFFAK_447.6_659.4
   GTYLYNDCPGPGQDTDCR_697.0_335.2
   TSYQVYSK_488.2_787.4
   ALNSIIDVYHK_424.9_774.4
   VELAPLPSWQPVGK_760.9_342.2
   LSETNR_360.2_330.2
   HFQNLGK_422.2_527.2
   ELPQSIVYK_538.8_417.7
   LPATEKPVLLSK_432.6_347.2
   SPEAEDPLGVER_649.8_314.1
   DEIPHNDIALLK_459.9_510.8
   FFQYDTWK_567.8_712.3
   LIEIANHVDK_384.6_498.3
   AGFAGDDAPR_488.7_701.3
   YEFLNGR_449.7_606.3
   VIAVNEVGR_478.8_284.2
   SLSQQIENIR_594.3_531.3
   EWVAIESDSVQPVPR_856.4_486.2
   YGLVTYATYPK_638.3_843.4
   SVVLIPLGAVDDGEHSQNEK_703.0_286.2
   NSDQEIDFK_548.3_409.2
   NVNQSLLELHK_432.2_543.3
   IAQYYYTFK_598.8_884.4
   GPITSAAELNDPQSILLR_632.4_601.4
   YTTEIIK_434.2_704.4
   GYVIIKPLVWV_643.9_304.2
   LDFHFSSDR_375.2_464.2
   IPSNPSHR_303.2_496.3
   APLTKPLK_289.9_357.2
   EVFSKPISWEELLQ_852.9_376.2
   YENYTSSFFIR_713.8_756.4
   SPQAFYR_434.7_556.3
   SVDEALR_395.2_488.3
   FVFGTTPEDILR_697.9_742.4
   FTFTLHLETPKPSISSSNLNPR_829.4_787.4
   VGEYSLYIGR_578.8_708.4
   ILPSVPK_377.2_244.2
   LFIPQITR_494.3_614.4
   TGYYFDGISR_589.8_857.4
   HYGGLTGLNK_530.3_759.4
   NQSPVLEPVGR_598.3_866.5
   IGKPAPDFK_324.9_294.2
   TSESTGSLPSPFLR_739.9_716.4
   ESDTSYVSLK_564.8_347.2
   ETPEGAEAKPWYEPIYLGGVFQLEK_951.1_877.5
   TSDQIHFFFAK_447.6_512.3
   SEYGAALAWEK_612.8_788.4
   VFQFLEK_455.8_811.4
   VLEPTLK_400.3_458.3
   SLDFTELDVAAEK_719.4_316.2
   TLAFVR_353.7_492.3
   YGIEEHGK_311.5_599.3
   LHEAFSPVSYQHDLALLR_699.4_251.2
   QGHNSVFLIK_381.6_520.4
   LLAPSDSPEWLSFDVTGVVR_730.1_430.3
   ELPQSIVYK_538.8_417.7
   VNHVTLSQPK_374.9_244.2
   VFQFLEK_455.8_811.4
   SEYGAALAWEK_612.8_788.4
   GEVTYTTSQVSK_650.3_750.4
   QVFAVQR_424.2_473.3
   VELAPLPSWQPVGK_760.9_342.2
   LHEAFSPVSYQHDLALLR_699.4_251.2
   SLDFTELDVAAEK_719.4_316.2
   YGIEEHGK_311.5_599.3
   YGIEEHGK_311.5_341.2
   HYINLITR_515.3_301.1
   TLAFVR_353.7_492.3
   GVTGYFTFNLYLK_508.3_260.2
   FSLVSGWGQLLDR_493.3_403.2
   DALSSVQESQVAQQAR_573.0_502.3
   VELAPLPSWQPVGK_760.9_400.3
   AVDIPGLEAATPYR_736.9_399.2
   ELPQSIVYK_538.8_417.7
   DVLLLVHNLPQNLTGHIWYK_791.8_310.2
   VPLALFALNR_557.3_620.4
   LLAPSDSPEWLSFDVTGVVR_730.1_430.3
   VNHVTLSQPK_374.9_244.2
   EVFSKPISWEELLQ_852.9_376.2
   SEYGAALAWEK_612.8_788.4
   VFQFLEK_455.8_811.4
   SLDFTELDVAAEK_719.4_316.2
   TLAFVR_353.7_492.3
   LHEAFSPVSYQHDLALLR_699.4_251.2
   HYINLITR_515.3_301.1
   VLEPTLK_400.3_458.3
   YGIEEHGK_311.5_599.3
   VELAPLPSWQPVGK_760.9_342.2
   QVFAVQR_424.2_473.3
   ANLINNIFELAGLGK_793.9_299.2
   DVLLLVHNLPQNLTGHIWYK_791.8_310.2
   VEHSDLSFSK_383.5_234.1
   AVDIPGLEAATPYR_736.9_399.2
   VPLALFALNR_557.3_620.4
   ELPQSIVYK_538.8_417.7
   LLAPSDSPEWLSFDVTGVVR_730.1_430.3
   EVFSKPISWEELLQ_852.9_376.2
   SEYGAALAWEK_612.8_845.5
   TLAFVR_353.7_492.3
   YGIEEHGK_311.5_599.3
   DFNQFSSGEK_386.8_333.2
   VFQFLEK_455.8_811.4
   LIEIANHVDK_384.6_683.4
   QINSYVK_426.2_496.3
   TYLHTYESEI_628.3_515.3
   SLQAFVAVAAR_566.8_804.5
   TEQAAVAR_423.2_615.4
   VLEPTLK_400.3_587.3
   AQPVQVAEGSEPDGFWEALGGK_758.0_574.3
   VPLALFALNR_557.3_620.4
   AVYEAVLR_460.8_587.4
   VFQFLEK_455.8_811.4
   SEYGAALAWEK_612.8_845.5
   YGIEEHGK_311.5_599.3
   DPTFIPAPIQAK_433.2_556.3
   TLAFVR_353.7_492.3
   NQSPVLEPVGR_598.3_866.5
   ALNHLPLEYNSALYSR_621.0_538.3
   TQILEWAAER_608.8_761.4
   VVGGLVALR_442.3_784.5
   QINSYVK_426.2_496.3
   YGIEEHGK_311.5_341.2
   ALEQDLPVNIK_620.4_570.4
   GTYLYNDCPGPGQDTDCR_697.0_666.3
   SLQNASAIESILK_687.4_860.5
   DLHLSDVFLK_396.2_260.2
   LIEIANHVDK_384.6_683.4
   NIQSVNVK_451.3_674.4
   FFQYDTWK_567.8_712.3
   ANLINNIFELAGLGK_793.9_299.2
   TYLHTYESEI_628.3_515.3
   AALAAFNAQNNGSNFQLEEISR_789.1_746.4
   AQPVQVAEGSEPDGFWEALGGK_758.0_574.3
   TSYQVYSK_488.2_787.4
   AVDIPGLEAATPYR_736.9_399.2
   TAHISGLPPSTDFIVYLSGLAPSIR_871.5_472.3
   AEIEYLEK_497.8_552.3
   LLAPSDSPEWLSFDVTGVVR_730.1_430.3
   VPLALFALNR_557.3_620.4
   AVYEAVLR_460.8_587.4
   ELPQSIVYK_538.8_417.7
   EVFSKPISWEELLQ_852.9_376.2
   VFQFLEK_455.8_811.4
   ALNHLPLEYNSALYSR_621.0_696.4
   YGIEEHGK_311.5_599.3
   SEPRPGVLLR_375.2_654.4
   TQILEWAAER_608.8_761.4
   NQSPVLEPVGR_598.3_866.5
   TEQAAVAR_423.2_615.4
   QINSYVK_426.2_496.3
   ALEQDLPVNIK_620.4_570.4
   NCSFSIIYPVVIK_770.4_555.4
   LIEIANHVDK_384.6_683.4
   SLQNASAIESILK_687.4_860.5
   ANLINNIFELAGLGK_793.9_299.2
   GTYLYNDCPGPGQDTDCR_697.0_666.3
   TYLHTYESEI_628.3_515.3
   NIQSVNVK_451.3_674.4
   LTTVDIVTLR_565.8_815.5
   FFQYDTWK_567.8_712.3
   TSYQVYSK_488.2_787.4
   AQPVQVAEGSEPDGFWEALGGK_758.0_574.3
   AEIEYLEK_497.8_552.3
   LLAPSDSPEWLSFDVTGVVR_730.1_430.3
   AVDIPGLEAATPYR_736.9_399.2
   EVFSKPISWEELLQ_852.9_376.2
   TGVAVNKPAEFTVDAK_549.6_258.1
   ELPQSIVYK_538.8_417.7
   VNHVTLSQPK_374.9_244.2
   VPLALFALNR_557.3_620.4
   ALNHLPLEYNSALYSR_621.0_538.3
   TLAFVR_353.7_492.3
   YGIEEHGK_311.5_599.3
   DPTFIPAPIQAK_433.2_461.2
   DFNQFSSGEK_386.8_333.2
   QINSYVK_426.2_496.3
   TYLHTYESEI_628.3_515.3
   AQPVQVAEGSEPDGFWEALGGK_758.0_574.3
   AEIEYLEK_497.8_552.3
   VNHVTLSQPK_374.9_244.2
   LLAPSDSPEWLSFDVTGWR_730.1_430.3
   EVFSKPISWEELLQ_852.9_376.2
   AVDIPGLEAATPYR_736.9_399.2
   VPLALFALNR_557.3_620.4
   AVYEAVLR_460.8_587.4
   QINSYVK_426.2_610.3
   ILDGGNK_358.7_603.3
   VFQYIDLHQDEFVQTLK_708.4_375.2
   SGVDLADSNQK_567.3_662.3
   YSHYNER_323.5_418.2
   DEIPHNDIALLK_459.9_510.8
   SGVDLADSNQK_567.3_591.3
   FGFGGSTDSGPIR_649.3_745.4
   GTYLYNDCPGPGQDTDCR_697.0_666.3
   TSYQVYSK_488.2_787.4
   TGVAVNKPAEFTVDAK_549.6_258.1
   SPEAEDPLGVER_649.8_314.1
   VPLALFALNR_557.3_620.4
   YYGYTGAFR_549.3_450.3
   AVYEAVLR_460.8_587.4
   AEIEYLEK_497.8_552.3
   AALAAFNAQNNGSNFQLEEISR_789.1_746.4
   SGVDLADSNQK_567.3_662.3
   ILDGGNK_358.7_603.3
   QINSYVK_426.2_610.3
   DEIPHNDIALLK_459.9_510.8
   VFQYIDLHQDEFVQTLK_708.4_375.2
   FGFGGSTDSGPIR_649.3_745.4
   LTTVDIVTLR_565.8_815.5
   YSHYNER_323.5_418.2
   FFQYDTWK_567.8_712.3
   IEVNESGTVASSSTAVIVSAR_693.0_545.3
   GTYLYNDCPGPGQDTDCR_697.0_666.3
   LIEIANHVDK_384.6_683.4
   VGVISFAQK_474.8_580.3
   TSYQVYSK_488.2_787.4
   ALNFGGIGVVVGHELTHAFDDQGR_837.1_360.2
   AYSDLSR_406.2_375.2
   TGVAVNKPAEFTVDAK_549.6_258.1
   SPEAEDPLGVER_649.8_314.1
   YYGYTGAFR_549.3_450.3
   VPLALFALNR_557.3_620.4
   YISPDQLADLYK_713.4_277.2
   AVDIPGLEAATPYR_736.9_286.1
   AEIEYLEK_497.8_552.3
   AVYEAVLR_460.8_587.4
   AALAAFNAQNNGSNFQLEEISR_789.1_746.4
   SGVDLADSNQK_567.3_662.3
   ILDGGNK_358.7_603.3
   QINSYVK_426.2_496.3
   YSHYNER_323.5_418.2
   ALEQDLPVNIK_620.4_798.5
   LIEIANHVDK_384.6_683.4
   GTYLYNDCPGPGQDTDCR_697.0_666.3
   IEVNESGTVASSSTAVIVSAR_693.0_545.3
   LTTVDIVTLR_565.8_815.5
   TSYQVYSK_488.2_787.4
   TGVAVNKPAEFTVDAK_549.6_258.1
   AYSDLSR_406.2_375.2
   SPEAEDPLGVER_649.8_314.1
   YYGYTGAFR_549.3_450.3
   YISPDQLADLYK_713.4_277.2
   AVDIPGLEAATPYR_736.9_286.1
   AEIEYLEK_497.8_552.3
   AALAAFNAQNNGSNFQLEEISR_789.1_746.4
   AVYEAVLR_460.8_587.4
   QINSYVK_426.2_496.3
   DEIPHNDIALLK_459.9_510.8
   ALEQDLPVNIK_620.4_570.4
   ILDGGNK_358.7_603.3
   LIEIANHVDK_384.6_683.4
   YYGYTGAFR_549.3_450.3
   AALAAFNAQNNGSNFQLEEISR_789.1_746.4
   AEIEYLEK_497.8_552.3
   AVYEAVLR_460.8_587.4
   ELIEELVNITQNQK_557.6_517.3
   AHYDLR_387.7_288.2
   FSVVYAK_407.2_381.2
   ITLPDFTGDLR_624.3_288.2
   ITGFLKPGK_320.9_301.2
   ITGFLKPGK_320.9_429.3
   ELIEELVNITQNQK_557.6_618.3
   VFQFLEK_455.8_811.4
   VQTAHFK_277.5_431.2
   FLNWIK_410.7_560.3
   DAGLSWGSAR_510.3_390.2
   TLLPVSKPEIR_418.3_288.2
   LIQDAVTGLTVNGQITGDK_972.0_798.4
   QALEEFQK_496.8_680.3
   FSVVYAK_407.2_579.4
   ITLPDFTGDLR_624.3_920.5
   AHYDLR_387.7_566.3
   ALNHLPLEYNSALYSR_621.0_538.3
   DVLLLVHNLPQNLPGYFWYK_810.4_967.5
   SFRPFVPR_335.9_635.3
   SDLEVAHYK_531.3_617.3
   VQEAHLTEDQIFYFPK_655.7_701.4
   VFQFLEK_455.8_276.2
   SLLQPNK_400.2_599.4
   SFRPFVPR_335.9_272.2
   FLNWIK_410.7_561.3
   LSSPAVITDK_515.8_743.4
   DVLLLVHNLPQNLPGYFWYK_810.4_594.3
   ALVLELAK_428.8_672.4
   DISEVVTPR_508.3_787.4
   ELIEELVNITQNQK_557.6_517.3
   LPNNVLQEK_527.8_844.5
   AHYDLR_387.7_288.2
   ITLPDFTGDLR_624.3_288.2
   IEGNLIFDPNNYLPK_874.0_414.2
   ITGFLKPGK_320.9_301.2
   FSVVYAK_407.2_381.2
   ITGFLKPGK_320.9_429.3
   VFQFLEK_455.8_811.4
   LIQDAVTGLTVNGQITGDK_972.0_798.4
   DADPDTFFAK_563.8_825.4
   FLNWIK_410.7_560.3
   TASDFITK_441.7_781.4
   DAGLSWGSAR_510.3_390.2
   VQTAHFK_277.5_431.2
   TLLPVSKPEIR_418.3_288.2
   ELIEELVNITQNQK_557.6_618.3
   TYLHTYESEI_628.3_908.4
   HFQNLGK_422.2_527.2
   TASDFITK_441.7_710.4
   ITLPDFTGDLR_624.3_920.5
   QALEEFQK_496.8_680.3
   AVLHIGEK_289.5_348.7
   FSVVYAK_407.2_579.4
   LPNNVLQEK_527.8_730.4
   AHYDLR_387.7_566.3
   IAPQLSTEELVSLGEK_857.5_333.2
   WWGGQPLWITATK_772.4_929.5
   TYLHTYESEI_628.3_515.3
   DALSSVQESQVAQQAR_573.0_502.3
   ELIEELVNITQNQK_557.6_517.3
   LPNNVLQEK_527.8_844.5
   ITLPDFTGDLR_624.3_288.2
   IEGNLIFDPNNYLPK_874.0_414.2
   VFQFLEK_455.8_811.4
   LIQDAVTGLTVNGQITGDK_972.0_798.4
   TASDFITK_441.7_781.4
   DAGLSWGSAR_510.3_390.2
   FSVVYAK_407.2_579.4
   AVLHIGEK_289.5_348.7
   FLNWIK_410.7_560.3
   QALEEFQK_496.8_680.3
   DVLLLVHNLPQNLPGYFWYK_810.4_967.5
   WWGGQPLWITATK_772.4_929.5
   VQEAHLTEDQIFYFPK_655.7_701.4
   NNQLVAGYLQGPNVNLEEK_700.7_999.5
   SLLQPNK_400.2_599.4
   DALSSVQESQVAQQAR_573.0_672.4
   NIQSVNVK_451.3_674.4
   HPWIVHWDQLPQYQLNR_744.0_1047.0
   TTSDGGYSFK_531.7_860.4
   ALNHLPLEYNSALYSR_621.0_538.3
   LSSPAVITDK_515.8_743.4
   TGVAVNKPAEFTVDAK_549.6_258.1
   TLNAYDHR_330.5_312.2
   DISEVVTPR_508.3_787.4
   LIENGYFHPVK_439.6_627.4
   SGVDLADSNQK_567.3_662.3
   ALQDQLVLVAAK_634.9_289.2
   ALVLELAK_428.8_672.4
   ELIEELVNITQNQK_557.6_517.3
   ITLPDFTGDLR_624.3_288.2
   AHYDLR_387.7_288.2
   FSVVYAK_407.2_381.2
   ITGFLKPGK_320.9_301.2
   VFQFLEK_455.8_811.4
   ELIEELVNITQNQK_557.6_618.3
   LPNNVLQEK_527.8_844.5
   IEGNLIFDPNNYLPK_874.0_414.2
   ITGFLKPGK_320.9_429.3
   FLNWIK_410.7_560.3
   LIQDAVTGLTVNGQITGDK_972.0_798.4
   ITLPDFTGDLR_624.3_920.5
   DAGLSWGSAR_510.3_390.2
   TLLPVSKPEIR_418.3_288.2
   FSVVYAK_407.2_579.4
   DADPDTFFAK_563.8_825.4
   AHYDLR_387.7_566.3
   QALEEFQK_496.8_680.3
   HTLNQIDEVK_598.8_951.5
   TASDFITK_441.7_781.4
   VFQFLEK_455.8_276.2
   DVLLLVHNLPQNLPGYFWYK_810.4_967.5
   VQTAHFK_277.5_431.2
   SFRPFVPR_335.9_635.3
   HFQNLGK_422.2_527.2
   SVSLPSLDPASAK_636.4_473.3
   FGFGGSTDSGPIR_649.3_745.4
   HFQNLGK_422.2_285.1
   AVLHIGEK_289.5_348.7
   ELIEELVNITQNQK_557.6_517.3
   ITLPDFTGDLR_624.3_288.2
   AHYDLR_387.7_288.2
   FSVVYAK_407.2_381.2
   ITGFLKPGK_320.9_301.2
   VFQFLEK_455.8_811.4
   ELIEELVNITQNQK_557.6_618.3
   LPNNVLQEK_527.8_844.5
   IEGNLIFDPNNYLPK_874.0_414.2
   ITGFLKPGK_320.9_429.3
   FLNWIK_410.7_560.3
   LIQDAVTGLTVNGQITGDK_972.0_798.4
   ITLPDFTGDLR_624.3_920.5
   DAGLSWGSAR_510.3_390.2
   TLLPVSKPEIR_418.3_288.2
   FSVVYAK_407.2_579.4
   DADPDTFFAK_563.8_825.4
   AHYDLR_387.7_566.3
   QALEEFQK_496.8_680.3
   HTLNQIDEVK_598.8_951.5
   TASDFITK_441.7_781.4
   VFQFLEK_455.8_276.2
   DVLLLVHNLPQNLPGYFWYK_810.4_967.5
   VQTAHFK_277.5_431.2
   SFRPFVPR_335.9_635.3
   HFQNLGK_422.2_527.2
   SVSLPSLDPASAK_636.4_473.3
   FGFGGSTDSGPIR_649.3_745.4
   HFQNLGK_422.2_285.1
   AVLHIGEK_289.5_348.7
   ELIEELVNITQNQK_557.6_517.3
   VFQFLEK_455.8_811.4
   FLNWIK_410.7_560.3
   ITLPDFTGDLR_624.3_288.2
   LIQDAVTGLTVNGQITGDK_972.0_798.4
   DAGLSWGSAR_510.3_390.2
   AHYDLR_387.7_288.2
   FSVVYAK_407.2_381.2
   LPNNVLQEK_527.8_844.5
   ITGFLKPGK_320.9_429.3
   ITGFLKPGK_320.9_301.2
   IEGNLIFDPNNYLPK_874.0_414.2
   VQTAHFK_277.5_431.2
   TLLPVSKPEIR_418.3_288.2
   QALEEFQK_496.8_680.3
   FSVVYAK_407.2_579.4
   ELIEELVNITQNQK_557.6_618.3
   TASDFITK_441.7_781.4
   DVLLLVHNLPQNLPGYFWYK_810.4_967.5
   AVLHIGEK_289.5_348.7
   ITLPDFTGDLR_624.3_920.5
   WWGGQPLWITATK_772.4_929.5
   VQEAHLTEDQIFYFPK_655.7_701.4
   DADPDTFFAK_563.8_825.4
   DALSSVQESQVAQQAR_573.0_502.3
   FGFGGSTDSGPIR_649.3_745.4
   FLPCENK_454.2_550.2
   VQTAHFK_277.5_502.3
   VFQFLEK_455.8_276.2
   TYLHTYESEI_628.3_908.4
   ALNHLPLEYNSALYSR_621.0_538.3
   NKPGVYTDVAYYLAWIR_677.0_545.3
   LEEHYELR_363.5_580.3
   HPWIVHWDQLPQYQLNR_744.0_1047.0
   HTLNQIDEVK_598.8_951.5
   LPNNVLQEK_527.8_730.4
   ALDLSLK_380.2_575.3
   YGIEEHGK_311.5_599.3
   LSSPAVITDK_515.8_830.5
   LEEHYELR_363.5_288.2
   NVIQISNDLENLR_509.9_402.3
   SGFSFGFK_438.7_732.4
   SDLEVAHYK_531.3_617.3
   NADYSYSVWK_616.8_333.2
   AHYDLR_387.7_566.3
   GAVHVVVAETDYQSFAVLYLER_822.8_580.3
   LIENGYFHPVK_439.6_627.4
   ALDLSLK_380.2_185.1
   WWGGQPLWITATK_772.4_373.2
   FGFGGSTDSGPIR_649.3_946.5
   GLQYAAQEGLLALQSELLR_1037.1_929.5
   HFQNLGK_422.2_527.2
   YSHYNER_323.5_581.3
   ISQGEADINIAFYQR_575.6_684.4
   TGVAVNKPAEFTVDAK_549.6_258.1
   NIQSVNVK_451.3_674.4
   DALSSVQESQVAQQAR_573.0_672.4
   SLLQPNK_400.2_599.4
   SFRPFVPR_335.9_635.3
   NNQLVAGYLQGPNVNLEEK_700.7_999.5
   LYYGDDEK_501.7_563.2
   SWNEPLYHLVTEVR_581.6_716.4
   SGVDLADSNQK_567.3_662.3
   GTYLYNDCPGPGQDTDCR_697.0_335.2
   HATLSLSIPR_365.6_472.3
   FIVGFTR_420.2_261.2
   QNYHQDSEAAINR_515.9_544.3
   DVLLLVHNLPQNLPGYFWYK_810.4_594.3
   FLNWIK_410.7_561.3
   SLQAFVAVAAR_566.8_487.3
   HFQNLGK_422.2_285.1
   GPGEDFR_389.2_322.2
   NKPGVYTDVAYYLAWIR_677.0_821.5
   LIQDAVTGLTVNGQITGDK_972.0_640.4
   YYGYTGAFR_549.3_450.3
   TLNAYDHR_330.5_312.2
   IQTHSTTYR_369.5_627.3
   TASDFITK_441.7_710.4
   ALNHLPLEYNSALYSR_621.0_696.4
   DADPDTFFAK_563.8_302.1
   SVSLPSLDPASAK_636.4_473.3
   TTSDGGYSFK_531.7_860.4
   AFTECCVVASQLR_770.9_574.3
   ELPQSIVYK_538.8_409.2
   TYLHTYESEI_628.3_515.3
   FQLSETNR_497.8_605.3
   GSLVQASEANLQAAQDFVR_6 6 8.7_806.4
   FSLVSGWGQLLDR_493.3_403.2
   ECEELEEK_533.2_405.2
   NADYSYSVWK_616.8_769.4
   SLLQPNK_400.2_358.2
   LIEIANHVDK_384.6_683.4
   DISEVVTPR_508.3_787.4
   AEVIWTSSDHQVLSGK_586.3_300.2
   SGVDLADSNQK_567.3_591.3
   SILFLGK_389.2_201.1
   AVLHIGEK_289.5_292.2
   QLYGDTGVLGR_589.8_501.3
   FSLVSGWGQLLDR_493.3_516.3
   DSPVLIDFFEDTER_841.9_399.2
   INPASLDK_429.2_630.4
   GAVHVVVAETDYQSFAVLYLER_822.8_863.5
   FLQEQGHR_338.8_497.3
   LNIGYIEDLK_589.3_837.4
   LSSPAVITDK_515.8_743.4
   GLQYAAQEGLLALQSELLR_1037.1_858.5
   SDGAKPGPR_442.7_213.6
   GYQELLEK_490.3_502.3
   GGEGTGYFVDFSVR_745.9_869.5
   ADLFYDVEALDLESPK_913.0_447.2
   ADLFYDVEALDLESPK_913.0_331.2
   EAQLPVIENK_570.8_699.4
   SILFLGK_389.2_577.4
   HTLNQIDEVK_598.8_958.5
   AQPVQVAEGSEPDGFWEALGGK_758.0_574.3
   FLPCENK_454.2_390.2
   LIEIANHVDK_384.6_498.3
   DEIPHNDIALLK_459.9_510.8
   SLQAFVAVAAR_566.8_804.5
   YISPDQLADLYK_713.4_277.2
   SLPVSDSVLSGFEQR_810.9_836.4
   DGSPDVTTADIGANTPDATK_973.5_531.3
   NTGVISVVTTGLDR_716.4_662.4
   ALVLELAK_428.8_672.4
   YEFLNGR_449.7_293.1
   WGAAPYR_410.7_577.3
   TFLTVYWTPER_706.9_401.2
   SEYGAALAWEK_612.8_845.5
   VGVISFAQK_474.8_693.4
   IIEVEEEQEDPYLNDR_9 9 6.0_777.4
   TGYYFDGISR_589.8_694.4
   LQGTLPVEAR_542.3_571.3
   DSPVLIDFFEDTER_841.9_512.3
   AALAAFNAQNNGSNFQLEEISR_789.1_746.4
   DGSPDVTTADIGANTPDATK_973.5_844.4
   ILILPSVTR_506.3_785.5
   SVSLPSLDPASAK_636.4_885.5
   TLAFVR_353.7_492.3
   VAPGVANPGTPLA_582.3_555.3
   HELTDEELQSLFTNFANWDK_817.1_906.5
   AGLLRPDYALLGHR_518.0_369.2
   GDTYPAELYITGSILR_885.0_1332.8
   LFIPQITR_494.3_727.4
   GYQELLEK_490.3_631.4
   VSEADSSNADWVTK_754.9_533.3
   LNIGYIEDLK_589.3_950.5
   SFRPFVPR_335.9_272.2
   ILDGGNK_358.7_490.2
   EANQSTLENFLER_775.9_678.4
   DFNQFSSGEK_386.8_189.1
   IEEIAAK_387.2_660.4
   TEFLSNYLTNVDDITLVPGTLGR_846.8_600.3
   LPATEKPVLLSK_432.6_347.2
   FLQEQGHR_338.8_369.2
   APLTKPLK_289.9_357.2
   YSHYNER_323.5_418.2
   TSYQVYSK_488.2_787.4
   IALGGLLFPASNLR_481.3_657.4
   TGISPLALIK_506.8_741.5
   LYYGDDEK_501.7_726.3
   IVLSLDVPIGLLQILLEQAR_735.1_503.3
   EAQLPVIENK_570.8_329.2
   TGYYFDGISR_589.8_857.4
   VGVISFAQK_474.8_580.3
   FQSVFTVTR_542.8_623.4
   TSDQIHFFFAK_447.6_659.4
   IAPQLSTEELVSLGEK_857.5_333.2
   EVFSKPISWEELLQ_852.9_260.2
   DEIPHNDIALLK_459.9_260.2
   NYFTSVAHPNLFIATK_608.3_319.2
   ITENDIQIALDDAK_779.9_632.3
   DTYVSSFPR_357.8_272.2
   TDAPDLPEENQAR_728.3_843.4
   LFYADHPFIFLVR_546.6_647.4
   TEQAAVAR_423.2_487.3
   AVGYLITGYQR_620.8_737.4
   HSHESQDLR_370.2_288.2
   IALGGLLFPASNLR_481.3_412.3
   IAQYYYTFK_598.8_884.4
   SLPVSDSVLSGFEQR_810.9_723.3
   IIGGSDADIK_494.8_762.4
   QTLSWTVTPK_580.8_545.3
   HYFIAAVER_553.3_658.4
   QVCADPSEEWVQK_788.4_374.2
   DLHLSDVFLK_396.2_366.2
   NIQSVNVK_451.3_546.3
   QTLSWTVTPK_580.8_818.4
   HSHESQDLR_370.2_403.2
   LLEVPEGR_456.8_356.2
   LIENGYFHPVK_439.6_343.2
   EDTPNSVWEPAK_686.8_630.3
   AFTECCVVASQLR_770.9_673.4
   VNHVTLSQPK_374.9_459.3
   VSFSSPLVAISGVALR_802.0_715.4
   DPDQTDGLGLSYLSSHIANVER_796.4_456.2
   VVGGLVALR_442.3_784.5
   NEIVFPAGILQAPFYTR_968.5_357.2
   QVCADPSEEWVQK_788.4_275.2
   LQDAGVYR_461.2_680.3
   IQTHSTTYR_369.5_540.3
   TPSAAYLWVGTGASEAEK_919.5_849.4
   ALALPPLGLAPLLNLWAKPQGR_770.5_256.2
   ALQDQLVLVAAK_634.9_289.2
   IEEIAAK_387.2_531.3
   TAVTANLDIR_537.3_288.2
   VSEADSSNADWVTK_754.9_347.2
   ITENDIQIALDDAK_779.9_873.5
   SSNNPHSPIVEEFQVPYNK_729.4_521.3
   HPWIVHWDQLPQYQLNR_744.0_918.5
   TPSAAYLWVGTGASEAEK_919.5_428.2
   AFLEVNEEGSEAAASTAVVIAGR_764.4_614.4
   WGAAPYR_410.7_634.3
   IEVNESGTVASSSTAVIVSAR_693.0_545.3
   AEAQAQYSAAVAK_654.3_908.5
   VPLALFALNR_557.3_917.6
   TLEAQLTPR_514.8_685.4
   QALEEFQK_496.8_551.3
   WNFAYWAAHQPWSR_607.3_545.3
   IYLQPGR_423.7_570.3
   FFQYDTWK_567.8_840.4
   NEIWYR_440.7_357.2
   GGEGTGYFVDFSVR_745.9_722.4
   VGEYSLYIGR_578.8_708.4
   TAHISGLPPSTDFIVYLSGLAPSIR_871.5_800.5
   GVTGYFTFNLYLK_508.3_260.2
   DYWSTVK_449.7_620.3
   YENYTSSFFIR_713.8_756.4
   YGLVTYATYPK_638.3_334.2
   LFIPQITR_494.3_614.4
   YEFLNGR_449.7_606.3
   SEPRPGVLLR_375.2_454.3
   NSDQEIDFK_548.3_294.2
   YEVQGEVFTKPQLWP_911.0_293.1
   GVTGYFTFNLYLK_508.3_683.9
   ISLLLIESWLEPVR_834.5_371.2
   ALLLGWVPTR_563.3_373.2
   VNHVTLSQPK_374.9_244.2
   SGAQATWTELPWPHEK_613.3_793.4
   AQPVQVAEGSEPDGFWEALGGK_758.0_623.4
   DLPHITVDR_533.3_490.3
   SEYGAALAWEK_612.8_788.4
   AVGYLITGYQR_620.8_523.3
   GFQALGDAADIR_617.3_717.4
   YEVQGEVFTKPQLWP_911.0_392.2
   SDGAKPGPR_442.7_459.2
   GFQALGDAADIR_617.3_288.2
   IAPQLSTEELVSLGEK_857.5_533.3
   DTDTGALLFIGK_625.8_217.1
   LHEAFSPVSYQHDLALLR_699.4_380.2
   IYLQPGR_423.7_329.2
   SEYGAALAWEK_612.8_788.4
   LLAPSDSPEWLSFDVTGWR_730.1_430.3
   ALNHLPLEYNSALYSR_621.0_696.4
   ELPQSIVYK_538.8_417.7
   VEHSDLSFSK_383.5_234.1
   VFQFLEK_455.8_811.4
   VNHVTLSQPK_374.9_244.2
   YGIEEHGK_311.5_599.3
   TLAFVR_353.7_492.3
   VLEPTLK_400.3_587.3
   VLEPTLK_400.3_458.3
   DALSSVQESQVAQQAR_573.0_502.3
   AQPVQVAEGSEPDGFWEALGGK_758.0_574.3
   LHEAFSPVSYQHDLALLR_699.4_251.2
   SEPRPGVLLR_375.2_654.4
   VEHSDLSFSK_383.5_468.2
   SLDFTELDVAAEK_719.4_316.2
   SEYGAALAWEK_612.8_845.5
   TYLHTYESEI_628.3_515.3
   VNHVTLSQPK_374.9_459.3
   IEEIAAK_387.2_660.4
   ALNHLPLEYNSALYSR_621.0_538.3
   TLLPVSKPEIR_418.3_514.3
   TEQAAVAR_423.2_615.4
   DLHLSDVFLK_396.2_260.2
   ALQDQLVLVAAK_634.9_289.2
   SLQAFVAVAAR_566.8_804.5
   QGHNSVFLIK_381.6_260.2
   ALQDQLVLVAAK_634.9_956.6
   SLDFTELDVAAEK_719.4_874.5
   SEYGAALAWEK_612.8_788.4
   ALNHLPLEYNSALYSR_621.0_696.4
   LLAPSDSPEWLSFDVTGWR_730.1_430.3
   ANLINNIFELAGLGK_793.9_299.2
   VFQFLEK_455.8_811.4
   VEHSDLSFSK_383.5_234.1
   ELPQSIVYK_538.8_417.7
   LNIGYIEDLK_589.3_950.5
   VLEPTLK_400.3_587.3
   VNHVTLSQPK_374.9_244.2
   VLEPTLK_400.3_458.3
   AQPVQVAEGSEPDGFWEALGGK_758.0_574.3
   TLFIFGVTK_513.3_811.5
   YGIEEHGK_311.5_599.3
   VPLALFALNR_557.3_620.4
   EVFSKPISWEELLQ_852.9_376.2
   VEHSDLSFSK_383.5_468.2
   TLAFVR_353.7_492.3
   DALSSVQESQVAQQAR_573.0_502.3
   SEYGAALAWEK_612.8_845.5
   SLDFTELDVAAEK_719.4_316.2
   SEPRPGVLLR_375.2_654.4
   LHEAFSPVSYQHDLALLR_699.4_251.2
   TYLHTYESEI_628.3_515.3
   AVDIPGLEAATPYR_736.9_399.2
   FSLVSGWGQLLDR_4 93.3_403.2
   GEVTYTTSQVSK_650.3_750.4
   VNHVTLSQPK_374.9_459.3
   HFQNLGK_422.2_527.2
   YGIEEHGK_311.5_341.2
   SEYGAALAWEK_612.8_788.4
   LLAPSDSPEWLSFDVTGWR_730.1_430.3
   VFQFLEK_455.8_811.4
   ANLINNIFELAGLGK_793.9_299.2
   VEHSDLSFSK_383.5_234.1
   ELPQSIVYK_538.8_417.7
   LNIGYIEDLK_589.3_950.5
   VLEPTLK_400.3_458.3
   VPLALFALNR_557.3_620.4
   TLAFVR_353.7_492.3
   TLFIFGVTK_513.3_811.5
   AQPVQVAEGSEPDGFWEALGGK_758.0_574.3
   DALSSVQESQVAQQAR_573.0_502.3
   YGIEEHGK_311.5_599.3
   EVFSKPISWEELLQ_852.9_376.2
   LHEAFSPVSYQHDLALLR_699.4_251.2
   SLDFTELDVAAEK_719.4_316.2
   TYLHTYESEI_628.3_515.3
   HFQNLGK_422.2_527.2
   AVDIPGLEAATPYR_736.9_399.2
   TLNAYDHR_330.5_312.2
   DVLLLVHNLPQNLTGHIWYK_791.8_310.2
   GPITSAAELNDPQSILLR_632.4_826.5
   VVLSSGSGPGLDLPLVLGLPLQLK_791.5_598.4
   SLQNASAIESILK_687.4_860.5
   HYINLITR_515.3_301.1
   VELAPLPSWQPVGK_760.9_342.2
   LQVNTPLVGASLLR_741.0_925.6
   TLEAQLTPR_514.8_814.4
   SDGAKPGPR_442.7_459.2
   SEYGAALAWEK_612.8_788.4
   ALNHLPLEYNSALYSR_621.0_696.4
   VFQFLEK_455.8_811.4
   LLAPSDSPEWLSFDVTGWR_730.1_430.3
   NADYSYSVWK_616.8_769.4
   VLEPTLK_400.3_458.3
   VEHSDLSFSK_383.5_234.1
   ELPQSIVYK_538.8_417.7
   VLEPTLK_400.3_587.3
   TLFIFGVTK_513.3_811.5
   ANLINNIFELAGLGK_793.9_299.2
   LNIGYIEDLK_589.3_950.5
   SEYGAALAWEK_612.8_845.5
   VNHVTLSQPK_374.9_244.2
   YGIEEHGK_311.5_599.3
   HFQNLGK_422.2_527.2
   AGITIPR_364.2_486.3
   AQPVQVAEGSEPDGFWEALGGK_758.0_574.3
   SLDFTELDVAAEK_719.4_316.2
   TLAFVR_353.7_492.3
   SEPRPGVLLR_375.2_654.4
   TLLIANETLR_572.3_703.4
   ALNHLPLEYNSALYSR_621.0_538.3
   LHEAFSPVSYQHDLALLR_699.4_251.2
   DGSPDVTTADIGANTPDATK_973.5_844.4
   VPLALFALNR_557.3_620.4
   AVDIPGLEAATPYR_736.9_399.2
   YGIEEHGK_311.5_341.2
   FSLVSGWGQLLDR_493.3_403.2
   IEEIAAK_387.2_660.4
   VPLALFALNR_557.3_620.4
   VFQFLEK_455.8_811.4
   AQPVQVAEGSEPDGFWEALGGK_758.0_574.3
   LIEIANHVDK_384.6_498.3
   DALSSVQESQVAQQAR_573.0_502.3
   TLAFVR_353.7_492.3
   ALNHLPLEYNSALYSR_621.0_696.4
   AVYEAVLR_460.8_587.4
   SEPRPGVLLR_375.2_654.4
   TYLHTYESEI_628.3_515.3
   ALNHLPLEYNSALYSR_621.0_538.3
   LIEIANHVDK_384.6_683.4
   YGIEEHGK_311.5_599.3
   VLEPTLK_400.3_587.3
   SEYGAALAWEK_612.8_788.4
   WSAGLTSSQVDLYIPK_883.0_515.3
   FGFGGSTDSGPIR_649.3_946.5
   SEYGAALAWEK_612.8_845.5
   QINSYVK_426.2_496.3
   QINSYVK_426.2_610.3
   TEQAAVAR_423.2_615.4
   AVYEAVLR_460.8_750.4
   TEQAAVAR_423.2_487.3
   DFNQFSSGEK_386.8_333.2
   SLQAFVAVAAR_566.8_804.5
   SLDFTELDVAAEK_719.4_316.2
   DPTFIPAPIQAK_433.2_556.3
   DPTFIPAPIQAK_433.2_461.2
   VLEPTLK_400.3_458.3
   YENYTSSFFIR_713.8_756.4
   VPLALFALNR_557.3_620.4
   VFQFLEK_455.8_811.4
   DALSSVQESQVAQQAR_573.0_502.3
   EVFSKPISWEELLQ_852.9_376.2
   AQPVQVAEGSEPDGFWEALGGK_758.0_574.3
   TLAFVR_353.7_492.3
   ANLINNIFELAGLGK_793.9_299.2
   ALNHLPLEYNSALYSR_621.0_538.3
   ELPQSIVYK_538.8_417.7
   LLAPSDSPEWLSFDVTGWR_730.1_430.3
   YGIEEHGK_311.5_341.2
   ALNHLPLEYNSALYSR_621.0_696.4
   TQILEWAAER_608.8_761.4
   AVYEAVLR_460.8_587.4
   AEIEYLEK_497.8_552.3
   SEPRPGVLLR_375.2_654.4
   AVDIPGLEAATPYR_736.9_399.2
   LIEIANHVDK_384.6_498.3
   YGIEEHGK_311.5_599.3
   VLEPTLK_400.3_587.3
   DVLLLVHNLPQNLTGHIWYK_791.8_883.0
   TAHISGLPPSTDFIVYLSGLAPSIR_871.5_472.3
   TLNAYDHR_330.5_312.2
   FGFGGSTDSGPIR_649.3_946.5
   LIEIANHVDK_384.6_683.4
   SEYGAALAWEK_612.8_788.4
   TYLHTYESEI_628.3_515.3
   FGFGGSTDSGPIR_649.3_745.4
   SEYGAALAWEK_612.8_845.5
   TPSAAYLWVGTGASEAEK_919.5_849.4
   VPLALFALNR_557.3_620.4
   ANLINNIFELAGLGK_793.9_299.2
   VFQFLEK_455.8_811.4
   AQPVQVAEGSEPDGFWEALGGK_758.0_574.3
   ALNHLPLEYNSALYSR_621.0_696.4
   TQILEWAAER_608.8_761.4
   ELPQSIVYK_538.8_417.7
   TGVAVNKPAEFTVDAK_549.6_258.1
   SEPRPGVLLR_375.2_654.4
   DALSSVQESQVAQQAR_573.0_502.3
   EVFSKPISWEELLQ_852.9_376.2
   LIEIANHVDK_384.6_683.4
   VLEPTLK_400.3_587.3
   TEQAAVAR_423.2_615.4
   LLAPSDSPEWLSFDVTGWR_730.1_430.3
   TLNAYDHR_330.5_312.2
   AVDIPGLEAATPYR_736.9_399.2
   DVLLLVHNLPQNLTGHIWYK_791.8_883.0
   AEIEYLEK_497.8_552.3
   TYLHTYESEI_628.3_515.3
   AGLLRPDYALLGHR_518.0_595.4
   YGIEEHGK_311.5_599.3
   QINSYVK_426.2_496.3
   LTTVDIVTLR_565.8_815.5
   AALAAFNAQNNGSNFQLEEISR_789.1_746.4
   AEVIWTSSDHQVLSGK_586.3_300.2
   ALEQDLPVNIK_620.4_570.4
   DFNQFSSGEK_386.8_333.2
   TSYQVYSK_488.2_787.4
   TLEAQLTPR_514.8_685.4
   VPLALFALNR_557.3_620.4
   VFQFLEK_455.8_811.4
   ALNHLPLEYNSALYSR_621.0_538.3
   YGIEEHGK_311.5_341.2
   ALNHLPLEYNSALYSR_621.0_696.4
   AQPVQVAEGSEPDGFWEALGGK_758.0_574.3
   ANLINNIFELAGLGK_793.9_299.2
   TQILEWAAER_608.8_761.4
   AVYEAVLR_460.8_587.4
   TLAFVR_353.7_492.3
   SEPRPGVLLR_375.2_654.4
   LIEIANHVDK_384.6_683.4
   ELPQSIVYK_538.8_417.7
   EVFSKPISWEELLQ_852.9_376.2
   LIEIANHVDK_384.6_498.3
   VLEPTLK_400.3_587.3
   YGIEEHGK_311.5_599.3
   DVLLLVHNLPQNLTGHIWYK_791.8_883.0
   TYLHTYESEI_628.3_515.3
   DALSSVQESQVAQQAR_573.0_502.3
   FGFGGSTDSGPIR_649.3_745.4
   AVDIPGLEAATPYR_736.9_399.2
   TGVAVNKPAEFTVDAK_549.6_258.1
   TLNAYDHR_330.5_312.2
   IYLQPGR_423.7_329.2
   AEVIWTSSDHQVLSGK_586.3_300.2
   VPSHAVVAR_312.5_515.3
   SEYGAALAWEK_612.8_845.5
   FGFGGSTDSGPIR_649.3_946.5
   SEYGAALAWEK_612.8_788.4
   AVYEAVLR_460.8_587.4
   AEIEYLEK_497.8_552.3
   AALAAFNAQNNGSNFQLEEISR_789.1_746.4
   TGVAVNKPAEFTVDAK_549.6_258.1
   AVYEAVLR_460.8_750.4
   LIEIANHVDK_384.6_683.4
   VPLALFALNR_557.3_620.4
   QINSYVK_426.2_610.3
   ITQDAQLK_458.8_702.4
   FGFGGSTDSGPIR_649.3_745.4
   LIEIANHVDK_384.6_498.3
   QINSYVK_426.2_496.3
   YSHYNER_323.5_418.2
   YYGYTGAFR_549.3_450.3
   VGVISFAQK_474.8_580.3
   VFQYIDLHQDEFVQTLK_708.4_375.2
   SPEAEDPLGVER_649.8_314.1
   DEIPHNDIALLK_459.9_510.8
   ALEQDLPVNIK_620.4_798.5
   ALEQDLPVNIK_620.4_570.4
   ILDGGNK_358.7_603.3
   TSYQVYSK_488.2_787.4
   YENYTSSFFIR_713.8_756.4
   SETEIHQGFQHLHQLFAK_717.4_447.2
   TLPFSR_360.7_506.3
   VLSSIEQK_452.3_691.4
   AEIEYLEK_497.8_389.2
   GTYLYNDCPGPGQDTDCR_697.0_666.3
   SGVDLADSNQK_567.3_662.3
   SGVDLADSNQK_567.3_591.3
   AVYEAVLR_460.8_587.4
   AEIEYLEK_497.8_552.3
   TGVAVNKPAEFTVDAK_549.6_258.1
   AVYEAVLR_460.8_750.4
   AALAAFNAQNNGSNFQLEEISR_789.1_746.4
   AYSDLSR_406.2_375.2
   VPLALFALNR_557.3_620.4
   QINSYVK_426.2_610.3
   LIEIANHVDK_384.6_498.3
   FGFGGSTDSGPIR_649.3_745.4
   WSAGLTSSQVDLYIPK_883.0_515.3
   ITQDAQLK_458.8_702.4
   LIEIANHVDK_384.6_683.4
   YYGYTGAFR_549.3_450.3
   VFQYIDLHQDEFVQTLK_708.4_375.2
   FQLPGQK_409.2_429.2
   AVDIPGLEAATPYR_736.9_286.1
   QINSYVK_426.2_496.3
   YISPDQLADLYK_713.4_277.2
   TLPFSR_360.7_506.3
   SPEAEDPLGVER_649.8_314.1
   ALEQDLPVNIK_620.4_798.5
   YSHYNER_323.5_418.2
   VGVISFAQK_474.8_580.3
   LQDAGVYR_461.2_680.3
   AEIEYLEK_497.8_389.2
   TSYQVYSK_488.2_787.4
   SWLIPLGAVDDGEHSQNEK_703.0_798.4
   ALEQDLPVNIK_620.4_570.4
   ILDGGNK_358.7_603.3
   AVYEAVLR_460.8_587.4
   AEIEYLEK_497.8_552.3
   AALAAFNAQNNGSNFQLEEISR_789.1_746.4
   TGVAVNKPAEFTVDAK_549.6_258.1
   AYSDLSR_406.2_375.2
   LIEIANHVDK_384.6_683.4
   QINSYVK_426.2_496.3
   ALEQDLPVNIK_620.4_798.5
   YISPDQLADLYK_713.4_277.2
   AVDIPGLEAATPYR_736.9_286.1
   SPEAEDPLGVER_649.8_314.1
   ILDGGNK_358.7_603.3
   VGVISFAQK_474.8_580.3
   YYGYTGAFR_549.3_450.3
   YSHYNER_323.5_418.2
   FQLPGQK_409.2_276.1
   YENYTSSFFIR_713.8_756.4
   TEQAAVAR_423.2_615.4
   SGVDLADSNQK_567.3_662.3
   VLSSIEQK_452.3_691.4
   GTYLYNDCPGPGQDTDCR_697.0_666.3
   TSYQVYSK_488.2_787.4
   TLEAQLTPR_514.8_685.4
   AEVIWTSSDHQVLSGK_586.3_300.2
   TAVTANLDIR_537.3_288.2
   ITENDIQIALDDAK_779.9_632.3
   TASDFITK_441.7_781.4
   SPQAFYR_434.7_556.3
   NHYTESISVAK_624.8_415.2
   LTTVDIVTLR_565.8_815.5
   AVYEAVLR_460.8_587.4
   AEIEYLEK_497.8_552.3
   AALAAFNAQNNGSNFQLEEISR_789.1_746.4
   TGVAVNKPAEFTVDAK_549.6_258.1
   AVYEAVLR_460.8_750.4
   AYSDLSR_406.2_375.2
   VPLALFALNR_557.3_620.4
   LIEIANHVDK_384.6_683.4
   ITQDAQLK_458.8_702.4
   WSAGLTSSQVDLYIPK_883.0_515.3
   FGFGGSTDSGPIR_649.3_745.4
   LIEIANHVDK_384.6_498.3
   QINSYVK_426.2_610.3
   QINSYVK_426.2_496.3
   FQLPGQK_409.2_429.2
   VFQYIDLHQDEFVQTLK_708.4_375.2
   ALEQDLPVNIK_620.4_798.5
   YYGYTGAFR_549.3_450.3
   ALFLDALGPPAVTR_720.9_640.4
   FQLPGQK_409.2_276.1
   SETEIHQGFQHLHQLFAK_717.4_447.2
   ALEQDLPVNIK_620.4_570.4
   VPSHAVVAR_312.5_515.3
   AVDIPGLEAATPYR_736.9_286.1
   FTFTLHLETPKPSISSSNLNPR_829.4_874.4
   VGVISFAQK_474.8_580.3
   TLPFSR_360.7_506.3
   SLDFTELDVAAEK_719.4_874.5
   SPEAEDPLGVER_649.8_314.1
   YGIEEHGK_311.5_341.2
   LIQDAVTGLTVNGQITGDK_972.0_798.4
   VQTAHFK_277.5_431.2
   FLNWIK_410.7_560.3
   ITGFLKPGK_320.9_429.3
   ALNHLPLEYNSALYSR_621.0_538.3
   TYLHTYESEI_628.3_908.4
   LIENGYFHPVK_439.6_627.4
   AVLHIGEK_289.5_292.2
   QALEEFQK_496.8_680.3
   TEFLSNYLTNVDDITLVPGTLGR_846.8_600.3
   TASDFITK_441.7_781.4
   LPNNVLQEK_527.8_844.5
   AHYDLR_387.7_288.2
   ITLPDFTGDLR_624.3_288.2
   IEGNLIFDPNNYLPK_874.0_414.2
   ITGFLKPGK_320.9_301.2
   FSWYAK_407.2_381.2
   ITGFLKPGK_320.9_429.3
   VFQFLEK_455.8_811.4
   LIQDAVTGLTVNGQITGDK_972.0_798.4
   DADPDTFFAK_563.8_825.4
   VPLALFALNR_557.3_620.4
   VFQFLEK_455.8_811.4
   AQPVQVAEGSEPDGFWEALGGK_758.0_574.3
   LIEIANHVDK_384.6_498.3
   TLAFVR_353.7_492.3
   ALNHLPLEYNSALYSR_621.0_696.4
   AVYEAVLR_460.8_587.4
   SEPRPGVLLR_375.2_654.4
   TYLHTYESEI_628.3_515.3
   ALNHLPLEYNSALYSR_621.0_538.3
   SPELQAEAK_486.8_659.4
   ALALPPLGLAPLLNLWAKPQGR_770.5_457.3
   ALALPPLGLAPLLNLWAKPQGR_770.5_256.2
   SPELQAEAK_486.8_788.4
   WLSSGSGPGLDLPLVLGLPLQLK_7 91.5_768.5
   WLSSGSGPGLDLPLVLGLPLQLK_791.5_598.4
   DYWSTVK_449.7_347.2
   DYWSTVK_449.7_620.3
   WGAAPYR_410.7_634.3
   DALSSVQESQVAQQAR_573.0_502.3
   DALSSVQESQVAQQAR_573.0_672.4
   LSIPQITTK_500.8_687.4
   GWVTDGFSSLK_598.8_854.4
   IALGGLLFPASNLR_481.3_657.4
   AKPALEDLR_506.8_813.5
   WGAAPYR_410.7_577.3
   VPLALFALNR_557.3_620.4
   FSLVSGWGQLLDR_493.3_447.3
   LSIPQITTK_500.8_800.5
   TLAFVR_353.7_274.2
   DVLLLVHNLPQNLPGYFWYK_810.4_960.5
   FSWYAK_407.2_381.2
   DVLLLVHNLPQNLPGYFWYK_810.4_215.1
   SEPRPGVLLR_375.2_654.4
   ALNHLPLEYNSALYSR_621.0_538.3
   WNFAYWAAHQPWSR_607.3_545.3
   ALNHLPLEYNSALYSR_621.0_696.4
   AQPVQVAEGSEPDGFWEALGGK_758.0_623.4
   GWVTDGFSSLK_598.8_953.5
   IALGGLLFPASNLR_481.3_412.3
   WNFAYWAAHQPWSR_607.3_673.3
   FGFGGSTDSGPIR_649.3_745.4
   SEYGAALAWEK_612.8_788.4
   DADPDTFFAK_563.8_302.1
   LFIPQITR_494.3_614.4
   DVLLLVHNLPQNLPGYFWYK_810.4_328.2
   TLAFVR_353.7_492.3
   AVLHIGEK_289.5_292.2
   IHPSYTNYR_384.2_452.2
   AGLLRPDYALLGHR_518.0_369.2
   ILILPSVTR_506.3_559.3
   YYLQGAK_421.7_516.3
   TPSAAYLWVGTGASEAEK_919.5_849.4
   AGLLRPDYALLGHR_518.0_595.4
   TYLHTYESEI_628.3_908.4
   FSLVSGWGQLLDR_493.3_403.2
   VNFTEIQK_489.8_765.4
   LFIPQITR_494.3_727.4
   DISEVVTPR_508.3_787.4
   SEPRPGVLLR_375.2_454.3
   SPELQAEAK_486.8_659.4
   DYWSTVK_449.7_347.2
   DYWSTVK_449.7_620.3
   DALSSVQESQVAQQAR_573.0_502.3
   GWVTDGFSSLK_598.8_854.4
   SPELQAEAK_486.8_788.4
   SEPRPGVLLR_375.2_654.4
   DALSSVQESQVAQQAR_573.0_672.4
   SEYGAALAWEK_612.8_788.4
   GWVTDGFSSLK_598.8_953.5
   ALNHLPLEYNSALYSR_621.0_696.4
   TYLHTYESEI_628.3_515.3
   ENPAVIDFELAPIVDLVR_670.7_601.4
   ALNHLPLEYNSALYSR_621.0_538.3
   TYLHTYESEI_628.3_908.4
   AGLLRPDYALLGHR_518.0_369.2
   AKPALEDLR_506.8_813.5
   SEYGAALAWEK_612.8_845.5
   FSLVSGWGQLLDR_493.3_447.3
   WGAAPYR_410.7_634.3
   ENPAVIDFELAPIVDLVR_670.7_811.5
   DVLLLVHNLPQNLPGYFWYK_810.4_215.1
   LFIPQITR_494.3_614.4
   WGAAPYR_410.7_577.3
   AGLLRPDYALLGHR_518.0_595.4
   TLAFVR_353.7_492.3
   FSLVSGWGQLLDR_493.3_403.2
   WWGGQPLWITATK_772.4_373.2
   GYVIIKPLVWV_643.9_304.2
   DVLLLVHNLPQNLPGYFWYK_810.4_960.5
   FGFGGSTDSGPIR_649.3_745.4
   DVLLLVHNLPQNLPGYFWYK_810.4_328.2
   WWGGQPLWITATK_772.4_929.5
   LDFHFSSDR_375.2_448.2
   LFIPQITR_494.3_727.4
   TEFLSNYLTNVDDITLVPGTLGR_846.8_600.3
   LSIPQITTK_500.8_800.5
   ALVLELAK_428.8_672.4
   SEPRPGVLLR_375.2_454.3
   LSIPQITTK_500.8_687.4
   LDFHFSSDR_375.2_611.3
   LDFHFSSDR_375.2_464.2
   GAVHVVVAETDYQSFAVLYLER_822.8_580.3
   HELTDEELQSLFTNFANWDK_817.1_854.4
   AYSDLSR_406.2_375.2
   YYLQGAK_421.7_516.3
   ILPSVPK_377.2_264.2
   ELLESYIDGR_597.8_710.4
   ALALPPLGLAPLLNLWAKPQGR_770.5_256.2
   VGEYSLYIGR_578.8_871.5
   ANDQYLTAAALHNLDEAVK_686.4_317.2
   YYGYTGAFR_549.3_551.3
   ENPAVIDFELAPIVDLVR_670.7_811.5
   ENPAVIDFELAPIVDLVR_670.7_601.4
   TYLHTYESEI_628.3_908.4
   TYLHTYESEI_628.3_515.3
   TEFLSNYLTNVDDITLVPGTLGR_846.8_600.3
   TEFLSNYLTNVDDITLVPGTLGR_846.8_699.4
   WWGGQPLWITATK_772.4_373.2
   WWGGQPLWITATK_772.4_929.5
   SEYGAALAWEK_612.8_788.4
   LDFHFSSDR_375.2_448.2
   ALVLELAK_428.8_672.4
   GLQYAAQEGLLALQSELLR_1037.1_929.5
   DVLLLVHNLPQNLPGYFWYK_810.4_960.5
   FGFGGSTDSGPIR_649.3_745.4
   DVLLLVHNLPQNLPGYFWYK_810.4_328.2
   GYVIIKPLVWV_643.9_304.2
   SPELQAEAK_486.8_659.4
   GLQYAAQEGLLALQSELLR_1037.1_858.5
   LDFHFSSDR_375.2_611.3
   LIEIANHVDK_384.6_683.4
   SFRPFVPR_335.9_272.2
   SFRPFVPR_335.9_635.3
   WSAGLTSSQVDLYIPK_883.0_515.3
   DLHLSDVFLK_396.2_260.2
   LIEIANHVDK_384.6_498.3
   DVLLLVHNLPQNLPGYFWYK_810.4_215.1
   LFIPQITR_494.3_614.4
   FGFGGSTDSGPIR_649.3_946.5
   YYLQGAK_421.7_516.3
   SEYGAALAWEK_612.8_845.5
   AITPPHPASQANIIFDITEGNLR_825.8_459.3
   LDFHFSSDR_375.2_464.2
   HELTDEELQSLFTNFANWDK_817.1_854.4
   ALNHLPLEYNSALYSR_621.0_696.4
   ITQDAQLK_458.8_702.4
   ITLPDFTGDLR_624.3_920.5
   SILFLGK_389.2_577.4
   WSAGLTSSQVDLYIPK_883.0_357.2
   GVTSVSQIFHSPDLAIR_609.7_472.3
   VGEYSLYIGR_578.8_871.5
   ITLPDFTGDLR_624.3_288.2
   YYLQGAK_421.7_327.1
   ALNHLPLEYNSALYSR_621.0_538.3
   DYWSTVK_449.7_620.3
   TVQAVLTVPK_528.3_855.5
   ITGFLKPGK_320.9_301.2
   LFIPQITR_494.3_727.4
   SPELQAEAK_486.8_788.4
   DYWSTVK_449.7_347.2
   ITGFLKPGK_320.9_429.3
   FLNWIK_410.7_560.3
   DLHLSDVFLK_396.2_366.2
   GVTSVSQIFHSPDLAIR_609.7_908.5
   VEHSDLSFSK_383.5_468.2
   LLDSLPSDTR_558.8_276.2
   LLDSLPSDTR_558.8_890.4
   QALEEFQK_496.8_551.3
   LLDSLPSDTR_558.8_575.3
   IIGGSDADIK_494.8_762.4
   AFIQLWAFDAVK_704.9_650.4
   GFQALGDAADIR_617.3_717.4
   LPNNVLQEK_527.8_730.4
   DTDTGALLFIGK_625.8_217.1
   VQTAHFK_277.5_502.3
   ILILPSVTR_506.3_559.3
   SILFLGK_389.2_201.1
   TVQAVLTVPK_528.3_428.3
   VEPLYELVTATDFAYSSTVR_754.4_712.4
   FSLVSGWGQLLDR_4 93. 3_447.3
   GWVTDGFSSLK_598.8_854.4
   LSETNR_36 0.2_519.3
   TQILEWAAER_608.8_632.3
   SETEIHQGFQHLHQLFAK_717.4_318.1
   TNLESILSYPK_632.8_936.5
   TNLESILSYPK_632.8_807.5
   AYSDLSR_406.2_375.2
   YEVQGEVFTKPQLWP_911.0_392.2
   AYSDLSR_406.2_577.3
   QALEEFQK_496.8_680.3
   APLTKPLK_289.9_398.8
   FQPTLLTLPR_593.4_276.1
   ITQDAQLK_458.8_803.4
   AVLHIGEK_289.5_292.2
   ANDQYLTAAALHNLDEAVK_686.4_317.2
   VGEYSLYIGR_578.8_708.4
   TLYSSSPR_455.7_533.3
   VEHSDLSFSK_383.5_234.1
   HELTDEELQSLFTNFANWDK_817.1_906.5
   TLYSSSPR_455.7_696.3
   GYVIIKPLVWV_643.9_854.6
   DEIPHNDIALLK_459.9_260.2
   ILILPSVTR_506.3_785.5
   WNFAYWAAHQPWSR_607.3_545.3
   FQPTLLTLPR_593.4_712.5
   AQPVQVAEGSEPDGFWEALGGK_758.0_623.4
   DEIPHNDIALLK_459.9_510.8
   GDSGGAFAVQDPNDK_739.3_716.3
   FLNWIK_410.7_561.3
   APLTKPLK_289.9_357.2
   ALDLSLK_380.2_185.1
   GWVTDGFSSLK_598.8_953.5
   SETEIHQGFQHLHQLFAK_717.4_447.2
   GDSGGAFAVQDPNDK_739.3_473.2
   YEVQGEVFTKPQLWP_911.0_293.1
   HVVQLR_376.2_614.4
   DTDTGALLFIGK_625.8_818.5
   EVPLSALTNILSAQLISHWK_740.8_996.6
   AFTECCVVASQLR_770.9_574.3
   TLAFVR_353.7_492.3
   LWAYLTIQELLAK_781.5_300.2
   DEIPHNDIALLK_459.9_245.1
   AGLLRPDYALLGHR_518.0_369.2
   AVYEAVLR_460.8_587.4
   LPNNVLQEK_527.8_844.5
   GAVHVVVAETDYQSFAVLYLER_822.8_580.3
   WNFAYWAAHQPWSR_607.3_673.3
   EAQLPVIENK_570.8_329.2
   VQEAHLTEDQIFYFPK-655.7-701.4
   AFIQLWAFDAVK_704.9_836.4
   SGFSFGFK_438.7_585.3
   SGFSFGFK_438.7_732.4
   DADPDTFFAK_563.8_302.1
   EAQLPVIENK_570.8_699.4
   IIGGSDADIK_494.8_260.2
   AVLTIDEK_444.8_718.4
   SEPRPGVLLR_375.2_654.4
   YHFEALADTGISSEFYDNANDLLSK_940.8_874.5
   HFQNLGK_422.2_527.2
   LEQGENVFLQATDK_796.4_822.4
   NTVISVNPSTK_580.3_732.4
   YYGYTGAFR_549.3_551.3
   ISLLLIESWLEPVR_834.5_500.3
   LQVLGK_32 9.2_416.3
   IALGGLLFPASNLR_481.3_657.4
   IALGGLLFPASNLR_481.3_412.3
   DALSSVQESQVAQQAR_573.0_672.4
   AVLHIGEK_289.5_292.2
   DVLLLVHNLPQNLPGYFWYK_810.4_960.5
   DALSSVQESQVAQQAR_573.0_502.3
   LFIPQITR_494.3_614.4
   DALSSVQESQVAQQAR_573.0_672.4
   DALSSVQESQVAQQAR_573.0_502.3
   LFIPQITR_494.3_614.4
   LFIPQITR_494.3_727.4
   DVLLLVHNLPQNLPGYFWYK_810.4_960.5
   AVLHIGEK_289.5_292.2
   ALNHLPLEYNSALYSR_621.0_696.4
   TYLHTYESEI_628.3_908.4
   FGFGGSTDSGPIR_649.3_946.5
   FGFGGSTDSGPIR_649.3_745.4
   TLFIFGVTK_513.3_215.1
   TLFIFGVTK_513.3_811.5
   ILILPSVTR_506.3_785.5
   ILILPSVTR_506.3_559.3
   FQLPGQK_409.2_429.2
   LFIPQITPK_528.8_261.2
   FQLPGQK_40 9.2_276.1
   LFIPQITPK_528.8_683.4
   SNPVTLNVLYGPDLPR_585.7_817.4
   SNPVTLNVLYGPDLPR_585.7_654.4
   SGVDLADSNQK_567.3_662.3
   ALVLELAK_428.8_331.2
   IHPSYTNYR_384.2_452.2
   LFIPQITR_494.3_727.4
   SGVDLADSNQK_567.3_591.3
   LFIPQITR_494.3_614.4
   DVLLLVHNLPQNLPGYFWYK_810.4_960.5
   IHPSYTNYR_384.2_338.2
   LIQDAVTGLTVNGQITGDK_972.0_640.4
   TYLHTYESEI_628.3_908.4
   WWGGQPLWITATK_772.4_373.2
   DVLLLVHNLPQNLPGYFWYK_810.4_328.2
   VEHSDLSFSK_383.5_468.2
   WWGGQPLWITATK_772.4_929.5
   ALQDQLVLVAAK_634.9_289.2
   VNHVTLSQPK_374.9_244.2
   LPNNVLQEK_527.8_730.4
   LPNNVLQEK_527.8_844.5
   AVLHIGEK_289.5_292.2
   TYLHTYESEI_628.3_515.3
   DIIKPDPPK_511.8_342.2
   DADPDTFFAK_563.8_302.1
   AVLHIGEK_289.5_348.7
   DVLLLVHNLPQNLPGYFWYK_810.4_215.1
   VEHSDLSFSK_383.5_234.1
   YYGYTGAFR_549.3_450.3
   QGHNSVFLIK_381.6_260.2
   LLELTGPK_435.8_227.2
   ALNHLPLEYNSALYSR_621.0_696.4
   ALNHLPLEYNSALYSR_621.0_538.3
   IRPHTFTGLSGLR_485.6_432.3
   LSNENHGIAQR_413.5_544.3
   TVQAVLTVPK_528.3_855.5
   HFQNLGK_422.2_527.2
   TGVAVNKPAEFTVDAK_549.6_258.1
   ALQDQLVLVAAK_634.9_956.6
   HFQNLGK_422.2_285.1
   LHKPGVYTR_357.5_692.4
   LHKPGVYTR_357.5_479.3
   FLNWIK_410.7_561.3
   HTLNQIDEVK_598.8_951.5
   DADPDTFFAK_563.8_825.4
   VNHVTLSQPK_374.9_459.3
   IRPHTFTGLSGLR_485.6_545.3
   LLELTGPK_435.8_644.4
   LIENGYFHPVK_439.6_343.2
   LSNENHGIAQR_413.5_519.8
   DALSSVQESQVAQQAR_573.0_672.4
   LIENGYFHPVK_439.6_627.4
   TVQAVLTVPK_528.3_428.3
   YEFLNGR_449.7_293.1
   QGHNSVFLIK_381.6_520.4
   HTLNQIDEVK_598.8_958.5
   LQGTLPVEAR_542.3_842.5
   YEFLNGR_449.7_606.3
   TLLPVSKPEIR_418.3_288.2
   FLNWIK_410.7_560.3
   DALSSVQESQVAQQAR_573.0_502.3
   LQGTLPVEAR_542.3_571.3
   DISEVVTPR_508.3_472.3
   TTIEKPVWLGFLGPIIK_638.0_640.4
   SLPVSDSVLSGFEQR_810.9_723.3
   FLQEQGHR_338.8_497.3
   VFQFLEK_455.8_811.4
   QALEEFQK_496.8_680.3
   DVLLLVHNLPQNLPGYFWYK_810.4_960.5
   VEHSDLSFSK_383.5_468.2
   DVLLLVHNLPQNLPGYFWYK_810.4_328.2
   TYLHTYESEI_628.3_908.4
   AVLHIGEK_289.5_292.2
   WWGGQPLWITATK_772.4_373.2
   LFIPQITR_494.3_614.4
   WWGGQPLWITATK_772.4_929.5
   TYLHTYESEI_628.3_515.3
   LFIPQITR_494.3_727.4
   DVLLLVHNLPQNLPGYFWYK_810.4_215.1
   AVLHIGEK_289.5_348.7
   LPNNVLQEK_527.8_730.4
   FGFGGSTDSGPIR_649.3_745.4
   TVQAVLTVPK_528.3_855.5
   LLELTGPK_435.8_227.2
   ALDLSLK_380.2_185.1
   FGFGGSTDSGPIR_649.3_946.5
   VEHSDLSFSK_383.5_234.1
   ALNHLPLEYNSALYSR_621.0_696.4
   YYGYTGAFR_549.3_450.3
   DADPDTFFAK_563.8_302.1
   ALNHLPLEYNSALYSR_621.0_538.3
   LPNNVLQEK_527.8_844.5
   TVQAVLTVPK_528.3_428.3
   EAQLPVIENK_570.8_329.2
   ITLPDFTGDLR_624.3_920.5
   VQEAHLTEDQIFYFPK_655.7_701.4
   YEFLNGR_449.7_606.3
   VNHVTLSQPK_374.9_459.3
   DISEVVTPR_508.3_472.3
   ALVLELAK_428.8_331.2
   INHBE_ALVLELAK_428.8_672.4
   EGLN_TQILEWAAER_608.8_761.4
   FA7_SEPRPGVLLR_375.2_654.4
   CBG_ITQDAQLK_458.8_702.4
   ITIH3_ALDLSLK_380.2_185.1
   ENPP2_WWGGQPLWITATK_772.4_929.5
   CBG_ITQDAQLK_458.8_803.4
   PSG1_LSETNR_360.2_519.3
   CAA60698_LEPLYSASGPGLRPLVIK_637.4_834.5
   INHBC_LDFHFSSDR_375.2_611.3
   CBG_QINSYVK_426.2_610.3
   LBP_GLQYAAQEGLLALQSELLR_1037.1_858.5
   A1BG_LLELTGPK_435.8_644.4
   CO6_DLHLSDVFLK_396.2_366.2
   VCAM1_TQIDSPLSGK_523.3_816.5
   LRP1_NAWQGLEQPHGLVVHPLR_688.4_285.2
   CBG_QINSYVK_426.2_496.3
   CO6_ENPAVIDFELAPIVDLVR_670.7_811.5
   ADA12_FGFGGSTDSGPIR_649.3_745.4
   CO6_DLHLSDVFLK_396.2_260.2
   ENPP2_TYLHTYESEI_628.3_908.4
   A1BG_LLELTGPK_435.8_227.2
   FRIH_QNYHQDSEAAINR_515.9_544.3
   ENPP2_TEFLSNYLTNVDDITLVPGTLGR_846.8_600.3
   CBG_WSAGLTSSQVDLYIPK_883.0_515.3
   C1QC_FQSVFTVTR_542.8_623.4
   GELS_DPDQTDGLGLSYLSSHIANVER_796.4_456.2
   A1BG_ATWSGAVLAGR_544.8_643.4
   APOA1_AKPALEDLR_506.8_813.5
   ENPP2_TYLHTYESEI_628.3_515.3
   PEPD_AVYEAVLR_460.8_750.4
   TIMP1_GFQALGDAADIR_617.3_717.4
   ADA12_LIEIANHVDK_384.6_498.3
   PGRP2_WGAAPYR_410.7_577.3
   PSG9_LFIPQITR_494.3_614.4
   HABP2_FLNWIK_410.7_560.3
   CBG_WSAGLTSSQVDLYIPK_883.0_357.2
   PSG2_IHPSYTNYR_384.2_452.2
   PSG5_LSIPQITTK_500.8_800.5
   HABP2_FLNWIK_410.7_561.3
   CO6_SEYGAALAWEK_612.8_788.4
   ADA12_LIEIANHVDK_384.6_683.4
   EGLN_TQILEWAAER_608.8_632.3
   CO6_ENPAVIDFELAPIVDLVR_670.7_601.4
   FA7_FSLVSGWGQLLDR_493.3_447.3
   ITIH3_ALDLSLK_380.2_575.3
   ADA12_FGFGGSTDSGPIR_649.3_946.5
   FETUA_AALAAFNAQNNGSNFQLEEISR_789.1_746.4
   FBLN1_AITPPHPASQANIIFDITEGNLR_825.8_459.3
   TSP1_FVFGTTPEDILR_697.9_843.5
   VCAM1_NTVISVNPSTK_580.3_732.4
   ENPP2_TEFLSNYLTNVDDITLVPGTLGR_846.8_699.4
   FBLN3_ELPQSIVYK_538.8_409.2
   ACTB_VAPEEHPVLLTEAPLNPK_652.0_892.5
   TSP1_FVFGTTPEDILR_697.9_742.4
   PLMN_EAQLPVIENK_570.8_699.4
   C1S_IIGGSDADIK_494.8_260.2
   IL1A_ANDQYLTAAALHNLDEAVK_686.4_317.2
   PGRP2_WGAAPYR_410.7_634.3
   PSG5_LSIPQITTK_500.8_687.4
   PSG6_SNPVTLNVLYGPDLPR_585. 7_654.4
   PRG2_WNFAYWAAHQPWSR_607.3_545.3
   CXCL2_CQCLQTLQGIHLK_13p8RT_533.6_567.4
   CXCL2_CQCLQTLQGIHLK_13p48RT_533.6_695.4
   G6PE_LLDFEFSSGR_585.8_553.3
   GELS_TASDFITK_441.7_710.4
   B2MG_VEHSDLSFSK_383.5_468.2
   CO8B-IPGIFELGISSQSDR_809.9_849.4
   PSG8_LQLSETNR_480.8-606.3
   LBP_GLQYAAQEGLLALQSELLR_1037.1_929.5
   AFAM_LPNNVLQEK_527.8_844.5
   MAGE
   PSG2_IHPSYTNYR_384.2_338.2
   PSG9_LFIPQITR_494.3_727.4
   TFR1_YNSQLLSFVR_613.8_734.5
   C1R_QRPPDLDTSSNAVDLLFFTDESGDSR_961.5_866.3
   PGH1_ILPSVPK_377.2_264.2
   FA7_SEPRPGVLLR_375.2_454.3
   FA7_TLAFVR_353.7_274.2
   PGRP2_DGSPDVTTADIGANTPDATK_973.5_844.4
   C1S_IIGGSDADIK_494.8_762.4
   PEPD_VPLALFALNR_557.3_620.4
   C1S_EDTPNSVWEPAK_686.8_630.3
   CHL1_TAVTANLDIR_537.3_802.4
   CHL1_VIAVNEVGR_478.8_744.4
   SDF1_ILNTPNCALQIVAR_791.9_341.2
   PAI1_EVPLSALTNILSAQLISHWK_740.8_996.6
   AMBP_AFIQLWAFDAVK_704.9_650.4
   G6PE_LLDFEFSSGR_585.8_944.4
   THBG_SILFLGK_389.2_577.4
   PRDX2_GLFIIDGK_431.8_545.3
   ENPP2_WWGGQPLWITATK_772.4_373.2
   VGFR3_SGVDLADSNQK_567.3_662.3
   C1S_EDTPNSVWEPAK_686.8_315.2
   AFAM_DADPDTFFAK_563.8_302.1
   CBG_SETEIHQGFQHLHQLFAK_717.4_447.2
   C1S_LLEVPEGR_456.8_686.4
   PTGDS_GPGEDFR_389.2_623.3
   ITIH2_IYLQPGR_423.7_329.2
   FA7_TLAFVR_353.7_492.3
   FA7_FSLVSGWGQLLDR_493.3_403.2
   SAMP_VGEYSLYIGR_578.8_871.5
   APOH_EHSSLAFWK_552.8_267.1
   PGRP2_AGLLRPDYALLGHR_518.0_595.4
   C1QC_FNAVLTNPQGDYDTSTGK_964.5_333.2
   PGRP2_AGLLRPDYALLGHR_518.0_369.2
   PGRP2_TFTLLDPK_467.8_686.4
   CO5_TDAPDLPEENQAR_728.3_843.4
   HRB_ELLESYIDGR_597.8_839.4
   GELS_DPDQTDGLGLSYLSSHIANVER_796.4_328.1
   TRFL_YYGYTGAFR_549.3_450.3
   HEMO_QGHNSVFLIK_381.6_260.2
   C1S_GDSGGAFAVQDPNDK_739.3_716.3
   B1A4H9_AHQLAIDTYQEFR_531. 3_450.3
   C1S_SSNNPHSPIVEEFQVPYNK_729.4_261.2
   HYOU1_LPATEKPVLLSK_432.6_347.2
   FETA_GYQELLEK_490.3_502.3
   LRP1_SERPPIFEIR_415.2_288.2
   CO6_SEYGAALAWEK_612.8_845.5
   CERU_TTIEKPVWLGFLGPIIK_638.0_844.5
   IBP1_AQETSGEEISK_589.8_850.4
   SHBG_VVLSSGSGPGLDLPLVLGLPLQLK_791.5_768.5
   CBG_SETEIHQGFQHLHQLFAK_717.4_318.1
   A1AT_LSITGTYDLK_555.8_696.4
   PGRP2_DGSPDVTTADIGANTPDATK_973.5_531.3
   C1QB_LEQGENVFLQATDK_796.4_675.4
   EGLN_GPITSAAELNDPQSILLR_632.4_826.5
   IL12B_YENYTSSFFIR_713.8_293.1
   B2MG_VEHSDLSFSK_383.5_234.1
   PGH1_AEHPTWGDEQLFQTTR_639.3_765.4
   INHBE_ALVLELAK_428.8_331.2
   HYOU1_LPATEKPVLLSK_432.6_460.3
   CXCL2_CQCLQTLQGIHLK_13p48RT_533.6_567.4
   PZP_AVGYLITGYQR_620.8_523.3
   AFAM_IAPQLSTEELVSLGEK_857.5_333.2
   ICAM1_VELAPLPSWQPVGK_760.9_400.3
   CHL1_VIAVNEVGR_478.8_284.2
   APOB_ITENDIQIALDDAK_779.9_632.3
   SAMP_AYSDLSR_406.2_577.3
   AMBP_AFIQLWAFDAVK_704.9_836.4
   EGLN_GPITSAAELNDPQSILLR_632.4_601.4
   LRP1_NAVVQGLEQPHGLVVHPLR_688.4_890.6
   SHBG_VVLSSGSGPGLDLPLVLGLPLQLK_791.5_598.4
   IBP1_AQETSGEEISK_589.8_979.5
   PSG6_SNPVTLNVLYGPDLPR_585.7_817.4
   APOC3_DYWSTVK_449.7_347.2
   C1R_WILTAAHTLYPK_471.9_621.4
   ANGT_ADSQAQLLLSTVVGVFTAPGLHLK_822.5_983.6
   PSG9_DVLLLVHNLPQNLPGYFWYK_810.4_328.2
   A1AT_AVLTIDEK_444.8_605.3
   PSGx_ILILPSVTR_506.3_785.5
   THRB_ELLESYIDGR_597.8_710.4
   SHBG_ALALPPLGLAPLLNLWAKPQGR_770.5_256.2
   PZP_QTLSWTVTPK_580.8_545.3
   SHBG_ALALPPLGLAPLLNLWAKPQGR_770.5_457.3
   PSG4_TLFIFGVTK_513.3_811.5
   PLMN_YEFLNGR_449.7_293.1
   PEPD_AVYEAVLR_460.8_587.4
   PLMN_LSSPAVITDK_515.8_830.5
   FINC_SYTITGLQPGTDYK_772.4_352.2
   C1R_WILTAAHTLYPK_471.9_407.2
   CHL1_TAVTANLDIR_537.3_288.2
   TENA_AVDIPGLEAATPYR_736.9_286.1
   CRP_YEVQGEVFTKPQLWP_911.0_293.1
   APOB_SVSLPSLDPASAK_636.4_885.5
   PRDX2_SVDEALR_395.2_488.3
   CO8A_YHFEALADTGISSEFYDNANDLLSK_940.8_301.1
   C1QC_FQSVFTVTR_542.8_722.4
   PSGx_ILILPSVTR_506.3_559.3
   VCAM1_TQIDSPLSGK_523.3_703.4
   VCAM1_NTVISVNPSTK_580.3_845.5
   CSH_ISLLLIESWLEPVR_834.5_500.3
   HGFA_EALVPLVADHK_397.9_439.8
   CGB1_CRPINATLAVEK_457.9_660.4
   APOB_IEGNLIFDPNNYLPK_874.0_414.2
   C1QB_LEQGENVFLQATDK_796.4_822.4
   APOH_EHSSLAFWK_552.8_838.4
   CO5_LQGTLPVEAR_542.3_842.5
   SHBG_IALGGLLFPASNLR_481.3_412.3
   B2MG_VNHVTLSQPK_374.9_459.3
   APOA2_SPELQAEAK_486.8_659.4
   FLNA_TGVAVNKPAEFTVDAK_549.6_258.1
   PLMN_YEFLNGR_449.7_606.3
   HABP2_FLNWIK_410.7_561.3
   ADA12_FGFGGSTDSGPIR_649.3_946.5
   A1BG_LLELTGPK_435.8_227.2
   B2MG_VEHSDLSFSK_383.5_234.1
   ADA12_FGFGGSTDSGPIR_649.3_745.4
   B2MG_VEHSDLSFSK_383.5_468.2
   APOB_IEGNLIFDPNNYLPK_874.0_414.2
   PSG9_DVLLLVHNLPQNLPGYFWYK_810.4_960.5
   A1BG_LLELTGPK_435.8_644.4
   ITIH3_ALDLSLK_380.2_185.1
   ENPP2_TYLHTYESEI_628.3_908.4
   IL12B_YENYTSSFFIR_713.8_293.1
   ENPP2_WWGGQPLWITATK_772.4_373.2
   ENPP2_TYLHTYESEI_628.3_515.3
   PSG9_LFIPQITR_494.3_614.4
   MAGE
   ENPP2_WWGGQPLWITATK_772.4_929.5
   CERU_TTIEKPVWLGFLGPIIK_638.0_640.4
   ITIH3_ALDLSLK_380.2_575.3
   PSG2_IHPSYTNYR_384.2_452.2
   ITIH1_LWAYLTIQELLAK_781.5_371.2
   INHBE_ALVLELAK_428.8_331.2
   HGFA_LHKPGVYTR_357.5_692.4
   TRFL_YYGYTGAFR_549.3_450.3
   THBG_AVLHIGEK_28 9.5_34 8.7
   GELS_TASDFITK_441.7_710.4
   PSG9_LFIPQITR_494.3_727.4
   VGFR3_SGVDLADSNQK_567.3_662.3
   INHA_TTSDGGYSFK_531.7_860.4
   PSG9_DVLLLVHNLPQNLPGYFWYK_810.4_328.2
   HYOU1_LPATEKPVLLSK_432.6_347.2
   CO8G_VQEAHLTEDQIFYFPK_655.7_701.4
   BMI
   HGFA_LHKPGVYTR_357.5_479.3
   PSG2_IHPSYTNYR_384.2_338.2
   GELS_AQPVQVAEGSEPDGFWEALGGK_758.0_623.4
   PSG6_SNPVTLNVLYGPDLPR_585.7_654.4
   HABP2_FLNWIK_410.7_560.3
   PGH1_ILPSVPK_377.2_527.3
   HYOU1_LPATEKPVLLSK_432.6_460.3
   CO6_ALNHLPLEYNSALYSR_621.0_696.4
   INHBE_ALVLELAK_428.8_672.4
   PLMN_LSSPAVITDK_515.8_743.4
   PSG9_DVLLLVHNLPQNLPGYFWYK_810.4_215.1
   BGH3_LTLLAPLNSVFK_658.4_875.5
   AFAM_LPNNVLQEK_527.8_730.4
   ITIH2_LSNENHGIAQR_413.5_519.8
   GELS_TASDFITK_441.7_781.4
   FETUA_AHYDLR_387.7_566.3
   CERU_TTIEKPVWLGFLGPIIK_638.0_844.5
   PSGx_ILILPSVTR_506.3_785.5
   APOB_SVSLPSLDPASAK_636.4_885.5
   FLNA_TGVAVNKPAEFTVDAK_549.6_258.1
   PLMN_YEFLNGR_449.7_606.3
PSGx_ILILPSVTR_506.3_559.3

## Claims

1. An isolated biomarker consisting of the sequence IALGGLLFPASNLR.

2. A method of determining probability for preterm birth or term birth in a pregnant female, the method comprising:
detecting a measurable feature of each of one or more biomarkers in a biological sample obtained from said pregnant female, and
analyzing said measurable feature to determine the probability for preterm birth or term birth in said pregnant female,
wherein the one or more biomarkers comprise a biomarker consisting of the sequence IALGGLLFPASNLR;
wherein said measurable feature is defined as the presence, absence, or concentration of the biomarker, or a fragment thereof; an altered structure, such as the presence or amount of a post-translational modification; or the presence of an altered conformation in comparison to the conformation of the biomarker in normal control subj ects; and
wherein the fragment comprises at least 5, at least 6, at least 7, at least 8, at least 9, at least 10, at least 11, at least 12, or at least 13 consecutive amino acid residues.

3. A method of predicting gestational age at birth (GAB) or time to birth in a pregnant female, the method comprising:
detecting a measurable feature of each of one or more biomarkers in a biological sample obtained from said pregnant female, and
analyzing said measurable feature to predict GAB or time to birth in said pregnant female,
wherein the one or more biomarkers comprise a biomarker consisting of the sequence IALGGLLFPASNLR;
wherein said measurable feature is defined as the presence, absence, or concentration of the biomarker, or a fragment thereof; an altered structure, such as the presence or amount of a post-translational modification; or the presence of an altered conformation in comparison to the conformation of the biomarker in normal control subj ects; and
wherein the fragment comprises at least 5, at least 6, at least 7, at least 8, at least 9, at least 10, at least 11, at least 12, or at least 13 consecutive amino acid residues.

4. The method according to claim 2 further comprising:
(a) quantifying an amount of the one or more biomarkers;
(b) multiplying said amount by a predetermined coefficient, and
(c) determining the probability for preterm birth in the pregnant female comprising adding said individual products to obtain a total risk score that corresponds to said probability.

5. The method according to claims 2 or 4, wherein said analyzing of said measurable feature initially comprises prediction of gestational age at birth (GAB) prior to said determining the probability for preterm birth;
optionally wherein said prediction of the GAB is used to determine the probability for preterm birth.

6. The method according to claim 3 further comprising:
(a) quantifying an amount of the one or more biomarkers;
(b) multiplying and/or thresholding said amount by a predetermined coefficient;
(c) determining the predicted GAB in the pregnant female, the determination comprising adding said individual products to obtain a total risk score that corresponds to said predicted GAB; and optionally
(d) subtracting the estimated gestational age (GA) at the time the biological sample was obtained from the predicted GAB to predict time to birth in said pregnant female.

7. The method according to any of claims 2 to 6, wherein the method further comprises calculating the probability for preterm birth or term birth, or GAB, or time to birth in said pregnant female based on quantifying an amount of each of the one or more biomarkers.

8. The method according to any of claims 2 to 7, wherein said analyzing comprises a use of a predictive model; optionally wherein said analyzing comprises comparing the measurable feature with a reference feature.

9. The method according to claim 8, wherein said analyzing comprises using one or more of: a linear discriminant analysis model, a support vector machine classification algorithm, a recursive feature elimination model, a prediction analysis of microarray model, a logistic regression model, a multiple regression model, a survival model, a CART algorithm, a flex tree algorithm, a LART algorithm, a random forest algorithm, a MART algorithm, a machine learning algorithm, a penalized regression method, and a combination thereof.

10. The method according to any of claims 2 to 9, wherein the biological sample is selected from the group consisting of whole blood, plasma, and serum.

11. The method according to any of claims 4 to 10, wherein said quantifying comprises mass spectrometry (MS) or liquid chromatography-mass spectrometry (LC-MS).

12. The method according to claim 11, wherein said MS comprises multiple reaction monitoring (MRM) or selected reaction monitoring (SRM); optionally wherein said MRM or SRM comprises scheduled MRM or SRM

13. The method according to any of claims 4 to 10, wherein said quantifying comprises an assay that utilizes a capture agent.

14. The method according to claim 13, wherein:
said capture agent is selected from the group consisting of an antibody, antibody fragment, nucleic acid-based protein binding reagent, and small molecule or variant thereof; and/or
said assay is selected from the group consisting of enzyme immunoassay (EIA), enzyme-linked immunosorbent assay (ELISA), and radioimmunoassay (RIA); and/or
said quantifying further comprises mass spectrometry (MS) or co-immunoprecipitation-mass spectrometry (co-IP MS).

15. The method according to any of claims 2 to 14, further comprising detecting one or more risk indicia;
optionally wherein the one or more risk indicia are selected from the group consisting of age, prior pregnancy, history of previous low birth weight or preterm delivery, multiple second trimester spontaneous abortion, prior first trimester induced abortion, familial and intergenerational factors, history of infertility, nulliparity, placental abnormalities, cervical and uterine anomalies, gestational bleeding, intrauterine growth restriction, in utero diethylstilbestrol exposure, multiple gestations, infant sex, short stature, low prepregnancy weight/low body mass index, diabetes, hypertension, hypothyroidism, asthma, education level, tobacco use, and urogenital infections.

## Patentansprüche

1. Isolierter Biomarker, bestehend aus der Sequenz IALGGLLFPASNLR.

2. Verfahren zum Bestimmen der Wahrscheinlichkeit einer Frühgeburt oder einer Reifgeburt bei einer schwangeren Frau, wobei das Verfahren umfasst:
Nachweisen eines messbaren Merkmals jedes von einem oder mehreren Biomarkern in einer biologischen Probe, die von der schwangeren Frau gewonnen wurde, und
Analysieren des messbaren Merkmals, um die Wahrscheinlichkeit einer Frühgeburt oder einer Reifgeburt bei der schwangeren Frau zu bestimmen, wobei der eine oder die mehreren Biomarker einen Biomarker umfassen, der aus der Sequenz IALGGLLFPASNLR besteht;
wobei das messbare Merkmal definiert ist als das Vorhandensein, das Fehlen oder die Konzentration des Biomarkers oder eines Fragments davon; eine veränderte Struktur, wie beispielsweise das Vorhandensein oder die Menge einer posttranslationalen Modifikation; oder das Vorhandensein einer veränderten Konformation im Vergleich zur Konformation des Biomarkers bei normalen Kontrollpersonen; und
wobei das Fragment mindestens 5, mindestens 6, mindestens 7, mindestens 8, mindestens 9, mindestens 10, mindestens 11, mindestens 12 oder mindestens 13 aufeinanderfolgende Aminosäurereste umfasst.

3. Verfahren zur Vorhersage des Gestationsalters bei der Geburt (GAB) oder der Zeit bis zur Geburt bei einer schwangeren Frau, wobei das Verfahren umfasst:
Nachweisen eines messbaren Merkmals jedes von einem oder mehreren Biomarkern in einer biologischen Probe, die von der schwangeren Frau gewonnen wurde, und
Analysieren des messbaren Merkmals, um das GAB oder die Zeit bis zur Geburt bei der schwangeren Frau vorherzusagen,
wobei der eine oder die mehreren Biomarker einen Biomarker umfassen, der aus der Sequenz IALGGLLFPASNLR besteht;
wobei das messbare Merkmal definiert ist als das Vorhandensein, das Fehlen oder die Konzentration des Biomarkers oder eines Fragments davon;
eine veränderte Struktur, wie beispielsweise das Vorhandensein oder die Menge einer posttranslationalen Modifikation; oder das Vorhandensein einer veränderten Konformation im Vergleich zur Konformation des Biomarkers bei normalen Kontrollpersonen; und
wobei das Fragment mindestens 5, mindestens 6, mindestens 7, mindestens 8, mindestens 9, mindestens 10, mindestens 11, mindestens 12 oder mindestens 13 aufeinanderfolgende Aminosäurereste umfasst.

4. Verfahren nach Anspruch 2, weiter umfassend:
(a) Quantifizieren einer Menge des einen oder der mehreren Biomarker;
(b) Multiplizieren der Menge mit einem vorbestimmten Koeffizienten, und
(c) Bestimmen der Wahrscheinlichkeit für eine Frühgeburt bei der schwangeren Frau, umfassend das Hinzufügen der einzelnen Produkte, um einen Gesamtrisikowert zu gewinnen, der der Wahrscheinlichkeit entspricht.

5. Verfahren nach den Ansprüchen 2 oder 4, wobei die Analyse des messbaren Merkmals zunächst die Vorhersage des Gestationsalters bei der Geburt (GAB) vor dem Bestimmen der Wahrscheinlichkeit für eine Frühgeburt umfasst;
optional wobei die Vorhersage des GAB verwendet wird, um die Wahrscheinlichkeit für eine Frühgeburt zu bestimmen.

6. Verfahren nach Anspruch 3, weiter umfassend:
(a) Quantifizieren einer Menge des einen oder der mehreren Biomarker;
(b) Multiplizieren und/oder Schwellenwertbildung des Betrags mit einem vorbestimmten Koeffizienten;
(c) Bestimmen des vorhergesagten GAB bei der schwangeren Frau, wobei die Bestimmung das Hinzufügen der einzelnen Produkte umfasst, um einen Gesamtrisikowert zu gewinnen, der dem vorhergesagten GAB entspricht; und optional
(d) Subtrahieren des geschätzten Gestationsalters (GA) zum Zeitpunkt der Gewinnung der biologischen Probe von dem vorhergesagten GAB, um die Zeit bis zur Geburt bei der schwangeren Frau vorherzusagen.

7. Verfahren nach einem der Ansprüche 2 bis 6, wobei das Verfahren ferner das Berechnen der Wahrscheinlichkeit für eine Frühgeburt oder eine Reifgeburt oder ein GAB oder eine Zeit bis zur Geburt bei der schwangeren Frau, basierend auf dem Quantifizieren einer Menge von jedem der einen oder mehreren Biomarker, umfasst.

8. Verfahren nach einem der Ansprüche 2 bis 7, wobei das Analysieren die Verwendung eines Prognosemodells umfasst; optional wobei das Analysieren ein Vergleichen des messbaren Merkmals mit einem Referenzmerkmal umfasst.

9. Verfahren nach Anspruch 8, wobei das Analysieren die Verwendung umfasst von einem oder mehreren von: einem linearen Diskriminanzanalysemodell, einem Unterstützungsvektor-Maschinenklassifikationsalgorithmus, einem rekursiven Merkmalseliminierungsmodell, einer Vorhersageanalyse des Microarray-Modells, einem logistischen Regressionsmodell, einem multiplen Regressionsmodell, einem Überlebensmodell, einem CART-Algorithmus, einem Flex-Tree-Algorithmus, einem LART-Algorithmus, einem Random-Forest-Algorithmus, einem MART-Algorithmus, einem maschinellen Lernalgorithmus, einem bestraften Regressionsverfahren und einer Kombination davon.

10. Verfahren nach einem der Ansprüche 2 bis 9, wobei die biologische Probe ausgewählt ist aus der Gruppe bestehend aus Vollblut, Plasma und Serum.

11. Verfahren nach einem der Ansprüche 4 bis 10, wobei die Quantifizierung eine Massenspektrometrie (MS) oder eine Flüssigchromatographie-Massenspektrometrie (LC-MS) umfasst.

12. Verfahren nach Anspruch 11, wobei die MS eine Mehrfachreaktionsüberwachung (MRM) oder eine ausgewählte Reaktionsüberwachung (SRM) umfasst; optional, wobei die MRM oder SRM eine geplante MRM oder SRM umfasst.

13. Verfahren nach einem der Ansprüche 4 bis 10, wobei die Quantifizierung einen Assay umfasst, der ein Erfassungsmittel verwendet.

14. Verfahren nach Anspruch 13, wobei:
das Einfangmittel ausgewählt ist aus der Gruppe bestehend aus einem Antikörper, einem Antikörperfragment, einem nukleinsäurebasierten Proteinbindungsreagenz, und einem kleinen Molekül oder einer Variante davon; und/oder
der Assay ausgewählt ist aus der Gruppe bestehend aus einem Enzymimmunoassay (EIA), einem enzymgebundenem Immunosorbentassay (ELISA) und einem Radioimmunoassay (RIA); und/oder
die Quantifizierung ferner eine Massenspektrometrie (MS) oder eine Ko-Immunoprezipitations-Massenspektrometrie (Co-IP MS) umfasst.

15. Verfahren nach einem der Ansprüche 2 bis 14, ferner umfassend das Nachweisen eines oder mehrerer Risikokennzeichen;
optional wobei das eine oder die mehreren Risikokennzeichen ausgewählt sind aus der Gruppe bestehend aus Alter, vorheriger Schwangerschaft, Anamnese eines früheren niedrigen Geburtsgewichts oder einer Frühgeburt, mehrfachen Spontanaborten im zweiten Trimester, früherem im ersten Trimester induziertem Abort, familiären und intergenerationellen Faktoren, Anamnese der Unfruchtbarkeit, Nulliparität, Anomalien der Plazenta, Gebärmutterhals- und Gebärmutteranomalien, Schwangerschaftsblutungen, intrauteriner Wachstumsretardierung, Belastung mit Diethylstilbestrol in utero, Mehrlingsschwangerschaften, Geschlecht des Kindes, Kleinwuchs, niedrigem Schwangerschaftsgewicht / niedrigem Körpermassenindex, Diabetes, Bluthochdruck, Hypothyreose, Asthma, Bildungsniveau, Tabakkonsum und Urogenitalinfektionen.

## Revendications

1. Biomarqueur isolé constituer de la séquence IALGGLLFPASNLR.

2. Procédé de détermination d'une probabilité d'une naissance prématurée ou d'une naissance à terme chez une femme enceinte, le procédé comprenant:
détecter une caractéristique mesurable de chacun d'un ou plusieurs biomarqueurs dans un échantillon biologique obtenu à partir de ladite femme enceinte, et
analyser ladite caractéristique mesurable pour déterminer la probabilité d'une naissance prématurée ou d'une naissance à terme chez ladite femme enceinte,
dans lequel l'un ou plusieurs biomarqueurs comprennent un biomarqueur consistant en la séquence IALGGLLFPASNLR;
dans lequel ladite caractéristique mesurable est définie comme la présence, l'absence, ou la concentration du biomarqueur, ou d'un fragment de celui-ci; une structure altérée, telle que la présence ou la quantité d'une modification post-translationnelle; ou la présence d'une conformation altérée en comparaison à la conformation du biomarqueur chez des sujets témoins normaux; et
dans lequel le fragment comprend au moins 5, au moins 6, au moins 7, au moins 8, au moins 9, au moins 10, au moins 11, au moins 12, ou au moins 13 résidus d'acides aminés consécutifs.

3. Procédé de prédiction d'un âge gestationnel à la naissance (GAB) ou d'un temps jusqu'à la naissance chez une femme enceinte, le procédé comprenant:
détecter une caractéristique mesurable de chacun d'un ou plusieurs biomarqueurs dans un échantillon biologique obtenu à partir de ladite femme enceinte, et
analyser ladite caractéristique mesurable pour prédire un GAB ou un temps jusqu'à la naissance chez ladite femme enceinte,
dans lequel l'un ou plusieurs biomarqueurs comprennent un biomarqueur consistant en la séquence IALGGLLFPASNLR;
dans lequel ladite caractéristique mesurable est définie comme la présence, l'absence, ou la concentration du biomarqueur, ou d'un fragment de celui-ci; une structure altérée, telle que la présence ou la quantité d'une modification post-translationnelle;
ou la présence d'une conformation altérée en comparaison à la conformation du biomarqueur chez des sujets témoins normaux; et
dans lequel le fragment comprend au moins 5, au moins 6, au moins 7, au moins 8, au moins 9, au moins 10, au moins 11, au moins 12, ou au moins 13 résidus d'acides aminés consécutifs.

4. Procédé selon la revendication 2 comprenant en outre:
a) quantifier une quantité de l'un ou plusieurs biomarqueurs;
b) multiplier ladite quantité par un coefficient prédéterminé, et
c) déterminer la probabilité d'une naissance prématurée chez la femme enceinte comprenant ajouter lesdits produits individuels pour obtenir un score de risque total qui correspond à ladite probabilité.

5. Procédé selon les revendications 2 ou 4, dans lequel ladite analyse de ladite caractéristique mesurable comprend initialement une prédiction d'un âge gestationnel à la naissance (GAB) avant ladite détermination de la probabilité d'une naissance prématurée;
optionnellement dans lequel ladite prédiction du GAB est utilisée pour déterminer la probabilité d'une naissance prématurée.

6. Procédé selon la revendication 3 comprenant en outre:
a) quantifier une quantité de l'un ou plusieurs biomarqueurs;
b) multiplier et/ou seuiller ladite quantité par un coefficient prédéterminé;
c) déterminer le GAB prédit chez la femme enceinte, la détermination comprenant ajouter lesdits produits individuels pour obtenir un score de risque total qui correspond audit GAB prédit; et optionnellement
d) soustraire l'âge gestationnel (GA) estimé au moment où l'échantillon biologique a été obtenu à partir du GAB prédit pour prédire un temps jusqu'à la naissance chez ladite femme enceinte.

7. Procédé selon l'une quelconque des revendications 2 à 6, dans lequel le procédé comprend en outre calculer la probabilité d'une naissance prématurée ou d'une naissance à terme, ou d'un GAB, ou d'un temps jusqu'à la naissance chez ladite femme enceinte sur la base de la quantification d'une quantité de chacun de l'un ou plusieurs biomarqueurs.

8. Procédé selon l'une quelconque des revendications 2 à 7, dans lequel ladite analyse comprend une utilisation d'un modèle prédictif; optionnellement dans lequel ladite analyse comprend comparer la caractéristique mesurable avec une caractéristique de référence.

9. Procédé selon la revendication 8, dans lequel ladite analyse comprend utiliser un ou plusieurs de : un modèle d'analyse discriminante linéaire, un algorithme de classification de machines à vecteurs de support, un modèle d'élimination de caractéristiques récursives, un modèle d'analyse de prédiction de biopuce, un modèle de régression logistique, un modèle de régression multiple, un modèle de survie, un algorithme CART, un algorithme à arbres flexibles, un algorithme LART, un algorithme à forêts aléatoires, un algorithme MART, un algorithme d'apprentissage automatique, un procédé de régression avec pénalité, et une combinaison de ceux-ci.

10. Procédé selon l'une quelconque des revendications 2 à 9, dans lequel l'échantillon biologique est sélectionné parmi le groupe consistant en du sang total, du plasma et du sérum.

11. Procédé selon l'une quelconque des revendications 4 à 10, dans lequel ladite quantification comprend une spectrométrie de masse (MS) ou une chromatographie en phase liquide-spectrométrie de masse (LC-MS).

12. Procédé selon la revendication 11, dans lequel ladite MS comprend un suivi de réactions multiples (MRM) ou un suivi de réactions de réactions choisies (SRM);
optionnellement dans lequel ladite MRM ou SRM comprend une MRM ou SRM planifiée.

13. Procédé selon l'une quelconque des revendications 4 à 10, dans lequel ladite quantification comprend un dosage qui utilise un agent de capture.

14. Procédé selon la revendication 13, dans lequel:
ledit agent de capture est sélectionné parmi le groupe consistant en un anticorps, un fragment d'anticorps, un réactif de liaison à une protéine d'acide nucléique, et une petite molécule ou une variante de ceux-ci; et/ou
ledit dosage est sélectionné parmi le groupe consistant en un immunodosage enzymatique (EIA), un dosage immuno-enzymatique sur support solide (ELISA), et un dosage radio-immunologique (RIA); et/ou
ladite quantification comprend en outre une spectrométrie de masse (MS) ou une co-immunoprécipitation-spectrométrie de masse (co-IP MS).

15. Procédé selon l'une quelconque des revendications 2 à 14, comprenant en outre détecter un ou plusieurs indices de risque;
optionnellement dans lequel l'un ou plusieurs indices de risque sont sélectionnés parmi le groupe consistant en l'âge, une grossesse antérieure, un historique d'un poids insuffisant à la naissance ou d'un accouchement prématuré précédents, des avortements spontanés du deuxième trimestre multiples, un avortement provoqué du premier trimestre antérieur, des facteurs familiaux et intergénérationnels, un historique d'infertilité, de nulliparité, des anormalités placentales, des anomalies cervicales et utérines, des saignements gestationnels, un retard de croissance intra-utérin, une exposition in utero au diéthylstilbestrol, des gestations multiples, le sexe du nourrisson, une petite taille, un poids de pré-grossesse faible/un indice de masse corporelle faible, du diabète, de l'hypertension, de l'hypothyroïdie, de l'asthme, le niveau d'éducation, l'usage du tabac, et des infections urogénitales.
